(19) Europäisches Patentamt
European Patent Office
Office européen des brevets

(11) **EP 1 865 053 B1**

(12) **EUROPEAN PATENT SPECIFICATION**

(45) Date of publication and mention of the grant of the patent:
**08.08.2012 Bulletin 2012/32**

(51) Int Cl.:
***C12N 9/06*** *(2006.01)* ***C12N 15/82*** *(2006.01)*

(21) Application number: **07113288.0**

(22) Date of filing: **10.12.2002**

(54) **Method for modifying plant morphology, biochemistry and physiology comprising expression of plant cytokinin oxidase**

Methode zur Veränderung der Morphologie, Biochemie und der Physiologie von Pflanzen, beinhaltend die Expression einer Pflanzlichen Cytokinin Oxidase

Procédé de modification de la morphologie, biochimie et physiologie des plantes comprenant l'expression d'une cytokinine oxydase des plantes

(84) Designated Contracting States:
**AT BE BG CH CY CZ DE DK EE ES FI FR GB GR IE IT LI LU MC NL PT SE SI SK TR**
Designated Extension States:
**AL LT LV MK RO**

(30) Priority: **10.12.2001 US 14101**

(43) Date of publication of application:
**12.12.2007 Bulletin 2007/50**

(62) Document number(s) of the earlier application(s) in accordance with Art. 76 EPC:
**02795139.1 / 1 458 874**

(73) Proprietors:
- **Schmulling, Thomas
14052 Berlin (DE)**
- **Werner, Tomas
10289 Berlin (DE)**

(72) Inventors:
- **Schmulling, Thomas
14052 Berlin (DE)**
- **Werner, Tomas
10289 Berlin (DE)**

(74) Representative: **De Clercq, Ann G. Y. et al
De Clercq & Partners cvba
Edgard Gevaertdreef 10 a
9830 Sint-Martens-Latem (BE)**

(56) References cited:
**WO-A-00/63401 WO-A-01/96580
WO-A-99/06571**

- **WERNER T ET AL: "Regulation of plant growth by cytokinin" PROCEEDINGS OF THE NATIONAL ACADEMY OF SCIENCES OF USA, NATIONAL ACADEMY OF SCIENCE, WASHINGTON, DC, US, vol. 98, no. 18, 28 August 2001 (2001-08-28), pages 10487-10492, XP002240696 ISSN: 0027-8424**
- **BILYEU K D ET AL: "Molecular and biochemical characterization of a cytokinin oxidase from maize" PLANT PHYSIOLOGY, AMERICAN SOCIETY OF PLANT PHYSIOLOGISTS, ROCKVILLE, MD, US, vol. 125, no. 1, January 2001 (2001-01), pages 378-386, XP002240697 ISSN: 0032-0889**
- **HOUBA-HERIN NICOLE ET AL: "Cytokinin oxidase from Zea mays: Purification, cDNA cloning and expression in moss protoplasts" PLANT JOURNAL, BLACKWELL SCIENTIFIC PUBLICATIONS, OXFORD, GB, vol. 17, no. 6, March 1999 (1999-03), pages 615-626, XP002233933 ISSN: 0960-7412**
- **JIANCHANG YANG ET AL: "Grain filling pattern and cytokinin content in the grains and roots of rice plants" PLANT GROWTH REGULATION, KLUWER ACADEMIC PUBLISHERS, DO, vol. 30, no. 3, 1 March 2000 (2000-03-01), pages 261-270, XP019243557 ISSN: 1573-5087**
- **SCHMUELLING T: "New insights into the functions of cytokinins in plant development" JOURNAL OF PLANT GROWTH REGULATION, SPRINGER VERLAG, NEW YORK, NY, US, vol. 21, no. 1, March 2002 (2002-03), pages 40-49, XP002240698 ISSN: 0721-7595**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **SASHA DASKALOVA ET AL: "Effect of seed-specific expression of the ipt Gene on Nicotiana tabacum L. seed composition" PLANT GROWTH REGULATION, KLUWER ACADEMIC PUBLISHERS, DO, vol. 51, no. 3, 1 February 2007 (2007-02-01), pages 217-229, XP019479918 ISSN: 1573-5087**
- **ASHIKARI MOTOYUKI ET AL: "Cytokinin oxidase regulates rice grain production" SCIENCE (WASHINGTON D C), vol. 309, no. 5735, July 2005 (2005-07), pages 741-745, XP009090630 ISSN: 0036-8075**

## Description

### FIELD OF THE INVENTION

The present invention generally relates to methods for modifying plant morphological, biochemical and physiological properties or characteristics, such as one or more developmental processes and/or environmental adaptive processes, including seed development . Methods for increasing seed size and/or weight, increasing embryo size and/or weight, and increasing cotyledon size and/or weight are also provided. The methods comprise expressing a cytokinin degradation control protein, in particular cytokinin oxidase, in the plant, operably under the control of a regulatable promoter sequence such as a cell-specific promoter, tissue-specific promoter, or organ-specific promoter sequence. Preferably, the characteristics modified by the present invention are cytokinin-mediated and/or auxin-mediated characteristics.

### BACKGROUND OF THE INVENTION

Roots are an important organ of higher plants. Their main functions are anchoring of the plant in the soil and uptake of water and nutrients (N-nutrition, minerals, etc.). Thus, root growth has a direct or indirect influence on growth and yield of aerial organs, particularly under conditions of nutrient limitation. Roots are also relevant for the production of secondary plant products, such as defense compounds and plant hormones.

Roots are also storage organs in a number of important staple crops. Sugar beet is the most important plant for sugar production in Europe (260 Mill t/year; 38 % of world production). Manioc (cassava), yarns and sweet potato (batate) are important starch producers (app. 150 Mill t/year each). Their content in starch can be twice as high as that of potato. Roots are also the relevant organ for consumption in a number of vegetables (e.g. carrots, radish), herbs (e.g. ginger, kukuma) and medicinal plants (e.g. ginseng). In addition, some of the secondary plant products found in roots are of economic importance for the chemical and pharmaceutical industry. An example is yarns, which contain basic molecules for the synthesis of steroid hormones. Another example is shikonin, which is produced by the roots of *Lithospermum erythrorhizon* in hairy root cultures. Shikonin is used for its anti-inflammatory, anti-tumor and wound-healing properties.

Moreover, improved root growth of crop plants will also enhance competitiveness with weedy plants and will improve growth in arid areas, by increasing water accessibility and uptake.

Improved root growth is also relevant for ecological purposes, such as bioremediation and prevention/arrest of soil erosion.

Root architecture is an area that has remained largely unexplored through classical breeding, because of difficulties with assessing this trait in the field. Thus, biotechnology could have significant impact on the improvement of this trait, because it does not rely on large-scale screenings in the field. Rather, biotechnological approaches require a basic understanding of the molecular components that determine a specific characteristic of the plant. Today, this knowledge is only fragmentary, and as a consequence, biotechnology was so far unable to realize a break-through in this area.

A well-established regulator of root growth is auxin. Application of indole-3-acetic acid (IAA) to growing plants stimulates lateral root development and lateral root elongation (Torrey, Am J Bot 37: 257-264, 1950; Blakely et al., Bot Gaz 143: 341-352, 1982; Muday and Haworth, Plant Physiol Biochem 32: 193-203, 1994). Roots exposed to a range of concentrations of IAA initiated increasing numbers of lateral roots (Kerk et al., Plant Physiol, 122: 925-932, 2000). Furthermore, when roots that had produced laterals in response to a particular concentration of exogenous auxin were subsequently exposed to a higher concentration of IAA, numerous supernumerary lateral roots spaced between existing ones were formed (Kerk et al., Plant Physiol, 122: 925-932, 2000). Conversely, growth of roots on agar containing auxin-transport inhibitors, including NPA, decreases the number of lateral roots (Muday and Haworth, Plant Physiol Biochem 32: 193-203, 1994).

Arabidopsis mutants containing increased levels of endogenous IAA have been isolated (Boerjan et al., Plant Cell 7: 1405-141, 1995; Celenza et al., Gene Dev 9: 2131-2142, 1995; King et al., Plant Cell 7: 2023-2037, 1995; Lehman et al., Cell 85: 183-194, 1996). They are now known to be alleles of a single locus located on chromosome 2. These mutant seedlings have excess adventitious and lateral roots, which is in accordance with the above-described effects of external auxin application.

The stimulatory effect of auxins on adventitious and lateral root formation suggests that overproduction of auxins in transgenic plants is a valid strategy for increasing root growth. Yet, it is also questionable whether this would yield a commercial product with improved characteristics. Apart from its stimulatory effect on adventitious and lateral root formation, auxin overproduction triggers other effects, such as reduction in leaf number, abnormal leaf morphology (narrow, curled leaves), aborted inflorescences, increased apical dominance, adventitious root formation on the stem, most of which are undesirable from an agronomic perspective (Klee et al., Genes Devel 1: 86-96, 1987; Kares et al.,

Plant Mol Biol 15: 225-236, 1990). Therefore, the major problem with approaches that rely on increased auxin synthesis is a problem of containment, namely to confine the effects of auxin to the root. This problem of containment is not likely overcome by using tissue-specific promoters: auxins are transported in the plant and their action is consequently not confined to the site of synthesis. Another issue is whether auxins will always enhance the total root biomass. For agar-grown plants, it has been noticed that increasing concentrations progressively stimulated lateral root formation but concurrently inhibited the outgrowth of these roots (Kerk et al., Plant Physiol, 122: 925-932, 2000).

Seeds are the reproduction unit of higher plants. Plant seeds contain reserve compounds to ensure nutrition of the embryo after germination. These storage organs contribute significantly to human nutrition as well as cattle feeding. Seeds consist of three major parts, namely the embryo, the endosperm and the seed coat. Reserve compounds are deposited in the storage organ which is either the endosperm (resulting form double fertilisation; e.g. in all cereals), the so-called perisperm (derived from the nucellus tissue) or the cotyledons (e.g. bean varieties). Storage compounds are lipids (oil seed rape), proteins (e.g. in the aleuron of cereals) or carbohydrates (starch, oligosaccharides like raffinose).

Starch is the storage compound in the seeds of cereals. The most important species are maize (yearly production ca. 570 mio t; according to FAO 1995), rice (540 mio t p.a.) and wheat (530 mio t p.a.). Protein rich seeds are different kinds of beans (*Phaseolus spec., Vicia faba, Vigna spec.;* ca. 20 mio t p.a.), pea (*Pisum sativum;* 14 mio t p.a.) and soybean (*Glycine max;* 136 mio t p.a.). Soybean seeds are also an important source of lipids. Lipid rich seeds are as well those of different *Brassica* species (app. 30 mio t p.a.), cotton, oriental sesame, flax, poppy, castor bean, sunflower, peanut, coconut, oilpalm and some other plants of less economic importance.

After fertilization, the developing seed becomes a sink organ that attracts nutritional compounds from source organs of the plant and uses them to produce the reserve compounds in the storage organ. Increases in seed size and weight, are desirable for many different crop species. In addition to increased starch, protein and lipid reserves and hence enhanced nutrition upon ingestion, increases in seed size and/or weight and cotyledon size and/or weight are correlated with faster growth upon germination (early vigor) and enhanced stress tolerance. Cytokinins are an important factor in determining sink strength. The common concept predicts that cytokinins are a positive regulator of sink strength.

Numerous reports ascribe a stimulatory or inhibitory function to cytokinins in different developmental processes such as root growth and branching, control of apical dominance in the shoot, chloroplast development, and leaf senescence (Mok M.C. (1994) in Cytokines: Chemistry, Activity and Function, eds., Mok, D.W.S. & Mok,M.C. (CRC Boca Raton, Fl), pp.155-166). Conclusions about the biological functions of cytokinins have mainly been derived from studies on the consequences of exogenous cytokinin application or endogenously enhanced cytokinin levels (Klee, H.J. & Lanehon, M.B. (1995) in Plant Hormones:Physiology, Biochemisry and Molecular Biology, ed. Davies, P.J. (Kluwer, Dordrdrocht, the Netherlands), pp. 340-353, Smulling, T., Rupp, H.M. Frank, M& Schafer, S. (1999) in Advances in Regulation of Plant Growth and Development, eds. Surnad, M. Pac P. & Beck, E. (Peres, Prague), pp. 85-96). Up to now, it has not been possible to address the reverse question: what are the consequences for plant growth and development if the endogenous cytokinin concentration is decreased? Plants with a reduced cytokinin content are expected to yield more precise information about processes cytokinins limit and, therefore, might regulate. Unlike other plant hormones such as abscisic acid, gibberellins, and ethylene, no cytokinin biosynthetic mutants have been isolated (Hooykens, P.J.J.,Hall, M.A. & Libbeuga, K.R.,eds.(1999) Biochemistry and Molecular Biology of Plant Hormones (Elsevier, Amsterdam).

The catabolic enzyme cytokinin oxidase (CKX) plays a principal role in controlling cytokinin levels in plant tissues. CKX activity has been found in a great number of higher plants and in different plant tissues. The enzyme is a FAD-containing oxidoreductase that catalyzes the degradation of cytokinins bearing unsaturated isoprenoid side chains. The free bases iP and Z, and their respective ribosides are the preferred substrates. The reaction products of iP catabolism are adenine and the unsaturated aldehyde 3-methyl-2-butonal (Armstrong, D.J. (1994) in Cytokinins: Chemistry, Activity and Functions, eds. Mok. D.W.S & Mok, M.C. (CRC Boca Raton, FL), pp. 139-154). Recently, a cytokinin oxidase gene from *Zea mays* has been isolated (Morris, R.O., Bilyeu, K.D., Laskey, J.G. & Cherich, N.N. (1999) Biochem. Biophys.Res. Commun. 255, 328-333, Houba-Heria, N.,Pethe, C. d'Alayer, J & Lelouc, M. (1999) Plant J. 17:615-626). The manipulation of CKX gene expression could partially overcome the lack of cytokinin biosynthetic mutants and can be used as a powerful tool to study the relevance of iP - and Z-type cytokinins during the whole life cycle of higher plants.

The present invention overcomes problems related to containment of auxin effects, maintenance of root outgrowth, and promotion of increased seed, embryo, and cotyledon size and/or weight through reduction of endogenous cytokinin concentration.

## SUMMARY OF THE INVENTION

The present invention relates to the subject-matter as defined in appended claims 1 to 29.

The present specification describes plant cytokinin oxidase proteins, nucleic acid sequences encoding such proteins, and vectors, host cells and transgenic plant cells, plants, and plant parts comprising the proteins, nucleic acid sequences, and vectors. For example, the present invention relates to a genetic construct comprising a gene encoding a protein with cytokinin oxidase activity from *Arabidopsis thaliana.* This gene may be expressed under control of a regulated promoter. This promoter may be regulated by endogenous tissue-specific or environment-specific factors or, alternatively, it may be induced by application of specific chemicals.

The present specification also describes a method to modify root architecture and biomass by expression of a cytokinin oxidase gene or expression of a nucleic acid encoding a protein that reduces the level of active cytokinins in plants or plant parts. Preferably, expression is under control of a promoter that is specific to the root or to certain tissues or cell types of the root.

Additionally, the present specification describes methods of increasing seed size and/or weight, embryo size and/or weight, and cotyledon size and/or weight. The methods involve expression of a cytokinin oxidase gene or expression of a nucleic acid encoding a protein that reduces the level of active cytokinins in plants or plant parts. Preferably, expression is under control of a promoter directs expression preferentially in the seed, embryo, or cotyledon.

## BRIEF DESCRIPTION OF THE DRAWINGS

**Figure 1. Schematic representation of plant cytokinin oxidase genes**.

Shown are the structures of different cytokinin oxidase genes isolated from maize *(ZmCKX1,* accession number AF044603, Biochem. Biophys. Res. Com. 255:328-333, 1999) and Arabidopsis (*AtCKX1 to AtCKX4*). Exons are denominated with 'E' and represented by shaded boxes. Introns are represented by white boxes. Further indicated are the gene sizes (in kb, on top of each structure), the gene accession numbers (under the names) and a size bar representing 0.5 kb.

**Figure 2. Alignment of plant cytokinin oxidase amino acid sequences**.

The amino acid sequences from cytokinin oxidases from maize (ZmCKX1) and Arabidopsis (AtCKX1 to AtCKX4) are aligned. Identical amino acid residues are marked by a black box, similar amino acid residues are in a grey box. Amino acid similarity groups: (M,I,L,V), (F,W,Y), (G,A), (S,T), (R,K,H), (E,D), (N,Q),

**Figure 3. Northern blot analysis of *AtCKX1*-expressing tobacco and *Arabidopsis* plants**.

(A) Northern blot analysis of constitutively expressing tobacco plants (lanes 1-8) compared to wild type SNN tobacco (lane 9)

(B) Comparison of tetracycline-induced gene expression in leaves after 12h of induction with a constitutively expressing clone. Lanes 2-9, leaves of four different *AtCKX1*-W38TetR clones (+,-, with or without tetracycline treatment), lane 1, constitutively expressing *35S:: AtCKX1* clone.

(C) Northern blot analysis of *Arabidopsis* plants constitutively expressing *AtCKX1* gene. Lanes 2-4, three different constitutively expressing *35S::AtCKX1* clones compared to wild type Arabidopsis plant (lane 1).

**Figure 4: Growth characteristics of *35S::AtCKX1* transgenic *Arabidopsis* plants.**

(A) Two wild type seedlings (left) compared to two *35S::AtCKX1* expressing seedlings (right). Note the increased formation of adventitious roots and increased root branching in the transgenic seedlings. Pictures were taken 14 days after germination. Plants were grown *in vitro* on MS medium in petri dishes in a vertical position.

(B) Like A, but roots stained with toluidine blue.

(C) Top view of a petri dish with *35S::AtCKX1* transgenic seedlings three weeks after germination.

(D) A *35S::AtCKX1* transgenic plants grown in liquid culture. Roots of wild type seedlings grow poorly under these conditions (not shown).

(E) Transformants (T0) that express the *35S::AtCKX1* gene (three plants on the right), a wild type plant is shown on the left.

(F) Phenotype of T1 plants grown in soil. Wild type plant (left) compared to two *35S::AtCKX1* transgenic plants.

**Figure 5: Phenotype of AtCKX2 overexpressing Arabidopsis plants.**

T1 generation of *35S::AtCKX2* expressing Arabidopsis plants (two plants on the right) compared to wild type (plant on the left).

**Figure 6. Northern blot analysis of *AtCKX2*-expressing tobacco and Arabidopsis plants.**

(A) Northern blot analysis of constitutively expressing tobacco plants (lanes 1-7) compared to wild type SNN tobacco (lane 8)
(B) Northern blot analysis of Arabidopsis plants constitutively expressing *AtCKX2* gene. Lanes 2-8, seven different constitutively expressing *35S::AtCKX2* clones compared to wild type Arabidopsis plant (lane 1).

**Figure 7. Shoot phenotype of *AtCKX1* and *AtCKX2* expressing tobacco plants.**

(A) Top view of six week old plants.
(B) Tobacco plants at the flowering stage.
(C) Kinetics of stem elongation. Arrows mark the onset of flowering. Age of plants (days after germination) and leaf number at that stage are indicated above the arrows. Bars indicate SD; $n$ = 12.
(D) Number of leaves ($n$ = 12) formed between day 68 and day 100 after germination and final surface area of these leaves (100% of wild type is 3646 ±144 $cm^2$; $n$ = 3).
(E) Comparison of leaf size and senescence. Leaves were from nodes number 4, 9, 12, 16 and 20 from the top (from left to right).

**Figure 8. Root phenotype of *AtCKX* expressing transgenic tobacco plants.**

(A) Seedlings 17 days after germination.
(B) Root system of soil grown plants at the flowering stage.
(C) Root length, number of lateral roots (LR) and adventitious roots (AR) on day 10 after germination.
(D) Dose-response curve of root growth inhibition by exogenous cytokinin. Bars indicate ±SD; $n$ = 30.

**Figure 9: Growth of axillary shoot meristems in *35S*::*AtCKX1* expressing tobacco plants.**

**Figure 10: Histology of shoot meristems, leaves and root meristems of *AtCKX1* overexpressing tobacco plants versus wild type (WT) tobacco**.

(A) Longitudinal median section through the vegetative shoot apical meristem. P, leaf primordia.
(B) Vascular tissue in second order veins of leaves. X, xylem, PH, a phloem bundle.
(C) Cross sections of fully developed leaves.
(D) Scanning electron microscopy of the upper leaf epidermis.
(E) Root apices stained with DAPI. RM, root meristem.
(F) Longitudinal median sections of root meristems ten days after germination. RC, root cap; PM, promeristem.
(G) Transverse root sections 10 mm from the apex. E, epidermis, C1-C4, cortical cell layer, X, xylem, PH, phloem. Bars are 100 µm.

**Figure 11: Northern blot analysis of *AtCKX3* and *AtCKX4*-expressing tobacco plants.**

(A) Northern blot analysis of constitutively expressing *AtCKX3* tobacco plants. Lane designations indicate individual transgenic plant numbers, WT is wild type SNN tobacco. The blot on top was probed with a *AtCKX3* specific probe, the lower blot with a probe specific for the 25S rRNA and serves as a control for RNA loading.
(B) Northern blot analysis of constitutively expressing *AtCKX4* tobacco plants. Lane designations indicate individual transgenic plant numbers, WT is wild type SNN tobacco. The blot on top was probed with an *AtCKX4* specific probe, the lower blot with a probe specific for the 25S rRNA and serves as a control for RNA loading.

**Figure 12: Reciprocal grafts of *AtCKX2* transgenic tobacco plants and wild type plants.**

(A) Two plants on the left: Control (WT scion grafted on a WT rootstock). Two plants on the right: WT scion grafted on a *AtCKX2-38* transgenic rootstock.
(B) Left: Control (WT scion grafted on a WT rootstock). Right: Scion of *AtCKX2-38* plant grafted on WT rootstock.
(C) Magnification of root area. Left: Control (WT scion grafted on a WT rootstock). Right: WT scion grafted on an *AtCKX2-38* transgenic rootstock.
(D) Formation of adventitious roots. Left: Control (WT scion grafted on an WT rootstock). Right: WT scion grafted on an *AtCKX2-38* transgenic rootstock.

**Figure 13: Phenotype of *Arabidopsis* seeds, embryos and seedlings.**

(A) Seeds of an *AtCKX1* transgenic line and wild type seeds. Bar size 1 mm.
(B) Seeds of *AtCKX1, AtCKX2, AtCKX3* and *AtCKX4* transgenic lines and wild type seeds. Bar size 1 mm.
(C) Mature embryos of *AtCKX1* transgenic *Arabidopsis* and of a wild type plant. Bar size 200 μm. Embryos were obtained from mature seeds that had been imbibed for 12 hours in 20% EtOH, squeezed out from the seed coat, cleared with chloralhydrate and photographed using Nomarski optics.
(D) Wild type (top) and *AtCKX1* expressing *Arabidopsis* seedlings 4 days after germination.
(E) Close-up of D.

**Figure 14:** Seed weight of wild type and two independent clones for each of the four investigated AtCKX genes. Average weight obtained by analysing five different batches of 200 seeds for each clone.

## DETAILED DESCRIPTION OF THE INVENTION

To by-pass above-mentioned problems associated with increasing auxin biosynthesis, it was decided to follow an alternative approach. We reasoned that downregulation of biological antagonists of auxins could evoke similar or even superior effects on root growth as compared to increasing auxin levels. Hormone actions and interactions are extremely complex, but we hypothesized that cytokinins could function as auxin antagonists with respect to root growth. Hormone studies on plant tissue cultures have shown that the ratio of auxin versus cytokinin is more important for organogenesis than the absolute levels of each of these hormones, which indeed indicates that these hormones function as antagonists - at least in certain biological processes. Furthermore, lateral root formation is inhibited by exogenous application of cytokinins. Interestingly, also root elongation is negatively affected by cytokinin treatment, which suggests that cytokinins control both root branching and root outgrowth.

Together, current literature data indicate that increasing cytokinin levels negatively affects root growth, but the mechanisms underlying this process are not understood. The sites of cytokinin synthesis in the plant are root tips and young tissues of the shoot. Endogenous concentrations of cytokinins are in the nM range. However, as their quantification is difficult, rather large tissue amounts need to be extracted and actual local concentrations are not known. Also the subcellular compartmentation of cytokinins is not known. It is generally thought that the free base and ribosides are localized in the cytoplasm and nucleus, while glucosides are localized in the vacuole. There exist also different cytokinins with slightly different chemical structure. As a consequence, it is not known whether the effects of exogenous cytokinins should be ascribed to a raise in total cytokinin concentration or rather to the competing out of other forms of plant-borne cytokinins (which differ either in structure, cellular or subcellular location) for receptors, translocators, transporters, and modifying enzymes.

In order to test the hypothesis that cytokinin levels in the root indeed exceed the level optimal for root growth, novel genes encoding cytokinin oxidases (which are cytokinin metabolizing enzymes) were cloned from *Arabidopsis thaliana* (designated *AtCKX)* and were subsequently expressed under a strong constitutive promoter in transgenic tobacco and *Arabidopsis.* Transformants showing *AtCKX* mRNA expression and increased cytokinin oxidase activity also manifested enhanced formation and growth of roots. Negative effects on shoot growth were also observed. The latter is in accordance with the constitutive expression of the cytokinin oxidase gene in these plants, illustrating the importance of confined expression of the cytokinin oxidase gene for general plant growth properties. Containment of cytokinin oxidase activity can be achieved by using cell-, tissue-or organ-specific promoters, since cytokinin degradation is a process limited to the tissues or cells that express the CKX protein, this in contrast to approaches relying on hormone synthesis, as explained above.

The observed negative effects of cytokinin oxidase expression on shoot growth demonstrate that cytokinin oxidases are interesting targets for the design of or screening for growth-promoting chemicals. Such chemicals should inhibit cytokinin oxidase activity, should preferably not be transported to the root and should be rapidly degraded in soil, so that application of these chemicals will not inhibit root growth. Cytokinins also delay leaf senescence, which means that positive effects will include both growth and maintenance of photosynthetic tissues. In addition, the observation that cytokinins delay senescence, enhance greening (chlorophyll content) of leaves and reduce shoot apical dominance shows that strategies based on suppressing CKX activity (such as antisense, ribozyme, and cosuppression technology) in the aerial parts of the plant could result in delayed senescence, enhanced leaf greening and increased branching.

Similarly, the observed positive effects of cytokinin oxidase expression on root growth demonstrate that cytokinin oxidases are interesting targets for the design of or screening for herbicides. Such herbicides should inhibit cytokinin oxidase activity, should preferably not be transported to the shoot, and should be soluble and relatively stable in a solvent that can be administered to the root through the soil.

These effects of cytokinin oxidase overexpression on plant development and architecture were hitherto unknown and, as a consequence, the presented invention and its embodiments could not be envisaged.

The observed negative effects on shoot growth demonstrate that manipulation of cytokinin oxidases can also be used for obtaining dwarfing phenotypes. Dwarfing phenotypes are particularly useful in commercial crops such as cereals and fruit trees for example.

In accordance with the present invention, it has also been surprisingly discovered that transgenic plants overexpressing a cytokinin oxidase gene develop seeds (including embryos) and cotyledons of increased size and/or weight. These results are surprising as a reduced cytokinin content would have been expected to be associated with a reduced organ growth.

The specification describes the positive effect of cytokinin oxidase expression on plant growth and architecture, and in particular on root growth and architecture, seed size and weight, embryo size and weight, and cotyledon size and weight. The cytokinin oxidase gene family contains at least six members in *Arabidopsis* (see examples below) and the present inventors have shown that there are quantitative differences in the effects achieved with some of these genes in transgenic plants. It is anticipated that functional homologs of the described *Arabidopsis* cytokinin oxidases can be isolated from other organisms, given the evidence for the presence of cytokinin oxidase activity in many green plants (Hare and van Staden, Physiol Plant 91:128-136, 1994; Jones and Schreiber, Plant Growth Reg 23:123-134, 1997), as well as in other organisms (Armstrong, in Cytokinins: Chemistry, Activity and Function. Eds Mok and Mok, CRC Press, pp139-154, 1994). Therefore, the sequence of the cytokinin oxidase, functional in the invention, need not to be identical to those described herein. This invention is particularly useful for cereal crops and monocot crops in general and cytokinin oxidase genes from for example wheat or maize may be used as well (Morris et al., 1999; Rinaldi and Comandini, 1999). It is envisaged that other genes with cytokinin oxidase activity or with any other cytokinin metabolizing activity (see Zažímalová et al., Biochemistry and Molecular Biology of Plant Hormones, Hooykaas, Hall and Libbenga (Eds.), Elsevier Science, pp141-160, 1997) can also be used for the purpose of this invention. Similarly, genes encoding proteins that would increase endogenous cytokinin metabolizing activity can also be used for the same purpose. In principle, similar phenotypes could also be obtained by interfering with genes that function downstream of cytokinin such as receptors or proteins involved in signal transduction pathways of cytokinin.

It should be understood that the term 'root growth' encompasses all aspects of growth of the different parts that make up the root system at different stages of its development, both in monocotyledonous and dicotyledonous plants. It is to be understood that enhanced growth of the root can result from enhanced growth of one or more of its parts including the primary root, lateral roots, adventitious roots, etc.

For purposes of this invention, it should be understood that increases in seed weight or seed size can include increases in the size of one or more of the embryo, the endosperm, aleurone, and seed coat. Moreover, increases in embryo size and/or weight can include increases in different organs associated therewith such as e.g., cotyledons, hypocotyl, and roots.

The present specification describes a method for stimulating root growth and/or enhancing the formation of lateral and/or adventitious roots and/or altering root geotropism comprising expression of a plant cytokinin oxidase or comprising expression of another protein that reduces the level of active cytokinins in plants or plant parts.

According to a first embodiment, the present invention relates to a method for increasing plant seed size and/or weight, by increasing the level or activity of a cytokinin oxidase in the plant or by expression of another protein that reduces the level of active cytokinins in a plant or plant part. Preferably, the increased level or activity of a cytokinin oxidase or expression of another protein that reduces the level of active cytokinins in a plant or plant part is localized in the seed including different tissues or cell types of the seed.

In another embodiment, the present invention relates to a method for increasing plant embryo size and/or weight, by increasing the level or activity of a cytokinin oxidase in the plant or by expression of another protein that reduces the level of active cytokinins in a plant or plant part. Preferably, the increased level or activity of a cytokinin oxidase or expression of another protein that reduces the level of active cytokinins in a plant or plant part is localized in the seed. Even more preferably, the increased level or activity of a cytokinin oxidase or expression of another protein that reduces the level of active cytokinins in a plant or plant part is localized in the embryo.

In yet another embodiment, the present invention relates to a method for increasing plant cotyledon size and/or weight, by increasing the level or activity of a cytokinin oxidase in the plant or by expression of another protein that reduces the level of active cytokinins in a plant or plant part. Preferably, the increased level or activity of a cytokinin oxidase or expression of another protein that reduces the level of active cytokinins in a plant or plant part is localized in the cotyledon.

In the context of the present invention it should be understood that the term "expression" and/or 'overexpression' are used interchangeably and both relate to an "enhanced and/or ectopic expression" of a plant cytokinin oxidase or any other protein that reduces the level of active cytokinins in plants. It should be clear that herewith an enhanced expression of the plant cytokinin oxidase as well as "de novo" expression of plant cytokinin oxidases or of said other proteins is meant. Alternatively, said other protein enhances the cytokinin metabolizing activity of a plant cytokinin oxidase.

It further should be understood that in the context of the present invention the expression "lateral and/or adventitious roots" can mean "lateral and adventitious roots" but also "lateral or adventitious roots". The enhancement can exist in the formation of lateral roots or in the formation of adventitious roots as well as in the formation of both types of non-primary roots, but not necessarily.

In addition, as used herein, "increasing seed size and/or weight," can mean increasing seed size and weight, but also size or weight. Thus, the enhancement can exist in an increase in the size of the seed or the weight of the seed or both. Similar interpretations should be applied to "increasing embryo size and/or weight" and "increasing cotyledon size and/or weight."

The terms "plant" and "plant part" are used interchangeably with the terms "plants" and "plant parts."

The present specification described a method for stimulating root growth and/or enhancing the formation of lateral or adventitious roots and/or altering root geotropism and/or increasing yield and/or enhancing early vigor and/or modifying root/shoot ratio and/or improving resistance to lodging and/or increasing drought tolerance and/or promoting in vitro propagation of explants, comprising expression of a plant cytokinin oxidase or comprising expression of another protein that reduces the level of active cytokinins in plants or plant parts.

The present specification also describes a method for stimulating root growth resulting in an increase of root mass by overexpression of a cytokinin oxidase, or another protein that reduces the level of active cytokinins in plants or plant parts, preferably in roots.

Higher root biomass production due to overexpression of growth promoting sequences has a direct effect on the yield and an indirect effect of production of compounds produced by root cells or transgenic root cells or cell cultures of said transgenic root cells. One example of an interesting compound produced in root cultures is shikonin, the yield of which can be advantageously enhanced by said methods.

The present specification also describe methods for stimulating root growth or for enhancing the formation of lateral and/or adventitious roots or for altering root geotropism or for increasing seed size and/or weight, or for increasing embryo size and/or weight, or for increasing cotyledon size and/or weight. The methods comprise expression of a nucleic acid encoding a plant cytokinin oxidase selected from the group consisting of:

(a) nucleic acids comprising a DNA sequence as given in any of SEQ ID NOs: 27, 1, 3, 5, 7, 9, 11, 25, 26, 28 to 31, 33 or 34, or the complement thereof,

(b) nucleic acids comprising the RNA sequences corresponding to any of SEQ ID NOs: 27, 1, 3, 5, 7, 9, 11, 25, 26, 28 to 31, 33 or 34, or the complement thereof,

(c) nucleic acids specifically hybridizing to any of SEQ ID NOs: 27, 1, 3, 5, 7, 9, 11, 25, 26, 28 to 31, 33 or 34, or to the complement thereof,

(d) nucleic acids encoding a protein comprising the amino acid sequence as given in any of SEQ ID NOs: 2, 4, 6, 8, 10, 12, 32 or 35, or the complement thereof,

(e) nucleic acids as defined in any of (a) to (d) characterized in that said nucleic acid is DNA, genomic DNA, cDNA, synthetic DNA or RNA wherein T is replaced by U,

(f) nucleic acids which are degenerated to a nucleic acid as given in any of SEQ ID NOs: 27, 1, 3, 5, 7, 9, 11, 25, 26, 28 to 31, 33 or 34, or which are degenerated to a nucleic acid as defined in any of (a) to (e) as a result of the genetic code,

(g) nucleic acids which are diverging from a nucleic acid encoding a protein as given in any of SEQ ID NOs: 2, 4, 6, 8, 10, 12 or 35 or which are diverging from a nucleic acid as defined in any of (a) to (e), due to the differences in codon usage between the organisms,

(h) nucleic acids encoding a protein as given in SEQ ID NOs: 2, 4, 6, 8, 10, 12 or 35 or nucleic acids as defined in (a) to (e) which are diverging due to the differences between alleles,

(i) nucleic acids encoding a protein as given in any of SEQ ID NOs: 2, 4, 6, 8, 10, 12 or 35,

(j) functional fragments of nucleic acids as defined in any of (a) to (i) having the biological activity of a cytokinin oxidase, and

(k) nucleic acids encoding a plant cytokinin oxidase,

or comprise expression, preferably in roots, or in seeds (including parts of seeds such as embryo, endosperm, seed coat or aleurone) or in cotyledons, of a nucleic acid encoding a protein that reduces the level of active cytokinins in plants or plant parts.

In the present specification nucleic acids encoding novel *Arabidopsis thaliana* cytokinin oxidases have been isolated and for the first time, the present inventors have surprisingly shown that the expression of cytokinin oxidases in transgenic plants or in transgenic plant parts resulted in the above-mentioned root and seed-related features. In order that root-related features be effected, the expression of the cytokinin oxidase(s) should take place in roots, preferably under the control of a root-specific promoter. In order that seed-related features be effected (including the embryo), expression of the cytokinin oxidase(s) should take place in seeds, preferably under the control of a seed-specific promoter. One example of such a root-specific promoter is provided in SEQ ID NO: 36. Examples of seed-specific promoters include but are not limited to those listed in Table 4.

In order that cotyledon-related features be effected, the expression of the cytokinin oxidase(s) should take place in the cotyledons, preferably under the control of a promoter which preferentially expresses in cotyledon.

It should be clear that, although the invention specification discloses in the examples section by several new AtCKX genes and proteins, the inventive concept also relates to the use of other cytokinin oxidases isolated from and expressed in other plants, preferably in the roots and/or seeds and/or cotyledons of said other plants to obtain similar effects in plants as described in the examples section.

Therefore, the present specification more generally describes the use of a nucleic acid encoding a plant cytokinin oxidase or encoding a protein that reduces the level of active cytokinins in plants or plant parts for stimulating root growth or for enhancing the formation of lateral or adventitious roots or for altering root geotropism. The present invention also relates to the use of a nucleic acid encoding a plant cytokinin oxidase or encoding a protein that reduces the level of active cytokinins in plants or plant parts for increasing seed size and/or weight, or for increasing embryo size and/or weight, or for increasing plant cotyledon size and/or weight. Preferred cytokinin oxidases to be used are encoded by the nucleic acids encoding the cytokinin oxidases as defined above and are encoded by the novel nucleic acids of the invention as defined hereunder.

The specification discloses an isolated nucleic acid encoding a novel plant protein having cytokinin oxidase activity selected from the group consisting of:

(a) a nucleic acid comprising a DNA sequence as given in any of SEQ ID NOs: 29, 3, 5, 9, 26, 27, 31, 33 or 34, or the complement thereof,

(b) a nucleic acid comprising the RNA sequences corresponding to any of SEQ ID NOs: 29, 3, 5, 9, 26, 27, 31, 33 or 34, or the complement thereof,

(c) a nucleic acid specifically hybridizing to a nucleic acid as given in any of SEQ ID NOs: 29, 3, 5, 9, 26, 27, 31, 33 or 34, or the complement thereof,

(d) a nucleic acid encoding a protein with an amino acid sequence comprising the polypeptide as given in SEQ ID NO: 32 and which is at least 70% similar, preferably at least 75%, 80% or 85%, more preferably at least 90% or 95%, most preferably at least 99% similar to the amino acid sequence as given in SEQ ID NO: 4,

(e) a nucleic acid encoding a protein with an amino acid sequence which is at least 35% similar, preferably 37%, 40%, 45%, 47% or 50%, similar, more preferably 55%, 60%, 65%, 70%, 75% or 80% similar, most preferably 85%, 90% or 95% similar to the amino acid sequence as given in SEQ ID NO: 6,

(f) a nucleic acid encoding a protein with an amino acid sequence which is at least 35% similar, preferably 37%, 40%, 45%, 47% or 50%, similar, more preferably 55%, 60%, 65%, 70%, 75% or 80% similar, most preferably 85%, 90% or 95% similar to the amino acid sequence as given in SEQ ID NO: 10 or 35,

(g) a nucleic acid encoding a protein comprising the amino acid sequence as given in any of SEQ ID NOs: 4, 6, 10, 32 or 35,

(h) a nucleic acid which is degenerated to a nucleic acid as given in any of SEQ ID NOs: 29, 3, 5, 9, 26, 27, 33 or 34 or which is degenerated to a nucleic acid as defined in any of (a) to (g) as a result of the genetic code,

(i) a nucleic acid which is diverging from a nucleic acid encoding a protein as given in any of SEQ ID NOs: 4, 6, 10 or 35 or which is diverging from a nucleic acid as defined in any of (a) to (g) due to the differences in codon usage between the organisms,

(j) a nucleic acid encoding a protein as given in SEQ ID NOs: 4, 6, 10 or 35, or a nucleic acid as defined in (a) to (g) which is diverging due to the differences between alleles,

(k) a nucleic acid encoding an immunologically active fragment of a cytokinin oxidase encoded by a nucleic acid as given in any of SEQ ID NOs: 29, 3, 5, 9, 26, 27, 31, 33 or 34, or an immunologically active fragment of a nucleic acid as defined in any of (a) to (j),

(l) a nucleic acid encoding a functional fragment of a cytokinin oxidase encoded by a nucleic acid as given in any of SEQ ID NOs: 29, 3, 5, 9, 26, 27, 31, 33 or 34, or a functional fragment of a nucleic acid as defined in any of (a) to (j), wherein said fragment has the biological activity of a cytokinin oxidase, and

(m) a nucleic acid encoding a protein as defined in SEQ ID NOs: 4, 6, 10 or 35, provided that said nucleic acid is not the nucleic acid as deposited under any of the following Genbank accession numbers: AC005917, AB024035, and AC023754

The specification discloses an isolated nucleic acid which is DNA, cDNA, genomic DNA or synthetic DNA, or RNA wherein T is replaced by U.

The specification discloses a nucleic acid molecule of at least 15 nucleotides in length hybridizing specifically with or specifically amplifying a nucleic acid of the invention.

Different cytokinin forms may have differing roles to play in the various developmental processes. Thus, differential effects of CKX1, CKX2, CKX 3 and CKX4 may relate to distinct effects on the pools of different cytokinins. For example, CKX1 and CKX3 mostly promote root elongation and branching, while CKX2 and CKX4 primarily stimulate the formation of adventitious roots. In addition, CKX1 and CKX3 increase seed size and weight to a greater degree than CKX2 and CKX4. Without being bound to a particular mode of action, this differential effect on cytokine pools may result from some differences in substrate specificity or from differential compartmentation of cytokinin oxidases in the cell (predicted to be mitochondrial for CKX1 and CKX3, while extracellular for CKX 2, CKX4, CKX5, and CKX6).

The specification further describes a a vector comprising a nucleic acid as described. Said vector can be an expression vector wherein the nucleic acid is operably linked to one or more control sequences allowing the expression of said sequence in prokaryotic and/or eukaryotic host cells.

It should be understood that for expression of the disclosed oxidase genes in monocots, a nucleic acid sequence corresponding to the cDNA sequence should be used to avoid mis-splicing of introns in monocots. Preferred cDNA sequences to be expressed in monocots have a nucleic acid sequence as represented in any of SEQ ID NOs: 25 to 30 and 34.

The specification also describes a host cell containing any of the nucleic acid molecules or vectors as described. Said host cell is chosen from the group comprising bacterial, insect, fungal, plant or animal cells.

The specification also describes an isolated polypeptide encodable by a nucleic acid as described, or a homologue or a derivative thereof, or an immunologically active or a functional fragment thereof. Polypeptides as described comprise the amino acid sequences as represented in any of SEQ ID NOs: 2, 4, 6, 8, 10, 12, 32 and 35, or a homologue or a derivative thereof, or an immunologically active and/or functional fragment thereof. The specification also describes a polypeptide which has an amino acid sequence as given in SEQ ID: NO 2, 4, 6, 8, 10,12 or 35, or a homologue or a derivative thereof, or an immunologically active and/or functional fragment thereof. Preferred functional fragments thereof are those fragments which are devoid of their signal peptide.

The specification also describes a method for producing a polypeptide of the invention comprising culturing a host cell as described under conditions allowing the expression of the polypeptide and recovering the produced polypeptide from the culture.

The specification also describes an antibody specifically recognizing a polypeptide as described or a specific epitope thereof.

The specification further describes a method for the production of transgenic plants, plant cells or plant tissues comprising the introduction of a disclosed nucleic acid molecule in an expressible format or a disclosed vector in said plant, plant cell or plant tissue. tissue.

The specification also describes a method for the production of altered plants, plant cells or plant tissues comprising the introduction of a disclosed polypeptide directly into a cell, a tissue or an organ of said plant.

The specification also discloses a method for effecting the expression of a described polypeptide comprising the introduction of a disclosed nucleic acid molecule operably linked to one or more control sequences or a disclosed vector stably into the genome of a plant cell. The specification further describe the method as described above further comprising regenerating a plant from said plant cell.

The specification also describes a transgenic plant cell comprising a disclosed nucleic acid sequence which is operably linked to regulatory elements allowing transcription and/or expression of said nucleic acid in plant cells or obtainable by a method as explained above.

The specification also describes a transgenic plant cell as described hereinabove wherein the discloses nucleic acid is stably integrated into the genome of said plant cell.

The specification describes also a transgenic plant or plant tissue comprising plant cells as herein described and also to a harvestable part of said transgenic plant, preferably selected from the group consisting of seeds, leaves, fruits, stem cultures, roots, tubers, rhizomes and bulbs. The invention also relates to the progeny derived from any of said transgenic plants or plant parts.

The specification also describes a method for stimulating root growth comprising expression of a disclosed nucleic acid or comprising expression of another protein that reduces the level of active cytokinins in plants or plant parts.

In another aspect of the invention, there is provided a method of increasing seed size and/or weight. The method comprises increasing the level or activity of a cytokinin oxidase in a plant or increasing the level or activity of a protein that reduces the level of active cytokinins in a plant or plant part, preferably seeds.

Various parts (organs) of the seed may also be increased in size and/or weight such as e.g., embryo, endosperm, seed coat, or aleurone. For example, in accordance with the present invention, there is provided a method of increasing embryo size and/or weight. The method comprises increasing the level or activity of a cytokinin oxidase in a plant or increasing the level or activity of a protein that reduces the level of active cytokinins in a plant or plant part, preferably embryos.

In still another aspect of the invention, there is provided a method of increasing cotyledon size and/or weight. The method comprises increasing the level or activity of a cytokinin oxidase in a plant or increasing the level or activity of a protein that reduces the level of active cytokinins in a plant or plant part, preferably cotyledons.

In accordance with the methods of increasing seed size and/or weight, there is a resultant increase in the speed of growth of seedlings or an increase in early vigor. Increases in yield are also obtained. Similarly, in accordance with the methods of increasing embryo size and/or weight, or cotyledon size and/or weight, there is a resultant increase in speed of growth of seedlings or an increase in early vigor. In many cases, increases in yield are also obtained. Increases in growth of seedlings or early vigor is often associated with increased stress tolerance. For example, faster development of seedlings, including the root systems of seedlings upon germination is critical for survival particularly under adverse conditions such as drought.

Any nucleotide sequence encoding a polypeptide with cytokinin oxidase activity may be used in the methods of the invention. For example, any of the various sequences provided herein encoding a polypeptide with cytokinin oxidase activity may be used in the methods of increasing seed, embryo, or cotyledon size and/or weight.

Preferably, transgenic plants are produced which express a nucleic acid as set forth in any of SEQ ID NOs:1, 5, 25, or 27 or an ortholog of said nucleic acid. Preferably, the ortholog is derived from a related species of the transgenic plant. Even more preferably, the ortholog is specific (native or endogenous) to the species of the transgenic plant.

As described above, promoters which control expression specifically, or preferentially may be used in the methods of the invention. Thus, where increases in seed size or weight are desired, a seed-specific promoter may be used. Where increases in embryo size or weight are desired, an embryo-specific promoter may be used. Where increases in cotyledon size or weight is desired, a promoter which controls expression in cotyledons is preferred. Such promoters are well known, widely available and listed herein in e.g., Table 4.

In another embodiment, the invention relates to a method for increasing size or seed weight, or both, said method comprising expression of a disclosed acid or comprising expression of another protein that reduces the level of active cytokinins in plants or plant parts

In yet another embodiment, the invention relates to a method for increasing embryo size or weight, or both, said method comprising expression of a disclosed acid of the or comprising expression of another protein that reduces the level of active cytokinins in plants or plant parts.

In still another embodiment, the invention relates a method for increasing cotyledon size comprising expression of a disclosed nucleic acid or comprising expression of another protein that reduces the level of active cytokinins in plants or plant parts. Localized expression of a subject cytokinin oxidase gene or part thereof, or of another protein that reduces the level of active cytokinins in plants or plant parts leads to enhanced growth of cotyledons. In species having cotyledons as storage organs, such enhanced growth of cotyledons leads to enhanced yields and/or to enhanced growth performance of seedlings. Further in this regard, carbohydrates, lipids and proteins are all stored within seeds and are metabolized during germination in order to provide energy and metabolites during early growth of the plant. Seed size is often associated with early vigor, since larger seeds contain more carbohydrates, lipids and proteins and thus confer faster growth. Thus, the methods of the present invention lead to faster growth of seedlings. Such early vigor is associated with enhanced stress tolerance. For example, faster development of a plant's root system is critical for survival, particularly under adverse conditions, such as drought. Early vigor is also related to enhanced yield and shortened time to flowering.

A plant cell or tissue culture is an artificially produced culture of plants cells or plant tissues that is grown in a special medium, either liquid or solid, which provides these plant cells or tissues with all requirements necessary for growth and/or production of certain compounds. Plant cell and/or tissue cultures can be used for the rapid propagation of plants and for the production of transgenic plant to name a few examples. Root formation can be difficult for some explants or under some conditions in said cultures and expression of a cytokinin oxidase gene in said cultured plant cells or tissue(s) can be used to enhance root formation. Plant cell and/or tissue culture can also be used for the industrial production of valuable compounds. Possible production compounds are pharmaceuticals, pesticides, pigments, cosmetics, perfumes, food additives, etc. An example of such a product is shikonin, which is produced by the roots of the plant *Lithospermum erythrorhizon.* An example of a plant tissue culture is a hairy root culture, which is an artificially produced mass of hairy roots. Roots of *L. erythrorhizon* are difficult to collect in large numbers and by preparing hairy root cultures, the end product shikonin could be industrially prepared at a faster rate than would normally occur. As disclosed herein, expression of cytokinin oxidases enhances root growth and development and can therefore be used advantageously in said plant cell and tissue culture procedures. The specification describes a method for stimulating root growth and development comprising expression of a nucleic acid encoding a plant cytokinin oxidase, preferably a disclosed cytokinin oxidase, in a transgenic plant cell or tissue culture comprising said transgenic plant cells.

The specification also describes a method for enhancing the formation of lateral or adventitious roots comprising expression of a nucleic acid of the invention or comprising expression of another protein that reduces the level of active cytokinins in plants or plant parts.

The specification also describes method for altering root geotropism comprising altering the expression of a nucleic acid of the invention or comprising expression of another protein that that reduces the level of active cytokinins in plants or plant parts.

The specification also describes methods for enhancing early vigor and/or for modifying root/shoot ratio and/or for improving resistance to lodging and/or for increasing drought tolerance and/or for promoting *in vitro* propagation of explants comprising expression of a disclosed nucleic acid comprising expression of another protein that reduces the level of active cytokinins in plants or plant parts.

The specification further describes methods for increasing the root size or the size of the root meristem comprising expression of a disclosed nucleic acid or comprising expression of another protein that reduces the level of active cytokinins in plants or plant parts, preferably in roots. The specification describes a method for increasing the size of the shoot meristem comprising downregulation of expression of a nucleic acid of the invention, preferably in shoots.

The specification also describes a method for delaying leaf senescence comprising downregulation of expression of any of the disclosed cytokinin oxidases in leaves, preferably in senescing leaves. Also the specification describes a mit method for altering leaf senescence comprising expression of one of the cytokinin oxidases in senescing leaves.

The specification also describes methods for increasing leaf thickness comprising expression of a disclosed nucleic acid or comprising expression of another protein that reduces the level of active cytokinins in plants or plant parts, preferably in leaves.

The specification also describes a method for reducing the vessel size comprising expression of a disclosed nucleic acid or comprising expression of another protein that reduces the level of active cytokinins in plants or plant parts, preferably in vessels.

The specification further describes a method for increasing the vessel size comprising downregulation of expression of a disclosed nucleic acid in plants or plant parts.

The specification also described a method for improving standability of seedlings comprising expression of a disclosed or comprising expression of another protein that reduces the level of active cytokinins in seedlings.

Furthermore, the specification describes any of the above described methods, said method leading to an increase in yield.

The specification further describes any of the methods wherein said expression of said nucleic acid occurs under the control of a strong constitutive promoter. With respect to those aspects having effects on plant roots such as e.g., methods for stimulating root growth, enhancing the formation of lateral or adventitious roots, or for altering root geotropism, preferably, expression of a subject nucleic acid preferably occurs under the control of a promoter that is preferentially expressed in roots. In Table 5 a non-exhaustive list of root specific promoters is included. A preferred promoter to be used in the methods of the specification is the root clavata homolog promoter, having a sequence as given in SEQ ID NO: 36.

With respect to those aspect of the invention having effects on plant seeds such as e.g., methods for increasing seed size or weight, embryo size or weight, or having effects on plant cotyledons such as methods for increasing cotyledon size of weight, expression of a subject nucleic acid occurs under the control of a promoter that is preferentially expressed in seeds. A seed specific promoter may be one which is expressed in all seed organs or one which shows a preference in expression to one or more organs or tissue such as the embryo, endosperm, or aleurone. Examples of such promoters are set forth herein at Table 4.

The specification also describes a method for modifying cell fate and/or modifying plant development and/or modifying plant morphology and/or modifying plant biochemistry and/or modifying plant physiology and/or modifying the cell cycle progression rate comprising the medication of expression in particular cells, tissues or organs of a plant, of a disclosed nucleic acid

The specification also describes a method for obtaining enhanced growth, and/or increased yield and/or altered senescence of a plant cell, tissue and/or organ and/or increased frequency of formation of lateral organs in a plant, comprising the ectopic expression of a disclosed nucleic acid

The specification also describes a method for promoting and extending cell division activity in cells in adverse growth conditions and/or in stress, comprising the ectopic expression of a disclosed nucleic acid sequence.

The specification also describes a method for identifying and obtaining proteins, interacting with a disclosed polypeptide comprising a screening assay wherein a disclosed is used.

The specification also describes a method for identifying and obtaining proteins interacting with a disclosed polypeptide comprising a two-hybrid screening assay wherein a disclosed polypeptide as a bait and a cDNA library as prey are used.

The specification further describes a method for modulating the interaction between a disclosed polypeptide and interacting protein partners obtainable by a method as described above.

The specification relates to a method for identifying and obtaining compounds interacting with a disclosed polypeptide comprising the steps of:

(a) providing a two-hybrid system wherein a disclosed polypeptide and an interacting protein partner obtainable by a method as described above,

(b) interacting said compound with the complex formed by the expressed polypeptides as defined in a), and,

(c) performing (real-time) measurement of interaction of said compound with said polypeptide or the complex formed by the expressed polypeptides as defined in a).

The specification further describes a method for identifying compounds or mixtures of compounds which specifically bind to a disclosed polypeptide, comprising:

(a) combining a disclosed polypeptide with said compound or mixtures of compounds under conditions suitable to allow complex formation, and,

(b) detecting complex formation, wherein the presence of a complex identifies a compound or mixture which specifically binds said polypeptide.

The specification also describes a method as described above wherein said compound or mixture inhibits the activity of said disclosed polypeptide and can be used for the rational design of chemicals.

The specification describes the use of a compound or mixture identified by means of a method as described above as a plant growth regulator or herbicide.

The specification also describes a method for production of a plant growth regulator or herbicide composition comprising the steps of the compound screening methods described above and formulating the compounds obtained from said steps in a suitable form for the application in agriculture or plant cell or tissue culture.

The specification also describes a method for increasing branching comprising expression of a disclosed nucleic acid in plants or plant parts, preferably in stems or axillary buds.

The specification also describes a method for improving lodging resistance comprising expression of a nucleic acid of the invention in plants or plant parts, preferably in stems or axillary buds.

The specification also describes a method for the design of or screening for growth-promoting chemicals or herbicides comprising the use of a disclosed nucleic acid or a dislosed vector.

The specification describes the use of a disclosed nucleic acid molecule, a disclosed vector or a disclosed polypeptide for increasing yield.

The specification also describes the use of a disclosed nucleic acid molecule, a disclosed vector or a disclosed polypeptide for stimulating root growth.

The specification also describes the use of a disclosed nucleic acid molecule, a disclosed vector or a disclosed polypeptide for enhancing the formation of lateral or adventitious roots.

The specification also describes the use of a disclosed nucleic acid molecule, a disclosed vector or a disclosed polypeptide for altering root geotropism.

The specification also describes the use of a disclosed nucleic acid molecule, a disclosed vector or a disclosed polypeptide for increasing at least one of seed size, seed weight, embryo size, embryo weight, cotyledon size, and cotyledon weight.

The specification further describes the use of a disclosed nucleic acid molecule, a disclosed vector or a disclosed polypeptide for enhancing early vigor and/or for modifying root/shoot ratio and/or for improving resistance to lodging and/or for increasing drought tolerance and/or for promoting *in vitro* propagation of explants.

The specification also describe the use of a disclosed nucleic acid molecule, a disclosed recombinant vector or a disclosed polypeptide for modifying plant development and/or for modifying plant morphology and/or for modifying plant biochemistry and/or for modifying plant physiology.

The specification describes a diagnostic composition comprising at least a describes nucleic acid molecule, a disclosed vector , a disclosed polypeptide or a disclosed antibody.

The current specification also describes the use of a transgenic rootstock that has an enhanced root growth and development due to expression of a cytokinin oxidase in grafting procedures with a scion to produce a plant or tree with improved agricultural or horticultural characteristics. The scion may be transgenic or non-transgenic. Specific characteristics envisaged are those conferred by root systems and include improved anchoring of the plant/tree in the soil and/or improved uptake of water resulting for example in improved drought tolerance, and/or improved nutrient uptake from the soil and/or improved transport of organic substances throughout the plant and/or enhanced secretion of substances into the soil such as for example phytosiderophores, and/or improved respiration and/or improved disease resistance and/or enhanced yield. An advantage of using *AtCKX* transformed rootstocks for grafting, in addition to their enhanced root system, is the delayed senescence of leaves on the graft, as disclosed herein (see Figure 12 A). Preferred plants or trees for this particular embodiment include plants or trees that do not grow well on their own roots and are grafted in cultivated settings such as commercially profitable varieties of grapevines, citrus, apricot, almond, plum, peach, apple, pear, cherry, walnut, fig, hazel and loquat.

As mentioned *supra,* auxins and cytokinins act as antagonists in certain biological processes. For example, the cytokinin/auxin ratio regulates the production of roots and shoots with a high concentration of auxin resulting in organized roots and a high concentration of cytokinins resulting in shoot production. As disclosed in this specification, expression of cytokinin oxidases in tobacco and Arabidopsis results in enhanced root development consistent with enhanced auxin effects. Auxins are also involved in the development of fruit. Treatment of female flower parts with auxin results in the development of parthenocarpic fruit in some plant species. Parthenocarpic fruit development has been genetically engineered in several horticultural crop plants through increased biosynthesis of auxins in the female reproductive organs (WO0105985).

Therefore, this specification describes a method for inducing the parthenocarpic trait in plants, said method consisting of downregulating the expression of one or more cytokinin oxidases or of another protein that reduces the level of active cytokinins in plants or plant parts, preferably in the female reproductive organs such as the placenta, ovules and tissues derived therefrom. The DefH9 promoter region from *Antirrhinum majus* or one of its homologues, which confer high expression specificity in placenta and ovules, can be used for this purpose.

Those skilled in the art will be aware that the invention described herein is subject to variations and modifications other than those specifically described. It is to be understood that the invention described herein includes all such variations and modifications. The invention also includes all such steps, features, compositions and compounds referred to or indicated in this specification, individually or collectively, and any and all combinations of any or more of said steps or features.

The present invention is applicable to any plant, in particular a monocotyledonous plants and dicotyledonous plants including a fodder or forage legume, ornamental plant, food crop, tree, or shrub selected from the list comprising *Acacia spp., Acer spp., Actinidia spp.,Aesculus spp., Agathis australis, Albizia amara, Alsophila tricolor, Andropogon spp., Arachis spp, Areca catechu, Astelia fragrans, Astragalus cicer, Baikiaea plurijuga, Betula spp., Brassica spp., Bruguiera gymnorrhiza, Burkea africana, Butea frondosa, Cadaba farinosa, Calliandra spp, Camellia sinensis, Canna indica, Capsicum spp., Cassia spp., Centroema pubescens, Chaenomeles spp.,Cinnamomum cassia, Coffea arabica, Colophospermum mopane, Coronillia varia, Cotoneaster serotina, Crataegus spp., Cucumis spp., Cupressus spp., Cyathea dealbata, Cydonia oblonga, Cryptomeria japonica, Cymbopogon spp., Cynthea dealbata, Cydonia oblonga, Dalbergia monetaria, Davallia divaricata, Desmodium spp., Dicksonia squarosa, Diheteropogon amplectens, Dioclea*

*spp, Dolichos spp., Dorycnium rectum*, *Echinochloa pyramidalis, Ehrartia spp., Eleusine coracana, Eragrestis spp., Erythrina spp., Eucalyptus spp., Euclea schimperi, Eulalia villosa, Fagopyrum spp., Feijoa sellowiana*, *Fragaria spp., Flemingia spp, Freycinetia banksii, Geranium thunbergii, Ginkgo biloba, Glycine javanica, Gliricidia spp, Gossypium hirsutum, Grevillea spp., Guibourtia coleosperma, Hedysarum spp., Hemarthia altissima, Heteropogon contortus, Hordeum vulgare, Hyparrhenia rufa, Hypericum erectum, Hyperthelia dissoluta, Indigo incarnata, Iris spp., Leptarrhena pyrolifolia, Lespediza spp., Lettuca spp., Leucaena leucocephala, Loudetia simplex, Lotonus bainesii, Lotus spp., Macrotyloma axillare, Malus spp., Manihot esculenta, Medicago sativa, Metasequoia glyptostroboides, Musa sapientum, Nicotianum spp., Onobrychis spp., Ornithopus spp., Oryza spp., Peltophorum africanum, Pennisetum spp., Persea gratissima, Petunia spp., Phaseolus spp., Phoenix canariensis, Phormium cookianum, Photinia spp., Picea glauca, Pinus spp., Pisum sativum, Podocarpus totara, Pogonarthria fleckii, Pogonarthria squarrosa, Populus spp., Prosopis cineraria, Pseudotsuga menziesii, Pterolobium stellatum, Pyrus communis, Quercus spp., Rhaphiolepsis umbellata, Rhopalostylis sapida, Rhus natalensis, Ribes grossularia, Ribes spp., Robinia pseudoacacia, Rosa spp., Rubus spp., Salix spp., Schyzachyrium sanguineum, Sciadopitys verticillata, Sequoia sempervirens, Sequoiadendron giganteum, Sorghum bicolor, Spinacia spp., Sporobolus fimbriatus, Stiburus alopecuroides, Stylosanthos humilis, Tadehagi spp, Taxodium distichum, Themeda triandra, Trifolium spp., Triticum spp., Tsuga heterophylla, Vaccinium spp., Vicia spp. Vitis vinifera, Watsonia pyramidata, Zantedeschia aethiopica, Zea mays,* amaranth, artichoke, asparagus, broccoli, brussel sprout, cabbage, canola, carrot, cauliflower, celery, collard greens, flax, kale, lentil, oilseed rape, okra, onion, potato, rice, soybean, straw, sugarbeet, sugar cane, sunflower, tomato, squash, and tea, amongst others, or the seeds of any plant specifically named above or a tissue, cell or organ culture of any of the above species.

Throughout this specification, unless the context requires otherwise the word "comprise", and variations such as "comprises" and "comprising", will be understood to imply the inclusion of a stated integer or step or group of integers or steps but not the exclusion of any other integer or step or group of integers or steps.

As used herein, the term "derived from" shall be taken to indicate that a particular integer or group of integers has originated from the species specified, but has not necessarily been obtained directly from the specified source.

The terms "protein(s)", "peptide(s)" or "oligopeptide(s)", when used herein refer to amino acids in a polymeric form of any length. Said terms also include known amino acid modifications such as disulphide bond formation, cysteinylation, oxidation, glutathionylation, methylation, acetylation, farnesylation, biotinylation, stearoylation, formylation, lipoic acid addition, phosphorylation, sulphation, ubiquitination, myristoylation, palmitoylation, geranylgeranylation, cyclization (e.g. pyroglutamic acid formation), oxidation, deamidation, dehydration, glycosylation (e.g. pentoses, hexosamines, N-acetylhexosamines, deoxyhexoses, hexoses, sialic acid etc.) and acylation as well as non-naturally occurring amino acid residues, L-amino acid residues and D-amino acid residues.

"Homologues" of a disclosed protein are those peptides, oligopeptides, polypeptides, proteins and enzymes which contain amino acid substitutions, deletions and/or additions relative to the said protein with respect to which they are a homologue, without altering one or more of its functional properties, in particular without reducing the activity of the resulting. For example, a homologue of said protein will consist of a bioactive amino acid sequence variant of said protein. To produce such homologues, amino acids present in the said protein can be replaced by other amino acids having similar properties, for example hydrophobicity, hydrophilicity, hydrophobic moment, antigenicity, propensity to form or break $\alpha$-helical structures or $\beta$-sheet structures, and so on. An overview of physical and chemical properties of amino acids is given in Table 1.

Substitutional variants of a disclosed protein are those in which at least one residue in said protein amino acid sequence has been removed and a different residue inserted in its place. Amino acid substitutions are typically of single residues, but may be clustered depending upon functional constraints placed upon the polypeptide; insertions will usually be of the order of about 1-10 amino acid residues and deletions will range from about 1-20 residues. Preferably, amino acid substitutions will comprise conservative amino acid substitutions, such as those described *supra.*

**Table 1.** Properties of naturally occurring amino acids.

| Charge properties / hydrophobicity | Side group | Amino Acid |
|---|---|---|
| Nonpolar<br>hydrophobic | Aliphatic<br>aliphatic, S-containing<br>aromatic<br>imino | ala, ile, leu, val<br>met<br>phe, trp<br>pro |
| polar uncharged | Aliphatic<br>Amide<br>Aromatic<br>Hydroxyl | gly<br>asn, gln<br>tyr<br>ser, thr |

(continued)

| Charge properties / hydrophobicity | Side group | Amino Acid |
|---|---|---|
| | Sulfhydryl | cys |
| Positively charged | Basic | arg, his, lys |
| Negatively charged | Acidic | asp, glu |

Insertional amino acid sequence variants of a disclosed protein of the invention are those in which one or more amino acid residues are introduced into a predetermined site in said protein. Insertions can comprise amino-terminal and/or carboxy-terminal fusions as well as intra-sequence insertions of single or multiple amino acids. Generally, insertions within the amino acid sequence will be smaller than amino or carboxyl terminal fusions, of the order of about 1 to 10 residues. Examples of amino- or carboxy-terminal fusion proteins or peptides include the binding domain or activation domain of a transcriptional activator as used in a two-hybrid system, phage coat proteins, $(histidine)_6$-tag, glutathione S-transferase, protein A, maltose-binding protein, dihydrofolate reductase, Tag•100 epitope (EETARFQPGYRS), c-myc epitope (EQKLISEEDL), FLAG®-epitope (DYKDDDK), lacZ, CMP (calmodulin-binding peptide), HA epitope (YPYDVPDYA), protein C epitope (EDQVDPRLIDGK) and VSV epitope (YTDIEMNRLGK).

Deletional variants of a protein of the invention are characterized by the removal of one or more amino acids from the amino acid sequence of said protein.

Amino acid variants of a disclosed protein may readily be made using peptide synthetic techniques well known in the art, such as solid phase peptide synthesis and the like, or by recombinant DNA manipulations. The manipulation of DNA sequences to produce variant proteins which manifest as substitutional, insertional or deletional variants are well known in the art. For example, techniques for making substitution mutations at predetermined sites in DNA having known sequence are well known to those skilled in the art, such as by M13 mutagenesis, T7-Gen in vitro mutagenesis kit (USB, Cleveland, OH), QuickChange Site Directed mutagenesis kit (Stratagene, San Diego, CA), PCR-mediated site-directed mutagenesis or other site-directed mutagenesis protocols.

In the current specification "identity" and/or "similarity" percentages between DNA sequences and/or proteins are calculated using computer programs known in the art such as the DNAstar/MegAlign programs in combination with the Clustal method.

"Derivatives" of a disclosed protein are those peptides, oligopeptides, polypeptides, proteins and enzymes which comprise at least about five contiguous amino acid residues of said polypeptide but which retain the biological activity of said protein. A "derivative" may further comprise additional naturally-occurring, altered glycosylated, acylated or non-naturally occurring amino acid residues compared to the amino acid sequence of a naturally-occurring form of said polypeptide. Alternatively or in addition, a derivative may comprise one or more non-amino acid substituents compared to the amino acid sequence of a naturally-occurring form of said polypeptide, for example a reporter molecule or other ligand, covalently or non-covalently bound to the amino acid sequence such as, for example, a reporter molecule which is bound thereto to facilitate its detection.

With "immunologically active" is meant that a molecule or specific fragments thereof such as specific epitopes or haptens are recognized by, i.e. bind to antibodies. Specific epitopes may be determined using, for example, peptide scanning techniques as described in Geysen *et al.* (1996) (Geysen, H.M., Rodda, S.J. and Mason, T.J. (1986). A priori delineation of a peptide which mimics a discontinuous antigenic determinant. Mol. Immunol. 23, 709-715.).

The term "fragment of a sequence" or "part of a sequence" means a truncated sequence of the original sequence referred to. The truncated sequence (nucleic acid or protein sequence) can vary widely in length; the minimum size being a sequence of sufficient size to provide a sequence with at least a comparable function and/or activity or the original sequence referred to (e. g. "functional fragment"), while the maximum size is not critical. In some applications, the maximum size usually is not substantially greater than that required to provide the desired activity and/or function (s) of the original sequence. Typically, the truncated amino acid sequence will range from about 5 to about 60 amino acids in length. More typically, however, the sequence will be a maximum of about 50 amino acids in length, preferably a maximum of about 60 amino acids. It is usually desirable to select sequences of at least about 10, 12 or 15 amino acids, up to a maximum of about 20 or 25 amino acids.

Functional fragments can also include those comprising an epitope which is specific for the disclosed proteins. Preferred functional fragments have a length of at least, for example, 5, 10, 25, 100, 150 or 200 amino acids.

It should thus be understood that functional fragments can also be immunologically active fragments or not.

In the context of the current specification are embodied homologues, derivatives and/or immunologically active and/or functional fragments of the cytokinin oxidases as defined supra. Particularly preferred homologues, derivatives and/or immunologically active and/or functional fragments of the cytokinin oxidase proteins which are contemplated for use in the current invention are derived from plants, more specifically from *Arabidopsis thaliana,* even more specifically said cytokinin oxidases are the *Arabidopsis thaliana* (At)CKX, or are capable of being expressed therein. The present invention clearly contemplates the use of functional homologues or derivatives and/or immunologically active fragments of the AtCKX proteins and is not to be limited in application to the use of a nucleotide sequence encoding one of said AtCKX proteins.

Any of said proteins, polypeptides, peptides and fragments thereof can be produced in a biological system, e.g. a cell culture. Alternatively any of said proteins, polypeptides, peptides and fragments thereof can be chemically manufactured e.g. by solid phase peptide synthesis. Said proteins or fragments thereof can be part of a fusion protein as is the case in e.g. a two-hybrid assay which enables e.g. the identification of proteins interacting with a disclosed cytokinin oxidase.

The proteins or fragments thereof are furthermore useful e.g. to modulate the interaction between a disclosed cytokinin oxidase and interacting protein partners obtained by a method of the invention. Chemically synthesized peptides are particularly useful e.g. as a source of antigens for the production of antisera and/or antibodies.

"Antibodies" include monoclonal, polyclonal, synthetic or heavy chain camel antibodies as well as fragments of antibodies such as Fab, Fv or scFv fragments. Monoclonal antibodies can be prepared by the techniques as described in e.g. Liddle and Cryer (1991) which comprise the fusion of mouse myeloma cells to spleen cells derived from immunized animals. Furthermore, antibodies or fragments thereof to a molecule or fragments thereof can be obtained by using methods as described in e.g. Harlow and Lane (1988). In the case of antibodies directed against small peptides such as fragments of a disclosed protein, said peptides are generally coupled to a carrier protein before immunization of animals. Such protein carriers include keyhole limpet hemocyanin (KLH), bovine serum albumin (BSA), ovalbumin and Tetanus toxoid. The carrier protein enhances the immune response of the animal and provides epitopes for T-cell receptor binding sites. The term "antibodies" furthermore includes derivatives thereof such as labeled antibodies. Antibody labels include alkaline phosphatase, PKH2, PKH26, PKH67, fluorescein (FITC), Hoechst 33258, R-phycoerythrin (PE), rhodamine (TRITC), Quantum Red, Texas Red, Cy3, biotin, agarose, peroxidase and gold spheres. Tools in molecular biology relying on antibodies against a protein include protein gel blot analysis, screening of expression libraries allowing gene identification, protein quantitative methods including ELISA and RIA, immunoaffinity purification of proteins, immunoprecipitation of proteins (see e.g. Example 6) and immunolocalization. Other uses of antibodies and especially of peptide antibodies include the study of proteolytic processing (Loffler et al. 1994, Woulfe et al. 1994), determination of protein active sites (Lerner 1982), the study of precursor and post-translational processing (Baron and Baltimore 1982, Lerner et al. 1981, Semier et al. 1982), identification of protein domains involved in protein-protein interactions (Murakami et al. 1992) and the study of exon usage in gene expression (Tamura et al. 1991).

Described are antibodies specifically recognizing a cytokinin oxidase or homologue, derivative or fragment thereof as defined supra. Preferably said cytokinin oxidase is a plant cytokinin oxidase, more specifically one of the *Arabidopsis thaliana* cytokinin oxidases (AtCKX).

The terms "gene(s)", "polynucleotide(s)", "nucleic acid(s)", "nucleic acid sequence(s)", "nucleotide sequence (s)", or "nucleic acid molecule(s)", when used herein refer to nucleotides, either ribonucleotides or deoxyribonucleotides or a combination of both, in a polymeric form of any length. Said terms furthermore include double-stranded and single-stranded DNA and RNA. Said terms also include known nucleotide modifications such as methylation, cyclization and 'caps' and substitution of one or more of the naturally occurring nucleotides with an analog such as inosine. Modifications of nucleotides include the addition of acridine, amine, biotin, cascade blue, cholesterol, Cy3®, Cy5®, Cy5.5® Dabcyl, digoxigenin, dinitrophenyl, Edans, 6-FAM, fluorescein, 3'-glyceryl, HEX, IRD-700, IRD-800, JOE, phosphate psoralen, rhodamine, ROX, thiol (SH), spacers, TAMRA, TET, AMCA-S®, SE, BOOIPY®, Marina Blue®, Pacific Blue®, Oregon Green®, Rhodamine Green®, Rhodamine Red®, Rhodol Green® and Texas Red®. Polynucleotide backbone modifications include methylphosphonate, 2'-OMe-methylphosphonate RNA, phosphorothiorate, RNA, 2'-OMeRNA. Base modifications include 2-amino-dA, 2-aminopurine, 3'-(ddA), 3'dA(cordycepin), 7-deaza-dA, 8-Br-dA, 8-oxo-dA, $N^6$-Me-dA, abasic site (dSpacer), biotin dT, 2'-OMe-5Me-C, 2'-OMe-propynyl-C, 3'-(5-Me-dC), 3'-(ddC), 5-Br-dC, 5-1-dC, 5-Me-dC, 5-F-dC, carboxy-dT, convertible dA, convertible dC, convertible dG, convertible dT, convertible dU, 7-deaza-dG, 8-Br-dG, 8-oxo-dG, $O^6$-Me-dG, S6-DNP-dG, 4-methyl-indole, 5-nitroindole, 2'-OMe-inosine, 2'-dl, $0^6$-phenyl-dl, 4-methyl-indole, 2'-deoxynebularine, 5-nitroindole, 2-aminopurine, dP(purine analogue), dK(pyrimidine analogue), 3-nitropyrrole, 2-thio-dT, 4-thio-dT, biotin-dT, carboxy-dT, $0^4$-Me-dT, $O^4$-triazol dT, 2'-OMe-propynyl-U, 5-Br-dU, 2'-dU, 5-F-dU, 5-I-dU, $O^4$-triazol dU. Said terms also encompass peptide nucleic acids (PNAs), a DNA analogue in which the backbone is a pseudopeptide consisting of N-(2-aminoethyl)-glycine units rather than a sugar. PNAs mimic the behavior of DNA and bind complementary nucleic acid strands. The neutral backbone of PNA results in stronger binding and greater specificity than normally achieved. In addition, the unique chemical, physical and biological properties of PNA have been exploited to produce powerful biomolecular tools, antisense and antigene agents, molecular probes and biosensors.

The present specification also advantageously provides nucleic acid sequences of at least approximately 15 contiguous nucleotides of a disclosed nucleic acid and preferably from 15 to 50 nucleotides. These sequences may, advantageously be used as probes to specifically hybridize to sequences of the invention as defined above or primers to initiate specific amplification or replication of disclosed sequences as defined above, or the like. Such nucleic acid sequences may be produced according to techniques well known in the art, such as by recombinant or synthetic means.

They may also be used in diagnostic kits or the like for detecting the presence of a nucleic acid . These tests generally comprise contacting the probe with the sample under hybridising conditions and detecting the presence of any duplex or triplex formation between the probe and any nucleic acid in the sample.

Advantageously, the nucleic acid sequences, according to the specification may be produced using such recombinant or synthetic means, such as for example using PCR cloning mechanisms which generally involve making a pair of primers, which may be from approximately 15 to 50 nucleotides to a region of the gene which is desired to be cloned, bringing the primers into contact with mRNA, cDNA or genomic DNA from a cell, performing a polymerase chain reaction under conditions which bring about amplification of the desired region, isolating the amplified region or fragment and recovering the amplified DNA. Generally, such techniques as defined herein are well known in the art, such as described in Sambrook et al. (Molecular Cloning: a Laboratory Manual, 1989).

A "coding sequence" or "open reading frame" or "ORF" is defined as a nucleotide sequence that can be transcribed into mRNA and/or translated into a polypeptide when placed under the control of appropriate control sequences or regulatory sequences, i.e. when said coding sequence or ORF is present in an expressible format. Said coding sequence of ORF is bounded by a 5' translation start codon and a 3' translation stop codon. A coding sequence or ORF can include, but is not limited to RNA, mRNA, cDNA, recombinant nucleotide sequences, synthetically manufactured nucleotide sequences or genomic DNA. Said coding sequence or ORF can be interrupted by intervening nucleic acid sequences.

Genes and coding sequences essentially encoding the same protein but isolated from different sources can consist of substantially divergent nucleic acid sequences. Reciprocally, substantially divergent nucleic acid sequences can be designed to effect expression of essentially the same protein. Said nucleic acid sequences are the result of e.g. the existence of different alleles of a given gene, of the degeneracy of the genetic code or of differences in codon usage. Thus, as indicated in Table 2, amino acids such as methionine and tryptophan are encoded by a single codon whereas other amino acids such as arginine, leucine and serine can each be translated from up to six different codons. Differences in preferred codon usage are illustrated in Table 3 for *Agrobacterium tumefaciens* (a bacterium), *A. thaliana, M. sativa* (two dicotyledonous plants) and *Oryza sativa* (a monocotyledonous plant). To extract one example, the codon GGC (for glycine) is the most frequently used codon in *A. tumefaciens* (36.2 ‰), is the second most frequently used codon in *O. sativa* but is used at much lower frequencies in *A. thaliana* and *M. sativa* (9 ‰ and 8.4 ‰, respectively). Of the four possible codons encoding glycine (see Table 2), said GGC codon is most preferably used in *A. tumefaciens* and *O. sativa*. However, in *A. thaliana* this is the GGA (and GGU) codon whereas in *M. sativa* this is the GGU (and GGA) codon.

DNA sequences as defined in the current specification can be interrupted by intervening sequences. With "intervening sequences" is meant any nucleic acid sequence which disrupts a coding sequence comprising said inventive DNA sequence or which disrupts the expressible format of a DNA sequence comprising said inventive DNA sequence. Removal of the intervening sequence restores said coding sequence or said expressible format.

Examples of intervening sequences include introns and mobilizable DNA sequences such as transposons. With "mobilizable DNA sequence" is meant any DNA sequence that can be mobilized as the result of a recombination event.

**Table 2.** Degeneracy of the genetic code.

| Amino Acid | Three -letter code | One-letter code | Possible codons | | | | | |
|---|---|---|---|---|---|---|---|---|
| Alanine | Ala | A | GCA | GCC | GCG | GCU | | |
| Arginine | Arg | R | AGA | AGG | CGA | CGC | CGG | CGU |
| Asparagine | Asn | N | AAC | AAU | | | | |
| Aspartic Acid | Asp | D | GAC | GAU | | | | |
| Cysteine | Cys | C | UGC | UGU | | | | |
| Glutamic Acid | Glu | E | GAA | GAG | | | | |
| Glutamine | Gln | Q | CAA | CAG | | | | |
| Glycine | Gly | G | GGA | GGC | GGG | GGU | | |
| Histidine | His | H | CAC | CAU | | | | |
| Isoleucine | Ile | I | AUA | AUC | AUU | | | |
| Leucine | Leu | L | UUA | UUG | CUA | CUC | CUG | CUU |
| Lysine | Lys | K | AAA | AAG | | | | |

(continued)

| Amino Acid | Three -letter code | One-letter code | Possible codons | | | | | |
|---|---|---|---|---|---|---|---|---|
| Methionine | Met | M | AUG | | | | | |
| Phenylalanine | Phe | F | UUC | UUU | | | | |
| Proline | Pro | P | CCA | CCC | CCG | CCU | | |
| Serine | Ser | S | AGC | AGU | UCA | UCC | UCG | UCU |
| Threonine | Thr | T | ACA | ACC | ACG | ACU | | |
| Tryptophan | Trp | W | UGG | | | | | |
| Tyrosine | Tyr | Y | UAC | UAU | | | | |
| Valine | Val | V | GUA | GUC | GUG | GUU | | |
| | | | Possible "STOP" codons | | | | | |
| | | | UAA | UAG | UGA | | | |

**Table 3.** Usage of the indicated codons in the different organisms given as frequency per thousand codons (http://www.kazusa.or.jp/codon).

| Codon | *Agrobacterium tumefaciens* | *Arabidopsis thaliana* | *Medicago sativa* | *Oryza sativa* |
|---|---|---|---|---|
| UUU | 13.9 | 22.5 | 24.1 | 11.3 |
| UUC | 24.3 | 20.7 | 16.9 | 26.3 |
| UUA | 3.5 | 12.9 | 10.4 | 4.7 |
| UUG | 13.2 | 21.0 | 22.4 | 11.8 |
| UCU | 7.0 | 24.6 | 19.8 | 10.1 |
| UCC* | 14.8 | 10.8 | 7.7 | 16.9 |
| UCA | 7.4 | 17.8 | 17.2 | 9.7 |
| UCG | 18.2 | 8.9 | 3.2 | 10.8 |
| UAU | 12.3 | 15.2 | 16.6 | 9.2 |
| UAC | 10.3 | 13.7 | 14.0 | 20.6 |
| UAA | 0.9 | 0.9 | 1.2 | 0.9 |
| UAG | 0.6 | 0.5 | 0.8 | 0.8 |
| UGU | 3.0 | 10.8 | 10.6 | 5.0 |
| UGC | 7.4 | 7.2 | 5.8 | 14.3 |
| UGA | 1.8 | 1.0 | 0.8 | 1.3 |
| UGG | 12.2 | 12.7 | 10.0 | 12.8 |
| CUU | 19.1 | 24.3 | 28.3 | 14.6 |
| CUC | 25.7 | 15.9 | 12.0 | 28.0 |
| CUA | 5.2 | 10.0 | 8.8 | 5.7 |
| CUG | 31.6 | 9.9 | 8.5 | 22.1 |
| CCU | 7.7 | 18.3 | 23.2 | 11.8 |
| CCC | 10.6 | 5.3 | 5.3 | 12.5 |
| CCA | 8.9 | 16.1 | 22.6 | 12.2 |

(continued)

| Codon | *Agrobacterium tumefaciens* | *Arabidopsis thaliana* | *Medicago sativa* | *Oryza sativa* |
|---|---|---|---|---|
| CCG | 20.7 | 8.3 | 3.6 | 16.7 |
| CAU | 10.6 | 14.0 | 14.6 | 9.2 |
| CAC | 9.1 | 8.7 | 9.1 | 14.6 |
| CAA | 11.2 | 19.7 | 23.2 | 11.9 |
| CAG | 24.9 | 15.2 | 12.3 | 24.6 |
| CGU | 12.2 | 8.9 | 10.1 | 6.8 |
| CGC | 25.5 | 3.7 | 4.2 | 15.9 |
| CGA | 8.2 | 6.2 | 4.2 | 4.2 |
| CGG | 13.2 | 4.8 | 1.8 | 9.7 |
| AUU | 15.4 | 22.0 | 29.4 | 13.8 |
| AUC | 36.9 | 18.5 | 14.7 | 25.5 |
| AUA | 6.2 | 12.9 | 11.7 | 7.2 |
| AUG | 24.7 | 24.5 | 21.7 | 24.4 |
| ACU | 6.4 | 17.8 | 20.8 | 10.3 |
| ACC | 20.9 | 10.3 | 11.7 | 18.6 |
| ACA | 9.1 | 15.9 | 18.9 | 10.0 |
| ACG | 18.8 | 7.6 | 2.8 | 10.8 |
| AAU | 13.5 | 22.7 | 25.0 | 12.9 |
| AAC | 18.7 | 20.9 | 18.7 | 25.1 |
| AAA | 13.6 | 31.0 | 32.2 | 12.0 |
| AAG | 24.4 | 32.6 | 35.1 | 39.4 |
| AGU | 5.7 | 14.0 | 12.6 | 7.3 |
| AGC | 15.8 | 11.1 | 8.8 | 16.9 |
| AGA | 5.3 | 18.7 | 13.6 | 7.7 |
| AGG | 6.5 | 10.9 | 11.7 | 14.9 |
| GUU | 16.6 | 27.3 | 34.7 | 15.0 |
| GUC | 29.3 | 12.7 | 9.9 | 22.8 |
| GUA | 6.1 | 10.1 | 10.0 | 5.7 |
| GUG | 19.7 | 17.5 | 16.5 | 25.0 |
| GCU | 17.4 | 28.0 | 34.6 | 19.8 |
| GCC | 35.8 | 10.3 | 11.4 | 33.2 |
| GCA | 19.5 | 17.6 | 25.9 | 15.6 |
| GCG | 31.7 | 8.8 | 3.4 | 25.3 |
| GAU | 25.8 | 36.8 | 40.0 | 21.5 |
| GAC | 28.0 | 17.3 | 15.5 | 31.6 |
| GAA | 29.9 | 34.4 | 35.9 | 17.1 |
| GAG | 26.3 | 32.2 | 27.4 | 41.1 |

(continued)

| Codon | *Agrobacterium tumefaciens* | *Arabidopsis thaliana* | *Medicago sativa* | *Oryza sativa* |
|---|---|---|---|---|
| GGU | 16.5 | 22.2 | 28.7 | 16.3 |
| GGC | 36.2 | 9.0 | 8.4 | 34.7 |
| GGA | 12.5 | 23.9 | 27.3 | 15.0 |
| GGG | 11.3 | 10.2 | 7.4 | 16.6 |

"Hybridization" is the process wherein substantially homologous complementary nucleotide sequences anneal to each other. The hybridization process can occur entirely in solution, i.e. both complementary nucleic acids are in solution. Tools in molecular biology relying on such a process include PCR, subtractive hybridization and DNA sequence determination. The hybridization process can also occur with one of the complementary nucleic acids immobilized to a matrix such as magnetic beads, Sepharose beads or any other resin. Tools in molecular biology relying on such a process include the isolation of poly (A+) mRNA. The hybridization process can furthermore occur with one of the complementary nucleic acids immobilized to a solid support such as a nitrocellulose or nylon membrane or immobilized by e.g. photolithography to e.g. a silicious glass support (the latter known as nucleic acid arrays or microarrays or as nucleic acid chips). Tools in molecular biology relying on such a process include RNA and DNA gel blot analysis, colony hybridization, plaque hybridization and microarray hybridization. In order to allow hybridization to occur, the nucleic acid molecules are generally thermally or chemically (e.g. by NaOH) denatured to melt a double strand into two single strands and/or to remove hairpins or other secondary structures from single stranded nucleic acids. The stringency of hybridization is influenced by conditions such as temperature, salt concentration and hybridization buffer composition. High stringency conditions for hybridization include high temperature and/or low salt concentration (salts include NaCl and Na3-citrate) and/or the inclusion of formamide in the hybridization buffer and/or lowering the concentration of compounds such as SDS (detergent) in the hybridization buffer and/or exclusion of compounds such as dextran sulfate or polyethylene glycol (promoting molecular crowding) from the hybridization buffer. Conventional hybridization conditions are described in e.g. Sambrook et al. (1989) but the skilled craftsman will appreciate that numerous different hybridization conditions can be designed in function of the known or the expected homology and/or length of the nucleic acid sequence. Sufficiently low stringency hybridization conditions are particularly preferred to isolate nucleic acids heterologous to the DNA sequences defined supra. Elements contributing to said heterology include allelism, degeneration of the genetic code and differences in preferred codon usage as discussed supra.

The term "specifically hybridizing" or "hybridizing specifically" refers to the binding, duplexing, or hybridizing of a molecule to a particular nucleotide sequence under medium to stringent conditions when that sequence is presented in a complex mixture e.g., total cellular DNA or RNA.

"Stringent hybridization conditions" and "stringent hybridization wash conditions" in the context of nucleic acid hybridization experiments such as Southern and Northern hybridizations are sequence dependent and are different under different environmental parameters. For example, longer sequences hybridize specifically at higher temperatures. The $T_m$ is the temperature under defined ionic strength and pH, at which 50% of the target sequence hybridizes to a perfectly matched probe. Specificity is typically the function of post-hybridization washes. Critical factors of such washes include the ionic strength and temperature of the final wash solution.

Generally, stringent conditions are selected to be about 50°C lower than the thermal melting point ($T_m$) for the specific sequence at a defined ionic strength and pH. The $T_m$ is the temperature (under defined ionic strength and pH) at which 50% of the target sequence hybridizes to a perfectly matched probe. The $T_m$ is dependent upon the solution conditions and the base composition of the probe, and may be calculated using the following equation:

$$T_m = 79.8°C + (18.5 \times \text{Log}[Na+]) + (58.4°C \times \%[G+C]) - (820 / \#\ \text{bp in duplex}) - (0.5 \times \%\ \text{formamide})$$

More preferred stringent conditions are when the temperature is 20°C below $T_m$, and the most preferred stringent conditions are when the temperature is 10°C below $T_m$. Nonspecific binding may also be controlled using any one of a number of known techniques such as, for example, blocking the membrane with protein-containing solutions, addition of heterologous RNA, DNA, and SDS to the hybridization buffer, and treatment with RNase.

Wash conditions are typically performed at or below stringency. Generally, suitable stringent conditions for nucleic acid hybridization assays or gene amplification detection procedures are as set forth above. More or less stringent conditions may also be selected.

For the purposes of defining the level of stringency, reference can conveniently be made to Sambrook, J., E.F. Fritsch, et al. 1989 "Molecular Cloning: a Laboratory Manual, 2nd Edition, Cold Spring Harbor, NY, Cold Spring Harbor Laboratory Press, at 11.45. An example of low stringency conditions is 4-6X SSC/0.1-0.5% w/v SDS at 37˚-45˚ C for 2-3 hours. Depending on the source and concentration of the nucleic acid involved in the hybridization, alternative conditions of stringency may be employed such as medium stringent conditions. Examples of medium stringent conditions include 1-4X SSC/0.25% w/v SDS at ≥ 45˚ C for 2-3 hours. An example of high stringency conditions includes 0.1-1X SSC/0.1% w/v SDS at 60 C for 1-3 hours. The skilled artisan is aware of various parameters which may be altered during hybridization and washing and which will either maintain or change the stringency conditions. For example, another stringent hibridization condition is hybridization at 4X SSC at 65˚ C, followed by a washing in 0.1X SSC at 65˚ C for about one hour. Alternatively, an exemplary stringent hybridization condition is in 50% formamide, 4XSSC, at 42˚ C. Still another example of stringent conditions include hybridization at 62˚ C in 6X SSC, .05X BLOTTO, and washing at 2X SSC, 0.1% SDS at 62˚ C.

Clearly, the current specification embodies the use of the disclosed DNA sequences encoding a cytokinin oxidase, homologue, derivative or immunologically active and/or functional fragment thereof as defined higher in any method of hybridization. The current specification furthermore also relates to DNA sequences hybridizing to said specification DNA sequences. Preferably said cytokinin oxidase is a plant cytokinin oxidase, more specifically the *Arabidopsis thaliana* (At)CKX.

To effect expression of a protein in a cell, tissue or organ, preferably of plant origin, either the protein may be introduced directly to said cell, such as by microinjection or ballistic means or alternatively, an isolated nucleic acid molecule encoding said protein may be introduced into said cell, tissue or organ in an expressible format.

Preferably, the disclosed DNA sequence comprises a coding sequence or open reading frame (ORF) encoding a cytokinin oxidase protein or a homologue or derivative thereof or an immunologically active and/or functional fragment thereof as defined *supra.* The preferred disclosed protein comprises the amino acid sequence of said cytokinin oxidase. Preferably said cytokinin oxidase is a plant cytokinin oxidase and more specifically a *Arabidopsis thaliana* (At)CKX.

With "vector" or "vector sequence" is meant a DNA sequence which can be introduced in an organism by transformation and can be stably maintained in said organism. Vector maintenance is possible in e.g. cultures of *Escherichia coli, A. tumefaciens, Saccharomyces cerevisiae* or *Schizosaccharomyces pombe.* Other vectors such as phagemids and cosmid vectors can be maintained and multiplied in bacteria and/or viruses. Vector sequences generally comprise a set of unique sites recognized by restriction enzymes, the multiple cloning site (MCS), wherein one or more non-vector sequence(s) can be inserted.

With "non-vector sequence" is accordingly meant a DNA sequence which is integrated in one or more of the sites of the MCS comprised within a vector.

"Expression vectors" form a subset of vectors which, by virtue of comprising the appropriate regulatory or control sequences enable the creation of an expressible format for the inserted non-vector sequence(s), thus allowing expression of the protein encoded by said non-vector sequence(s). Expression vectors are known in the art enabling protein expression in organisms including bacteria (e.g. *E. coli),* fungi (e.g. *S. cerevisiae, S. pombe, Pichia pastoris),* insect cells (e.g. baculoviral expression vectors), animal cells (e.g. COS or CHO cells) and plant cells (e.g. potato virus X-based expression vectors).

The current specification clearly includes any cytokinin oxidase, homologue, derivative and/or immunologically active and/or functional fragment thereof as defined supra. Preferably said cytokinin oxidase is a plant cytokinin oxidase, more specifically a *Arabidopsis thaliana* (At)CKX.

As an alternative to expression vector-mediated protein production in biological systems, chemical protein synthesis can be applied. Synthetic peptides can be manufactured in solution phase or in solid phase. Solid phase peptide synthesis (Merrifield 1963) is, however, the most common way and involves the sequential addition of amino acids to create a linear peptide chain. Solid phase peptide synthesis includes cycles consisting of three steps: (i) immobilization of the carboxy-terminal amino acid of the growing peptide chain to a solid support or resin; (ii) chain assembly, a process consisting of activation, coupling and deprotection of the amino acid to be added to the growing peptide chain; and (iii) cleavage involving removal of the completed peptide chain from the resin and removal of the protecting groups from the amino acid side chains. Common approaches in solid phase peptide synthesis include Fmoc/tBu (9-fluorenylmethyloxycarbonyl/t-butyl) and Boc (t-butyloxycarbonyl) as the amino-terminal protecting groups of amino acids. Amino acid side chain protecting groups include methyl (Me), formyl (CHO), ethyl (Et), acetyl (Ac), t-butyl (t-Bu), anisyl, benzyl (Bzl), trifluroacetyl (Tfa), N-hydroxysuccinimide (ONSu, OSu), benzoyl (Bz), 4-methylbenzyl (Meb), thioanizyl, thiocresyl, benzyloxymethyl (Bom), 4-nitrophenyl (ONp), benzyloxycarbonyl (Z), 2-nitrobenzoyl (NBz), 2-nitrophenylsulphenyl (Nps), 4-toluenesulphonyl (Tosyl,Tos), pentafluorophenyl (Pfp), diphenylmethyl (Dpm), 2-chlorobenzyloxycarbonyl (Cl-Z), 2,4,5-trichlorophenyl, 2-bromobenzyloxycarbonyl (Br-Z), tripheylmethyl (Trityl, Trt), and 2,5,7,8-pentamethyl-chroman-6-sulphonyl (Pmc). During chain assembly, Fmoc or Boc are removed resulting in an activated amino-terminus of the amino acid residue bound to the growing chain. The carboxy-terminus of the incoming amino acid is activated by conversion into a highly reactive ester, e.g. by HBTU. With current technologies (e.g. PerSeptive Biosystems 9050 synthesizer, Applied Biosystems Model 431A Peptide Synthesizer), linear peptides of up to 50 residues can be manufactured. A number of guidelines is available to produce peptides that are suitable for use in biological systems including (i) limiting the use of difficult amino acids such as cys, met, trp (easily oxidized and/or degraded during peptide synthesis) or arg; (ii) minimize hydrophobic amino acids (can impair peptide solubility); and (iii) prevent an amino-terminal glutamic acid (can cyclize to pyroglutamate).

By "expressible format" is meant that the isolated nucleic acid molecule is in a form suitable for being transcribed into mRNA and/or translated to produce a protein, either constitutively or following induction by an intracellular or extracellular signal, such as an environmental stimulus or stress (mitogens, anoxia, hypoxia, temperature, salt, light, dehydration, etc) or a chemical compound such as IPTG (isopropyl-β-D-thiogalactopyranoside) or such as an antibiotic (tetracycline, ampicillin, rifampicin, kanamycin), hormone (e.g. gibberellin, auxin, cytokinin, glucocorticoid, brassinosteroid, ethylene, abscisic acid etc), hormone analogue (indoleacetic acid (IAA), 2,4-D, etc), metal (zinc, copper, iron, etc), or dexamethasone, amongst others. As will be known to those skilled in the art, expression of a functional protein may also require one or more post-translational modifications, such as glycosylation, phosphorylation, dephosphorylation, or one or more protein-protein interactions, amongst others. All such processes are included within the scope of the term "expressible format".

Preferably, expression of a protein in a specific cell, tissue, or organ, preferably of plant origin, is effected by introducing and expressing an isolated nucleic acid molecule encoding said protein, such as a cDNA molecule, genomic gene, synthetic oligonucleotide molecule, mRNA molecule or open reading frame, to said cell, tissue or organ, wherein said nucleic acid molecule is placed operably in connection with suitable regulatory or control sequences including a promoter, preferably a plant-expressible promoter, and a terminator sequence.

Reference herein to a "promoter" is to be taken in its broadest context and includes the transcriptional regulatory sequences derived from a classical eukaryotic genomic gene, including the TATA box which is required for accurate transcription initiation, with or without a CCAAT box sequence and additional regulatory or control elements (i.e. upstream activating sequences, enhancers and silencers) which alter gene expression in response to developmental and/or external stimuli, or in a tissue-specific manner.

The term "promoter" also includes the transcriptional regulatory sequences of a classical prokaryotic gene, in which case it may include a -35 box sequence and/or a -10 box transcriptional regulatory sequences.

The term "promoter" is also used to describe a synthetic or fusion molecule, or derivative which confers, activates or enhances expression of a nucleic acid molecule in a cell, tissue or organ.

Promoters may contain additional copies of one or more specific regulatory elements, to further enhance expression and/or to alter the spatial expression and/or temporal expression of a nucleic acid molecule to which it is operably connected. Such regulatory elements may be placed adjacent to a heterologous promoter sequence to drive expression of a nucleic acid molecule in response to e.g. copper, glucocorticoids, dexamethasone, tetracycline, gibberellin, cAMP, abscisic acid, auxin, wounding, ethylene, jasmonate or salicylic acid or to confer expression of a nucleic acid molecule to specific cells, tissues or organs such as meristems, leaves, roots, embryo, flowers, seeds or fruits.

In the context of the present specification, the promoter preferably is a plant-expressible promoter sequence. Promoters that also function or solely function in non-plant cells such as bacteria, yeast cells, insect cells and animal cells are not excluded. By "plant-expressible" is meant that the promoter sequence, including any additional regulatory elements added thereto or contained therein, is at least capable of inducing, conferring, activating or enhancing expression in a plant cell, tissue or organ, preferably a monocotyledonous or dicotyledonous plant cell, tissue, or organ.

The terms "plant-operable" and "operable in a plant" when used herein, in respect of a promoter sequence, shall be taken to be equivalent to a plant-expressible promoter sequence.

Regulatable promoters as part of a binary viral plant expression system are also known to the skilled artisan (Yadav 1999 - WO9922003; Yadav 2000 - W00017365).

In the present context, a "regulatable promoter sequence" is a promoter that is capable of conferring expression on a structural gene in a particular cell, tissue, or organ or group of cells, tissues or organs of a plant, optionally under specific conditions, however does generally not confer expression throughout the plant under all conditions. Accordingly, a regulatable promoter sequence may be a promoter sequence that confers expression on a gene to which it is operably connected in a particular location within the plant or alternatively, throughout the plant under a specific set of conditions, such as following induction of gene expression by a chemical compound or other elicitor.

Preferably, the regulatable promoter used in the performance of the present invention confers expression in a specific location within the plant, either constitutively or following induction, however not in the whole plant under any circumstances. Included within the scope of such promoters are cell-specific promoter sequences, tissue-specific promoter sequences, organ-specific promoter sequences, cell cycle specific gene promoter sequences, inducible promoter sequences and constitutive promoter sequences that have been modified to confer expression in a particular part of the plant at any one time, such as by integration of said constitutive promoter within a transposable genetic element (*Ac, Ds, Spm, En,* or other transposon).

Similarly, the term "tissue-specific" shall be taken to indicate that expression is predominantly in a particular tissue or tissue-type, preferably of plant origin, albeit not necessarily exclusively in said tissue or tissue-type.

Similarly, the term "organ-specific" shall be taken to indicate that expression is predominantly in a particular organ, preferably of plant origin, albeit not necessarily exclusively in said organ.

Similarly, the term "cell cycle specific" shall be taken to indicate that expression is predominantly cyclic and occurring in one or more, not necessarily consecutive phases of the cell cycle albeit not necessarily exclusively in cycling cells, preferably of plant origin.

Those skilled in the art will be aware that an "inducible promoter" is a promoter the transcriptional activity of which is increased or induced in response to a developmental, chemical, environmental, or physical stimulus. Similarly, the skilled craftsman will understand that a "constitutive promoter" is a promoter that is transcriptionally active throughout most, but not necessarily all parts of an organism, preferably a plant, during most, but not necessarily all phases of its growth and development.

Those skilled in the art will readily be capable of selecting appropriate promoter sequences for use in regulating appropriate expression of the cytokinin oxidase protein from publicly-available or readily-available sources, without undue experimentation.

Placing a nucleic acid molecule under the regulatory control of a promoter sequence, or in operable connection with a promoter sequence, means positioning said nucleic acid molecule such that expression is controlled by the promoter sequence. A promoter is usually, but not necessarily, positioned upstream, or at the 5'-end, and within 2 kb of the start site of transcription, of the nucleic acid molecule which it regulates. In the construction of heterologous promoter/structural gene combinations it is generally preferred to position the promoter at a distance from the gene transcription start site that is approximately the same as the distance between that promoter and the gene it controls in its natural setting (i.e., the gene from which the promoter is derived). As is known in the art, some variation in this distance can be accommodated without loss of promoter function. Similarly, the preferred positioning of a regulatory sequence element with respect to a heterologous gene to be placed under its control is defined by the positioning of the element in its natural setting (i.e., the gene from which it is derived). Again, as is known in the art, some variation in this distance can also occur.

Examples of promoters suitable for use in disclosed gene constructs include those listed in Table 4, amongst others. The promoters listed in Table 4 are provided for the purposes of exemplification only and are not to be limited by the list provided therein. Those skilled in the art will readily be in a position to provide additional promoters that are useful.

In the case of constitutive promoters or promoters that induce expression throughout the entire plant, it is preferred that such sequences are modified by the addition of nucleotide sequences derived from one or more of the tissue-specific promoters listed in Table 4, or alternatively, nucleotide sequences derived from one or more of the above-mentioned tissue-specific inducible promoters, to confer tissue-specificity thereon. For example, the CaMV 35S promoter may be modified by the addition of maize *Adh1* promoter sequence, to confer anaerobically-regulated root-specific expression thereon, as described previously (Ellis *et al.,* 1987). Another example describes conferring root specific or root abundant gene expression by fusing the CaMV35S promoter to elements of the maize glycine-rich protein GRP3 gene (Feix and Wulff 2000 - WO0015662). Such modifications can be achieved by routine experimentation by those skilled in the art.

The term "terminator" refers to a DNA sequence at the end of a transcriptional unit which signals termination of transcription. Terminators are 3'-non-translated DNA sequences containing a polyadenylation signal, which facilitates the addition of polyadenylate sequences to the 3'-end of a primary transcript. Terminators active in cells derived from viruses, yeasts, molds, bacteria, insects, birds, mammals and plants are known and described in the literature. They may be isolated from bacteria, fungi, viruses, animals and/or plants.

**Table 4.** Exemplary plant-expressible promoters for use in the performance of the present specification

| **I: CELL-SPECIFIC,TISSUE-SPECIFIC, AND ORGAN-SPECIFIC PROMOTERS** | | |
|---|---|---|
| **GENE SOURCE** | **EXPRESSION PATTERN** | **REFERENCE** |
| α-amylase (Amy32b) | aleurone | Lanahan, M.B., et al., Plant Cell 4: 203-211, 1992; Skriver, K., et al. Proc. Natl. Acad. Sci. (USA) 88: 7266-7270, 1991 |
| cathepsin β-like gene | aleurone | Cejudo, F.J., et al. Plant Molecular Biology 20:849-856, 1992. |
| *Agrobacterium rhizogenes rolb* | cambium | Nilsson et al., Physiol. Plant. 100: 456-462, 1997 |

(continued)

| I: CELL-SPECIFIC,TISSUE-SPECIFIC, AND ORGAN-SPECIFIC PROMOTERS | | |
|---|---|---|
| GENE SOURCE | EXPRESSION PATTERN | REFERENCE |
| AtPRP4 | flowers | http://salus.medium.edu/mmg/ tierney/ html |
| chalcone synthase (chsA) | flowers | Van der Meer, et al., Plant Mol. Biol. 15, 95-109, 1990. |
| LAT52 | anther | Twell et al Mol. Gen Genet. 217: 240-245 (1989) |
| *apetala-3* | flowers | |
| Chitinase | fruit (berries, grapes, etc) | Thomas et al. CSIRO Plant Industry, Urrbrae, South Australia, Australia; http://winetitles.com.au/ gwrdc/csh95-1.html |
| rbcs-3A | green tissue (eg leaf) | Lam, E. et al., The Plant Cell 2: 857-866, 1990.; Tucker et al., Plant Physiol. 113: 1303-1308, 1992. |
| leaf-specific genes | leaf | Baszczynski, et al., Nucl. Acid Res. 16: 4732, 1988. |
| AtPRP4 | leaf | http://salus.medium.edu/mmg/ tierney/ html |
| chlorella virus adenine methyltransferase gene promoter | leaf | Mitra and Higgins, 1994, Plant Molecular Biology 26: 85-93 |
| aldP gene promoter from rice | leaf | Kagaya et al., 1995, Molecular and General Genetics 248: 668-674 |
| rbcs promoter from rice or tomato | leaf | Kyozuka et al., 1993, Plant Physiology 102: 991-1000 |
| Pinus cab-6 | leaf | Yamamoto et al., Plant Cell Physiol. 35:773-778, 1994. |
| rubisco promoter | leaf | |
| cab (chlorophyll a/b/binding protein | leaf | |
| SAM22 | senescent leaf | Crowell, et al., Plant Mol. Biol. 18: 459-466, 1992. |
| *Itp gene (lipid transfer gene)* | | Fleming, et al, Plant J. 2, 855-862. |
| *R. japonicum nif* gene | Nodule | United States Patent No. 4, 803, 165 |
| *B. japonicum nifH* gene | Nodule | United States Patent No. 5, 008, 194 |
| GmENOD40 | Nodule | Yang, et al., The Plant J. 3: 573-585. |
| PEP carboxylase (PEPC) | Nodule | Pathirana, et al., Plant Mol. Biol. 20: 437-450, 1992. |
| Leghaemoglobin (Lb) | Nodule | Gordon, et al., J. Exp. Bot. 44: 1453-1465, 1993. |
| *Tungro bacilliform* virus gene | phloem | Bhattacharyya-Pakrasi, et al, The Plant J. 4: 71-79, 1992. |
| pollen-specific genes | pollen; microspore | Albani, et al., Plant Mol. Biol. 15: 605, 1990; Albani, et al., Plant Mol. Biol. 16: 501, 1991) |

(continued)

| I: CELL-SPECIFIC,TISSUE-SPECIFIC, AND ORGAN-SPECIFIC PROMOTERS | | |
|---|---|---|
| **GENE SOURCE** | **EXPRESSION PATTERN** | **REFERENCE** |
| Zm13 | pollen | Guerrero et al Mol. Gen. Genet. 224: 161-168 (1993) |
| apg gene | microspore | Twell et al Sex. Plant Reprod. 6: 217-224 (1993) |
| maize pollen-specific gene | pollen | Hamilton, et al., Plant Mol. Biol. 18: 211-218, 1992. |
| sunflower pollen-expressed gene | pollen | Baltz, et al., The Plant J. 2: 713-721, 1992. |
| *B. napus* pollen-specific gene | pollen;anther tapetum | Arnoldo, et al., J. Cell. Biochem., Abstract No. Y101, 204, 1992. |
| root-expressible genes | roots | Tingey, et al., EMBO J. 6: 1, 1987. |
| tobacco auxin-inducible gene | root tip | Van der Zaal, et al., Plant Mol. Biol. 16,983,1991. |
| β-tubulin | root | Oppenheimer, et al., Gene 63: 87, 1988. |
| tobacco root-specific genes | root | Conkling, et al., Plant Physiol. 93: 1203,1990. |
| *B. napus* G1-3b gene | root | United States Patent No. 5, 401, 836 |
| SbPRP1 | roots | Suzuki et al., Plant Mol. Biol. 21: 109-119, 1993. |
| AtPRP1; AtPRP3 | roots; root hairs | http://saius.medium.edu/mmg/ tierney/ html |
| RD2 gene | root cortex | http://www2.cnsu.edu/ncsu/research |
| TobRB7 gene | root vasculature | http://www2.cnsu.edu/ncsu/research |
| AtPRP4 | leaves; flowers; lateral root primordia | http://salus.medium.edu/mmg/ tierney/ html |
| seed-specific genes | seed | Simon, et al., Plant Mol. Biol. 5: 191, 1985; Scofield, et al., J. Biol. Chem. 262: 12202, 1987.; Baszczynski, et al., Plant Mol. Biol. 14: 633, 1990. |
| Brazil Nut albumin | seed | Pearson, et al., Plant Mol. Biol. 18: 235-245,1992. |
| Legumin | seed | Ellis, et al., Plant Mol. Biol. 10: 203-214, 1988. |
| glutelin (rice) | seed | Takaiwa, et al., Mol. Gen. Genet. 208: 15-22, 1986; Takaiwa, et al., FEBS Letts. 221: 43-47, 1987. |
| Zein | seed | Matzke et al Plant Mol Biol, 14(3): 323-32 1990 |
| NapA | seed | Stalberg, et al, Planta 199: 515-519, 1996. |
| wheat LMW and HMW glutenin-1 | endosperm | Mol Gen Genet 216:81-90, 1989; NAR 17:461-2, 1989 |

(continued)

| I: CELL-SPECIFIC,TISSUE-SPECIFIC, AND ORGAN-SPECIFIC PROMOTERS | | |
|---|---|---|
| **GENE SOURCE** | **EXPRESSION PATTERN** | **REFERENCE** |
| wheat SPA | seed | Albani et al, Plant Cell, 9: 171-184, 1997 |
| wheat $\alpha$, $\beta$, $\gamma$-gliadins | endosperm | EMBO 3:1409-15, 1984 |
| barley *Itr1* promoter | endosperm | |
| barley B1, C, D, hordein | endosperm | Theor Appl Gen 98:1253-62, 1999; Plant J 4:343-55, 1993; Mol Gen Genet 250:750-60, 1996 |
| barley DOF | endosperm | Mena et al, The Plant Journal, 116(1): 53-62, 1998 |
| *blz2* | endosperm | EP99106056.7 |
| synthetic promoter | endosperm | Vicente-Carbajosa et al., Plant J. 13: 629-640, 1998. |
| rice prolamin NRP33 | endosperm | Wu et al, Plant Cell Physiology 39(8) 885-889, 1998 |
| rice $\alpha$-globulin Glb-1 | endosperm, | Wu et al, Plant Cell Physiology 39(8) 885-889,1998 |
| rice OSH1 | embryo | Sato et al, Proc. Natl. Acad. Sci. USA, 93: 8117-8122, 1996 |
| rice $\alpha$-globulin REB/OHP-1 | endosperm | Nakase et al. Plant Mol. Biol. 33: 513-522, 1997 |
| rice ADP-glucose PP | endosperm | Trans Res 6:157-68,1997 |
| maize ESR gene family | endosperm | Plant J 12:235-46, 1997 |
| sorgum $\gamma$-kafirin | endosperm | PMB 32:1029-35, 1996 |
| KNOX | embryo | Postma-Haarsma et al, Plant Mol. Biol. 39:257-71, 1999 |
| rice oleosin | embryo and aleuron | Wu et at, J. Biochem., 123:386, 1998 |
| sunflower oleosin | seed (embryo and dry seed) | Cummins, et al., Plant Mol. Biol. 19: 873-876,1992 |
| *LEAFY* | shoot meristem | Weigel et al., Cell 69:843-859, 1992. |
| *Arabidopsis thaliana knat1* | shoot meristem | Accession numberAJ131822 |
| *Malus domestica kn 1* | shoot meristem | Accession number Z71981 |
| *CLAVATA1* | shoot meristem | Accession number AF049870 |
| stigma-specific genes | stigma | Nasrallah, et al., Proc. Natl. Acad. |
| | | Sci. USA 85: 5551, 1988; Trick, et al., Plant Mol. Biol. 15: 203, 1990. |
| class I patatin gene | tuber | Liu et al., Plant Mol. Biol. 153: 386-395, 1991. |
| PCNA rice | meristem | Kosugi et al, Nucleic Acids Research 19:1571-1576, 1991; Kosugi S. and Ohashi Y, Plant Cell 9:1607-1619, 1997. |

(continued)

| I: CELL-SPECIFIC,TISSUE-SPECIFIC, AND ORGAN-SPECIFIC PROMOTERS | | |
|---|---|---|
| **GENE SOURCE** | **EXPRESSION PATTERN** | **REFERENCE** |
| Pea TubA1 tubulin | Dividing cells | Stotz and Long, Plant Mol.Biol. 41, 601-614. 1999 |
| *Arabidopsis* cdc2a | cycling cells | Chung and Parish, FEBS Lett, 3;362 (2):215-9, 1995 |
| *Arabidopsis* Rop1A | Anthers; mature pollen + pollen tubes | Li et al. 1998 Plant Physiol 118, 407-417. |
| *Arabidopsis* AtDMC1 | Meiosis-associated | Klimyuk and Jones 1997 Plant J. 11, 1-14. |
| Pea PS-IAA4/5 and PS-IAA6 | Auxin-inducible | Wong et al. 1996 Plant J. 9, 587-599. |
| Pea farnesyltransferase | Meristematic tissues; phloem near growing tissues; light- and sugar-repressed | Zhou et al. 1997 Plant J. 12, 921-930 |
| Tobacco *(N. sylvestris)* cyclin B1;1 | Dividing cells / meristematic tissue | Trehin et al. 1997 Plant Mol.Biol. 35, 667-672. |
| Mitotic cyclins CYS (A-type) and CYM (B-type) | Dividing cells / meristematic tissue | Ito et al. 1997 Plant J. 11, 983-992 |
| *Arabidopsis* cyc1At (=cyc B1;1) and cyc3aAt (A-type) | Dividing cells / meristematic tissue | Shaul et al. 1996 Proc.Nati.Acad.Sci.U.S.A 93, 4868-4872. |
| *Arabidopsis* tef1 promoter box | Dividing cells / meristematic tissue | Regad et al. 1995 Mol. Gen. Genet. 248, 703-711. |
| *Catharanthus roseus* cyc07 | Dividing cells / meristematic tissue | Ito et al. 1994 Plant Mol.Biol. 24, 863-878. |
| **II: EXEMPLARY CONSTITUTIVE PROMOTERS** | | |
| **GENE SOURCE** | **EXPRESSION PATTERN** | **REFERENCE** |
| Actin | constitutive | McElroy et al, Plant Cell, 2: 163-171,1990 |
| CAMV 35S | constitutive | Odell et al, Nature, 313: 810-812, 1985 |
| CaMV 19S | constitutive | Nilsson et al., Physiol. Plant. 100:462, 1997 |
| GOS2 | constitutive | de Pater et al, Plant J. 2:837-844, 1992 |
| Ubiquitin | constitutive | Christensen et al, Plant Mol. Biol. 18: 675-689, 1992 |
| rice cyclophilin | constitutive | Buchholz et al, Plant Mol Biol. 25: 837-843, 1994 |
| maize histone H3 | constitutive | Lepetit et al, Mol. Gen. Genet. 231: 276-285,1992 |
| alfalfa histone H3 | constitutive | Wu et al., Nucleic Acids Res. 17: 3057-3063, 1989; Wu et al., Plant Mol. Biol. 11:641-649,1988 |

(continued)

| II: EXEMPLARY CONSTITUTIVE PROMOTERS | | |
|---|---|---|
| **GENE SOURCE** | **EXPRESSION PATTERN** | **REFERENCE** |
| actin 2 | constitutive | An et al, Plant J. 10(1); 107-121, 1996 |
| **III: EXEMPLARY STRESS-INDUCIBLE PROMOTERS** | | |
| **NAME** | **STRESS** | **REFERENCE** |
| P5CS (delta(1)-pyrroline-5-carboxylate syntase) | salt, water | Zhang et al. Plant Science. 129: 81-89, 1997 |
| cor15a | cold | Hajela et al., Plant Physiol. 93: 1246-1252,1990 |
| cor15b | cold | Wlihelm et al., Plant Mol Biol. 23: 1073-1077, 1993 |
| cor15a (-305 to +78 nt) | cold, drought | Baker et al., Plant Mol Biol. 24: 701-713, 1994 |
| rd29 | salt, drought, cold | Kasuga et al., Nature Biotechnology 18:287-291, 1999 |
| heat shock proteins, including artificial promoters containing the heat shock element (HSE) | heat | Barros et al., Plant Mol Biol 19: 665-75, 1992. Marrs et al., Dev Genet.14: 27-41, 1993. Schoffl et al., Mol Gen Gent, 217: 246-53, 1989. |
| smHSP (small heat shock proteins) | heat | Waters et al, J Experimental Botany 47:325-338, 1996 |
| wcs 120 | cold | Ouellet et al., FEBS Lett. 423: 324-328,1998 |
| ci7 | cold | Kirch et al., Plant Mol Biol 33: 897-909, 1997 |
| Adh | cold, drought, hypoxia | Dolferus et al., Plant Physiol 105: 1075-87, 1994 |
| pwsi 18 | water: salt and drought | Joshee et al., Plant Cell Physiol 39: 64-72, 1998 |
| ci21A | cold | Schneider et al., Plant Physiol 113: 335-45, 1997 |
| Trg-31 | drought | Chaudhary et al., Plant Mol Biol 30: 1247-57, 1996 |
| Osmotin | osmotic | Raghothama et al., Plant Mol Biol 23: 1117-28, 1993 |
| Rab17 | osmotic, ABA | Vilardell et al., Plant Mol Biol 17: 985-93, 1991 |
| LapA | wounding, enviromental | WO99/03977 University of California/ INRA |

(continued)

| IV: EXEMPLARY PATHOGEN-INDUCIBLE PROMOTERS | | |
|---|---|---|
| **NAME** | **PATHOGEN** | **REFERENCE** |
| RB7 | Root-knot nematodes (Meloidogyne spp.) | US5760386 - North Carolina State University; Opperman et al (1994) Science 263: 221-23. |
| PR-1, 2, 3, 4, 5, 8, 11 | fungal, viral, bacterial | Ward et al (1991) Plant Cell 3: 1085-1094; Reiss et al 1996; Lebel et al (1998), Plant J, 16(2):223-33; |
| | | Melchers et al (1994), Plant J, 5(4): 469-80; Lawton et al (1992), Plant Mol Biol, 19(5):735-43. |
| HMG2 | nematodes | WO9503690 - Virginia Tech Intellectual Properties Inc. |
| Abi3 | Cyst nematodes (Heterodera spp.) | Unpublished |
| ARM1 | nematodes | Barthels et al., (1997) The Plant Cell 9, 2119-2134.<br>WO 98/31822 - Plant Genetic Systems |
| Att0728 | nematodes | Barthels et al., (1997) The Plant Cell 9, 2119-2134.<br>PCT/EP98/07761 |
| Att1712 | nematodes | Barthels et al., (1997) The Plant Cell 9, 2119-2134.<br>PCT/EP98/07761 |
| Gst1 | Different types of pathogens | Strittmatter et al (1996) Mol. Plant-Microbe Interact. 9, 68-73. |
| *LEMMI* | nematodes | *WO 92/21757 - Plant Genetic Systems* |
| CLE | geminivirus | *PCT/EP99/03445 - CINESTAV* |
| PDF1.2 | Fungal including *Alternaria brassicicola* and *Botrytis cinerea* | Manners et al (1998), Plant Mol Biol, 38(6):1071-80. |
| Thi2.1 | Fungal - *Fusarium oxysporum f sp. matthiolae* | Vignutelli et al (1998) Plant J;14(3): 285-95 |
| DB#226 | nematodes | Bird and Wilson (1994) Mol. Plant-Microbe Interact., 7, 419-42 WO 95.322888 |
| DB#280 | nematodes | Bird and Wilson (1994) Mol. Plant-Microbe Interact., 7,419-42 WO 95.322888 |
| Cat2 | nematodes | Niebel et al (1995) Mol Plant Microbe Interact 1995 May-Jun;8(3):371-8 |
| □Tub | nematodes | Aristizabal et al (1996), 8th International Congress on Plant-Microbe Interaction, Knoxville US B-29 |

(continued)

| IV: EXEMPLARY PATHOGEN-INDUCIBLE PROMOTERS | | |
|---|---|---|
| **NAME** | **PATHOGEN** | **REFERENCE** |
| SHSP | nematodes | Fenoll et al (1997) In: Cellular and molecular aspects of plant-nematode interactions. Kluwer Academic, C. Fenoll, F.M.W. Grundler and S.A. Ohl (Eds.), |
| Tsw12 | nematodes | Fenoll et al (1997) In: Cellular and molecular aspects of plant-nematode interactions. Kluwer Academic, C. Fenoll, F.M.W. Grundler and S.A. Ohl (Eds.) |
| Hs1(pro1) | nematodes | WO 98/122335 - Jung |
| NsLTP | viral, fungal, bacterial | Molina & Garcia-Olmedo (1993) FEBS Lett. 316(2):119-22 |
| RIP | viral, fungal | Turner et al (1997) Proc Natl Acad Sci U S A, 94(8):3866-71 |

Examples of terminators particularly suitable for use in the disclosed gene constructs include the *Agrobacterium tumefaciens* nopaline synthase (NOS) gene terminator, the *Agrobacterium tumefaciens* octopine synthase (OCS) gene terminator sequence, the Cauliflower mosaic virus (CaMV) 35S gene terminator sequence, the *Oryza sativa* ADP-glucose pyrophosphorylase terminator sequence (t3'Bt2), the *Zea mays* zein gene terminator sequence, the *rbcs-1A* gene terminator, and the *rbcs-3A* gene terminator sequences, amongst others.

Preferred promoter sequences of the invention include root specific promoters and seed-specific promoters such as but not limited to the ones listed in Table 5, Table 4, and as outlined in the Examples.

**Table 5.** Exemplary, root specific promoters for use in the performance of the present specification

| NAME | ORIGIN | REFERENCE |
|---|---|---|
| SbPRP1 | Soybean | Suzuki et al., Plant Mol Biol, 21: 109-119, 1993 |
| 636 bp fragment of TobRB7 | Tobacco | Yamamoto et al., Plant Cell 3:371-382, 1991 |
| GGPS3 | Arabidopsis | Okada et al.,Plant Physiol 122: 1045-1056, 2000 |
| 580 bp fragment of prxEa | Arabidopsis | Wanapu and Shinmyo, Ann N Y Acad Sci 782: 107-114, 1996 |
| Ids2 promoter | Barley | Okumura et al., Plant Mol Biol 25: 705-719, 1994 |
| AtPRP3 | Arabidopsis | Fowler et al., Plant Physiol 121: 1081-1092, 1999 |

Those skilled in the art will be aware of additional promoter sequences and terminator sequences which may be suitable for use in performing the invention. Such sequences may readily be used without any undue experimentation.

In the context of the current invention, "ectopic expression" or "ectopic overexpression" of a gene or a protein are conferring to expression patterns and/or expression levels of said gene or protein normally not occurring under natural conditions, more specifically is meant increased expression and/or increased expression levels. Ectopic expression can be achieved in a number of ways including operably linking of a coding sequence encoding said protein to an isolated homologous or heterologous promoter in order to create a chimeric gene and/or operably linking said coding sequence to its own isolated promoter (i.e. the unisolated promoter naturally driving expression of said protein) in order to create a recombinant gene duplication or gene multiplication effect. With "ectopic co-expression" is meant the ectopic expression or ectopic overexpression of two or more genes or proteins. The same or, more preferably, different promoters are used to confer ectopic expression of said genes or proteins.

Preferably, the promoter sequence used in the context of the present invention is operably linked to a coding sequence or open reading frame (ORF) encoding a cytokinin oxidase protein or a homologue, derivative or an immunologically active and/or functional fragment thereof as defined supra.

"Downregulation of expression" as used herein means lowering levels of gene expression and/or levels of active gene product and/or levels of gene product activity. Decreases in expression may be accomplished by e.g. the addition of coding sequences or parts thereof in a sense orientation (if resulting in co-suppression) or in an antisense orientation relative to a promoter sequence and furthermore by e.g. insertion mutagenesis (e.g. T-DNA insertion or transposon insertion) or by gene silencing strategies as described by e.g. Angell and Baulcombe (1998 WO9836083), Lowe et al. (1989 - WO9853083), Lederer et al. (1999 - WO9915682) or Wang et al. (1999 - WO9953050). Genetic constructs aimed at silencing gene expression may have the nucleotide sequence of said gene (or one or more parts thereof) contained therein in a sense and/or antisense orientation relative to the promoter sequence. Another method to downregulate gene expression comprises the use of ribozymes.

Modulating, including lowering, the level of active gene products or of gene product activity can be achieved by administering or exposing cells, tissues, organs or organisms to said gene product, a homologue, derivative and/or immunologically active fragment thereof. Immunomodulation is another example of a technique capable of downregulation levels of active gene product and/or of gene product activity and comprises administration of or exposing to or expressing antibodies to said gene product to or in cells, tissues, organs or organisms wherein levels of said gene product and/or gene product activity are to be modulated. Such antibodies comprise "plantibodies", single chain antibodies, IgG antibodies and heavy chain camel antibodies as well as fragments thereof.

Modulating, including lowering, the level of active gene products or of gene product activity can furthermore be achieved by administering or exposing cells, tissues, organs or organisms to an agonist of said gene product or the activity thereof. Such agonists include proteins (comprising e.g. kinases and proteinases) and chemical compounds identified according to the current specification as described supra.

In the context of the current specification is envisaged the downregulation of the expression of a cytokinin oxidase gene as defined earlier. Preferably said cytokinin oxidase gene is a plant cytokinin oxidase gene, more specifically an *AtCKX.* The specification further discloses downregulation of levels of a cytokinin oxidase protein or of a cytokinin oxidase activity whereby said cytokinin oxidase protein has been defined supra. Preferably said cytokinin oxidase protein is a plant cytokinin oxidase, more specifically an AtCKX.

By "modifying cell fate and/or plant development and/or plant morphology and/or biochemistry and/or physiology" is meant that one or more developmental and/or morphological and/or biochemical and/or physiological characteristics of a plant is altered by the performance of one or more steps pertaining to the invention described herein.

"Cell fate" refers to the cell-type or cellular characteristics of a particular cell that are produced during plant development or a cellular process therefor, in particular during the cell cycle or as a consequence of a cell cycle process.

"Plant development" or the term "plant developmental characteristic" or similar term shall, when used herein, be taken to mean any cellular process of a plant that is involved in determining the developmental fate of a plant cell, in particular the specific tissue or organ type into which a progenitor cell will develop. Cellular processes relevant to plant development will be known to those skilled in the art. Such processes include, for example, morphogenesis, photomorphogenesis, shoot development, root development, vegetative development, reproductive development, stem elongation, flowering, and regulatory mechanisms involved in determining cell fate, in particular a process or regulatory process involving the cell cycle.

"Plant morphology" or the term "plant morphological characteristic" or similar term will, when used herein, be understood by those skilled in the art to refer to the external appearance of a plant, including any one or more structural features or combination of structural features thereof. Such structural features include the shape, size, number, position, color, texture, arrangement, and patternation of any cell, tissue or organ or groups of cells, tissues or organs of a plant, including the root, stem, leaf, shoot, petiole, trichome, flower, petal, stigma, style, stamen, pollen, ovule, seed, embryo, endosperm, seed coat, aleurone, fiber, fruit, cambium, wood, heartwood, parenchyma, aerenchyma, sieve element, phloem or vascular tissue, amongst others.

"Plant biochemistry" or the term "plant biochemical characteristic" or similar term will, when used herein, be understood by those skilled in the art to refer to the metabolic and catalytic processes of a plant, including primary and secondary metabolism and the products thereof, including any small molecules, macromolecules or chemical compounds, such as but not limited to starches, sugars, proteins, peptides, enzymes, hormones, growth factors, nucleic acid molecules, celluloses, hemicelluloses, calloses, lectins, fibers, pigments such as anthocyanins, vitamins, minerals, micronutrients, or macronutrients, that are produced by plants.

"Plant physiology" or the term "plant physiological characteristic" or similar term will, when used herein, be understood to refer to the functional processes of a plant, including developmental processes such as growth, expansion and differentiation, sexual development, sexual reproduction, seed set, seed development, grain filling, asexual reproduction, cell division, dormancy, germination, light adaptation, photosynthesis, leaf expansion, fiber production, secondary growth or wood production, amongst others; responses of a plant to externally-applied factors such as metals, chemicals, hormones, growth factors, environment and environmental stress factors (e.g. anoxia, hypoxia, high temperature, low temperature, dehydration, light, daylength, flooding, salt, heavy metals, amongst others), including adaptive responses of plants to said externally-applied factors.

Means for introducing recombinant DNA into plant tissue or cells include, but are not limited to, transformation using $CaCl_2$ and variations thereof, in particular the method described by Hanahan (1983), direct DNA uptake into protoplasts (Krens *et al,* 1982; Paszkowski *et al,* 1984), PEG-mediated uptake to protoplasts (Armstrong *et al,* 1990) microparticle bombardment, electroporation (Fromm *et al*., 1985), microinjection of DNA (Crossway *et al.,* 1986), microparticle bombardment of tissue explants or cells (Christou *et al*, 1988; Sanford, 1988), vacuum-infiltration of tissue with nucleic acid, or in the case of plants, T-DNA-mediated transfer from *Agrobacterium* to the plant tissue as described essentially by An *et al*. (1985), Dodds *et al*., (1985), Herrera-Estrella *et al.* (1983a, 1983b, 1985). Methods for transformation of monocotyledonous plants are well known in the art and include *Agrobacterium*-mediated transformation (Cheng et al., 1997 - WO9748814; Hansen 1998 - WO9854961; Hiei et al., 1994 - WO9400977; Hiei et al., 1998 - WO9817813; Rikiishi et al., 1999 - WO9904618; Saito et al., 1995 - WO9506722), microprojectile bombardment (Adams et al., 1999 - US5969213; Bowen et al., 1998 - US5736369; Chang et al., 1994 - WO9413822; Lundquist et al., 1999 - US5874265/US5990390; Vasil and Vasil, 1995 - US5405765. Walker et al., 1999 - US5955362), DNA uptake (Eyal et al., 1993 - WO9318168), microinjection of Agrobacterium cells (von Holt, 1994 - DE4309203) and sonication (Finer et al., 1997 - US5693512).

For microparticle bombardment of cells, a microparticle is propelled into a cell to produce a transformed cell. Any suitable ballistic cell transformation methodology and apparatus can be used in performing the present invention. Exemplary apparatus and procedures are disclosed by Stomp et al. (U.S. Patent No. 5,122,466) and Sanford and Wolf (U.S. Patent No. 4,945,050). When using ballistic transformation procedures, the gene construct may incorporate a plasmid capable of replicating in the cell to be transformed. Examples of microparticles suitable for use in such systems include 1 to 5 $\mu$m gold spheres. The DNA construct may be deposited on the microparticle by any suitable technique, such as by precipitation.

A whole plant may be regenerated from the transformed or transfected cell, in accordance with procedures well known in the art. Plant tissue capable of subsequent clonal propagation, whether by organogenesis or embryogenesis, may be transformed with a disclosed gene construct and a whole plant regenerated therefrom. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem).

The term "organogenesis", as used herein, means a process by which shoots and roots are developed sequentially from meristematic centers.

The term "embryogenesis", as used herein, means a process by which shoots and roots develop together in a concerted fashion (not sequentially), whether from somatic cells or gametes.

Preferably, the plant is produced according to the inventive method is transfected or transformed with a genetic sequence, or amenable to the introduction of a protein, by any art-recognized means, such as microprojectile bombardment, microinjection, *Agrobacterium*-mediated transformation (including *in planta* transformation), protoplast fusion, or electroporation, amongst others. Most preferably said plant is produced by *Agrobacterium*-mediated transformation.

*Agrobacterium*-mediated transformation or agrolistic transformation of plants, yeast, molds or filamentous fungi is based on the transfer of part of the transformation vector sequences, called the T-DNA, to the nucleus and on integration of said T-DNA in the genome of said eukaryote.

With "Agrobacterium" is meant a member of the Agrobacteriaceae, more preferably Agrobacterium or Rhizobacterium and most preferably Agrobacterium tumefaciens.

With "T-DNA", or transferred DNA, is meant that part of the transformation vector flanked by T-DNA borders which is, after activation of the *Agrobacterium vir* genes, nicked at the T-DNA borders and is transferred as a single stranded DNA to the nucleus of an eukaryotic cell.

When used herein, with "T-DNA borders", "T-DNA border region", or "border region" are meant either right T-DNA border (RB) or left T-DNA border (LB). Such a border comprises a core sequence flanked by a border inner region as part of the T-DNA flanking the border and/or a border outer region as part of the vector backbone flanking the border. The core sequences comprise 22 bp in case of octopine-type vectors and 25 bp in case of nopaline-type vectors. The core sequences in the right border region and left border region form imperfect repeats. Border core sequences are indispensable for recognition and processing by the *Agrobacterium* nicking complex consisting of at least VirD1 and VirD2. Core sequences flanking a T-DNA are sufficient to promote transfer of said T-DNA. However, efficiency of transformation using transformation vectors carrying said T-DNA solely flanked by said core sequences is low. Border inner and outer regions are known to modulate efficiency of T-DNA transfer (Wang et al. 1987). One element enhancing T-DNA transfer has been characterized and resides in the right border outer region and is called *overdrive* (Peralta et al. 1986, van Haaren et al. 1987).

With "T-DNA transformation vector" or "T-DNA vector" is meant any vector encompassing a T-DNA sequence flanked by a right and left T-DNA border consisting of at least the right and left border core sequences, respectively, and used for transformation of any eukaryotic cell.

With "T-DNA vector backbone sequence" or "T-DNA vector backbone sequences" is meant all DNA of a T-DNA containing vector that lies outside of the T-DNA borders and, more specifically, outside the nicking sites of the border core imperfect repeats.

The current specification includes optimized T-DNA vectors such that vector backbone integration in the genome of a eukaryotic cell is minimized or absent. With "optimized T-DNA vector" is meant a T-DNA vector designed either to decrease or abolish transfer of vector backbone sequences to the genome of a eukaryotic cell. Such T-DNA vectors are known to the one familiar with the art and include those described by Hanson et al. (1999) and by Stuiver et al. (1999 - W09901563).

The current invention clearly considers the inclusion of a DNA sequence encoding a cytokinin oxidase, homologue, derivative or immunologically active and/or functional fragment thereof as defined supra, in any T-DNA vector comprising binary transformation vectors, super-binary transformation vectors, co-integrate transformation vectors, Ri-derived transformation vectors as well as in T-DNA carrying vectors used in agrolistic transformation. Preferably, said cytokinin oxidase is a plant cytokinin oxidase, more specifically an *Arabidopsis thaliana* (At)CKX.

With "binary transformation vector" is meant a T-DNA transformation vector comprising:

(a) a T-DNA region comprising at least one gene of interest and/or at least one selectable marker active in the eukaryotic cell to be transformed; and

(b) a vector backbone region comprising at least origins of replication active in *E. coli* and *Agrobacterium* and markers for selection in *E. coli* and *Agrobacterium.*

The T-DNA borders of a binary transformation vector can be derived from octopine-type or nopaline-type Ti plasmids or from both. The T-DNA of a binary vector is only transferred to a eukaryotic cell in conjunction with a helper plasmid.

With "helper plasmid" is meant a plasmid that is stably maintained in *Agrobacterium* and is at least carrying the set of *vir* genes necessary for enabling transfer of the T-DNA. Said set of *vir* genes can be derived from either octopine-type or nopaline-type Ti plasmids or from both.

With "super-binary transformation vector" is meant a binary transformation vector additionally carrying in the vector backbone region a *vir* region of the Ti plasmid pTiBo542 of the super-virulent *A. tumefaciens* strain A281 (EP0604662, EP0687730). Super-binary transformation vectors are used in conjunction with a helper plasmid.

With "co-integrate transformation vector" is meant a T-DNA vector at least comprising:

(a) a T-DNA region comprising at least one gene of interest and/or at least one selectable marker active in plants; and

(b) a vector backbone region comprising at least origins of replication active in *Escherichia coli* and *Agrobacterium,* and markers for selection in *E. coli* and *Agrobacterium,* and a set of *vir* genes necessary for enabling transfer of the T-DNA.

The T-DNA borders and said set of *vir* genes of a said T-DNA vector can be derived from either octopine-type or nopaline-type Ti plasmids or from both.

With "Ri-derived plant transformation vector" is meant a binary transformation vector in which the T-DNA borders are derived from a Ti plasmid and said binary transformation vector being used in conjunction with a 'helper' Ri-plasmid carrying the necessary set of *vir* genes.

As used herein, the term "selectable marker gene" or "selectable marker" or "marker for selection" includes any gene which confers a phenotype on a cell in which it is expressed to facilitate the identification and/or selection of cells which are transfected or transformed with a disclosed gene construct or a derivative thereof. Suitable selectable marker genes contemplated herein include the ampicillin resistance (Amp’), tetracycline resistance gene (Tc’), bacterial kanamycin resistance gene (Kan’), phosphinothricin resistance gene, neomycin phosphotransferase gene (*npt*II), hygromycin resistance gene, β-glucuronidase (GUS) gene, chloramphenicol acetyltransferase (CAT) gene, green fluorescent protein (*gfp*) gene (Haseloff *et al,* 1997), and luciferase gene, amongst others.

With "agrolistics", "agrolistic transformation" or "agrolistic transfer" is meant here a transformation method combining features of *Agrobacterium*-mediated transformation and of biolistic DNA delivery. As such, a T-DNA containing target plasmid is co-delivered with DNA/RNA enabling in planta production of VirD1 and VirD2 with or without VirE2 (Hansen and Chilton 1996; Hansen et al. 1997; Hansen and Chilton 1997 - W09712046).

With "foreign DNA" is meant any DNA sequence that is introduced in the host’s genome by recombinant techniques. Said foreign DNA includes e.g. a T-DNA sequence or a part thereof such as the T-DNA sequence comprising the selectable marker in an expressible format. Foreign DNA furthermore include intervening DNA sequences as defined supra.

With "recombination event" is meant either a site-specific recombination event or a recombination event effected by transposon 'jumping'.

With "recombinase" is meant either a site-specific recombinase or a transposase.

With "recombination site" is meant either site-specific recombination sites or transposon border sequences.

With "site specific recombination event" is meant an event catalyzed by a system generally consisting of three elements: a pair of DNA sequences (the site-specific recombination sequences or sites) and a specific enzyme (the site-specific recombinase). The site-specific recombinase catalyzes a recombination reaction only between two site-specific recombination sequences depending on the orientation of the site-specific recombination sequences. Sequences intervening between two site-specific recombination sites will be inverted in the presence of the site-specific recombinase when the site-specific recombination sequences are oriented in opposite directions relative to one another (i.e. inverted repeats). If the site-specific recombination sequences are oriented in the same direction relative to one another (i.e. direct repeats), then any intervening sequences will be deleted upon interaction with the site-specific recombinase. Thus, if the site-specific recombination sequences are present as direct repeats at both ends of a foreign DNA sequence integrated into a eukaryotic genome, such integration of said sequences can subsequently be reversed by interaction of the site-specific recombination sequences with the corresponding site specific recombinase.

A number of different site specific recombinase systems can be used including but not limited to the Cre/lox system of bacteriophage P1, the FLP/FRT system of yeast, the Gin recombinase of phage Mu, the Pin recombinase of *E. coli,* the PinB, PinD and PinF from *Shigella,* and the R/RS system of the pSR1 plasmid. Recombinases generally are integrases, resolvases or flippases. Also dual-specific recombinases can be used in conjunction with direct or indirect repeats of two different site-specific recombination sites corresponding to the dual-specific recombinase (WO99/25840). The two preferred site-specific recombinase systems are the bacteriophage P1 Cre/lox and the yeast FLP/FRT systems. In these systems a recombinase (Cre or FLP) interact specifically with its respective site-specific recombination sequence (lox or FRT respectively) to invert or excise the intervening sequences. The site-specific recombination sequences for each of these two systems are relatively short (34 bp for lox and 47 bp for FRT). Some of these systems have already been used with high efficiency in plants such as tobacco (Dale et al. 1990) and *Arabidopsis* (Osborne et al. 1995). Site-specific recombination systems have many applications in plant molecular biology including methods for control of homologous recombination (e.g. US5527695), for targeted insertion, gene stacking, etc. (WO99/25821) and for resolution of complex T-DNA integration patterns or for excision of a selectable marker (WO99/23202).

Although the site-specific recombination sequences must be linked to the ends of the DNA to be excised or to be inverted, the gene encoding the site specific recombinase may be located elsewhere. For example, the recombinase gene could already be present in the eukaryote's DNA or could be supplied by a later introduced DNA fragment either introduced directly into cells, through crossing or through cross-pollination. Alternatively, a substantially purified recombinase protein could be introduced directly into the eukaryotic cell, e.g. by micro-injection or particle bombardment. Typically, the site-specific recombinase coding region will be operably linked to regulatory sequences enabling expression of the site-specific recombinase in the eukaryotic cell.

With "recombination event effected by transposon jumping" or "transposase-mediated recombination" is meant a recombination event catalyzed by a system consisting of three elements: a pair of DNA sequences (the transposon border sequences) and a specific enzyme (the transposase). The transposase catalyzes a recombination reaction only between two transposon border sequences which are arranged as inverted repeats.

A number of different transposon/transposase systems can be used including but not limited to the Ds/Ac system, the Spm system and the Mu system. These systems originate from corn but it has been shown that at least the Ds/Ac and the Spm system also function in other plants (Fedoroff et al. 1993, Schlappi et al. 1993, Van Sluys et al. 1987). Preferred are the Ds- and the Spm-type transposons which are delineated by 11 bp- and 13 bp- border sequences, respectively.

Although the transposon border sequences must be linked to the ends of the DNA to be excised, the gene encoding the transposase may be located elsewhere. For example, the recombinase gene could already be present in the eukaryote's DNA or could be supplied by a later introduced DNA fragment either introduced directly into cells, through crossing or through cross-pollination. Alternatively, a substantially purified transposase protein could be introduced directly into cells, e.g. by microinjection or by particle bombardment.

As part of the current specification, transposon border sequences are included in a foreign DNA sequence such that they lie outside said DNA sequence and transform said DNA into a transposon-like entity that can move by the action of a transposase.

As transposons often reintegrate at another locus of the host's genome, segregation of the progeny of the hosts in which the transposase was allowed to act might be necessary to separate transformed hosts containing e.g. only the transposon footprint and transformed hosts still containing the foreign DNA.

In performing the present invention, the genetic element is preferably induced to mobilize, such as, for example, by the expression of a recombinase protein in the cell which contacts the integration site of the genetic element and facilitates a recombination event therein, excising the genetic element completely, or alternatively, leaving a "footprint", generally of about 20 nucleotides in length or greater, at the original integration site. Those hosts and host parts that have been produced according to the inventive method can be identified by standard nucleic acid hybridization and/or amplification techniques to detect the presence of the mobilizable genetic element or a gene construct comprising the same. Alternatively, in the case of transformed host cells, tissues, and hosts wherein the mobilizable genetic element has been excised, it is possible to detect a footprint in the genome of the host which has been left following the excision event, using such techniques. As used herein, the term "footprint" shall be taken to refer to any derivative of a mobilizable genetic element or gene construct comprising the same as described herein which is produced by excision, deletion or other removal of the mobilizable genetic element from the genome of a cell transformed previously with said gene construct. A footprint generally comprises at least a single copy of the recombination loci or transposon used to promote excision. However, a footprint may comprise additional sequences derived from the gene construct, for example nucleotide sequences derived from the left border sequence, right border sequence, origin of replication, recombinase-encoding or transposase-encoding sequence if used, or other vector-derived nucleotide sequences. Accordingly, a footprint is identifiable according to the nucleotide sequence of the recombination locus or transposon of the gene construct used, such as, for example, a sequence of nucleotides corresponding or complementary to a *lox* site or *frt* site.

The term "cell cycle" means the cyclic biochemical and structural events associated with growth and with division of cells, and in particular with the regulation of the replication of DNA and mitosis. Cell cycle includes phases called: G0, Gap1 (G1), DNA synthesis (S), Gap2 (G2), and mitosis (M). Normally these four phases occur sequentially, however, the cell cycle also includes modified cycles wherein one or more phases are absent resulting in modified cell cycle such as endomitosis, acytokinesis, polyploidy, polyteny, and endoreduplication.

The term "cell cycle progression" refers to the process of passing through the different cell cycle phases. The term "cell cycle progression rate" accordingly refers to the speed at which said cell cycle phases are run through or the time spans required to complete said cell cycle phases.

With "two-hybrid assay" is meant an assay that is based on the observation that many eukaryotic transcription factors comprise two domains, a DNA-binding domain (DB) and an activation domain (AD) which, when physically separated (i.e. disruption of the covalent linkage) do not effectuate target gene expression. Two proteins able to interact physically with one of said proteins fused to DB and the other of said proteins fused to AD will re-unite the DB and AD domains of the transcription factor resulting in target gene expression. The target gene in the yeast two-hybrid assay is usually a reporter gene such as the β-galactosidase gene. Interaction between protein partners in the yeast two-hybrid assay can thus be quantified by measuring the activity of the reporter gene product (Bartel and Fields 1997). Alternatively, a mammalian two-hybrid system can be used which includes e.g. a chimeric green fluorescent protein encoding reporter gene (Shioda *et al.,* 2000).

Furthermore folding simulations and computer redesign of structural motifs of the disclosed protein can be performed using appropriate computer programs (Olszewski, Proteins 25 (1996), 286-299; Hoffman, Comput. Appl. Biosci. 1 (1995), 675-679). Computer modeling of protein folding can be used for the conformational and energetic analysis of detailed peptide and protein models (Monge, J. Mol. Biol. 247 (1995), 995-1012; Renouf, Adv. Exp. Med. Biol. 376 (1995), 37-45). In particular, the appropriate programs can be used for the identification of interactive sites of the cytokinin oxidases, its ligands or other interacting proteins by computer assistant searches for complementary peptide sequences (Fassina, Immunomethods 5 (1994), 114-120). Further appropriate computer systems for the design of protein and peptides are described in the prior art, for example in Berry, Biochem. Soc. Trans. 22 (1994), 1033-1036; Wodak, Ann, N. Y. Acac. Sci. 501 (1987), 1-13; Pabo, Biochemistry 25 (1986), 5987-5991. The results obtained form the above-described computer analysis can be used for, e.g. the preparation of peptidomimetics of the disclosed protein or fragments thereof. Such pseudopeptide analogues of the natural amino acid sequence of the protein may very efficiently mimic the parent protein (Benkirane, J. Biol. Chem. 271 (1996), 33218-33224). For example, incorporation of easily available achiral Ω-amino acid residues into a disclosed protein or a fragment thereof results in the substitution of amino bonds by polymethylene units of an aliphatic chain, thereby providing a convenient strategy for constructing a peptidomimetic (Banerjee, Biopolymers 39 (1996), 769-777). Superactive peptidomimetic analogues of small peptide hormones in other systems are described in the prior art (Zhang, Biochem. Biophys. Res. Commun. 224 (1996), 327-331). Appropriate peptidomimetics of the disclosed protein invention can also be identified by the synthesis of peptidomimetic combinatorial libraries through successive amine alkylation and testing the resulting compounds, e.g., for their binding, kinase inhibitory and/or immunological properties. Methods for the generation and use of peptidomimetic combinatorial libraries are described in the prior art, for example in Ostresh, Methods in Enzymology 267 (1996), 220-234 and Dorner, Bioorg. Med. Chem. 4 (1996), 709-715.

Furthermore, a three-dimensional and/or crystallographic structure of the disclosed protein the invention can be used for the design of peptidomimetic inhibitors of the biological activity of the disclosed protein (Rose, Biochemistry 35 (1996), 12933-12944; Ruterber, Bioorg. Med. Chem. 4 (1996), 1545-1558).

The compounds to be obtained or identified in the methods of the specification can be compounds that are able to bind to any of the disclosed nucleic acids, peptides or proteins . Other interesting compounds to be identified are compounds that modulate the expression of the genes or the proteins of the specification in such a way that either the expression of said gene or protein is enhanced or decreased by the action of said compound. Alternatively the compound can exert his action by enhancing or decreasing the activity of any of the proteins of the specification. Herein, preferred proteins are novel cytokinin oxidases.

Said compound or plurality of compounds may be comprised in, for example, samples, e.g., cell extracts from, e.g., plants, animals or microorganisms. Furthermore, said compound(s) may be known in the art but hitherto not known to be capable of suppressing or activating cytokinin oxidase interacting proteins. The reaction mixture may be a cell free extract of may comprise a cell or tissue culture. Suitable set ups for the disclosed method are known to the person skilled in the art and are, for example, generally described in Alberts et al., Molecular Biology of the Cell, third edition (1994), in particular Chapter 17. The plurality of compounds may be, e.g., added to the reaction mixture, culture medium or injected into the cell.

If a sample containing a compound or a plurality of compounds is identified in the method of the the specification, then it is either possible to isolate the compound form the original sample identified as containing the compound capable of acting as an agonist, or one can further subdivide the original sample, for example, if it consists of a plurality of different compounds, so as to reduce the number of different substances per sample and repeat the method with the subdivisions of the original sample. Depending on the complexity of the samples, the steps described above can be performed several times, preferably until the sample identified according to the disclosed method only comprises a limited number of or only one substance(s). Preferably said sample comprises substances or similar chemical and/or physical properties, and most preferably said substances are identical. Preferably, the compound identified according to the above-described method or its derivative is further formulated in a form suitable for the application in plant breeding or plant cell and tissue culture.

The term "early vigor" refers to the ability of a plant to grow rapidly during early development, and relates to the successful establishment, after germination, of a well-developed root system and a well-developed photosynthetic apparatus.

The term "resistance to lodging" or "standability" refers to the ability of a plant to fix itself to the soil. For plants with an erect or semi-erect growth habit this term also refers to the ability to maintain an upright position under adverse (environmental) conditions. This trait relates to the size, depth and morphology of the root system.

The term 'grafting' as used herein, refers to the joining together of the parts of two different plants so that they bind together and the sap can flow, thus forming a single new plant that can grow and develop. A graft therefore consists of two parts: (i) the lower part is the rootstock as referred to herein and essentially consists of the root system and a portion of the stem, and (ii) the upper part, the scion or graft, which gives rise to the aerial parts of the plant.

As used herein, tblastn refers to an alignment tool that is part of the BLAST (Basic Local Alignment Search Tool) family of programs (http://www.ncbi.nlm.nih.gov/BLAST/). BLAST aims to identify regions of optimal local alignment, i.e. the alignment of some portion of two nucleic acid or protein sequences, to detect relationships among sequences which share only isolated regions of similarity (Altschul et al., 1990). In the present invention, tblastn of the BLAST 2.0 suite of programs was used to compare the maize cytokinin oxidase protein sequence against a nucleotide sequence database dynamically translated in all reading frames (Altschul et al., Nucleic Acids Res. 25: 3389-3402 (1997)).

The following examples are given by means of illustration of the present invention and are in no way limiting.

## EXAMPLES

### Example 1. Brief description of the sequences of the specification

| SEQ ID NO: | DESCRIPTION |
|---|---|
| 1 | *AtCKX1* genomic |
| 2 | *AtCKX1* protein |
| 3 | *AtCKX2* genomic |
| 4 | *AtCKX2* protein |
| 5 | *AtCKX3* genomic |
| 6 | *AtCKX3* protein |
| 7 | *AtCKX4* genomic |

(continued)

| SEQ ID NO: | DESCRIPTION |
|---|---|
| 8 | *AtCKX4* protein |
| 9 | *AfCKX5* genomic (short version) |
| 10 | *AtCKX5* protein (short version) |
| 11 | *AtCKX6* genomic |
| 12 | *AtCKX6* protein |
| 13 | 5'primer *AtCKX1* |
| 14 | 3'primer *AtCKX1* |
| 15 | 5'primer *AtCKX2* |
| 16 | 3'primer *AtCKX2* |
| 17 | 5'primer *AtCKX3* |
| 18 | 3'primer *AtCKX3* |
| 19 | 5'primer *AtCKX4* |
| 20 | 3'primer *AtCKX4* |
| 21 | 5'primer *AtCKX5* |
| 22 | 3'primer *AtCKX5* |
| 23 | 5'primer *AtCKX6* |
| 24 | 3'primer *AtCKX6* |
| 25 | *AtCKX1* cDNA |
| 26 | *AtCKX2* cDNA |
| 27 | *AtCKX3* cDNA |
| 28 | *AtCKX4* cDNA |
| 29 | *AtCKX5* cDNA (short version) |
| 30 | *AtCKX6* cDNA |
| 31 | *AtCKX2* cDNA fragment |
| 32 | *AtCKX2* peptide fragment |
| 33 | *AtCKX5* genomic (long version) |
| 34 | *AtCKX5* cDNA (long version) |
| 35 | *AtCKX5* protein (long version) |
| 36 | root clavata homolog promoter |

**Example 2. Identification of candidate cytokinin oxidase encoding genes from *Arabidopsis thaliana***

Six different genes were identified from *Arabidopsis thaliana* that bear sequence similarity to a cytokinin oxidase gene from maize (Morris et al., Biochem Biophys Res Comm 255:328-333, 1999; Houda-Herin et al. Plant J 17:615-626; WO 99/06571). These genes were found by screening 6-frame translations of nucleotide sequences from public genomic databases with the maize protein sequence, employing tblastn program. These sequences were designated as *Arabidopsis thaliana* cytokinin oxidase-like genes or *AtCKX.* They were arbitrarily numbered as *AtCKX1 to AtCKX6.* The below list summarizes the information on these genes. The predicted ORF borders and protein sequences are indicative, in order to illustrate by approximation the protein sequence divergence between the Arabidopsis and maize cytokinin oxidases, as well as amongst the different Arabidopsis cytokinin oxidases. The ORF borders and protein sequences shown should not be taken as conclusive evidence for the mode of action of these *AtCKX* genes. For DNA and protein sequence comparisons the program MegAlign from DNAstar was used. This program uses the Clustal method for alignments. For multiple alignments of protein and cDNA sequences the gap penalty and gap length penalty was set at 10 each. For pairwise alignments of proteins the parameters were as follows: Ktuple at 1; Gap penalty at 3; window at 5; diagonals saved at 5. For pairwise alignments of cDNA's the parameters were as follows: Ktuple at 2; Gap penalty at 5; window at 4; diagonals saved at 4. The similarity groups for protein alignments was: (M,I,L,V), (F,W,Y), (G,A), (S, T), (R,K,H), (E,D), (N,Q). The values that are indicated amongst the Arabidopsis cDNA and protein sequences represent the lowest and highest values found with all combinations.

**A. Gene name:***AtCKX1* *(Arabidopsis thaliana* cytokinin oxidase-like protein 1 SEQ ID NO: 1)

**Location in database** (accession number, location on bac): AC002510, *Arabidopsis thaliana* chromosome II section 225 of 255 of the complete sequence. Sequence from clones T32G6.

**ORF predicted in the database:**

15517..16183, 16415..16542, 16631..16891, 16995..17257, 17344..17752

The *AtCKX1* cDNA sequence is listed as SEQ ID NO: 25

**Predicted protein sequence:** SEQ ID NO: 2:

**Homologies**

*% identity with Z. mays cDNA:*

31.5% (Dnastar/MegAlign - Clustal method)

*% similarity with Z. mays protein:*

32.2% (Dnastar/MegAlign - Clustal method)

*% identity with other Arabidopsis cDNA's (range):*

38.2 % (*AtCKX2*) - 54.1 % (*AtCKX6*) (Dnastar/MegAlign - Clustal method)

*% similarity with other Arabidopsis proteins (range):*

37.1 % (*AtCKX2*) - 58.1 % (*AtCKX6*) (Dnastar/MegAlign - Clustal method)

**B. Gene name:** *AtCKX2* (*Arabidopsis thaliana* cytokinin oxidase-like protein 2, SEQ ID NO: 3)

**Location in database** (accession number, location on bac): AC005917, *Arabidopsis thaliana* chromosome II section 113 of 255 of the complete sequence. Sequence from clones F27F23, F3P11.

**ORF predicted in the database:**

complement, 40721..41012, 41054..41364, 41513..41770, 42535..42662, 43153..43711

**Please note:** The cDNA sequence identified by the inventor using the gene prediction program NetPlantGene (hftp://www.cbs.dtu.dk/services/NetGene2/) was different than the one annotated in the database. Based on the new cDNA sequence the ORF predicted in the database was revised:

complement, 40721..41012, 41095..41364, 41513..41770, 42535..42662, 43153..43711

The protein sequence encoded by this cDNA is listed as SEQ ID NO: 4. The cDNA of *AtCKX2* was cloned by RT-PCR from total RNA of *AtCKX2* transgenic plant tissue with the one-step RT-PCR kit (Qiagen, Hilden, Germany) and sequenced using an ABI PRISM Big Dye Terminator cycle sequencing reaction kit (Perkin Elmer Applied Biosystems Division). This confirmed that the cDNA sequence identified and predicted by the inventor was correct. The new *AtCKX2* cDNA sequence is listed as SEQ ID NO: 26. An 84-bp fragment corresponding to nucleotides 1171 through 1254 of the *AtCKX2* cDNA is listed as SEQ ID NO: 31. The corresponding peptide sequence of this 84-bp cDNA sequence is listed as SEQ ID NO: 32.

**Homologies**

*% identity with Z. mays cDNA:*

38.4% (Dnastar/MegAlign - Clustal method)

*% similarity with Z. mays protein:*

37.5% (Dnastar/MegAlign - Clustal method)

*% identity with other Arabidopsis cDNA's (range):*

34.9% *(AtCKX6)* - 64.5% (*AtCKX4*) (Dnastar/MegAlign - Clustal method)

*% similarity with other Arabidopsis proteins (range):*

36.5% (*AtCKX6*) - 66.1 % (*AtCKX4*) (Dnastar/MegAlign - Clustal method)

**C. Gene name:** *AtCKX3* (*Arabidopsis thaliana* cytokinin oxidase-like protein 3, SEQ ID NO: 5)

**Location in database** (accession number, location on bac): AB024035, *Arabidopsis thaliana* genomic DNA, chromosome 5, P1 clone: MHM17, complete sequence.

**No prediction of the ORF in the database.**

The gene was identified by the inventor using several gene prediction programs including GRAIL (ftp: //arthur.epm.ornl.gov/pub/xgrail), Genscan (http://CCR-081.mit.edu/ GENSCAN html) and NetPlantGene (http: //www.cbs.dtu.dk/services/NetGene2/):

complement, 29415..29718, 29813..30081, 30183..30443, 30529..30656, 32107..32716

The new *AtCKX3* cDNA sequence identified by the inventor is listed as SEQ ID NO: 27

**Predicted protein sequence, based on own ORF prediction:** SEQ ID NO: 6

**Homologies**

*% identity with Z. mays cDNA:*

38.7% (Dnastar/MegAlign - Clustal method)

*% similarity with Z. mays protein:*

39.2% (Dnastar/MegAlign - Clustal method)

*% identity with other Arabidopsis cDNA's (range):*

38.8% *(AtCKX6)* - 51.0% (*AtCKX2*) (Dnastar/MegAlign - Clustal method)

*% similarity with other Arabidopsis proteins (range):*

39.9% *(AtCKX6)* - 46.7% *(AtCKX2)* (Dnastar/MegAlign - Clustal method)

**D. Gene name**: *AtCKX4* (*Arabidopsis thaliana* cytokinin oxidase-like protein 4, SEQ ID NO: 7)

**Location in database** (accession number, location on bac):

1) AL079344, *Arabidopsis thaliana* DNA chromosome 4, BAC clone T16L4 (ESSA project)

2) AL161575, *Arabidopsis thaliana* DNA chromosome 4, contig fragment No. 71.

**ORF predicted in the database:**

1) 76187..76814, 77189..77316, 77823..78080, 78318..78586, 78677..78968

2) 101002..101629, 102004..102131, 102638..102895, 103133..103401, 103492..103783

The *AtCKX4* cDNA sequence is listed as SEQ ID NO: 28
**Predicted protein sequence:** SEQ ID NO: 8

**Homologies**

*% identity with Z. mays cDNA:*

41.0% (Dnastar/MegAlign - Clustal method)

*% similarity with Z. mays protein:*

41.0% (Dnastar/MegAlign - Clustal method)

*% identity with other Arabidopsis cDNA's (range):*

35.2% *(AtCKX6)* - 64.5% *(AtCKX2)* (Dnastar/MegAlign - Clustal method)

*% similarity with other Arabidopsis proteins (range):*

35.1 % (*AtCKX6*) - 66.1 % *(AtCKX2)* (Dnastar/MegAlign - Clustal method)

**E. Gene name:** *AtCKX5 (Arabidopsis thaliana* cytokinin oxidase-like protein 5, SEQ ID NO: 9)

**Location in database** (accession number, location on bac): AC023754, F1B16, complete sequence, chromosome 1

**No prediction of the ORF in the database.**

The gene was identified by the inventors using several gene prediction programs including GRAIL (ftp://arthur.epm.ornl.gov/pub/xgrail), Genscan (http://CCR-081.mit.edu/ GEN SCAN.html) and NetPlantGene (http://www.cbs.dtu.dk/services/NetGene2/).

43756..44347, 44435..44562, 44700..44966, 45493..45755, 46200..46560

The new *AtCKX5* cDNA sequence identified and predicted by the inventor is listed as SEQ ID NO: 29. The predicted protein sequence for this cDNA is listed as SEQ ID NO: 10. A second potential ATG start codon is present 9 nucleotides more upstream in the genomic sequence. It is unclear which of these 2 start codons encodes the first amino acid of the protein. Therefore, a second potential *AtCKX5* cDNA starting at this upstream start codon is also listed in this invention as SEQ ID NO: 34. The corresponding genomic sequence is listed as SEQ ID NO: 33 and the encoded protein as SEQ ID NO: 35.

**Homologies**

*% identity with Z. mays cDNA:*

39.1% (Dnastar/MegAlign - Clustal method)

*% similarity with Z. mays protein:*

36.6% (Dnastar/MegAlign - Clustal method)

*% identity with other Arabidopsis cDNA's (range):*

40.1 % (*AtCKX2*) - 44.0% (*AtCKX3*) (Dnastar/MegAlign - Clustal method)

*% similarity with other Arabidopsis proteins (range):*

41.6% (*AtCKX4*) - 46.4% (*AtCKX6*) (Dnastar/MegAlign - Clustal method)

**F. Gene name:** *AtCKX6 (Arabidopsis thaliana* cytokinin oxidase-like protein 6, SEQ ID NO: 11)

**Location in database** (accession number, location on bac): AL163818, *Arabidopsis thaliana* DNA chromosome 3, P1 clone MAA21 (ESSA project).

**ORF predicted in the database:**

46630..47215, 47343..47470, 47591..47806, 47899..48161, 48244..48565
The *AtCKX6* cDNA sequence is listed as SEQ ID NO: 30

**Predicted protein sequence:** SEQ ID NO: 12

**Homologies**

*% identity with Z. mays cDNA:*

37.3% (Dnastar/MegAlign - Clustal method)

*% similarity with Z. mays protein:*

36.1% (Dnastar/MegAlign - Clustal method)

*% identity with other Arabidopsis cDNA's (range):*

34.9% (*AtCKX2*) - 54.1 % (*AtCKX1*) (Dnastar/MegAlign - Clustal method)

*% similarity with other Arabidopsis proteins (range):*

35. 1 % (*AtCKX4*) - 58.1 % (*AtCKX1*) (Dnastar/MegAlign - Clustal method)

Genes *AtCKX3* and *AtCKX5* were not annotated as putative cytokinin oxidases in the database and ORFs for these genes were not given. Furthermore, the ORF (and consequently the protein structures) predicted for *AtCKX2* was different from our own prediction and our prediction was confirmed by sequencing the *AtCKX2* cDNA.

A comparison of the gene structure of the *Arabidopsis AtCKX* genes 1 to 4 and the maize *CKX* gene is shown in Fig 1.

The predicted proteins encoded by the *Arabidopsis AtCKX* genes show between 32% and 41% sequence similarity with the maize protein, while they show between 35% and 66% sequence similarity to each other. Because of this reduced sequence conservation, it is not clear *a priori* whether the *Arabidopsis AtCKX* genes encode proteins with cytokinin oxidase activity. An alignment of the *Arabidopsis AtCKX* predicted proteins 1 to 4 and the maize CKX gene is shown in Fig 2.

**Example 3. Transgenic plants overexpressing *AtCKX1* showed increased cytokinin oxidase activity and altered plant** morphology

**1. Description of the cloning process**

The following primers were used to PCR amplify the *AtCKX1* gene from *Arabidopsis thaliana,* accession Columbia (non-homologous sequences used for cloning are in lower case):

Sequence of 5' primer: cggtcgacATGGGATTGACCTCATCCTTACG (SEQ ID NO:13)

Sequence of 3' primer: gcgtcgacTTATACAGTTCTAGGTTTCGGCAGTAT (SEQ ID NO: 14)

A 2235-bp PCR fragment, amplified by these primers, was inserted in the Sal I site of pUC19. The insert was sequenced and confirmed that the PCR amplification product did not contain any mutations. The SalI/SalI fragment of this vector was subcloned in the SalI site downstream of a modified CaMV 35S promoter (carrying three tetracycline operator sequences) in the binary vector pBinHyg-Tx (Gatz *et al*., 1992). The resulting construct was introduced into tobacco and *Arabidopsis thaliana* through *Agrobacterium*-mediated transformation, using standard transformation protocols.

**2. Molecular analysis of the transgenic lines**

Several transgenic lines were identified that synthesize the *AtCKX1* transcript at high levels (Fig 3). Transgenic lines expressing *AtCKX1* transcript also showed increased cytokinin oxidase activity as determined by a standard assay for cytokinin oxidase activity based on conversion of [2-$^3$H]iP to adenine as described (Motyka et al., 1996). This is exemplified for 2 tobacco and 2 *Arabidopsis* lines in Table 6. This result proves that the *AtCKX1* gene encodes a protein with cytokinin oxidase activity.

**Table 6.** Cytokinin oxidase activity in *AtCKX1* transgenic plant tissues

| **Leaf sample** | | |
|---|---|---|
| **Plant species** | **Plant line** | **Cytokinin oxidase activity (nmol Ade/mg protein.h)** |
| *Arabidopsis* | Col-0 wild-type | 0.009 |
| | CKX1-11 | 0.024 |
| | CKX1-22 | 0.026 |
| | CKX1-22 | 0.027 |
| Tobacco | SNN wild-type | 0.004 |
| | CKX1-SNN-8 | 0.016 |
| | CKX1-SNN-28 | 0.021 |

**3. Phenotypic description of the transgenic lines**

3.1 In tobacco:

The plants had a dwarfed phenotype with reduced apical dominance (Figure 7 A, B and C) and increased root production (Figure 8).

Five categories of phenotype:

1) strong - 2 clones

2) intermediate - 3 clones

3) weak - 4 clones

4) tall plants (as WT) with large inflorescence - 5 clones

5) similar to WT, 9 clones

Height (see Fig. 7 B and C)

- WT: between 100-150 cm
- weak: approximately 75 cm
- intermediate: appr. 40-45 cm (main stem app. 25 cm but overgrown by side branches.
- strong: appr. 10 cm

The transgenics *AtCKX1-48* and *AtCKX1-50* displayed a strong phenotype. Below are measurements for stem elongation as compared to WT plants:

| Line | Wild-type | AtCKX1-48 | AtCKX1-50 |
|---|---|---|---|
| Days after germination | Height (cm) | Height (cm) | Height (cm) |
| 47 | 9.5 ± 0.5 | 1.3 ± 0.3 | 1.2 ± 0.2 |
| 58 | 22.4 ± 2.3 | 2.2 ± 0.3 | 2.3 ± 0.3 |
| 68 | 35.3 ± 2.6 | 3.1 ± 0.5 | 2.6 ± 0.5 |
| 100 | 113.3 ± 9.8 | 7.1 ± 0.8 | 4.8 ± 0.9 |
| 117 | 138.6 ± 8.1 | 9.7 ± 0.7 | 3.6 ± 0.9 |
| 131 | 39.0 ± 9.3 | 9.3 ± 0.7 | 3.6 ± 1.0 |
| 152 | 136.6 ± 10.4 | 10.9 ± 1.1 | 10.0 ± 1.0 |
| 165 | | 11.8 ± 1.9 | 11.4 ± 1.4 |
| 181 | | 16.5 ± 1.7 | 14.9 ± 1.2 |
| 198 | | 19.5 ± 1.5 | 18.1 ± 1.3 |

*Experimental*: Plants were grown in soil in a greenhouse. Data were collected from at least ten plants per line.

Leaves (see Figure 7 D and E)

The shape of leaves of *AtCKX1* transgenic expressors was lanceolate (longer and narrow): the width-to-length ratio of mature leaves was reduced from 1:2 in wild type plants to 1:3 in *AtCKX1* transgenics (Figure 7 E). The number of leaves and leaf surface was reduced compared to WT (see Figure 7 D). A prominent difference was also noted for progression of leaf senescence. In WT tobacco, leaf senescence starts in the most basal leaves and leads to a uniform reduction of leaf pigment (Figure 7 E). By contrast, ageing leaves of strongly expressing *AtCKX1* plants stayed green along the leaf veins and turned yellow in the intercostal regions, indicating altered leaf senescence. The texture of older leaves was more rigid.

Roots

In vitro grown plants highly expressing the gene were easily distinguishable from the WT by their ability to form more roots which are thicker (stronger) (Figure 8 A), as well as by forming aerial roots along the stem.

The primary root was longer and the number of lateral and adventitious roots was higher as illustrated in Figure 8C for *AtCKX1-50* overexpressing seedlings (see also Example 9).

The dose-response curve of root growth inhibition by exogenous cytokinin showed that roots of transgenic seedlings are more cytokinin resistant than WT roots (Figure 8 D). The resistance of *AtCKX1* transgenics to iPR was less marked than for *AtCKX2,* which is consistent with the smaller changes in iP-type cytokinins in the latter (see Table 10).

A large increase in root biomass was observed for adult plants grown in soil (see Figure 8B for a plant grown in soil for 4 to 5 months) despite the fact that growth of the aerial plant parts was highly reduced.

Internode distance

- intermediate phenotype: the 5$^{th}$ internode below inflorescence is about 2.5 cm long and 9$^{th}$ internode was about 0,5 cm long compared to 5 cm and 2 cm for the length of the 5$^{th}$ and 9$^{th}$ internode respectively, in WT plants.

- strong phenotype: plant *AtCKX1-50* The length of the 20$^{th}$ internode from the bottom measured at day 131 after germination was 1.3 $\pm$ 0.4 mm compared to 39.2 $\pm$ 3.8 mm for WT

Apical dominance and branching

More side branches were formed indicating reduced apical dominance compared to WT plants during vegetative growth (see Figure 9). The side branches overgrew the main stem, reaching a height of 40-45 cm for intermediate *AtCKX1* expressors. Even secondary branches appeared. However, the buds were not completely released from apical dominance, i.e. lateral shoots did not really continue to develop. The reduced apical dominance might be due to reduced auxin production by the smaller shoot apical meristem (see Example 10).

Reproductive development

The onset of flowering in *AtCKX1* transgenics was delayed, the number of flowers and the seed yield per capsule was reduced. The size of flowers was not altered in transgenic plants and the weight of the individual seeds was comparable to the weight of seeds from wild type plants. Data for two representative *AtCKX1* transgenics is summarized below:

A. Onset of flowering

| Line | Wild-type | AtCKX1-48 | AtCKX1-50 |
|---|---|---|---|
| Flowering time (DAG) | 106.2 $\pm$ 3.3 | 193.3 $\pm$ 4.3 | 191.8 $\pm$ 3.8 |

*Experimental:* Data collected for at least ten plants per line. The full elongation of the first flower was defined as onset of flowering. DAG = days after germination.

B. Number of seed capsules per plant

| Line | Wild-type | AtCKX1-48 | AtCKX1-50 |
|---|---|---|---|
| Number of capsules | 83.33 ± 5.13 | 2.00 ± 1.00 | 2.60 ± 1.67 |

*Experimental:* Number of seed capsules was determined at least from 5 different plants. Please note that these plants were grown under greenhouse conditions during winter time. This affects negatively the number of flowers that are formed, in particular in the transgenic clones. However, the general picture that they form a reduced number of flowers is correct. n.d., not determined

C. Seed yield / capsule (mg)

| Line | Wild-type | AtCKX1-48 | AtCKX1-50 |
|---|---|---|---|
| Seed/capsule (mg) | 87.41 ± 28.75 | 23.83 ± 13.36 | 61.8 ± 40.66 |

*Experimental:* Seed yield was determined for at least 12 seed capsules. The size of seed capsules was very variable, hence the large standard deviations. n.d., not determined

D. Weight of 100 seeds (mg)

| Line | Wild-type | AtCKX1-48 | AtCKX1-50 |
|---|---|---|---|
| Seeds weight (mg) | 9.73 ± 0.44 | 10.70 ± 1.60 | 9.54 ± 0.94 |

Experimental: *The seed biomass was determined* as *the weight of 100 seed from at least 5 different seed capsules. n.d., not determined*

3.2 In *Arabidopsis*

- onset of germination was same as for WT
- the total root system was enlarged and the number of side roots and adventitious roots was enhanced (see Figure 4A through D)
- the growth of aerial organs was reduced resulting in a dwarfed phenotype (see Figure 4E and F) and the leaf biomass was reduced. Leaf and flower formation is delayed.
- the life cycle was longer compared to WT and the seed yield was lower compared to WT

The following morphometric data illustrate these phenotypes:

Root development

A. Total length of the root system

| Line | Wild-type | AtCKX1-11 | AtCKX1-15 |
|---|---|---|---|
| Length (mm) | 32.5 | 76.5 | 68.4 |

B. Primary root length

| Line | Wild-type | AtCKX1-11 | AtCKX1-15 |
|---|---|---|---|
| Length (mm) | 32.3 ± 3.8 | 52.3 ± 4.8 | 39.9 ± 4.2 |

C. Lateral roots (LR) length

| Line | Wild-type | AtCKX1-11 | AtCKX1-15 |
|---|---|---|---|
| Length (mm) | 0.2 ± 0.4 | 15.6 ± 11.0 | 10.4 ± 7.6 |

D. Adventitious roots length

| Line | Wild-type | AtCKX1-11 | AtCKX1-15 |
|---|---|---|---|
| Length (mm) | 0.03 ± 0.18 | 8.6±8.5 | 19.1 ± 11.0 |

E. Number of lateral roots (LR)

| Line | Wild-type | AtCKX1-11 | AtCKX1-15 |
|---|---|---|---|
| Number of LR | 0.3 ± 0.5 | 10.4 ± 5.4 | 2.6 ± 1.1 |

F. Number of adventitious roots (AR)

| Line | Wild-type | AtCKX1-11 | AtCKX1-15 |
|---|---|---|---|
| Number of AR | 3.03 ± 0.18 | 1.6 ± 1.1 | 2.6 ± 1.1 |

*Experimental*: Measurements were carried out on plants 8 days after germination in vitro on MS medium. At least 17 plants per line were scored.

Shoot development

A. Leaf surface

| Line | Wild-type | AtCKX1-11-7 T3 homozygous T3 plants | AtCKX1-11-12 homozygous T3 plants | AtCKX1-15-1 homozygous plants |
|---|---|---|---|---|
| Leaf surface ($cm^2$) | 21.16 ± 1.73 | 2.28 ± 0.58 | 2.62 ± 0.28 | 1.66 ± 0.22 |

*Experimental:* Leaf surface area of main rosette leaves formed after 30 days after germination was measured. 3 plants per clone were analyzed.

Reproductive development

Onset of flowering

| Line | Wild-type | AtCKX1-11 T3 heterozygous plants | AtCKX2-2 T2 heterozygous plants | AtCKX2-5 T2 heterozygous plants |
|---|---|---|---|---|
| Flowering time (DAG) | 43.6 ± 5.8 | 69.7 ± 9.4 | 51.2 ± 4.1 | 45.1 ± 6.9 |

*Experimental:* Plants were grown under greenhouse condition. At least 13 plants per clone were analyzed. DAG = days after germination

**Conclusion**: The analysis of *AtCKX1* transgenic *Arabidopsis* plants confirmed largely the results obtained from tobacco and indicates the general nature of the consequences of a reduced cytokinin content. The total root system was enlarged (the total root length was increased app. 110-140% in *AtCKX1* transgenics), the shoot developed more slowly (retarded flowering) and the leaf biomass was reduced. The seed yield was lower in the transgenics as well.

**Example 4. Transgenic plants overexpression *AtCKX2* showed increased cytokinin oxidase activity and altered plant** morphology

### 1. Description of the cloning process

The following primers were used to PCR amplify the AtCKX2 gene from *Arabidopsis thaliana,* accession Columbia (non-homologous sequences used for cloning are in lower case):

Sequence of 5' primer: gcggtaccAGAGAGAGAAACATAAACAAATGGC (SEQ ID N0:15)

Sequence of 3' primer: gcggtaccCAATTTTACTTCCACCAAAATGC (SEQ ID NO:16)

A 3104-bp PCR fragment, amplified by these primers, was inserted in the Kpnl site of pUC19. The insert was sequenced to check that no differences to the published sequence were introduced by the PCR procedure. The Kpnl/Kpnl fragment of this vector was subcloned in the Kpnl site downstream of a modified CaMV 35S promoter (carrying three tetracycline operator sequences) in the binary vector pBinHyg-Tx (Gatz *et al*., 1992). The resulting construct was introduced into tobacco and *Arabidopsis thaliana* through Agrobacterium-mediated transformation, using standard transformation protocols.

### 2. Molecular analysis of the transgenic lines

Several transgenic lines were identified that synthesize the *AtCKX2* transcript at high levels (Fig 6). Transgenic lines expressing *AtCKX2* transcript also showed increased cytokinin oxidase activity. This is exemplified for 2 tobacco and 3 Arabidopsis lines in Table 7. This result proves that the *AtCKX2* gene encodes a protein with cytokinin oxidase activity.

Table 7. Cytokinin oxidase activity in AtCKX2 transgenic plant tissues

| **Sample** | | |
|---|---|---|
| **Plant species and tissue** | **Plant line** | **Cytokinin oxidase activity (nmol Ade/mg protein.h)** |
| *Arabidopsis* callus | Col-0 wild-type | 0.037 |
| | CKX2-15 | 0.351 |
| | CKX2-17 | 0.380 |
| | CKX2-55 | 0.265 |
| Tobacco leaves | SNN wild-type | 0.009 |
| | CKX2-SNN-18 | 0.091 |
| | CKX2-SNN-19 | 0.091 |

**3. Phenotypic description of the transgenic lines**

3.1 In tobacco (see Fig 7 to 10):

Three categories of phenotype:

1) strong - 15 clones (similar to intermediate phenotype of *AtCKX1*)

2) weak - 6 clones

3) others - similar to WT plants, 7 clones

Aerial plant parts

The observations concerning plant height, internode distance, branching, leaf form and yellowing were similar as for *AtCKX1* transgenics with some generally minor quantitative differences in that the dwarfing characteristics were more severe in *AtCKX1* transgenics than in *AtCKX2* transgenics (compare *AtCKX1* plants with *AtCKX2* plants in Figure 7A and B). This is illustrated below for stem elongation and internode distance measurements of clones with a strong phenotype *AtCKX2-38* and *AtCKX2-40*:

Stem elongation

| Line | Wild-type | AtCKX2-38 | AtCKX2-40 |
|---|---|---|---|
| Days after germination | Height (cm) | Height (cm) | Height (cm) |
| 47 | 9.5 ± 0.5 | 2.4 ± 0.1 | 2.6 ± 0.2 |
| 58 | 22.4 ± 2.3 | 5.5 ± 0.7 | 5.3 ± 0.5 |
| 68 | 35.3 ± 2.6 | 7.1 ± 0.8 | 7.0 ± 0.7 |
| 100 | 113.3 ± 9.8 | 15.5 ± 2.5 | 20.3 ± 6.4 |
| 117 | 138.6 ± 8.1 | 19.8 ± 3.8 | 29.5 ± 6.0 |
| 131 | 139.0 ± 9.3 | 26.5 ± 7.0 | 33.4 ± 5.8 |
| 152 | 136.6 ± 10.4 | 33.7 ± 6.3 | 33.9 ± 6.4 |
| 165 | | 36.2 ± 4.3 | |

*Experimental*: Plants were grown in soil in a green house. Data were collected from at least ten plants per line.

Internode distance

| Line | Wild-type | AtCKX2-38 |
|---|---|---|
| Internode distance (mm) | 39.2 ± 3.8 | 7.2 ± 1.6 |

*Experimental:* The length of the $20^{th}$ internode from the bottom was measured at day 131 after germination.

Roots

In vitro grown plants highly expressing the gene were easily distinguishable from WT plants by their ability to form more roots which are thicker (stronger) as well as by forming aerial roots along the stem.
The primary root was longer and the number of lateral and adventitious roots was higher as illustrated in Figure 8C for *AtCKX2-38* overexpressing seedlings (see also Example 9).

The dose-response curve of root growth inhibition by exogenous cytokinin showed that roots of transgenic seedlings were more cytokinin resistant than WT roots (Figure 8 D). The resistance of *AtCKX1-28* transgenics to iPR was less marked than for *AtCKX2-38,* which is consistent with the smaller changes in iP-type cytokinins in the latter (see Table 10).

An increase in fresh and dry weight of the root biomass of TO lines of AtCKX2 transgenic plants compared to WT was observed for plant grown in soil, as illustrated in the following table:

| Line | Wild-type | AtCKX2 (T0) |
|---|---|---|
| Fresh weight (g) | 45.2 ± 15.4 | 77.1 ± 21.3 |
| Dry weight (g) | 6.3 ± 1.9 | 9.6 ± 2.2 |

Experimental: Six WT plants and six independent T0 lines of 35S::AtCKX2 clone were grown on soil. After flowering the root system was washed with water, the soil was removed as far as possible and the fresh weight and dry weight was measured.

An increase in fresh and dry weight of the root biomass was also observed for F1 progeny of *AtCKX2* transgenics grown in hydroponics as compared to WT, as illustrated in the following table:

| Line | Wild-type | AtCKX2-38 | AtCKX2-40 |
|---|---|---|---|
| Fresh weight ROOT (9) | 19.76 ± 6.79 | 33.38 ± 7.76 | 50.04 ± 15.59 |
| Dry weight ROOT (g) | 2.36 ± 0.43 | 2.61 ± 0.39 | 3.52 ± 1.06 |
| Fresh weight SHOOT (g) | 159.8 ± 44.53 | 33.66 ± 2.67 | 48.84 ± 11.83 |
| Fresh weight SHOOT/ROOT ratio | 8.24 ± 0.63 | 1.04 ± 0.18 | 1.08 ± 0.51 |

Experimental: Soil grown plants were transferred 60 days after germination to a hydroponic system (Hoagland's solution) and grown for additional 60 days. The hydroponic solution was aerated continuously and replaced by fresh solution every third day.

In summary, transgenic plants grown in hydroponic solution formed approximately 65-150% more root biomass (fresh weight) than wild type plants. The increase in dry weight was 10-50%. This difference is possibly in part due to the larger cell volume of the transgenics. This reduces the relative portion of cell walls, which forms the bulk of dry matter material. The shoot biomass was reduced to 20%-70% of wild type shoots. The difference in fresh weight leads to a shift in the shoot/root ratio, which was approximately 8 in wild type but approximately 1 in the transgenic clones.

**Conclusion:**

An increase in root growth and biomass was observed for *AtCKX2* transgenic seedlings and adult plants grown under different conditions compared to WT controls despite the fact that growth of the aerial plant parts is reduced. Quantitative differences were observed between different transgenic plants: higher increases in root biomass were observed for the strongest expressing clones.

**Reproductive development**

The onset of flowering in *AtCKX2* transgenics was delayed, the number of flowers and the seed yield per capsule was reduced. These effects were very similar to those observed in the *AtCKX1* transgenic plants but they were less prominent in the *AtCKX2* transgenics, as indicated in the tables below. The size of flowers was not altered in transgenic plants and the weight of the individual seeds was comparable to the weight of seeds from wild type plants.

A. Onset of flowering

| Line | Wild-type | AtCKX1-48 | AtCKX1-50 | AtCKX2-38 | AtCKX2-40 |
|---|---|---|---|---|---|
| Flowering time (DAG) | 106.2 ± 3.3 | 193.3 ± 4.3 | 191.8 ± 3.8 | 140.6 ± 6.5 | 121.9 ± 9.8 |

*Experimental:* Data collected for at least ten plants per line. The full elongation of the first flower was defined as onset of flowering. DAG = days after germination.

B. Number of seed capsules per plant

| Line | Wild-type | AtCKX1-48 | AtCKX1-50 | AtCKX2-38 | AtCKX2-40 |
|---|---|---|---|---|---|
| Number of capsules | 83.33 ± 5.13 | 2.00 ± 1.00 | 2.60 ± 1.67 | 4.30 ± 2.58 | n.d. |

*Experimental:* Number of seed capsules was determined at least from 5 different plants. Please note that these plants were grown under green house conditions during winter time. This affects negatively the number of flowers that are formed, in particular in the transgenic clones. However, the general picture that they form a reduced number of flowers is correct. n.d., not determined

C. Seed yield / capsule (mg)

| Line | Wild-type | AtCKX1-48 | AtCKX1-50 | AtCKX2-38 | AtCKX2-40 |
|---|---|---|---|---|---|
| Seed/capsule (mg) | 87.41 ± 28.75 | 23.83 ± 13.36 | 61.8 ± 40.66 | 46.98 ± 29.30 | n.d. |

*Experimental:* Seed yield was determined for at least 12 seed capsules. The size of seed capsules was very variable, hence the large standard deviations. n.d., not determined

D. Weight of 100 seeds (mg)

| Line | Wild-type | AtCKX1-48 | AtCKX1-50 | AtCKX2-38 | AtCKX2-40 |
|---|---|---|---|---|---|
| Seeds weight (mg) | 9.73 ± 0.44 | 10.70 ± 1.60 | 9.54 ± 0.94 | 10.16 ± 0.47 | n.d. |

Experimental: *The seed biomass was determined as the weight of 100 seed from at least 5 different seed capsules. n.d., not determined*

3.2 In Arabidopsis:

The following morphometric data were obtained for *AtCKX2* transgenics:

Root development

A. Total length of the root system

| Line | Wild-type | AtCKX2-2 | AtCKX2-5 |
|---|---|---|---|
| Length (mm) | 32.5 | 50.6 | 48.5 |

B. Primary root length

| Line | Wild-type | AtCKX2-2 | AtCKX2-5 |
|---|---|---|---|
| Length (mm) | 32.3 ± 3.8 | 30.7 ± 4.8 | 31.6 ± 6.8 |

C. Lateral roots length

| Line | Wild-type | AtCKX2-2 | AtCKX2-5 |
|---|---|---|---|
| Length (mm) | 0.2 ± 0.4 | 5.5 ± 9.0 | 1.9 ± 2.5 |

D. Adventitious roots length

| Line | Wild-type | AtCKX2-2 | AtCKX2-5 |
|---|---|---|---|
| Length (mm) | 0.03 ± 0.18 | 14.4 ± 10.2 | 14.9 ± 9.1 |

E. Number of lateral roots (LR)

| Line | Wild-type | AtCKX2-2 | AtCKX2-5 |
|---|---|---|---|
| Number of LR | 0.3 ± 0.5 | 2.9 ± 2.3 | 1.9 ± 1.0 |

F. Number of adventitious roots (AR)

| Line | Wild-type | AtCKX2-2 | AtCKX2-5 |
|---|---|---|---|
| Number of AR | 3.03 ± 0.18 | 1.8 ± 0.9 | 1.8 ± 1.0 |

*Experimental:* Measurements were carried out on plants 8 d.a.g. in vitro on MS medium. At least 17 plants per line were scored.

Shoot development

Leaf surface

| Line | Wild-type | AtCKX2-2 T2 heterozygous plants | AtCKX2-5 T2 heterozygous plants | AtCKX2-9 T2 heterozygous plants |
|---|---|---|---|---|
| Leaf surface ($cm^2$) | 21.16 ± 1.73 | 8.20 ± 2.35 | 8.22 ± 0.55 | 7.72 ± 0.85 |

*Experimental:* Leaf surface area of main rosette leaves formed after 30 days after germination was measured. 3 plants per clone were analyzed.

Reproductive development

Onset of flowering

| Line | Wild-type heterozygous | [AtCKX1-11 T3 plants | AtCKX2-2 T2 heterozygous plants | AtCKX2-5 T2 heterozygous plants |
|---|---|---|---|---|
| Flowering time (DAG) | 43.6 ± 5.8 | 69.7 ± 9.4 | 51.2 ± 4.1 | 45.1 ± 6.9 |

*Experimental:* Plants were grown under greenhouse condition. At least 13 plants per clone were analyzed. DAG = days after germination.

**Conclusion:** Arabidopsis *AtCKX2* transgenics had reduced leaf biomass and a dwarfing phenotype similar to *AtCKX1* transgenics (compare Figure 5 with Figure 4 F). The total root system was also enlarged in *AtCKX2* transgenic Arabidopsis. The total root length is increased approximately 50% in *AtCKX2* transgenics. The *AtCKX1* transgenics have longer primary roots, more side roots and form more adventitious roots. *AtCKX2* transgenics lack the enhanced growth of the primary root but form more side roots and lateral roots than WT.

**Summary:**

The phenotypes observed for *AtCKX2* transgenics were very similar but not identical to the *AtCKX1* transgenics, which in turn were very similar but not identical to the results obtained for the tobacco transgenics. This confirms the general nature of the consequences of a reduced cytokinin content in these two plant species and therefore, similar phenotypes can be expected in other plant species as well. The main difference between tobacco and Arabidopsis is the lack of enhanced primary root growth in *AtCKX2* overexpressing plants.

**Example 5. Transgenic plants overexpressing *AtCKX3* showed increased cytokinin oxidase activity and altered plant morchology**

**1. Description of the cloning process**

The following primers were used to PCR amplify the *AtCKX3* gene from *Arabidopsis thaliana,* accession Columbia (non-homologous sequences used for cloning are in lower case):

Sequence of 5' primer: gcggtaccTTCATTGATAAGAATCAAGCTATTCA (SEQ ID NO:17)

Sequence of 3' primer: gcggtaccCAAAGTGGTGAGAACGACTAACA (SEQ ID NO:18)

A 3397-bp PCR fragment, produced by this PCR amplification, was inserted in the KpnI site of pBluescript. The insert was sequenced to confirm that the PCR product has no sequence changes as compared to the gene. The KpnI/KpnI fragment of this vector was subcloned in the KpnI site downstream of a modified CaMV 35S promoter (carrying three tetracycline operator sequences) in the binary vector pBinHyg-Tx (Gatz *et al.,* 1992). The resulting construct was introduced into tobacco and *Arabidopsis thaliana* through *Agrobacterium*-mediated transformation, using standard transformation protocols.

**2. Molecular analysis of the transgenic lines**

Several transgenic tobacco lines were identified that synthesize the *AtCKX3* transcript at high levels (Fig 11A.). Transgenic tobacco lines expressing *AtCKX3* transcript also showed increased cytokinin oxidase activity. This is exemplified for three plants in

Table 8. This proves that the *AtCKX3* gene encodes a protein with cytokinin oxidase activity.

**Table 8.** Cytokinin oxidase activity in *AtCKX4* transgenic plant tissues

| Sample | | |
|---|---|---|
| **Plant species and tissue** | **Plant line** | **Cytokinin oxidase activity (nmol Ade/mg protein.h)** |
| tobacco leaves | SNN wild-type | 0.011 |
| | CKX3-SNN-3 | 0.049 |
| | CKX3-SNN-6 | 0.053 |
| | CKX3-SNN-21 | 0.05 |

**3. Plant phenotypic analysis**

The phenotypes generated by overexpression of the *AtCKX3* gene in tobacco and *Arabidopsis* were basically similar as those of *AtCKX1* and *AtCKX2* expressing plants, i.e. enhanced rooting and dwarfing. However, overexpression of the *AtCKX3* gene in tobacco resulted in a stronger phenotype compared to *AtCKX2.* In this sense *AtCKX3* overexpression was more similar to *AtCKX1* overexpression.

**Example 6. Transgenic plants overexpressing *AtCKX4* showed increased cytokinin oxidase activity and altered plant morphology**

**1. Description of the cloning process**

The following primers were used to PCR amplify the AtCKX4 gene from *Arabidopsis thaliana,* accession Columbia (non-homologous sequences used for cloning are in lower case):

Sequence of 5' primer: gcggtaccCCCATTAACCTACCCGTTTG (SEQ ID NO:19)

Sequence of 3' primer: gcggtaccAGACGATGAACGTACTTGTCTGTA (SEQ ID NO:20)

A 2890-bp PCR fragment, produced by this PCR amplification, was inserted in the KpnI site of pBluescript. The insert was sequenced to confirm that the PCR product has no sequence changes as compared to the gene. The KpnI/KpnI fragment of this vector was subcloned in the KpnI site downstream of a modified CaMV 35S promoter (carrying three tetracycline operator sequences) in the binary vector pBinHyg-Tx (Gatz *et al*., 1992). The resulting construct was introduced into tobacco and *Arabidopsis thaliana* through *Agrobacterium*-mediated transformation, using standard transformation protocols.

**2. Molecular analysis of the transgenic lines**

Several transgenic tobacco lines synthesized the *AtCKX4* transcript at high levels (Fig 11B.). Transgenic lines expressing *AtCKX4* transcript also showed increased cytokinin oxidase activity. This is exemplified for 3 *Arabidopsis* and 3 tobacco lines in Table 9. This result proves that the *AtCKX4* gene encodes a protein with cytokinin oxidase activity.

**Table 9.** Cytokinin oxidase activity in *AtCKX4* transgenic plant tissues

| Sample | | |
|---|---|---|
| **Plant species and tissue** | **Plant line** | **Cytokinin oxidase activity (nmol Ade/mg protein.h)** |
| *Arabidopsis* callus | Col-0 wild-type | 0.037 |
| | CKX4-37 | 0.244 |
| | CKX4-40 | 0.258 |
| | CKX4-41 | 0.320 |
| tobacco leaves | SNN wild-type | 0.011 |
| | CKX4-SNN-3 | 0.089 |
| | CKX4-SNN-18 | 0.085 |

(continued)

| Sample | | |
|---|---|---|
| **Plant species and tissue** | **Plant line** | **Cytokinin oxidase activity (nmol Ade/mg protein.h)** |
| | CKX4-SNN-27 | 0.096 |

Overall, the data showed that the apparent $K_m$ values for the four cytokinin oxidases were in the range of 0.2 to 9.5 μM with iP as substrate, which further demonstrates that the proteins encoded by *AtCKX1* through *4* are indeed cytokinin oxidase enzymes as disclosed herein.

**3. Plant phenotypic analysis**

The phenotypes generated by overexpression of the *AtCKX4* gene in tobacco and *Arabidopsis* were basically similar as those of *AtCKX1* and *AtCKX2* expressing plants, i.e. enhanced rooting, reduced apical dominance, dwarfing and yellowing of intercostal regions in older leaves of tobacco. An additional phenotype in tobacco was lanceolate leaves (altered length-to-width ratio).

General observations of *AtCKX* overexpressing tobacco plants

Overall, the phenotypic analysis demonstrated that *AtCKX* gene overexpression caused drastic developmental alterations in the plant shoot and root system in tobacco, including enhanced development of the root system and dwarfing of the aerial plant part. Other effects such as altered leaf senescence, formation of adventitious root on stems, and others were also observed as disclosed herein. The alterations were very similar, but not identical, for the different genes. In tobacco, *AtCKX1* and *AtCKX3* overexpressors were alike as were *AtCKX2* and *AtCKX4.* Generally, the two former showed higher expression of the traits, particularly in the shoot. Therefore, a particular cytokinin oxidase gene may be preferred for achieving the phenotypes that are described in the embodiments of this invention.

**Example 7. Cloning of the *AtCKX5* gene**

The following primers were used to PCR amplify the AtCKX5 gene from *Arabidopsis thaliana,* accession Columbia (non-homologous sequences used for cloning are in lower case):

Sequence of 5' primer: ggggtaccTTGATGAATCGTGAA*ATG*AC (SEQ ID NO:21)

Sequence of 3' primer: ggggtaccCTTTCCTCTTGGTTTTGTCCTGT (SEQ ID NO:22)

The sequence of the 5' primer includes the two potential start codons of the AtCKX5 protein, the most 5' start codon is underlined and a second ATG is indicated in italics.

A 2843-bp PCR fragment, produced by this PCR amplification, was inserted as a blunt-end product in pCR-Blunt II-TOPO cloning vector (Invitrogen).

**Example 8. Cloning of the *AtCKX6* gene**

The following primers were used to PCR amplify the AtCKX6 gene from *Arabidopsis thaliana,* accession Columbia (non-homologous sequences used for cloning are in lower case):

Sequence of 5' primer: gctctagaTCAGGAAAAGAACCATGCTTATAG (SEQ ID NO:23)

Sequence of 3' primer: gctctagaTCATGAGTATGAGACTGCCTTTTG (SEQ ID NO:24)

A 1949-bp PCR fragment, produced by this PCR amplification, was inserted as a blunt-end product in pCR-Blunt II-TOPO cloning vector (Invitrogen).

**Example 9. Tobacco seedling growth test demonstrated early vigor of *AtCKX* transgenics**

Seeds of *AtCKX1-50* and *AtCKX2-38* overexpressing transgenics and WT tobacco were sown *in vitro* on MS medium, brought to culture room 4 days after cold treatment and germinated after 6 days. Observations on seedling growth were made 10 days after germination (see also Figure 8C) and are summarized below. At least 20 individuals were scored per clone. Similar data have been obtained in two other experiments.

A. Total length of the root system

| Line | Wild-type | AtCKX1-50 | AtCKX2-38 |
|---|---|---|---|
| Length (mm) | 61.1 | 122.0 | 106.5 |

B. Primary root length

| Line | Wild-type | AtCKX1-50 | AtCKX2-38 |
|---|---|---|---|
| Length (mm) | 32.3 ± 2.6 | 50.8 ± 4.5 | 52.4 ± 4.8 |

C. Lateral roots length

| Line | Wild-type | AtCKX1-50 | AtCKX2-38 |
|---|---|---|---|
| Length (mm) | 9.8 ± 5.5 | 18.0 ± 8.1 | 13.0 ± 6.0 |

D. Adventitious roots length

| Line | Wild-type | AtCKX1-50 | AtCKX2-38 |
|---|---|---|---|
| Length (mm) | 19.0 ± 5.0 | 53.0 ± 12.0 | 42.0 ± 9.8 |

E. Number of lateral roots (LR)

| Line | Wild-type | 4tCKX1-50 | AtCKX2-38 |
|---|---|---|---|
| Number of LR | 1.9 ± 0.9 | 6.5 ± 2.2 | 5.6 ± 2.0 |

F. Number of adventitious roots (AR)

| Line | Wild-type | AtCKX1-50 | AtCKX2-38 |
|---|---|---|---|
| Number of AR | 2.2 ± 0.6 | 3.5 ± 0.9 | 3.6 ± 1.3 |

*AtCKX1* and *AtCKX2* plants, general observations:

Seedlings of *AtCKX1* and *AtCKX2* overexpressing tobacco plants had 60% more adventitious roots and three times more lateral roots than untransformed control plants 10 days after germination. The length of the primary root was increased by about 70%. This - together with more and longer side roots and secondary roots - resulted in a 70-100% increase in total root length. These results showed that overexpression of cytokinin oxidase enhances the growth and development of both the main root and the adventitious roots, resulting in early vigor.

**Example 10. Histological analysis of altered plant morphology in *AtCKX1* overexpressing tobacco plants**

Microscopic analysis of different tissues revealed that the morphological changes in *AtCKX* transgenics are reflected by distinct changes in cell number and rate of cell formation (see Figure 10). The shoot apical meristem (SAM) of *AtCKX1* transgenics was smaller than in wild type and fewer cells occupy the space between the central zone and the peripheral zone of lateral organ formation, but the cells were of the same size (Figure 10 A). The reduced cell number and size of the SAM as a consequence of a reduced cytokinin content indicates that cytokinins have a role in the control of SAM proliferation. No obvious changes in the differentiation pattern occurred, suggesting that the spatial organization of the differentiation zones in the SAM is largely independent from cell number and from the local cytokinin concentration. The overall tissue pattern of leaves in cytokinin oxidase overexpressors was unchanged. However, the size of the phloem and xylem was significantly reduced (Figure 10 B). By contrast, the average cell size of leaf parenchyma and epidermal cells was increased four- to fivefold (Figure 10C, D). New cells of *AtCKX1* transgenics are formed at 3-4% of the rate of wild type leaves and final leaf cell number was estimated to be in the range of 5-6% of wild type. This indicates an absolute requirement for cytokinins in leaves to maintain the cell division cycle. Neither cell size nor cell form of floral organs was altered and seed yield per capsule was similar in wild type and *AtCKX* transgenic plants. The cell population of root meristems of *AtCKX1* transgenic plants was enlarged approximately 4-fold and the cell numbers in both the central and lateral columnella were enhanced (Figure 10E, F). The final root diameter was increased by 60% due to an increased diameter of all types of root cells. The radial root patterns was identical in wild type and transgenics, with the exception that frequently a fourth layer of cortex cells was noted in transgenic roots (Figure 10 G). The increased cell number and the slightly reduced cell length indicates that the enhanced root growth is due to an increased number of cycling cells rather than increased cell growth. In the presence of lowered cytokinin content, root meristem cells must undergo additional rounds of mitosis before they leave the meristem and start to elongate. The exit from the meristem is therefore regulated by a mechanism that is sensitive to cytokinins. Apparently, cytokinins have a negative regulatory role in the root meristem and wild type cytokinin concentrations are inhibitory to the development of a maximal root system. Therefore, reducing the level of active cytokinins by overexpressing cytokinin oxidases stimulates root development, which results in an increase in the size of the root with more lateral and adventitious roots as compared to WT plants.

**Example 11. *AtCKX1* and *AtCKX2*- overexpressing tobacco plants had a reduced cytokinin content.**

Among the 16 different cytokinin metabolites that were measured, the greatest change occurred in the iP-type cytokinins in *AtCKX2* overexpressers (Table 10): the overall decrease in the content of iP-type cytokinins is more pronounced in *AtCKX2* expressing plants than in *AtCKX1* transgenics. *AtCKX1* transgenics showed a stronger phenotype in the shoot. It is not known which cytokinin metabolite is relevant for the different traits that were analysed. It may be that different cytokinin forms play different roles in the various development processes. Smaller alterations were noted for Z-type cytokinins, which could be due to a different accessibility of the substrate or a lower substrate specificity of the protein. The total content of iP and Z metabolites in individual transgenic clones was between 31% and 63% of wild type. The cytokinin reserve pool of *O*-glucosides was also lowered in the transgenics (Table 10). The concentration of *N*-glucosides and DHZ-type cytokinins was very low and was not or only marginally, altered in transgenic seedlings (data not shown).

**Table 10.** Cytokinin content of *AtCKX* transgenic plants. Cytokinin extraction, immunopurification, HPLC separation and quantification by ELISA methods was carried out as described by Faiss et al., 1997. Three independently pooled samples of approximately 100 two week old seedlings (2.5 g per sample) were analysed for each clone. Concentrations are in pmol x g fresh weight$^{-1}$. Abbreviations: iP, $N^6$-($\Delta^2$isopentenyl)adenine; iPR, $N^6$-($\Delta^2$isopentenyl)adenine riboside; iPRP, $N^6$-($\Delta^2$isopentenyl)adenine riboside 5'-monophosphate; Z, *trans*-zeatin; ZR, zeatin riboside; ZRP, zeatin riboside 5'-monophosphate; ZOG, zeatin *O*-glucoside; ZROG, zeatin riboside *O*-glucoside.

| Line | WT | AtCKX1-2 | | AtCKX1-28 | | AtCKX2-38 | | AtCKX2-40 | |
|---|---|---|---|---|---|---|---|---|---|
| Cytokinin metabolite | Concentration | Concentration | % of WT | Concentration | % of WT | Concentration | % of WT | Concentration | % of WT |
| iP | 5.90<br>1.80 | ± 4.76<br>0.82 | ± 81 | 4.94<br>2.62 | ± 84 | 1.82<br>0.44 | ± 31 | 2.85<br>0.62 | ± 48 |
| IPR | 2.36<br>0.74 | ± 1.53<br>0.14 | ± 65 | 0.75<br>0.27 | ± 32 | 0.55<br>0.39 | ± 23 | 0.89<br>0.07 | ± 38 |
| IPRP | 3.32<br>0.73 | ± 0.87<br>0.26 | ± 26 | 1.12<br>0.13 | ± 34 | 0.80<br>0.48 | ± 24 | 1.68<br>0.45 | ± 51 |
| Z | 0.24<br>0.06 | ± 0.17<br>0.02 | ± 71 | 0.22<br>0.03 | ± 92 | 0.21<br>0.06 | ± 88 | 0.22<br>0.02 | ± 92 |
| ZR | 0.60<br>0.13 | ± 0.32<br>0.12 | ± 53 | 0.34<br>0.03 | ± 57 | 0.34<br>0.15 | ± 57 | 0.32<br>0.05 | ± 53 |
| ZRP | 0.39<br>0.17 | ± 0.42<br>0.11 | ± 107 | 0.28<br>0.15 | ± 72 | 0.06<br>0.01 | ± 15 | 0.17<br>0.06 | ± 44 |
| ZOG | 0.46<br>0.20 | ± 0.32<br>0.09 | ± 70 | 0.26<br>0.13 | ± 57 | 0.20<br>0.07 | ± 43 | 0.12<br>0.02 | ± 26 |
| ZROG | 0.48<br>0.17 | ± 0.30<br>0.06 | ± 63 | 0.47<br>0.02 | ± 98 | 0.23<br>0.05 | ± 48 | 0.30<br>0.13 | ± 63 |
| Total | 13.75 | 8.69 | 63 | 8.38 | 61 | 4.21 | 31 | 6.55 | 48 |

**Example 12. Grafting experiments showed that dwarfing and enhanced root development due to *AtCKX* overexpression is confined to transgenic tissues**

To investigate which phenotypic effects of cytokinin oxidase overexpression are restricted to expressing tissues, i.e. are cell- or organ-autonomous traits, grafting experiments were performed. Reciprocal grafts were made between an *AtCKX2* transgenic tobacco plant and a WT tobacco. The transgenic plant used in this experiment was AtCKX2-38, which displayed a strong phenotype characterized by enhanced root growth and reduced development of the aerial plant parts. As described in Example 3 through 6, these were two important phenotypes that resulted from cytokinin oxidase overexpression in tobacco and arabidopsis.

Plants were about 15 cm tall when grafted and the graft junction was about 10 cm above the soil. Figure 12 shows plants 15 weeks after grafting. The main results were that : (i) the aerial phenotype of a WT scion grafted on a transgenic rootstock was similar to the WT control graft (= WT scion on WT rootstock). Importantly, this showed that overexpression of the *AtCKX2* transgene in the rootstock did not induce dwarfing of the non-transgenic aerial parts of the plant (see Figure 12 A). Improved root growth of the transgenic rootstock was maintained, indicating that improved root growth of *AtCKX* transgenics is autonomous and does not depend on an *AtCKX* transgenic shoot (Figure 12C). Interestingly, the WT scions grafted on the transgenic rootstocks looked healthier and were better developed. Notably, senescence of the basal leaves was retarded in these plants (see Figure 12 A); (ii) the transgenic scion grafted on the WT rootstock looked similar to the aerial part of the transgenic plant from which it was derived, i.e. the shoot dwarfing phenotype is also autonomous and not dependent on the improved root growth (see Figure 12 B).

In addition to the above-mentioned better appearance of WT shoots grafted on a transgenic rootstock, the formation of adventitious roots on the basal part of WT shoots was noted (Figure 12D, right plant). Formation of adventitious roots also occurred on the stem of *AtCKX* transgenics but not on stems of WT control grafts (Figure 12D, left plant) and therefore seems to be a non-autonomous trait.

In summary, it is disclosed in this invention that enhanced root formation and dwarfing of the shoot in *AtCKX* overexpressing tobacco are autonomous traits and can be uncoupled by grafting procedures. Surprisingly, grafting of a WT scion on an *AtCKX* transgenic rootstock resulted in more vigorously growing plants and retardation of leaf senescence.

As an alternative to grafting, tissue-specific promoters could be used for uncoupling the autonomous phenotypic effects of cytokinin overexpression. Therefore, it is disclosed in this invention that cytokinin oxidase overexpression in a tissue specific manner can be used to alter the morphology of a plant such as the shoot or root system.

**Example 13. Expression of an *AtCKX* gene under a root-specific promoter in transgenic plants leads to increased root production**

An *AtCKX* gene (see example 4) is cloned under control of the root clavata homolog promoter of *Arabidopsis* (SEQ ID NO: 36), which is a promoter that drives root-specific expression. Other root-specific promoters may also be used for the purpose of this invention. See Table 5 for exemplary root-specific promoters.

Transgenic plants expressing the *AtCKX* gene specifically in the roots show increased root production without negatively affecting growth and development of the aerial parts of the plant. Positive effects on leaf senescence and growth of aerial plant parts are observed.

**Example 14. Suppression of an *AtCKX* gene under a senescence-induced promoter in transgenic plants leads to delayed leaf senescence and enhanced seed yield.**

A chimeric gene construct derived from an *AtCKX* gene and designed to suppress expression of endogenous cytokinin oxidase gene(s) is cloned under control of a senescence-induced promoter. For example, promoters derived from senescence-associated genes (SAG) such as the SAG12 promoter can be used (Quirino et al., 2000). Transgenic plants suppressing endogenous cytokinin oxidase gene(s) specifically in senescing leaves show delayed leaf senescence and higher seed yield without negatively affecting the morphology and growth and development of the plant.

**Example 15. Overexpression of an *AtCKX* gene in the female reproductive organs leads to parthenocarpic fruit development**

The open reading frame of an *AtCKX* gene is cloned under control of a promoter that confers overexpression in the female reproductive organs such as for example the DefH9 promoter from *Antirrhinum majus* or one of its homologues, which have high expression specificity in the placenta and ovules. Transgenic plants with enhanced cytokinin oxidase activity in these tissues show parthenocarpic fruit development.

## Example 16. Overexpression of AtCKX genes result in increased seed and cotyledon size

Transgenic *Arabidopsis thaliana* plants that overexpress cytokinin oxidase *(AtCKX)* genes under control of the 35S promoter as described supra. Transgenic plants, in particular those expressing the *AtCKX1* and *AtCKX3* genes, developed seeds with increased size which was almost entirely due to an enlarged embryo. Details of the seed, embryo and early postembryonic phenotypes are shown in Figures 13A through 13E. Table 11 shows seed weight of wild type and two independent clones for each of the four investigated AtCKX genes. Average weight was obtained by analysing five different batches of 200 seeds for each clone. A quantitative evaluation showed that the seed weight of *AtCKX1* and *AtCKX3* expressing clones was app. 1.8-2.3-fold higher than in wild type. Gain of weight for seeds of *AtCKX2* and *AtCKX4* expressing lines was in the range of 10-25% (Table 11 and Fig. 14).

The increases in size and weight for seeds, embryos, and cotyledons are unexpected as a reduced cytokinin content would have been expected to be associated with a reduced organ growth. One possible reason for the increases in seed, embryo, and cotyledon size is a previously unknown negative regulatory function of cytokinins in these storage organs. A negative regulatory functions of cytokinins in the control of organ growth is so far only known from roots (Werner et al. 2001). We propose, therefore, that localized expression of cytokinin oxidase genes in tissues where growth is negatively regulated by cytokinins leads to enhanced growth of this tissue. For example, localized expression of *CKX* genes during cotyledon development likely leads to enhanced growth of cotyledons and in species with cotyledons as storage organs, to enhanced yield and to an enhanced growth performance of seedlings. Total number of seeds is lowered in *AtCKX1* and *AtCKX3* expressers. There have been no previous reports however, of lower seed number in *Arabidopsis* being linked to an increase in size.

## Example 17.

Nicotiana tabacum L. cv. Samsun NN leaf explants were transformed with the vector Bin-Hyg-TX carrying the AtCKX1 gene or the AtCKX 2 gene under control of CaMV 35 S promoter. Several lines originating from these transformed plants were further cultivated and their seed size was analysed (Table 12).

Tobacco Plant having the transgene CKX1and CKX2 all showed an increase in seed area, a parameter for seed size.

**TABLE 11**

| | WT | CKX1-11-7 | CKX1-15-1 | CKX2-2-4 | CKX2-9-3 | CKX3-9-4 | CKX3-12-13 | CKX4-37-2 | CKX4-41-7 |
|---|---|---|---|---|---|---|---|---|---|
| **Seed Weight** | 0.0158± 0.0009 | 0.0372± 0.0015 | 0.0352± 0.0023 | 0.0201± 0.0017 | 0.0180± 0.0001 | 0.0340± 0.0027 | 0.0280± 0.0027 | 0.0185± 0.0004 | 0.0179± 0.0007 |
| **% of WT** | 100 | 235.5 | 222.6 | 126.7 | 113.7 | 215.0 | 176.7 | 116.8 | 112.7 |

**TABLE 12**

| Tobacco plant | Description | transgene | Average seed area |
|---|---|---|---|
| 2 | T1 38 nullizygote | | 0 |
| 3 | T1 38 nullizygote | | 0.279 |
| 4 | T1 38 nullizygote | | 0.297 |
| 5 | WT | | 0.248 |
| 6 | WT | | 0.243 |
| 7 | WT | | 0.264 |
| 8 | WT | | 0.277 |
| 1 | T1 38 transgenic | CKX2 | 0.353 |
| 9 | T1 38 transgenic | CKX2 | 0.281 |
| 10 | T1 38 transgenic | CKX2 | 0.293 |
| 11 | T1 38 transgenic | CKX2 | 0.329 |
| 12 | T1 38 transgenic | CKX2 | 0.282 |
| 13 | T1 8 transgenic | CKX1 | 0.278 |
| 14 | T1 8 transgenic | CKX1 | 0.315 |
| 15 | T1 8 transgenic | CKX1 | 0.322 |
| 16 | T1 8 transgenic | CKX1 | 0.312 |

**REFERENCES**

WO0105985. Method to modulate the expression of genes inducing the parthenocarpic trait in plants.

Alberts, B., Bray, D., Lewis, J., Raff, M., Roberts, K., and Watson, J. D. (1994). "Molecular Biology of the Cell." Garland Publishing Inc.

Altschul, S.F., Madden, T.L., Schäffer, A.A., Zhang, J., Zhang, Z., Miller, W. & Lipman, D.J. (1997) "Gapped BLAST and PSI-BLAST: a new generation of protein database search programs." Nucl. Acids Res. 25, 3389-3402.

Armstrong, D.J. (1994) in Cytokinins: Chemistry, Activity and Functions, eds. Mok. D.W.S & Mok, M.C. (CRC Boca Raton, FL), pp. 139-154.

An, G., Watson, B. D., Stachel, S., Gordon, M. P., and Nester, E. W. (1985). New cloning vehicles for transformation of higher plants. EMBO J. 4, 277-284.

Armstrong, C. L., Petersen, W. P., Buchholz, W. G., Bowen, B. A., and Sulc, S. L. (1990). Factors affecting PEG-mediated stable transformation of maize protoplasts. Plant Cell Reports 9, 335-339.

Banerjee, A., Pramanik, A., Bhattacharjya, S., and Balaram, P. (1996). Omega amino acids in peptide design: incorporation into helices. Biopolymers 39, 769-777.

Baron, M. H. and Baltimore, D. (1982). Antibodies against the chemically synthesized genome-linked protein of poliovirus react with native virus-specific proteins. Cell 28, 395-404.

Bartel, P. L. and Fields, S. (1997). "The Yeast Two-Hybrid System." Oxford University Press.

Benkirane, N., Guichard, G., Briand, J. P., and Muller, S. (1996). Exploration of requirements for peptidomimetic immune recognition. Antigenic and immunogenic properties of reduced peptide bond pseudopeptide analogues of a histone hexapeptide. J. Biol Chem. 271, 33218-33224.

Berry, A. and Brenner, S. E. (1994). A prototype computer system for de novo protein design. Biochem.Soc. Trans. 22, 1033-1036.

Christou, P., McCabe, D. E., and Swain, W. F. (1988). Stable transformation of soybean callus by DNA-coated gold particles. Plant Physiol. 87, 671-674.

Crossway, A., Oakes, J. V., Irvine, J. M., Ward, B., Knauf, V. C., and Shewmaker, C. K. (1986). Integration of foreign DNA following microinjection of tobacco mesophyll protoplasts. Mol. Gen. Genet. 202, 179-185.

Dale, E. C. and Ow, D. W. (1990). Intra- and intermolecular site-specific recombination in plant cells mediated by bacteriophage P1 recombinase. Gene 91, 79-85.

Dodds, J. H. (1985). "Plant genetic engineering." Cambridge University Press.

Doerner, P., Jorgensen, J. E., You, R., Steppuhn, J., and Lamb, C. (1996). Control of root growth and development by cyclin expression. Nature 380, 520-523.

Dorner, B., Husar, G. M., Ostresh, J. M., and Houghten, R. A. (1996). The synthesis of peptidomimetic combinatorial libraries through successive amide alkylations. Bioorg.Med.Chem. 4, 709-715.

Ellis, J. G., Llewellyn, D. J., Dennis, E. S., and Peacock, W. J. (1987). Maize Adh-1 promoter sequences control anaerobic regulation: addition of upstream promoter elements from constitutive genes is necessary for expression in tobacco. EMBO J. 6, 11-16.

Faiss, M., Zalubilová, J., Strnad, M., Schmülling, T. (1997). Conditional transgenic expression of the ipt gene indicates a function for cytokinins in paracrine signaling in whole tobacco plants. Plant J. 12, 401-415.

Fassina, G. and Melli, M. (1994). Identification of interactive sites of proteins and protein receptors by computer-assisted searches for complementary peptide sequences. Immunomethods. 5, 114-120.

Fedoroff, N. V. and Smith, D. L. (1993). A versatile system for detecting transposition in Arabidopsis. Plant J. 3, 273-289.

Hanahan, D. (1983). Studies on transformation of Escherichia coli with plasmids. J.Mol.Biol 166, 557-580.

Hansen, G. and Chilton, M. D. (1996). "Agrolistic" transformation of plant cells: integration of T-strands generated in planta. Proc.Natl.Acad.Sci.U.S.A 93, 14978-14983.

Hansen, G., Shillito, R. D., and Chilton, M. D. (1997). T-strand integration in maize protoplasts after codelivery of a T-DNA substrate and virulence genes. Proc.Natl.Acad.Sci.U.S.A 94, 11726-11730.

Hanson, B., Engler, D., Moy, Y., Newman, B., Ralston, E., and Gutterson, N. (1999). A simple method to enrich an Agrobacterium-transformed population for plants containing only T-DNA sequences. Plant J. 19, 727-734.

Harlow, E. and Lane, D. (1988). "Antibodies: A Laboratory Manual." Cold Spring Harbor Laboratory Press.

Herrera-Estrella, L., De Block, M., Messens, E. H. J. P., Van Montagu, M., and Schell, J. (1983). Chimeric genes as dominant selectable markers in plant cells. EMBO J. 2, 987-995.

Hoffman, D. L., Laiter, S., Singh, R. K., Vaisman, I. I., and Tropsha, A. (1995). Rapid protein structure classification using one-dimensional structure profiles on the bioSCAN parallel computer. Comput.Appl.Biosci. 11, 675-679.

Hooykens, P.J.J.,Hall, M.A. & Libbeuga, K.R.,eds.(1999) Biochemistry and Molecular Biology of Plant Hormones (Elsevier, Amsterdam).

Houba-Heria, N.,Pethe, C. d'Alayer, J & Lelouc, M. (1999) Plant J. 17:615-626.

Klee, H.J. & Lanehon, M.B. (1995) in Plant Hormones:Physiology, Biochemisry and Molecular Biology, ed. Davies, P.J. (Kluwer, Dordrdrocht, the Netherlands), pp. 340-353.

Krens, F. A., Molendijk, L., Wullems, G. J., and Schilperoort, R. A. (1982). In vitro transformation of plant protoplasts with Ti-plasmid DNA. Nature 296, 72-74.

Lerner, R. A. (1982). Tapping the immunological repertoire to produce antibodies of predetermined specificity. Nature 299, 593-596.

Lerner, R. A., Green, N., Alexander, H., Liu, F. T., Sutcliffe, J. G., and Shinnick, T. M. (1981). Chemically synthesized peptides predicted from the nucleotide sequence of the hepatitis B virus genome elicit antibodies reactive with the native envelope protein of Dane particles. Proc.Natl.Acad.Sci.U.S.A 78, 3403-3407.

Liddle, J. E. and Cryer, A. (1991). "A Practical Guide to Monoclonal Antibodies." Wiley New York.

Loffler, J., Langui, D., Probst, A., and Huber, G. (1994). Accumulation of a 50 kDa N-terminal fragment of beta-APP695 in Alzheimer's disease hippocampus and neocortex. Neurochem.Int. 24, 281-288.

Mok M.C. (1994) in Cytokines: Chemistry, Activity and Function, eds., Mok, D.W.S. & Mok,M.C. (CRC Boca Raton, Fl), pp.155-166.

Monge, A., Lathrop, E. J., Gunn, J. R., Shenkin, P. S., and Friesner, R. A. (1995). Computer modeling of protein folding: conformational and energetic analysis of reduced and detailed protein models. J.Mol.Biol 247, 995-1012.

Morris, R.O. et al. (1999). Isolation of a gene encoding a glycosylated cytokinin oxidase from maize. Bioechem. Biophys. Res. Commun. 255, 328-333

Motyka, V., Faiss, M., Strnad, M., Kaminek, M. and Schmuelling, T. (1996). Changes in cytokinin content and cytokinin oxidase activity in response to derepression of ipt gene transcription in transgenic tobacco calli and plants. Plant Physiol. 112, 1035-1043.

Murakami, T., Simonds, W. F., and Spiegel, A. M. (1992). Site-specific antibodies directed against G protein beta and gamma subunits: effects on alpha and beta gamma subunit interaction. Biochemistry 31, 2905-2911.

Olszewski, K. A., Kolinski, A., and Skolnick, J. (1996). Folding simulations and computer redesign of protein A three-helix bundle motifs. Proteins 25, 286-299.

Osborne, B. I., Wirtz, U., and Baker, B. (1995). A system for insertional mutagenesis and chromosomal rearrangement using the Ds transposon and Cre-lox. Plant J. 7, 687-701.

Ostresh, J. M., Blondelle, S. E., Dorner, B., and Houghten, R. A. (1996). Generation and use of nonsupport-bound peptide and peptidomimetic combinatorial libraries. Methods Enzymol. 267, 220-234.

Pabo, C. O. and Suchanek, E. G. (1986). Computer-aided model-building strategies for protein design. Biochemistry 25, 5987-5991.

Paszkowski, J., Shillito, R. D., Saul, M., Mandak, V., and Hohn, T. H. B. P. I. (1984). Direct gene transfer to plants. EMBO J. 3, 2717-2722.

Peralta, E. G., Hellmiss, R., and Ream, W. (1986). Overdrive, a T-DNA transmission enhancer on the A. tumefaciens tumour-inducing plasmid. EMBO J. 5, 1137-1142.

Quirino, B.F., Noh, Y.-S., Himelbau, E., and Amasino, R.M. (2000). Molecular aspects of leaf senescence. Trends in Plant Science 5, 278-282.

Renouf, D. V. and Hounsell, E. F. (1995). Molecular modelling of glycoproteins by homology with non-glycosylated protein domains, computer simulated glycosylation and molecular dynamics. Adv.Exp.Med.Biol 376, 37-45.

Rinaldi, A.C. and Comandini, O. (1999). Cytokinin oxidase strikes again. Trends in Plant Sc. 4, 300.

Rose, R. B., Craik, C. S., Douglas, N. L., and Stroud, R. M. (1996). Three-dimensional structures of HIV-1 and SIV protease product complexes. Biochemistry 35, 12933-12944.

Rutenber, E. E., McPhee, F., Kaplan, A. P., Gallion, S. L., Hogan, J. C., Jr., Craik, C. S., and Stroud, R. M. (1996). A new class of HIV-1 protease inhibitor: the crystallographic structure, inhibition and chemical synthesis of an aminimide peptide isostere. Bioorg.Med.Chem. 4, 1545-1558.

Sambrook, J., Fritsch, E. F., and Maniatis, T. (1989). "Molecular Cloning: A Laboratory Manual." Cold Spring Harbor Laboratory Press.

Schlappi, M., Smith, D., and Fedoroff, N. (1993). TnpA trans-activates methylated maize Suppressor-mutator transposable elements in transgenic tobacco. Genetics 133, 1009-1021.

Shioda, T., Andriole, S., Yahata, T., and Isselbacher, K. J. (2000). A green fluorescent protein-reporter mammalian two-hybrid system with extrachromosomal maintenance of a prey expression plasmid: Application to interaction screening. Proc.Natl.Acad.Sci.U.S.A 97, 5220-5224.

Smulling, T., Rupp, H.M. Frank, M& Schafer, S.. (1999) in Advances in Regulation of Plant Growth and Development, eds. Surnad, M. Pac P. & Beck, E. (Peres, Prague), pp. 85-96.

Tamura, R. N., Cooper, H. M., Collo, G., and Quaranta, V. (1991). Cell type-specific integrin variants with alternative alpha chain cytoplasmic domains. Proc.Natl.Acad.Sci.U.S.A 88, 10183-10187.

Werner, T., Vadau Motyka, Miroslav Strnad, and Thomas Schmülling (2001) Regulation of plant growth by cytokinin. Proc. Nat. Acad. Sci., 58 (18) 10487-10492.

Van Haaren, M. J., Sedee, N. J., Schilperoort, R. A., and Hooykaas, P. J. (1987). Overdrive is a T-region transfer enhancer which stimulates T-strand production in Agrobacterium tumefaciens. Nucleic Acids Res. 15, 8983-8997.

Van Sluys, M. A., Tempe, J., and Fedoroff, N. (1987). Studies on the introduction and mobility of the maize Activator element in Arabidopsis thaliana and Daucus carota. EMBO J. 6, 3881-3889.

Wang, K., Genetello, C., Van Montagu, M., and Zambryski, P. C. (1987). Sequence context of the T-DNA border repeat element determines its relative activity during T-DNA transfer to plant cells. Mol. Gen. Genet. 210, 338-346.

Woulfe, J., Lafortune, L., de Nadai, F., Kitabgi, P., and Beaudet, A. (1994). Post-translational processing of the neurotensin/neuromedin N precursor in the central nervous system of the rat-II. Immunohistochemical localization of maturation products. Neuroscience 60, 167-181.

Zhang, Y. L., Dawe, A. L., Jiang, Y., Becker, J. M., and Naider, F. (1996). A superactive peptidomimetic analog of a farnesylated dodecapeptide yeast pheromone. Biochem.Biophys.Res.Commun. 224, 327-331.

## Claims

1. A method of increasing seed size or weight which comprises increasing the level or activity of a cytokinin oxidase in seeds in a plant.

2. A method of increasing embryo size or weight which comprises increasing the level or activity of a cytokinin oxidase in seeds in a plant, preferably in embryos.

3. A method of increasing cotyledon size which comprises increasing the level or activity of a cytokinin oxidase in cotyledons in a plant.

**4.** A method according to claim 1 for increasing seed size or weight which comprises expression of a nucleic acid selected from the group consisting of:

(a) a nucleic acid comprising a DNA sequence as given in any of SEQ ID NOs: 26, 3, 1, 7, 11, 25, 28, 30, 29, 5, 9, 27, 31, 33 or 34,
(b) a nucleic acid comprising the RNA sequences corresponding to any of SEQ ID NOs: 26, 3, 1, 7, 11, 25, 28, 30, 29, 5, 9, 27, 31, 33 or 34,
(c) a nucleic acid specifically hybridizing to the complement of a nucleic acid as given in any of SEQ ID NOs: 26, 3, 1, 7, 11, 25, 28, 30, 29, 5, 9, 27, 31, 33 or 34,
(d) a nucleic acid encoding a protein with an amino acid sequence comprising the polypeptide as given in SEQ ID NO: 32 and which is at least 70% similar to the amino acid sequence as given in SEQ ID NO: 4,
(e) a nucleic acid encoding a protein with an amino acid sequence which is at least 47% similar to the amino acid sequence as given in SEQ ID NO: 6,
(f) a nucleic acid encoding a protein with an amino acid sequence which is at least 47% similar to the amino acid sequence as given in SEQ ID NO: 10 or 35,
(g) a nucleic acid encoding a protein comprising or consisting of the amino acid sequence as given in any of SEQ ID NOs: 4, 2, 6, 8, 10, 12, 32 or 35,
(h) a nucleic acid which is degenerated to a nucleic acid as given in any of SEQ ID NOs: 26, 3, 1, 7, 11, 25, 28, 30, 29, 5, 9, 27, 33 or 34 or which is degenerated to a nucleic acid as defined in any of (a) to (g) as a result of the genetic code,
(i) a nucleic acid which is diverging from a nucleic acid encoding a protein as given in any of SEQ ID NOs: 4, 2, 6, 8, 10, 12 or 35 or which is diverging from a nucleic acid as defined in any of (a) to (g) due to the differences in codon usage between the organisms,
(j) a nucleic acid encoding a protein as given in SEQ ID NOs: 4, 2, 6, 8, 10, 12 or 35, or a nucleic acid as defined in (a) to (g) which is diverging due to the differences between alleles,
(k) a nucleic acid encoding a functional fragment of a cytokinin oxidase encoded by a nucleic acid as given in any of SEQ ID NOs: 26, 3, 1, 7, 11, 25, 28, 30, 29, 5, 9, 27, 31, 33 or 34, or a functional fragment of a nucleic acid as defined in any of (a) to (j), wherein said fragment has the biological activity of a cytokinin oxidase,
(l) a nucleic acid as defined in any of (a) to (k) **characterized in that** said nucleic acid is DNA, cDNA, genomic DNA or synthetic DNA, or RNA wherein T is replaced by U.

**5.** A method according to claim 2 for increasing embryo size or weight which comprises expression of a nucleic acid selected from the group consisting of.

(a) a nucleic acid comprising a DNA sequence as given in any of SEQ ID NOs: 26, 3, 1, 7, 11, 25, 28, 30, 29, 5, 9, 27, 31, 33 or 34,
(b) a nucleic acid comprising the RNA sequences corresponding to any of SEQ ID NOs: 26, 3, 1, 7, 11, 25, 28, 30, 29, 5, 9, 27, 31, 33 or 34,
(c) a nucleic acid specifically hybridizing to the complement of a nucleic acid as given in any of SEQ ID NOs: 26, 3, 1, 7, 11, 25, 28, 30, 29, 5, 9, 27, 31, 33 or 34,
(d) a nucleic acid encoding a protein with an amino acid sequence comprising the polypeptide as given in SEQ ID NO: 32 and which is at least 70% similar to the amino acid sequence as given in SEQ ID NO: 4,
(e) a nucleic acid encoding a protein with an amino acid sequence which is at least 47% similar to the amino acid sequence as given in SEQ ID NO: 6,
(f) a nucleic acid encoding a protein with an amino acid sequence which is at least 47% similar to the amino acid sequence as given in SEQ ID NO: 10 or 35,
(g) a nucleic acid encoding a protein comprising or consisting of the amino acid sequence as given in any of SEQ ID NOs: 4, 2, 6, 8, 10, 12, 32 or 35,
(h) a nucleic acid which is degenerated to a nucleic acid as given in any of SEQ ID NOs: 26, 3, 1, 7, 11, 25, 28, 30, 29, 5, 9, 27, 33 or 34 or which is degenerated to a nucleic acid as defined in any of (a) to (g) as a result of the genetic code,
(i) a nucleic acid which is diverging from a nucleic acid encoding a protein as given in any of SEQ ID NOs: 4, 2, 6, 8, 10, 12 or 35 or which is diverging from a nucleic acid as defined in any of (a) to (g) due to the differences in codon usage between the organisms,
(j) a nucleic acid encoding a protein as given in SEQ ID NOs: 4, 2, 6, 8, 10, 12 or 35, or a nucleic acid as defined in (a) to (g) which is diverging due to the differences between alleles,

(k) a nucleic acid encoding a functional fragment of a cytokinin oxidase encoded by a nucleic acid as given in any of SEQ ID NOs: 26, 3, 1, 7, 11, 25, 28, 30, 29, 5, 9, 27, 31, 33 or 34, or a functional fragment of a nucleic acid as defined in any of (a) to (j), wherein said fragment has the biological activity of a cytokinin oxidase,
(l) a nucleic acid as defined in any of (a) to (k) **characterized in that** said nucleic acid is DNA, cDNA, genomic DNA or synthetic DNA, or RNA wherein T is replaced by U.

**6.** A method according to claim 3 for increasing cotyledon size which comprises expression of a nucleic acid selected from the group consisting of:

(a) a nucleic acid comprising a DNA sequence as given in any of SEQ ID NOs: 26, 3, 1, 7, 11, 25, 28, 30, 29, 5, 9, 27, 31, 33 or 34,
(b) a nucleic acid comprising the RNA sequences corresponding to any of SEQ ID NOs: 26, 3, 1, 7, 11, 25, 28, 30, 29, 5, 9, 27, 31, 33 or 34,
(c) a nucleic acid specifically hybridizing to the complement of a nucleic acid as given in any of SEQ ID NOs: 26, 3, 1, 7, 11, 25, 28, 30, 29, 5, 9, 27, 31, 33 or 34,
(d) a nucleic acid encoding a protein with an amino acid sequence comprising the polypeptide as given in SEQ ID NO: 32 and which is at least 70% similar to the amino acid sequence as given in SEQ ID NO: 4,
(e) a nucleic acid encoding a protein with an amino acid sequence which is at least 47% similar to the amino acid sequence as given in SEQ ID NO: 6,
(f) a nucleic acid encoding a protein with an amino acid sequence which is at least 47% similar to the amino acid sequence as given in SEQ ID NO: 10 or 35,
(g) a nucleic acid encoding a protein comprising or consisting of the amino acid sequence as given in any of SEQ ID NOs: 4, 2, 6, 8, 10, 12, 32 or 35,
(h) a nucleic acid which is degenerated to a nucleic acid as given in any of SEQ ID NOs: 26, 3, 1, 7, 11, 25, 28, 30, 29, 5, 9, 27, 33 or 34 or which is degenerated to a nucleic acid as defined in any of (a) to (g) as a result of the genetic code,
(i) a nucleic acid which is diverging from a nucleic acid encoding a protein as given in any of SEQ ID NOs: 4, 2, 6, 8, 10, 12 or 35 or which is diverging from a nucleic acid as defined in any of (a) to (g) due to the differences in codon usage between the organisms,
(j) a nucleic acid encoding a protein as given in SEQ ID NOs: 4, 2, 6, 8, 10, 12 or 35, or a nucleic acid as defined in (a) to (g) which is diverging due to the differences between alleles,
(k) a nucleic acid encoding a functional fragment of a cytokinin oxidase encoded by a nucleic acid as given in any of SEQ ID NOs: 26, 3, 1, 7, 11, 25, 28, 30, 29, 5, 9, 27, 31, 33 or 34, or a functional fragment of a nucleic acid as defined in any of (a) to (j), wherein said fragment has the biological activity of a cytokinin oxidase,
(l) a nucleic acid as defined in any of (a) to (k) **characterized in that** said nucleic acid is DNA, cDNA, genomic DNA or synthetic DNA, or RNA wherein T is replaced by U.

**7.** The method of claim 4 wherein the nucleic acid is under control of a promoter that controls expression preferentially in seeds.

**8.** The method of claim 5 wherein the nucleic acid is under the control of a promoter that controls expression preferentially in embryos.

**9.** The method of claim 6 wherein the nucleic acid is under the control of a promoter that controls expression preferentially in cotyledons.

**10.** The method of claim 7 wherein the promoter is further specific to the endosperm or aleurone.

**11.** The method of claim 4 wherein said method leads to an increase in growth of seedlings or an increase in early vigor.

**12.** The method of claim 5 wherein said method leads to an increase in growth of seedlings or an increase in early vigor.

**13.** The method of claim 6 wherein said method leads to an increase in growth of seedlings or an increase in early vigor.

**14.** The method of claim 11 wherein the increase in growth of seedlings or early vigor is associated with increased stress tolerance.

**15.** The method of claim 12 wherein the increase in growth of seedlings or early vigor is associated with increased stress tolerance.

**16.** The method of claim 13 wherein the increase in growth of seedlings or early vigor is associated with increased stress tolerance.

**17.** The method according to claim 4, which comprises expression in a plant of a nucleic acid as set forth in any of SEQ ID NOs:1, 5, 25, or 27 or an ortholog of said nucleic acid, wherein said ortholog is specific to the species of the plant.

**18.** The method according to claim 5, which comprises expression in a plant of a nucleic acid as set forth in any of SEQ ID NOs:1, 5, 25, or 27 or an ortholog of said nucleic acid, wherein said ortholog is specific to the species of the plant.

**19.** The method according to claim 6, which comprises expression in a plant of a nucleic acid as set forth in any of SEQ ID NOs:1, 5, 25, or 27 or an ortholog of said nucleic acid, wherein said ortholog is specific to the species of the plant.

**20.** The method of claim 17 wherein the nucleic acid is under control of a promoter that controls expression preferentially in seeds.

**21.** The method of claim 18 wherein the nucleic acid is under the control of a promoter that controls expression preferentially in embryos.

**22.** The method of claim 19 wherein the nucleic acid is under the control of a promoter that controls expression preferentially in cotyledons.

**23.** The method of claim 20 wherein the promoter is further specific to the endosperm or aleurone.

**24.** The method of claim 17 wherein said method leads to an increase in growth of seedlings or an increase in early vigor.

**25.** The method of claim 18 wherein said method leads to an increase in growth of seedlings or an increase in early vigor.

**26.** The method of claim 19 wherein said method leads to an increase in growth of seedlings or an increase in early vigor.

**27.** The method of claim 24 wherein the increase in growth of seedlings or early vigor is associated with increased stress tolerance.

**28.** The method of claim 25 wherein the increase in growth of seedlings or early vigor is associated with increased stress tolerance.

**29.** The method of claim 26 wherein the increase in growth of seedlings or early vigor is associated with increased stress tolerance.

**Patentansprüche**

**1.** Eine Methode zur Steigerung der Samengröße oder der Samengewichts, die die Steigerung des Levels oder der Aktivität einer Cytokinin-Oxidase in den Samen einer Pflanze umfasst.

**2.** Eine Methode zur Steigerung Embryogröße oder Gewicht, die die Steigerung des Levels oder der Aktivität einer Cytokinin-Oxidase in den Samen einer Pflanze umfasst, vorzugsweise in Embryonen.

**3.** Eine Methode zur Steigerung der Keimblattgröße, die die Steigerung des Levels oder der Aktivität einer Cytokinin-Oxidase in den Keimblättern einer Pflanze umfasst.

**4.** Eine Methode nach Anspruch 1 zur Steigerung der Samengröße oder des Samengewichts, die die Expression einer Nukleinsäure umfasst, die ausgewählt wurde aus der Gruppe bestehend aus:

(a) einer Nukleinsäure, die eine DNA-Sequenz umfasst, wie in einer der folgenden SEQ-ID-NR. gegeben: 26, 3, 1, 7, 11, 25, 28, 30, 29, 5, 9, 27, 31, 33 oder 34,
(b) einer Nukleinsäure, die die RNA-Sequenzen umfasst, die einer der folgenden SEQ-ID-NR. entsprechen:

26, 3, 1, 7, 11, 25, 28, 30, 29, 5, 9, 27, 31, 33 oder 34,
(c) einer Nukleinsäure, die spezifisch an das Komplement von einer Nukleinsäure hybridisiert, wie in einer der folgenden SEQ-ID-NR. gegeben: 26, 3, 1, 7, 11, 25, 28, 30, 29, 5, 9, 27, 31, 33 oder 34,
(d) einer Nukleinsäure, die ein Protein mit einer Aminosäuresequenz kodiert, welche das Polypeptid umfasst, wie in folgender SEQ-ID-NR. gegeben: 32 und die zu mindesten 70% der Aminosäuresequenz ähnlich ist, wie in folgender SEQ-ID-NR. gegeben: 4,
(e) einer Nukleinsäure, die ein Protein mit einer Aminosäuresequenz kodiert, die zu mindesten 47% der Aminosäuresequenz ähnlich ist, wie in folgender SEQ-ID-NR. gegeben: 6,
(f) einer Nukleinsäure, die ein Protein mit einer Aminosäuresequenz kodiert, die zu mindesten 47% der Aminosäuresequenz ähnlich ist, wie in folgender SEQ-ID-NR. gegeben: 10 oder 35,
(g) einer Nukleinsäure, die ein Protein kodiert, das die Aminosäuresequenz umfasst oder aus ihr besteht, wie in einer der folgenden SEQ-ID-NR. gegeben: 4, 2, 6, 8,10, 12, 32 oder 35,
(h) einer Nukleinsäure, die aufgrund des genetischen Codes, zu einer Nukleinsäure degeneriert wird, wie in einer der folgenden SEQ-ID-NR. gegeben: 26, 3, 1, 7, 11, 25, 28, 30, 29, 5, 9, 27, 33 oder 34 oder die zu einer Nukleinsäure degeneriert wird, wie in (a) bis (g) definiert wird,
(i) einer Nukleinsäure, die aufgrund der unterschiedlichen Codon-Usage zwischen den Organismen, von einer Nukleinsäure abweicht, die ein Protein kodiert, wie in einer der folgenden SEQ-ID-NR. gegeben: 4, 2, 6, 8, 10, 12 oder 35 oder die von einer Nukleinsäure abweicht, wie in irgendeinem der Punkte (a) bis (g) definiert,
(j) einer Nukleinsäure, die ein Protein kodiert, wie in folgender SEQ-ID-NR. gegeben: 4, 2, 6, 8, 10, 12 oder 35, oder einer Nukleinsäure, wie in (a) bis (g) definiert, die aufgrund der Unterschiede zwischen den Allelen abweicht,
(k) einer Nukleinsäure, die ein Funktionelles Fragment einer Cytokinin-Oxidase kodiert, die durch eine Nukleinsäure kodiert wird, wie in einer der folgenden SEQ-ID-NR. gegeben: 26, 3, 1, 7, 11, 25, 28, 30, 29, 5, 9, 27, 31, 33 oder 34, oder ein Funktionelles Fragment einer Nukleinsäure, wie in irgendeinem der Punkte (a) bis (j) definiert, worin besagtes Fragment die biologische Aktivität einer Cytokinin-Oxidase hat,
(l) einer Nukleinsäure, wie in irgendeinem der Punkte (a) bis (k) definiert, dadurch charakterisiert, dass besagte Nukleinsäure DNA, cDNA, genomische DNA oder synthetische DNA oder RNA ist, worin T durch U ersetzt wird.

**5.** Eine Methode nach Anspruch 2 zur Steigerung der Embryogröße oder des Embryogewichts, die die Expression einer Nukleinsäure umfasst, die aus der Gruppe gewählt wird, die besteht aus:

(a) einer Nukleinsäure, die eine DNA-Sequenz umfasst, wie in einer der folgenden SEQ-ID-NR. gegeben: 26, 3, 1, 7, 11, 25, 28, 30, 29, 5, 9, 27, 31, 33 oder 34,
(b) einer Nukleinsäure, die die RNA-Sequenzen umfasst, die einer der folgenden SEQ-ID-NR. entsprechen: 26, 3, 1, 7, 11, 25, 28, 30, 29, 5, 9, 27, 31, 33 oder 34,
(c) einer Nukleinsäure, die spezifisch an das Komplement von einer Nukleinsäure hybridisiert, wie in einer der folgenden SEQ-ID-NR. gegeben: 26, 3, 1, 7, 11, 25, 28, 30, 29, 5, 9, 27, 31, 33 oder 34,
(d) einer Nukleinsäure, die ein Protein mit einer Aminosäuresequenz kodiert, welche das Polypeptid umfasst, wie in folgender SEQ-ID-NR. gegeben: 32 und die zu mindesten 70% der Aminosäuresequenz ähnlich ist, wie in folgender SEQ-ID-NR. gegeben: 4,
(e) einer Nukleinsäure, die ein Protein mit einer Aminosäuresequenz kodiert, die zu mindestens 47% der Aminosäuresequenz ähnlich ist, wie in folgender SEQ-ID-NR. gegeben: 6,
(f) einer Nukleinsäure, die ein Protein mit einer Aminosäuresequenz kodiert, die zu mindestens 47% der Aminosäuresequenz ähnlich ist, wie in folgender SEQ-ID-NR. gegeben: 10 oder 35,
(g) einer Nukleinsäure, die ein Protein kodiert, das die Aminosäuresequenz umfasst oder aus ihr besteht, wie in einer der folgenden SEQ-ID-NR. gegeben: 4, 2, 6, 8, 10, 12, 32 oder 35,
(h) einer Nukleinsäure, die aufgrund des genetischen Codes, zu einer Nukleinsäure degeneriert wird, wie in einer der folgenden SEQ-ID-NR. gegeben: 26, 3, 1, 7, 11, 25, 28, 30, 29, 5, 9, 27, 33 oder 34 oder die zu einer Nukleinsäure degeneriert wird, wie in irgendeinem der Punkte (a) bis (g) definiert,
(i) einer Nukleinsäure, die aufgrund der unterschiedlichen Codon-Usage zwischen den Organismen von einer Nukleinsäure abweicht, die ein Protein kodiert, wie in einer der folgenden SEQ-ID-NR. gegeben: 4, 2, 6, 8, 10, 12 oder 35 oder die von einer Nukleinsäure abweicht, wie in irgendeinem der Punkte (a) bis (g) definiert,
(j) einer Nukleinsäure, die ein Protein kodiert, wie in folgender SEQ-ID-NR. gegeben: 4, 2, 6, 8, 10, 12 oder 35, oder einer Nukleinsäure, wie in (a) bis (g) definiert, die Aufgrund der Unterschiede zwischen den Allelen abweicht,
(k) einer Nukleinsäure, die ein Funktionelles Fragment einer Cytokinin-Oxidase kodiert, die durch eine Nukleinsäure kodiert wird, wie in einer der folgenden SEQ-ID-NR. gegeben: 26, 3, 1, 7, 11, 25, 28, 30, 29, 5, 9, 27, 31, 33 oder 34, oder ein Funktionelles Fragment einer Nukleinsäure, wie in irgendeinem der Punkte (a) bis (j) definiert, worin besagtes Fragment die biologische Aktivität einer Cytokinin-Oxidase hat,
(l) einer Nukleinsäure, wie in irgendeinem der Punkte (a) bis (k) definiert, dadurch charakterisiert, dass besagte Nukleinsäure DNA, cDNA, genomische DNA oder synthetische DNA oder RNA ist, worin T durch U ersetzt wird.

**6.** Eine Methode nach Anspruch 3 zur Steigerung der Keimblattgröße, die die Expression einer Nukleinsäure umfasst, die aus der Gruppe gewählt wird, die besteht aus:

(a) einer Nukleinsäure, die eine DNA-Sequenz umfasst, wie in einer der folgenden SEQ-ID-NR. gegeben: 26, 3, 1, 7, 11, 25, 28, 30, 29, 5, 9, 27, 31, 33 oder 34,
(b) einer Nukleinsäure, die die RNA-Sequenzen umfasst, die einer der folgenden SEQ-ID-NR. entsprechen: 26, 3, 1, 7, 11, 25, 28, 30, 29, 5, 9, 27, 31, 33 oder 34,
(c) einer Nukleinsäure, die spezifisch an das Komplement von einer Nukleinsäure hybridisiert, wie in einer der folgenden SEQ-ID-NR. gegeben: 26, 3, 1, 7, 11, 25, 28, 30, 29, 5, 9, 27, 31, 33 oder 34,
(d) einer Nukleinsäure, die ein Protein mit einer Aminosäuresequenz kodiert, welche das Polypeptid umfasst, wie in folgender SEQ-ID-NR. gegeben: 32 und die zu mindesten 70% der Aminosäuresequenz ähnlich ist, wie in folgender SEQ-ID-NR. gegeben: 4,
(e) einer Nukleinsäure, die ein Protein mit einer Aminosäuresequenz kodiert, die zu mindestens 47% der Aminosäuresequenz ähnlich ist, wie in folgender SEQ-ID-NR. gegeben: 6,
(f) einer Nukleinsäure, die ein Protein mit einer Aminosäuresequenz kodiert, die zu mindestens 47% der Aminosäuresequenz ähnlich ist, wie in folgender SEQ-ID-NR. gegeben: 10 oder 35,
(g) einer Nukleinsäure, die ein Protein kodiert, das die Aminosäuresequenz umfasst oder aus ihr besteht, wie in einer der folgenden SEQ-ID-NR. gegeben: 4, 2, 6, 8, 10, 12, 32 oder 35,
(h) einer Nukleinsäure, die aufgrund des genetischen Codes, zu einer Nukleinsäure degeneriert wird, wie in einer der folgenden SEQ-ID-NR. gegeben: 26, 3, 1, 7, 11, 25, 28, 30, 29, 5, 9, 27, 33 oder 34 oder die zu einer Nukleinsäure degeneriert wird, wie in irgendeinem der Punkte (a) bis (g) definiert wird,
(i) einer Nukleinsäure, die aufgrund der unterschiedlichen Codon-Usage zwischen den Organismen von einer Nukleinsäure abweicht, die ein Protein kodiert, wie in einer der folgenden SEQ-ID-NR. gegeben: 4, 2, 6, 8, 10,12 oder 35 oder die von einer Nukleinsäure abweicht, wie in irgendeinem der Punkte (a) bis (g) definiert,
(j) einer Nukleinsäure, die ein Protein kodiert, wie in folgender SEQ-ID-NR. gegeben: 4, 2, 6, 8, 10, 12 oder 35, oder einer Nukleinsäure, wie in (a) bis (g) definiert, die aufgrund der Unterschiede zwischen den Allelen abweicht,
(k) einer Nukleinsäure, die ein Funktionelles Fragment einer Cytokinin-Oxidase kodiert, die durch eine Nukleinsäure kodiert wird, wie in einer der folgenden SEQ-ID-NR. gegeben: 26, 3, 1, 7, 11, 25, 28, 30, 29, 5, 9, 27, 31, 33 oder 34, oder ein Funktionelles Fragment einer Nukleinsäure, wie in irgendeinem der Punkte (a) bis (j) definiert, worin besagtes Fragment die biologische Aktivität einer Cytokinin-Oxidase hat,
(l) einer Nukleinsäure, wie in irgendeinem der Punkte (a) bis (k) definiert, dadurch charakterisiert, dass besagte Nukleinsäure DNA, cDNA, genomische DNA oder synthetische DNA oder RNA ist, worin T durch U ersetzt wird.

**7.** Die Methode nach Anspruch 4, worin die Nukleinsäure sich unter der Kontrolle eines Promotors befindet, der die Expression vorzugsweise in Samen kontrolliert.

**8.** Die Methode nach Anspruch 5, worin die Nukleinsäure sich unter der Kontrolle eines Promotors befindet, der die Expression vorzugsweise in Embryonen kontrolliert.

**9.** Die Methode nach Anspruch 6, worin die Nukleinsäure sich unter der Kontrolle eines Promotors befindet, der die Expression vorzugsweise in Keimblättern kontrolliert.

**10.** Die Methode nach Anspruch 7, worin der Promotor außerdem spezifisch für das Endosperm oder Aleuron ist.

**11.** Die Methode nach Anspruch 4, worin besagte Methode zu einer Steigerung des Wachstums von Sätzlingen oder einer Steigerung der frühen Vitalität führt.

**12.** Die Methode nach Anspruch 5, worin besagte Methode zu einer Steigerung des Wachstums von Sätzlingen oder einer Steigerung der frühen Vitalität führt.

**13.** Die Methode nach Anspruch 6, worin besagte Methode zu einer Steigerung des Wachstums von Sätzlingen oder einer Steigerung der frühen Vitalität führt.

**14.** Die Methode nach Anspruch 11, worin die Steigerung des Wachstums von Sätzlingen oder frühe Vitalität mit erhöhter Stresstoleranz verbunden ist.

**15.** Die Methode nach Anspruch 12, worin die Steigerung des Wachstums von Sätzlingen oder frühe Vitalität mit erhöhter Stresstoleranz verbunden ist.

**16.** Die Methode nach Anspruch 13, worin die Steigerung des Wachstums von Sätzlingen oder frühe Vitalität mit erhöhter Stresstoleranz verbunden ist.

**17.** Die Methode gemäß Anspruch 4, die die Expression einer Nukleinsäure in einer Pflanze umfasst, wie in irgendeiner der SEQ-ID-NR. 1, 5, 25, oder 27 dargelegt oder einen Ortholog besagter Nukleinsäure, worin besagter Ortholog für die Pflanzenspezies spezifisch ist.

**18.** Die Methode gemäß Anspruch 5, die die Expression einer Nukleinsäure in einer Pflanze umfasst, wie in irgendeiner der SEQ-ID-NR. 1, 5, 25, oder 27 dargelegt oder einen Ortholog besagter Nukleinsäure, worin besagter Ortholog für die Pflanzenspezies spezifisch ist.

**19.** Die Methode gemäß Anspruch 6, die die Expression einer Nukleinsäure in einer Pflanze umfasst, wie in irgendeiner der SEQ-ID-NR. 1, 5, 25, oder 27 dargelegt oder einen Ortholog besagter Nukleinsäure, worin besagter Ortholog für die Pflanzenspezies spezifisch ist.

**20.** Die Methode nach Anspruch 17, worin die Nukleinsäure sich unter der Kontrolle eines Promotors befindet, der die Expression vorzugsweise in Samen kontrolliert.

**21.** Die Methode nach Anspruch 18, worin die Nukleinsäure sich unter der Kontrolle eines Promotors befindet, der die Expression vorzugsweise in Embryonen kontrolliert.

**22.** Die Methode nach Anspruch 19, worin die Nukleinsäure sich unter der Kontrolle eines Promotors befindet, der die Expression vorzugsweise in Keimblättern kontrolliert.

**23.** Die Methode nach Anspruch 20, worin der Promotor außerdem spezifisch für das Endosperm oder Aleuron ist.

**24.** Die Methode nach Anspruch 17 worin besagte Methode zu einer Steigerung des Wachstums von Sätzlingen oder einer Steigerung der frühen Vitalität führt.

**25.** Die Methode nach Anspruch 18 worin besagte Methode zu einer Steigerung des Wachstums von Sätzlingen oder einer Steigerung der frühen Vitalität führt.

**26.** Die Methode nach Anspruch 19, worin besagte Methode zu einer Steigerung des Wachstums von Sätzlingen oder einer Steigerung der frühen Vitalität führt.

**27.** Die Methode nach Anspruch 24, worin die Steigerung des Wachstums von Sätzlingen oder frühe Vitalität mit erhöhter Stresstoleranz verbunden ist.

**28.** Die Methode nach Anspruch 25, worin die Steigerung des Wachstums von Sätzlingen oder frühe Vitalität mit erhöhter Stresstoleranz verbunden ist.

**29.** Die Methode nach Anspruch 26, worin die Steigerung des Wachstums von Sätzlingen oder frühe Vitalität mit erhöhter Stresstoleranz verbunden ist.

## Revendications

**1.** Un procédé de l’accroissement de la taille ou du poids de la semence comprenant l’augmentation du niveau de cytokinine oxydase ou son activité dans les semences d’une plante.

**2.** Un procédé de l’accroissement de la taille ou du poids d’un embryon comprenant l’augmentation du niveau de cytokinine oxydase ou son activité dans les semences d’une plante, de préférence dans les embryons.

**3.** Un procédé de l’accroissement de la taille ou du poids du cotylédon comprenant l’augmentation du niveau de cytokinine oxydase ou son activité dans les cotylédons d’une plante.

**4.** Un procédé selon le revendication 1 pour l'accroissement de la taille ou du poids de la semence, et comprenant l'expression d'un acide nucléique sélectionné dans le groupe constitué par :

(a) un acide nucléique comprenant une séquence d'ADN telle que donnée dans l'une des SEQ ID Nos: 26, 3, 1, 7, 11, 25, 28, 30, 29, 5, 9, 27, 31, 33 ou 34,
(b) un acide nucléique comprenant les séquences d'ARN correspondant à l'une des SEQ ID Nos: 26, 3, 1, 7, 11, 25, 28, 30, 29, 5, 9, 27, 31, 33 ou 34,
(c) un acide nucléique s'hybridant spécifiquement au complément de l'une des SEQ ID Nos: 26, 3, 1, 7, 11, 25, 28, 30, 29, 5, 9, 27, 31, 33 ou 34,
(d) un acide nucléique codant une protéine avec une séquence d'acides aminés comprenant le polypeptide tel que donné dans SEQ ID NO: 32 et ayant au moins 70 % similaire à la séquence d'acides aminés donnée dans SEQ ID NO: 4,
(e) un acide nucléique codant pour une protéine comprenant une séquence d'acides aminés d'au moins 47 % similaire à la séquence d'acides aminés telle que donnée dans SEQ ID NO: 6,
(f) un acide nucléique codant une protéine comprenant une séquence d'acides aminés d'au moins 47 % similaire à la séquence d'acides aminés telle que donnée dans SEQ ID NO: 10 ou 35,
(g) un acide nucléique codant une protéine comprenant ou constitué par la séquence d'acides aminés telle que donnée dans l'une des SEQ ID Nos: 4, 2, 6, 8,10, 12, 32 ou 35,
(h) un acide nucléique dégénéré à un acide nucléique comme dans l'une des SEQ ID Nos: 26, 3, 1, 7, 11, 25, 28, 30, 29, 5, 9, 27, 33 ou 34, ou qui est dégénéré à un acide nucléique tel que défini dans l'un des points (a) à (g) comme résultat du code génétique,
(i) un acide nucléique qui diverge d'un acide nucléique codant pour une protéine telle que donnée dans l'une des SEQ ID Nos: 4, 2, 6, 8, 10, 12 ou 35, ou qui diverge d'un acide nucléique tel que défini dans l'un des points (a) à (g) en raison des différences d'usages de codons entre les organismes,
(j) un acide nucléique codant une protéine telle que définie dans l'une des SEQ ID Nos: 4, 2, 6, 8, 10, 12 ou 35, ou un acide nucléique tel que défini dans les points (a) à (g) qui diverge en raison de différences entre les allèles,
(k) un acide nucléique codant pour un fragment fonctionnel d'une cytokinine oxydase codée par un acide nucléique tel que donné dans l'une des SEQ ID Nos: 26, 3, 1, 7, 11, 25, 28, 30, 29, 5, 9, 27, 31, 33 ou 34, ou un fragment fonctionnel d'un acide nucléique tel que défini dans l'un des points (a) à (j), ledit fragment ayant l'activité biologique d'une cytokinine oxydase,
(l) un acide nucléique tel que défini dans l'un des points (a) à (k) où ledit acide nucléique est l'ADN, l'ADNc, l'ADN génomique ou l'ADN synthétique, ou l'ARN où T est remplacé par U.

**5.** Un procédé selon le revendication 2 pour augmenter la taille ou le poids de l'embryon, comprenant l'expression d'un acide nucléique sélectionné dans le groupe constitué par :

(a) un acide nucléique comprenant une séquence d'ADN telle que dans l'une des SEQ ID Nos: 26, 3, 1, 7, 11, 25, 28, 30, 29, 5, 9, 27, 31, 33 ou 34,
(b) un acide nucléique comprenant les séquences d'ARN correspondant à l'une des SEQ ID Nos: 26, 3, 1, 7, 11, 25, 28, 30, 29, 5, 9, 27, 31, 33 ou 34,
(c) un acide nucléique s'hybridant spécifiquement au complément d'un acide aminé de l'une des SEQ ID Nos: 26, 3, 1, 7, 11, 25, 28, 30, 29, 5, 9, 27, 31, 33 ou 34,
(d) un acide nucléique codant une protéine avec une séquence d'acides aminés comprenant le polypeptide comme donné dans la SEQ ID NO: 32 et ayant au moins de 70 % similaire à la séquence d'acides aminés donnée dans SEQ ID NO: 4,
(e) un acide nucléique codant une protéine comprenant une séquence d'acides aminés d'au moins 47 % similaire à la séquence d'acides aminés donnée dans SEQ ID NO: 6,
(f) un acide nucléique codant une protéine comprenant une séquence d'acides aminés d'au moins 47 % similaire à la séquence d'acides aminés telle que donnée dans SEQ ID NO: 10 ou 35,
(g) un acide nucléique codant une protéine comprenant ou consistant en la séquence d'acides aminés telle que donnée dans l'une des SEQ ID Nos: 4, 2, 6, 8, 10, 12, 32 ou 35,
(h) un acide nucléique dégénéré à un acide nucléique tel que donné dans l'une des SEQ ID Nos: 26, 3, 1, 7, 11, 25, 28, 30, 29, 5, 9, 27, 33 ou 34, ou qui est dégénéré à un acide nucléique comme défini dans l'un des points (a) à (g) en tant que résultat du code génétique,
(i) un acide nucléique qui diverge d'un acide nucléique codant une protéine telle que donnée dans l'une des SEQ ID Nos: 4, 2, 6, 8, 10, 12 ou 35, ou qui diverge d'un acide nucléique tel que donné dans l'un des points (a) à (g) en raison des différences d'usages de codons entre les organismes,

(j) un acide nucléique codant une protéine telle que donnée dans l'une des SEQ ID Nos: 4, 2, 6, 8, 10, 12 ou 35, ou un acide nucléique tel que défini dans les points (a) à (g) qui diverge en raison de différences entre les allèles,
(k) un acide nucléique codant pour un fragment fonctionnel d'une cytokinine oxydase codée par un acide nucléique tel que donné dans l'une des SEQ ID Nos: 26, 3, 1, 7, 11, 25, 28, 30, 29, 5, 9, 27, 31, 33 ou 34, ou un fragment fonctionnel d'un acide nucléique tel que défini dans l'un des points (a) à (j), ledit fragment ayant l'activité biologique d'une cytokinine oxydase,
(l) un acide nucléique tel que défini dans l'un des points (a) à (k) où ledit acide nucléique est l'ADN, l'ADNc, l'ADN génomique ou l'ADN synthétique, ou l'ARN où T est remplacé par U.

**6.** Un procédé selon le revendication 3 pour l'augmentation de la taille du cotylédon comprenant l'expression d'un acide nucléique sélectionné à partir du groupe constitué par :

(a) un acide nucléique comprenant une séquence d'ADN telle que donnée dans l'une des SEQ ID Nos: 26, 3, 1, 7, 11, 25, 28, 30, 29, 5, 9, 27, 31, 33 ou 34,
(b) un acide nucléique comprenant les séquences d'ARN correspondant à l'une des SEQ ID Nos: 26, 3, 1, 7, 11, 25, 28, 30, 29, 5, 9, 27, 31, 33 ou 34,
(c) un acide nucléique s'hybridant spécifiquement au complément d'un acide aminé de l'une des SEQ ID Nos: 26, 3, 1, 7, 11, 25, 28, 30, 29, 5, 9, 27, 31, 33 ou 34,
(d) un acide nucléique codant une protéine avec une séquence d'acides aminés comprenant le polypeptide de SEQ ID NO: 32 et ayant au moins de 70 % similaire à la séquence d'acides aminés de SEQ ID NO: 4,
(e) un acide nucléique codant une protéine comprenant une séquence d'acides aminés d'au moins 47 % similaire à la séquence d'acides aminés telle que donnée dans SEQ ID NO: 6,
(f) un acide nucléique codant pour une protéine comprenant une séquence d'acides aminés d'au moins 47 % similaire à la séquence d'acides aminés telle que donnée dans SEQ ID NO: 10 ou 35,
(g) un acide nucléique codant pour une protéine comprenant ou constituée par la séquence d'acides aminés telle que donnée dans l'une des SEQ ID Nos: 4, 2, 6, 8, 10, 12, 32 ou 35,
(h) un acide nucléique dégénéré à un acide nucléique telle que donnée dans l'une des SEQ ID Nos: 26, 3, 1, 7, 11, 25, 28, 30, 29, 5, 9, 27, 33 ou 34 ou qui est dégénéré à un acide nucléique comme défini dans l'un des points (a) à (g) en tant que résultat du code génétique,
(i) un acide nucléique qui diverge d'un acide nucléique codant une protéine telle que donnée dans l'une des SEQ ID Nos: 4, 2, 6, 8, 10,12 ou 35 ou qui diverge d'un acide nucléique tel que défini dans l'un des points (a) à (g) en raison des différences d'usages de codons entre les organismes,
(j) un acide nucléique codant une protéine telle que définie dans l'une des SEQ ID Nos: 4, 2, 6, 8, 10, 12 ou 35 ou un acide nucléique tel que défini dans les points (a) à (g) qui diverge en raison de différences entre les allèles,
(k) un acide nucléique codant pour un fragment fonctionnel d'une cytokinine oxydase codée par un acide nucléique tel que proposé dans l'une des SEQ ID Nos: 26, 3, 1, 7, 11, 25, 28, 30, 29, 5, 9, 27, 31, 33 ou 34, ou un fragment fonctionnel d'un acide nucléique tel que défini dans l'un des points (a) à (j), ledit fragment ayant l'activité biologique d'une cytokinine oxydase,
(l) un acide nucléique tel que défini dans l'un des points (a) à (k) où ledit acide nucléique est l'ADN, l'ADNc, l'ADN génomique ou l'ADN synthétique, ou l'ARN où T est remplacé par U.

**7.** Le procédé du revendication 4 où l'acide nucléique est sous le contrôle d'un promoteur contrôlant l'expression de préférence dans les semences.

**8.** Le procédé du revendication 5 où l'acide nucléique est sous le contrôle d'un promoteur contrôlant l'expression de préférence dans les embryons.

**9.** Le procédé du revendication 6 où l'acide nucléique est sous le contrôle d'un promoteur contrôlant l'expression de préférence dans les cotylédons.

**10.** Le procédé du revendication 7, dans lequel le promoteur est en plus spécifique à l'endosperme ou l'aleurone.

**11.** Le procédé du revendication 4, dans lequel ledit procédé débouche sur une augmentation de croissance des semis ou une augmentation de la vigueur précoce.

**12.** Le procédé du revendication 5, dans lequel ledit procédé débouche sur une augmentation de croissance des semis ou une augmentation de la vigueur précoce.

**13.** Le procédé du revendication 6, dans lequel ledit procédé débouche sur une augmentation de croissance des semis ou une augmentation de la vigueur précoce.

**14.** Le procédé du revendication 11, dans lequel l'augmentation de croissance des semis ou l'augmentation de la vigueur précoce est doublée d'une tolérance au stress accrue.

**15.** Le procédé du revendication 12, dans lequel l'augmentation de croissance des semis ou l'augmentation de la vigueur précoce est doublée d'une tolérance au stress accrue.

**16.** Le procédé du revendication 13, dans lequel l'augmentation de croissance des semis ou l'augmentation de la vigueur précoce est doublée d'une tolérance au stress accrue.

**17.** Le procédé du revendication 4, lequel comprend l'expression dans une plante d'un acide nucléique comme proposé dans l'une des SEQ ID Nos: 1, 5, 25 ou 27 ou bien un orthologue dudit acide nucléique où cet orthologue est spécifique aux espèces de la plante.

**18.** Le procédé du revendication 5, qui comprend l'expression dans une plante d'un acide nucléique tel que proposé dans l'une des SEQ ID Nos: 1, 5, 25 ou 27, ou un orthologue dudit acide nucléique où cet orthologue est spécifique aux espèces de la plante.

**19.** Le procédé du revendication 6, qui comprend l'expression dans une plante d'un acide nucléique tel que proposé dans l'une des SEQ ID Nos: 1, 5, 25 ou 27, ou un orthologue dudit acide nucléique, où cet orthologue est spécifique aux espèces de la plante.

**20.** Le procédé du revendication 17 où l'acide nucléique est sous le contrôle d'un promoteur contrôlant l'expression de préférence dans les semences.

**21.** Le procédé du revendication 18 où l'acide nucléique est sous le contrôle d'un promoteur contrôlant l'expression de préférence dans les embryons.

**22.** Le procédé du revendication 19 où l'acide nucléique est sous le contrôle d'un promoteur contrôlant l'expression de préférence dans les cotylédons.

**23.** Le procédé du revendication 20, dans lequel le promoteur est en plus spécifique à l'endosperme ou l'aleurone.

**24.** Le procédé du revendication 17, dans lequel ledit procédé débouche sur une augmentation de croissance des semis ou une augmentation de la vigueur précoce.

**25.** Le procédé du revendication 18, dans lequel ledit procédé débouche sur une augmentation de croissance des semis ou une augmentation de la vigueur précoce.

**26.** Le procédé du revendication 19, dans lequel ledit procédé débouche sur une augmentation de croissance des semis ou une augmentation de la vigueur précoce.

**27.** Le procédé du revendication 24, dans lequel l'augmentation de croissance des semis ou l'augmentation de la vigueur précoce est doublée d'une tolérance au stress accrue.

**28.** Le procédé du revendication 25, dans lequel l'augmentation de croissance des semis ou l'augmentation de la vigueur précoce est doublée d'une tolérance au stress accrue.

**29.** Le procédé du revendication 26, dans lequel l'augmentation de croissance des semis ou l'augmentation de la vigueur précoce est doublée d'une tolérance au stress accrue.

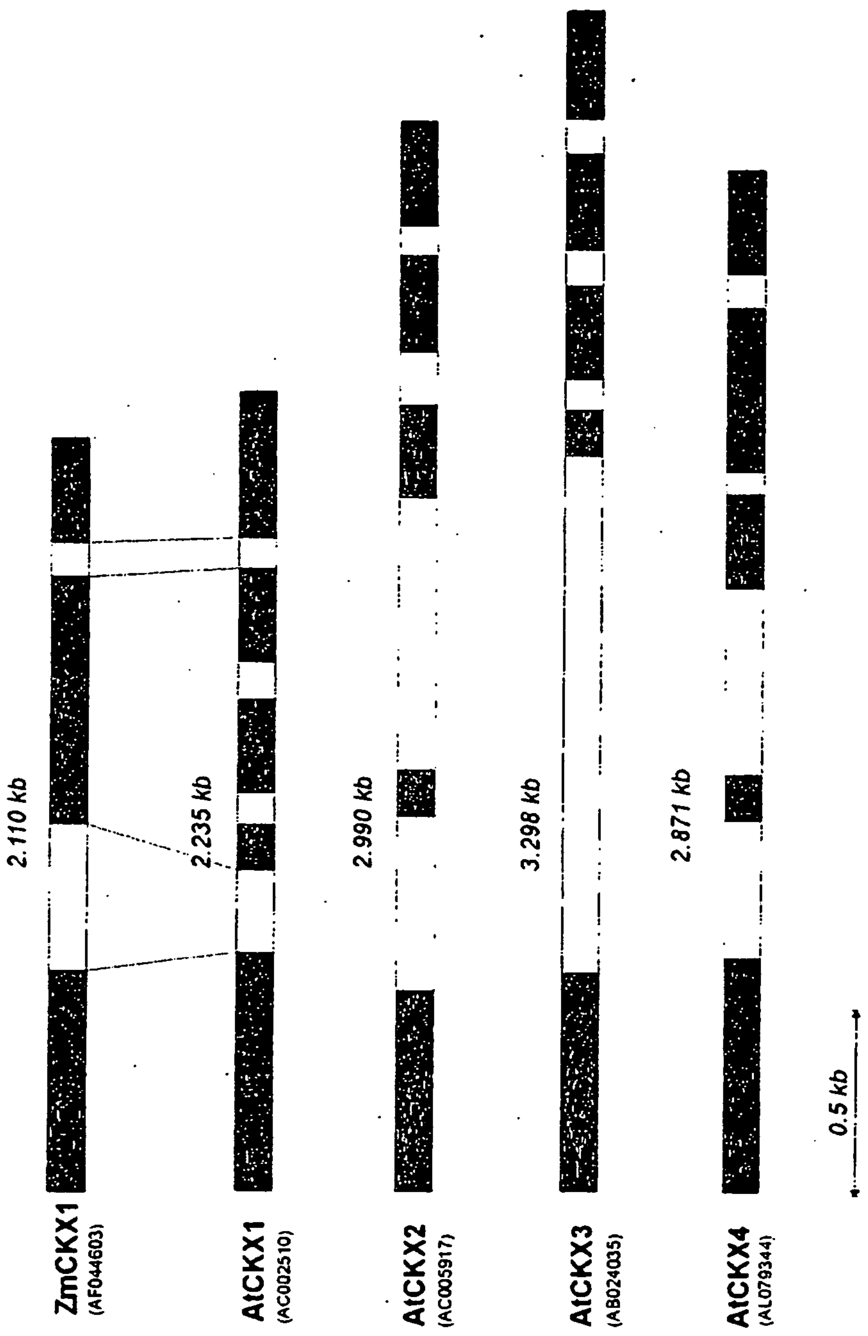
ZmCKX1
(AF044603)
2.110 kb
AtCKX1
(AC002510)
2.235 kb
AtCKX2
(AC005917)
2.990 kb
AtCKX3
(AB024035)
3.298 kb
AtCKX4
(AL079344)
2.871 kb
0.5 kb

FIGURE 1

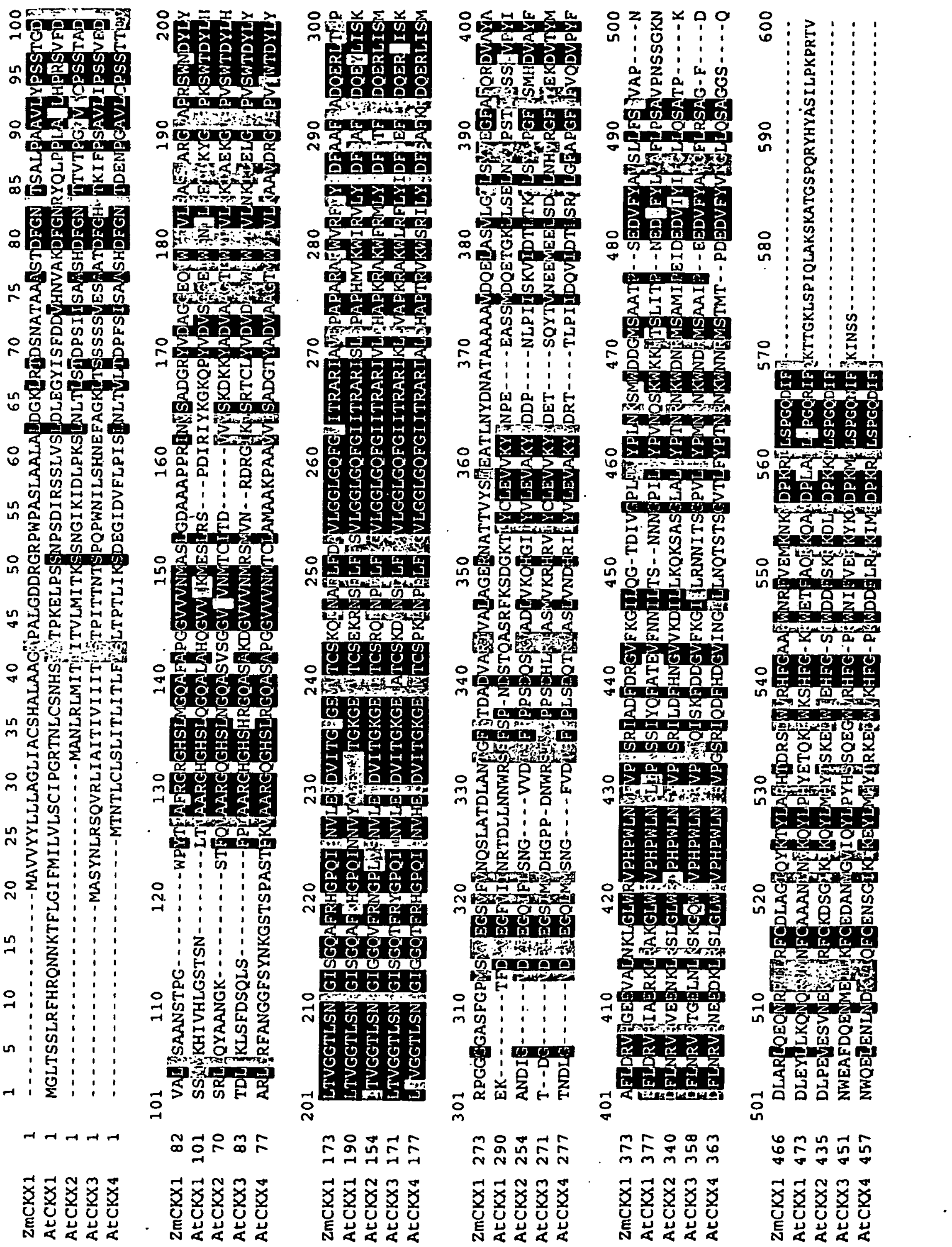

FIGURE 2

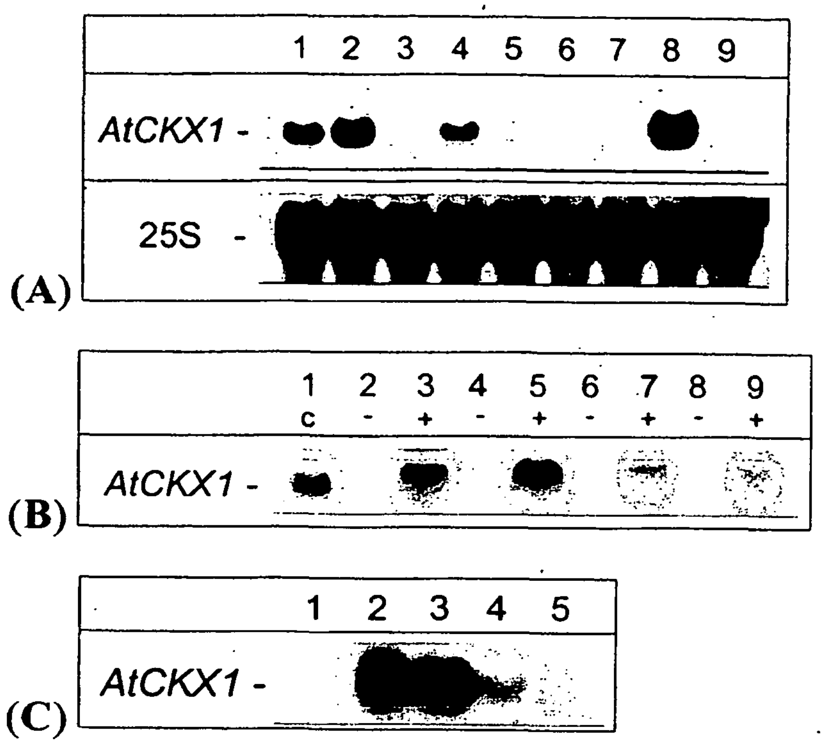


Figure 3

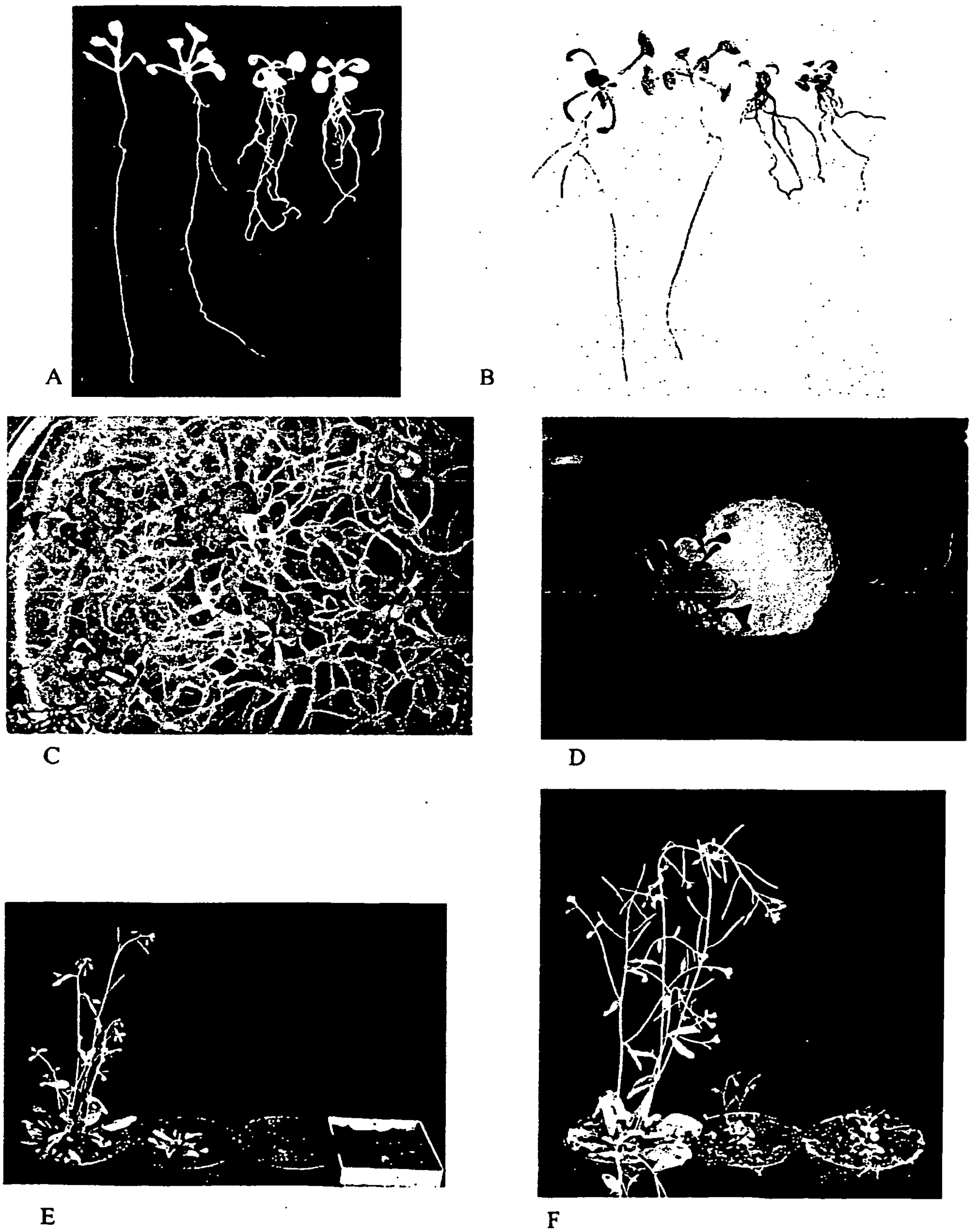

A B C D E F

Figure 4

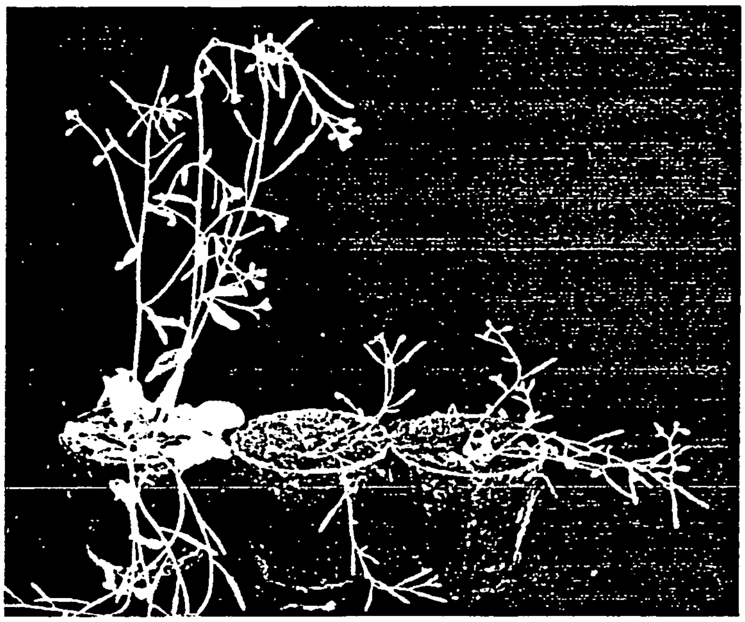

Figure 5

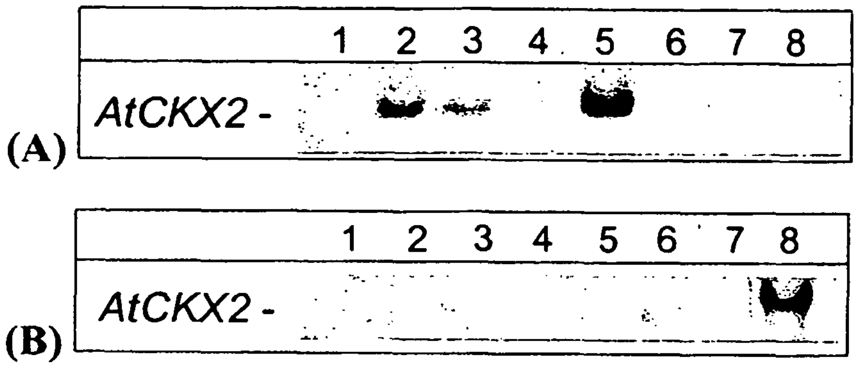


Figure 6

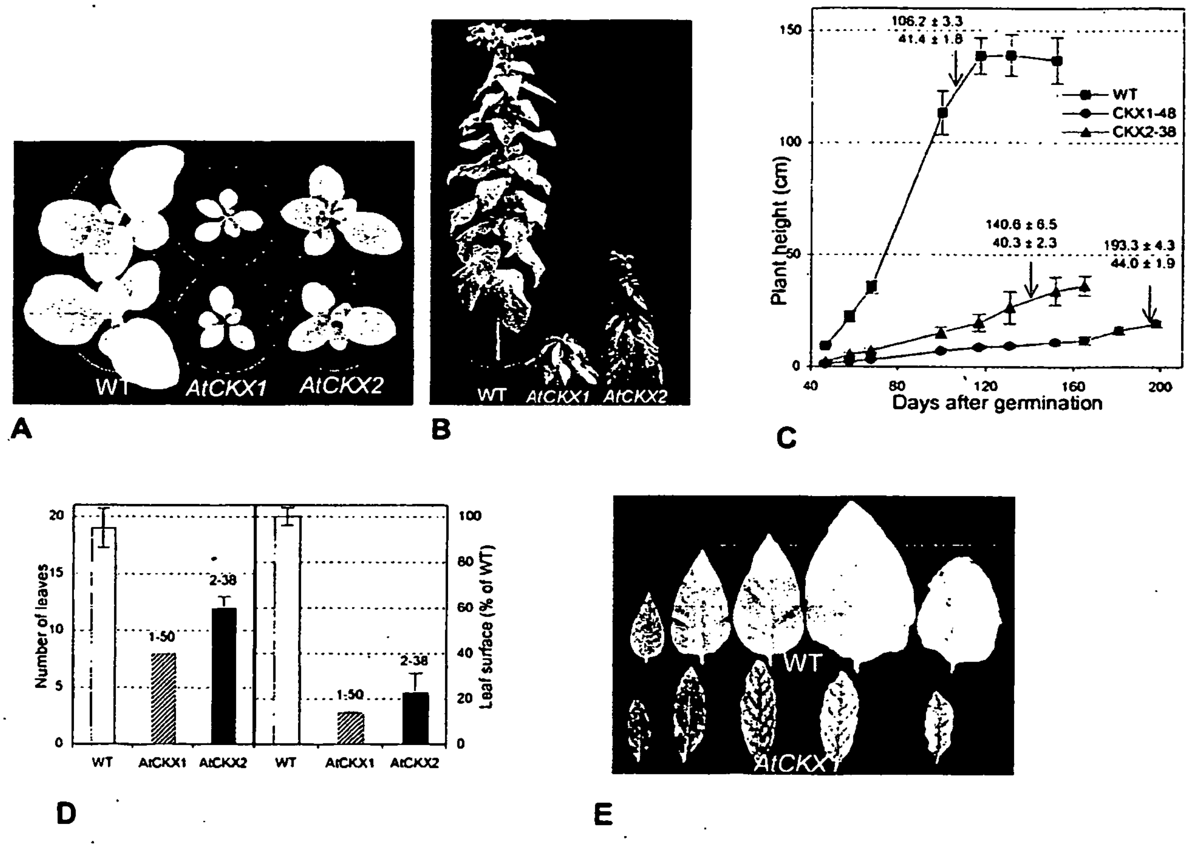
WT
AtCKX1
AtCKX2
A
WT AtCKX1 AtCKX2
B
106.2 ± 3.3
41.4 ± 1.8
140.6 ± 6.5
40.3 ± 2.3
193.3 ± 4.3
44.0 ± 1.9
WT
CKX1-48
CKX2-38
Plant height (cm)
Days after germination
C
Number of leaves
Leaf surface (% of WT)
1-50
2-38
WT AtCKX1 AtCKX2 WT AtCKX1 AtCKX2
D
WT
AtCKX1
E

Figure 7

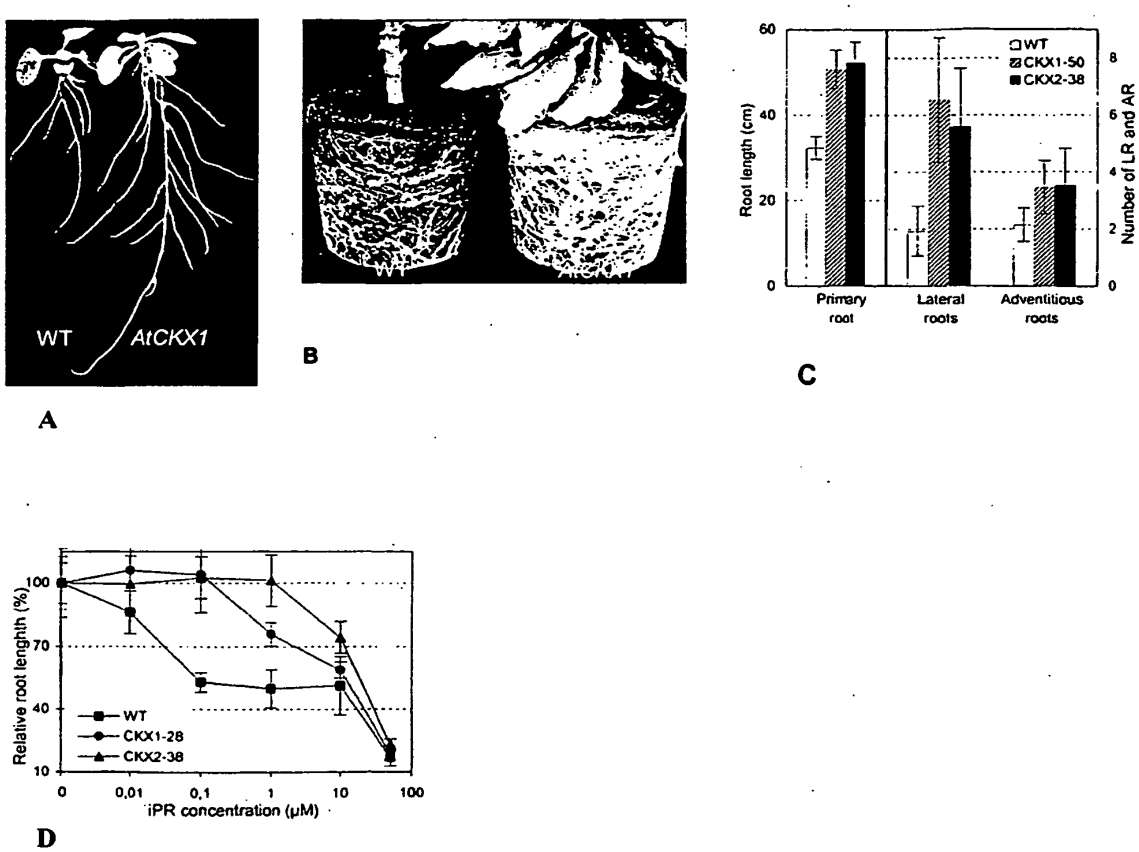
WT
AtCKX1
A
B
Root length (cm)
Number of LR and AR
WT
CKX1-50
CKX2-38
Primary root
Lateral roots
Adventitious roots
C
Relative root lenghth (%)
WT
CKX1-28
CKX2-38
iPR concentration (µM)
D

**Figure 8**

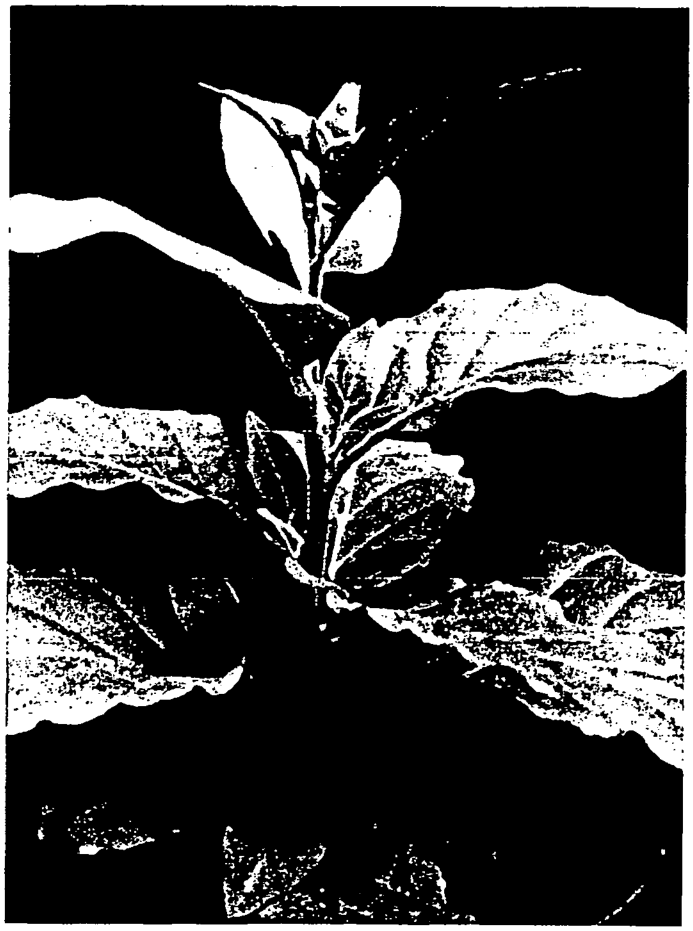

Figure 9

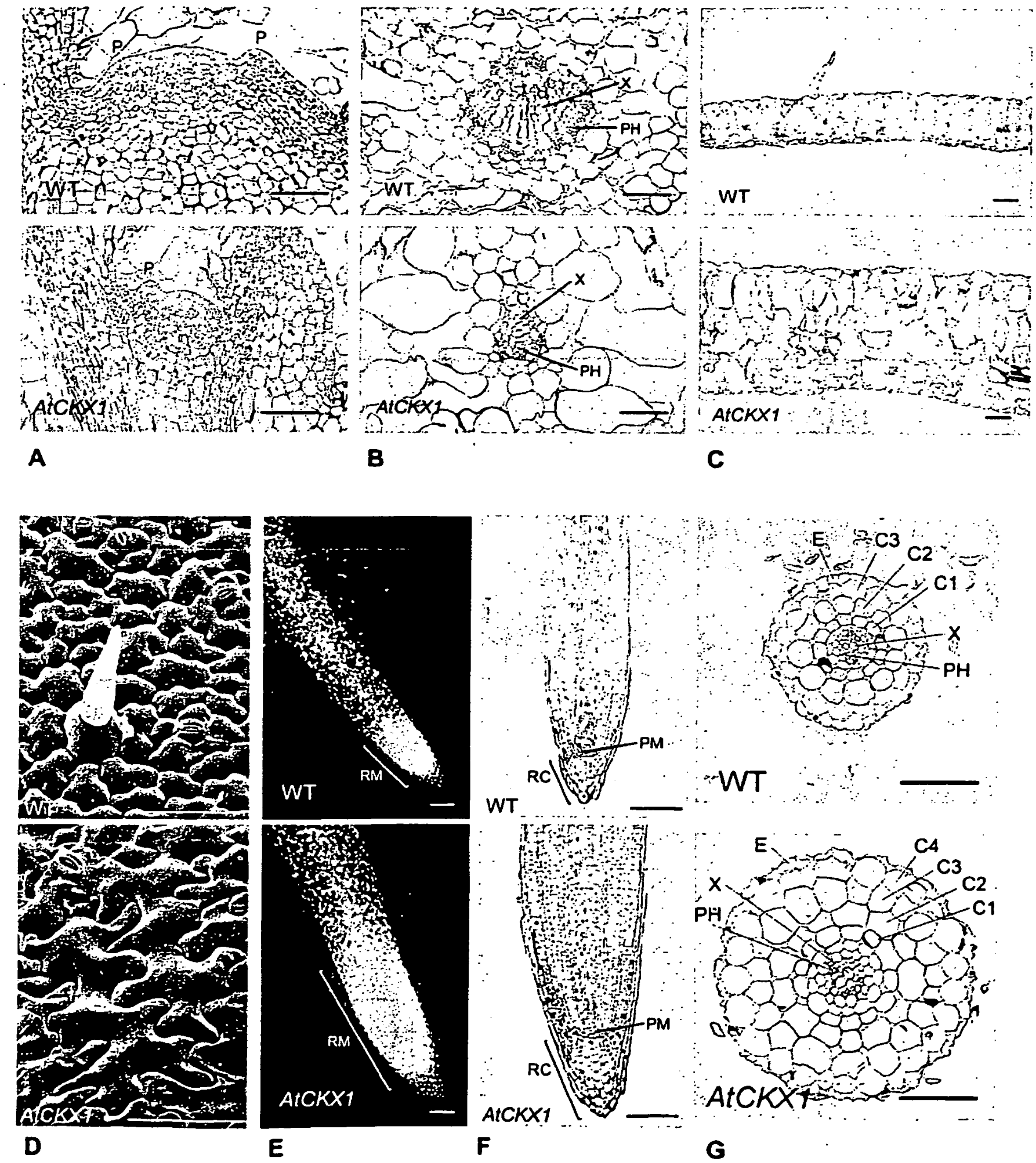
P
P
WT
P
AtCKX1
A
X
PH
WT
X
PH
AtCKX1
B
WT
AtCKX1
C
WT
AtCKX1
D
WT
RM
RM
AtCKX1
E
PM
RC
WT
PM
RC
AtCKX1
F
E
C3
C2
C1
X
PH
WT
E
C4
C3
C2
C1
X
PH
AtCKX1
G

**Figure 10**

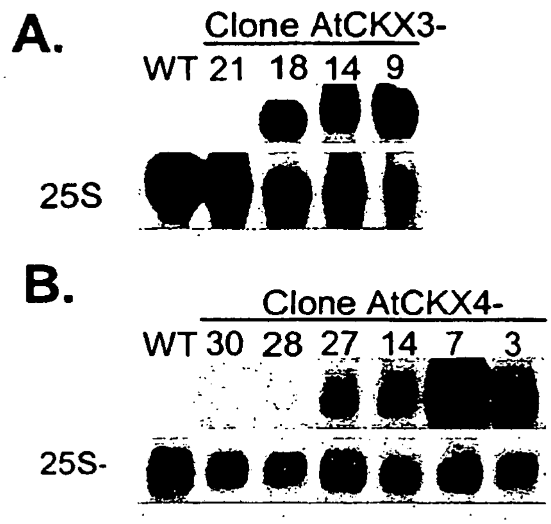
A.
Clone AtCKX3-
WT 21 18 14 9
25S
B.
Clone AtCKX4-
WT 30 28 27 14 7 3
25S-

**Figure 11**

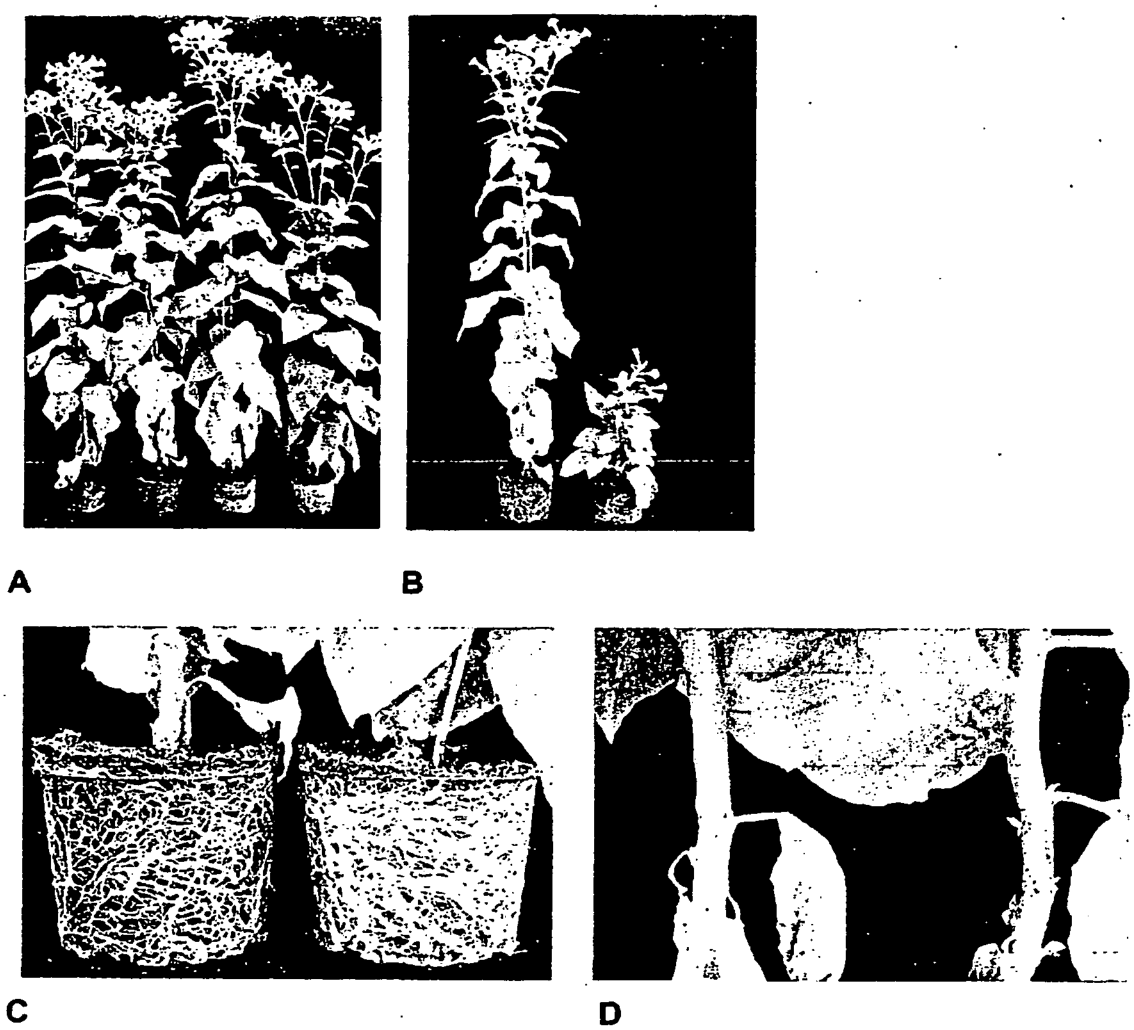


Figure 12

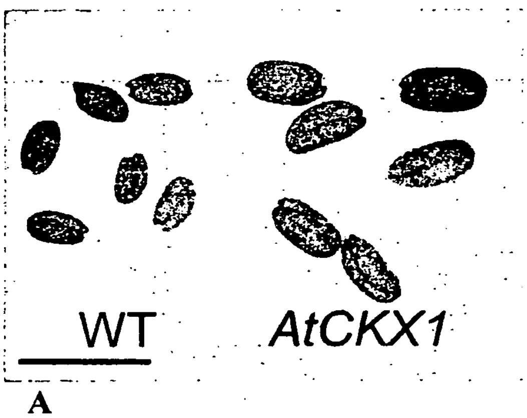


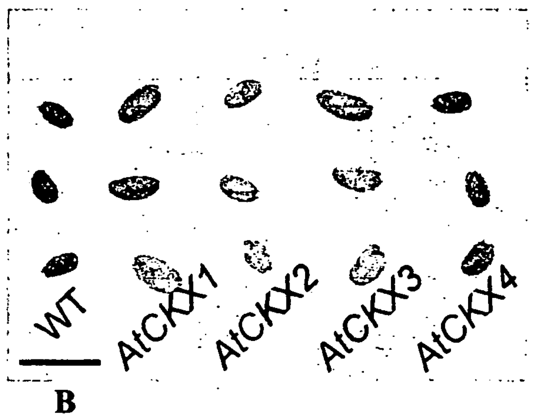


Figure 13A

Figure 13B

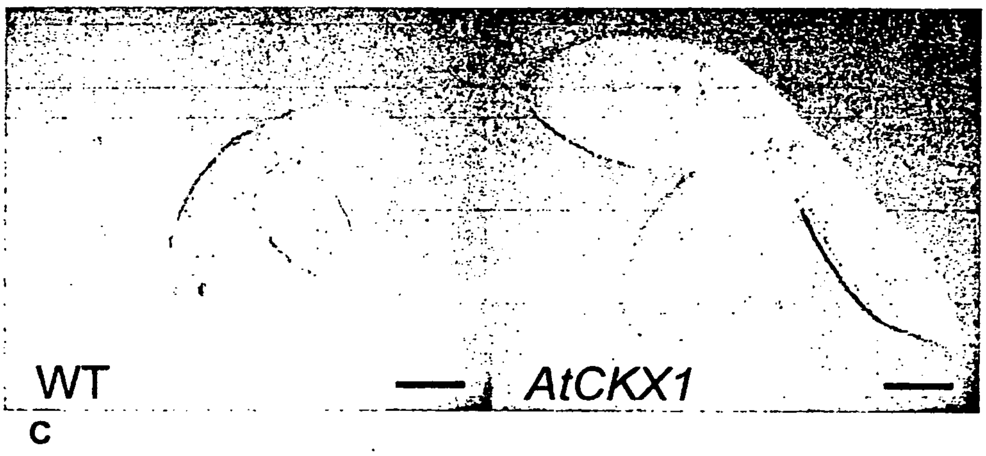


Figure 13C

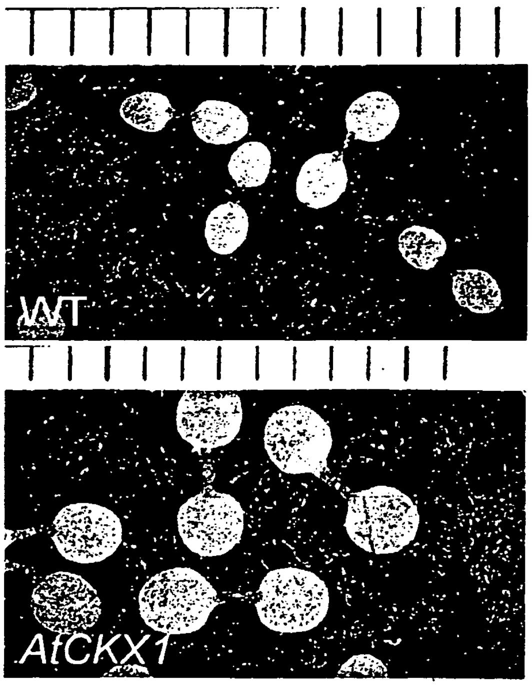


D

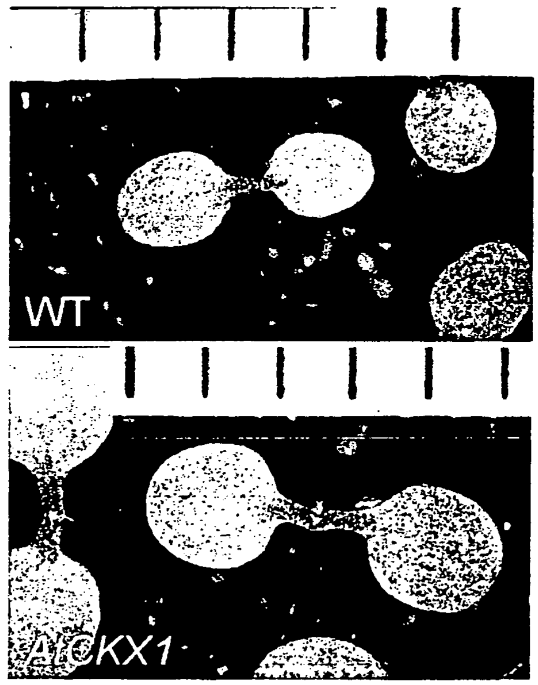


E

Figure 13D

Figure 13E

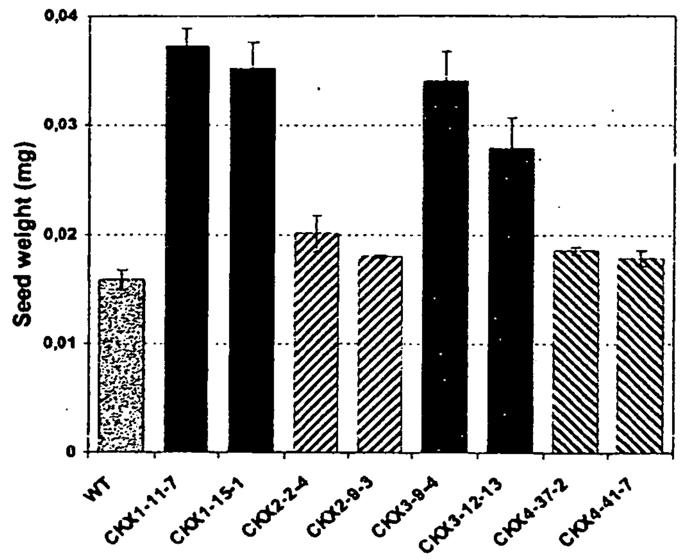
0,04
0,03
0,02
0,01
0
Seed weight (mg)
WT
CKX1-11-7
CKX1-15-1
CKX2-2-4
CKX2-9-3
CKX3-9-4
CKX3-12-13
CKX4-37-2
CKX4-41-7

Figure 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0105985 A
- WO 9922003 A, Yadav
- WO 0017365 A, Yadav
- WO 0015662 A, Feix and Wulff
- US 4803165 A
- US 5008194 A
- US 5401836 A
- EP 99106056 A
- WO 9903977 A
- US 5760386 A
- WO 9503690 A
- WO 9831822 A
- EP 9807761 W
- WO 95322888 A
- WO 98122335 A
- WO 9836083 A, Angell and Baulcombe
- WO 9853083 A, Lowe
- WO 9915682 A, Lederer
- WO 9953050 A, Wang
- WO 9748814 A, Cheng
- WO 9854961 A, Hansen
- WO 9400977 A, Hiei
- WO 9817813 A, Hiei
- WO 9904618 A, Rikiishi
- WO 9506722 A, Saito
- US 5969213 A, Adams
- US 5736369 A, Bowen
- WO 9413822 A, Chang
- US 5874265 A, Lundquist
- US 5990390 A
- US 5405765 A, Vasil and Vasil
- US 5955362 A, Walker
- WO 9318168 A, Eyal
- DE 4309203, von Holt
- US 5693512 A, Finer
- US 5122466 A, Stomp
- US 4945050 A, Sanford and Wolf
- WO 9901563 A, Stuiver
- EP 0604662 A
- EP 0687730 A
- WO 9712046 A, Hansen and Chilton
- WO 9925840 A
- US 5527695 A
- WO 9925821 A
- WO 9923202 A
- WO 9906571 A
- US 10014101 B


### Non-patent literature cited in the description


- **Torrey.** *Am J Bot,* 1950, vol. 37, 257-264
- **Blakely et al.** *Bot Gaz,* 1982, vol. 143, 341-352
- **Muday ; Haworth.** *Plant Physiol Biochem,* 1994, vol. 32, 193-203
- **Kerk et al.** *Plant Physiol,* 2000, vol. 122, 925-932
- **Boerjan et al.** *Plant Cell,* 1995, vol. 7, 1405-141
- **Celenza et al.** *Gene Dev,* 1995, vol. 9, 2131-2142
- **King et al.** *Plant Cell,* 1995, vol. 7, 2023-2037
- **Lehman et al.** *Cell,* 1996, vol. 85, 183-194
- **Klee et al.** *Genes Devel,* 1987, vol. 1, 86-96
- **Kares et al.** *Plant Mol Biol,* 1990, vol. 15, 225-236
- **Mok M.C.** Cytokines: Chemistry, Activity and Function. CRC Boca Raton, 1994, 155-166
- **Klee, H.J. ; Lanehon, M.B.** Plant Hormones:Physiology, Biochemisry and Molecular Biology. Kluwer, Dordrdrocht, the Netherlands, 1995, 340-353
- **Smulling, T. ; Rupp, H.M. ; Frank, M ; Schafer, S.** Advances in Regulation of Plant Growth and Development. Peres, Prague, 1999, 85-96
- Biochemistry and Molecular Biology of Plant Hormones. Elsevier, 1999
- **Armstrong, D.J.** Cytokinins: Chemistry, Activity and Functions. CRC Boca Raton, 1994, 139-154
- **Morris, R.O. ; Bilyeu, K.D. ; Laskey, J.G. ; Cherich, N.N.** *Biochem. Biophys.Res. Commun.,* 1999, vol. 255, 328-333
- **Houba-Heria, N. ; Pethe, C. ; d'Alayer, J ; Lelouc, M.** *Plant J.,* 1999, vol. 17, 615-626
- *Biochem. Biophys. Res. Com.,* 1999, vol. 255, 328-333
- **Hare ; van Staden.** *Physiol Plant,* 1994, vol. 91, 128-136
- **Jones ; Schreiber.** *Plant Growth Reg,* 1997, vol. 23, 123-134
- Armstrong, in Cytokinins: Chemistry, Activity and Function. CRC Press, 1994, 139-154

- **ímalová et al.** Biochemistry and Molecular Biology of Plant Hormones. Elsevier Science, 1997, 141-160
- **Geysen, H.M. ; Rodda, S.J. ; Mason, T.J.** A priori delineation of a peptide which mimics a discontinuous antigenic determinant. *Mol. Immunol.,* 1986, vol. 23, 709-715
- **Sambrook et al.** Molecular Cloning: a Laboratory Manual. 1989
- **Sambrook, J. ; E.F. Fritsch et al.** Molecular Cloning: a Laboratory Manual. Cold Spring Harbor Laboratory Press, 1989
- **Lanahan, M.B.** *Plant Cell,* 1992, vol. 4, 203-211
- **Skriver, K. et al.** *Proc. Natl. Acad. Sci. (USA),* 1991, vol. 88, 7266-7270
- **Cejudo, F.J. et al.** *Plant Molecular Biology,* 1992, vol. 20, 849-856
- **Nilsson et al.** *Physiol. Plant.,* 1997, vol. 100, 456-462
- **Van der Meer et al.** *Plant Mol. Biol.,* 1990, vol. 15, 95-109
- **Twell et al.** *Mol. Gen Genet.,* 1989, vol. 217, 240-245
- **Thomas et al.** *CSIRO Plant Industry, Urrbrae, South Australia, Australia, winetitles.com.au/gwrdc/csh95-1.html*
- **Lam, E. et al.** *The Plant Cell,* 1990, vol. 2, 857-866
- **Tucker et al.** *Plant Physiol.,* 1992, vol. 113, 1303-1308
- **Baszczynski et al.** *Nucl. Acid Res.,* 1988, vol. 16, 4732
- **Mitra ; Higgins.** *Plant Molecular Biology,* 1994, vol. 26, 85-93
- **Kagaya et al.** *Molecular and General Genetics,* 1995, vol. 248, 668-674
- **Kyozuka et al.** *Plant Physiology,* 1993, vol. 102, 991-1000
- **Yamamoto et al.** *Plant Cell Physiol.,* 1994, vol. 35, 773-778
- **Crowell et al.** *Plant Mol. Biol.,* 1992, vol. 18, 459-466
- **Fleming et al.** *Plant J.,* vol. 2, 855-862
- **Yang et al.** *The Plant J.,* vol. 3, 573-585
- **Pathirana et al.** *Plant Mol. Biol.,* 1992, vol. 20, 437-450
- **Gordon et al.** *J. Exp. Bot.,* 1993, vol. 44, 1453-1465
- **Bhattacharyya-Pakrasi et al.** *The Plant J.,* 1992, vol. 4, 71-79
- **Albani et al.** *Plant Mol. Biol.,* 1990, vol. 15, 605
- **Albani et al.** *Plant Mol. Biol.,* 1991, vol. 16, 501
- **Guerrero et al.** *Mol. Gen. Genet.,* 1993, vol. 224, 161-168
- **Twell et al.** *Sex. Plant Reprod.,* 1993, vol. 6, 217-224
- **Hamilton et al.** *Plant Mol. Biol.,* 1992, vol. 18, 211-218
- **Baltz et al.** *The Plant J.,* 1992, vol. 2, 713-721
- **Arnoldo et al.** *J. Cell. Biochem.,* 1992, (Y101), 204
- **Tingey et al.** *EMBO J.,* 1987, vol. 6, 1
- **Van der Zaal et al.** *Plant Mol. Biol.,* 1991, vol. 16, 983
- **Oppenheimer et al.** *Gene,* 1988, vol. 63, 87
- **Conkling et al.** *Plant Physiol.,* 1990, vol. 93, 1203
- **Suzuki et al.** *Plant Mol. Biol.,* 1993, vol. 21, 109-119
- **Simon et al.** *Plant Mol. Biol.,* 1985, vol. 5, 191
- **Scofield et al.** *J. Biol. Chem.,* 1987, vol. 262, 12202
- **Baszczynski et al.** *Plant Mol. Biol.,* 1990, vol. 14, 633
- **Pearson et al.** *Plant Mol. Biol.,* 1992, vol. 18, 235-245
- **Ellis et al.** *Plant Mol. Biol.,* 1988, vol. 10, 203-214
- **Takaiwa et al.** *Mol. Gen. Genet.,* 1986, vol. 208, 15-22
- **Takaiwa et al.** *FEBS Letts.,* 1987, vol. 221, 43-47
- **Matzke et al.** *Plant Mol Biol,* 1990, vol. 14 (3), 323-32
- **Stalberg et al.** *Planta,* 1996, vol. 199, 515-519
- *Mol Gen Genet,* 1989, vol. 216, 81-90
- *NAR,* 1989, vol. 17, 461-2
- **Albani et al.** *Plant Cell,* 1997, vol. 9, 171-184
- *EMBO,* 1994, vol. 3, 1409-15
- *Theor Appl Gen,* 1999, vol. 98, 1253-62
- *Plant J,* 1993, vol. 4, 343-55
- *Mol Gen Genet,* 1996, vol. 250, 750-60
- **Mena et al.** *The Plant Journal,* 1998, vol. 116 (1), 53-62
- **Vicente-Carbajosa et al.** *Plant J.,* 1998, vol. 13, 629-640
- **Wu et al.** *Plant Cell Physiology,* 1998, vol. 39 (8), 885-889
- **Sato et al.** *Proc. Natl. Acad. Sci. USA,* 1996, vol. 93, 8117-8122
- **Nakase et al.** *Plant Mol. Biol.,* 1997, vol. 33, 513-522
- *Trans Res,* 1997, vol. 6, 157-68
- *Plant J,* 1997, vol. 12, 235-46
- *PMB,* 1996, vol. 32, 1029-35
- **Postma-Haarsma et al.** *Plant Mol. Biol.,* 1999, vol. 39, 257-71
- **Wu.** *J. Biochem.,* 1998, vol. 123, 386
- **Cummins et al.** *Plant Mol. Biol.,* 1992, vol. 19, 873-876
- **Weigel et al.** *Cell,* 2007, vol. 69, 843-859

- **Nasrallah et al.** *Proc. Natl. Acad.*
- *Sci. USA,* 1988, vol. 85, 5551
- **Trick et al.** *Plant Mol. Biol.,* 1990, vol. 15, 203
- **Liu et al.** *Plant Mol. Biol.,* 1991, vol. 153, 386-395
- **Kosugi et al.** *Nucleic Acids Research,* 1991, vol. 19, 1571-1576
- **Kosugi S. ; Ohashi Y.** *Plant Cell,* 1997, vol. 9, 1607-1619
- **Stotz ; Long.** *Plant Mol.Biol.,* 1999, vol. 41, 601-614
- **Chung ; Parish.** *FEBS Lett,* March 1995, vol. 362 (2), 215-9
- **Li et al.** *Plant Physiol,* 1998, vol. 118, 407-417
- **Klimyuk ; Jones.** *Plant J.,* 1997, vol. 11, 1-14
- **Wong et al.** *Plant J.,* 1996, vol. 9, 587-599
- **Zhou et al.** *Plant J.,* 1997, vol. 12, 921-930
- **Trehin et al.** *Plant Mol.Biol.,* 1997, vol. 35, 667-672
- **Ito et al.** *Plant J.,* 1997, vol. 11, 983-992
- **Shaul et al.** *Proc.Nati.Acad.Sci.U.S.A,* 1996, vol. 93, 4868-4872
- **Regad et al.** *Mol. Gen. Genet.,* 1995, vol. 248, 703-711
- **Ito et al.** *Plant Mol.Biol.,* 1994, vol. 24, 863-878
- **McElroy et al.** *Plant Cell,* 1990, vol. 2, 163-171
- **Odell et al.** *Nature,* 1995, vol. 313, 810-812
- **Nilsson et al.** *Physiol. Plant.,* 1997, vol. 100, 462
- **de Pater et al.** *Plant J.,* 1992, vol. 2, 837-844
- **Christensen et al.** *Plant Mol. Biol.,* 1992, vol. 18, 675-689
- **Buchholz et al.** *Plant Mol Biol.,* 1994, vol. 25, 837-843
- **Lepetit et al.** *Mol. Gen. Genet.,* 1992, vol. 231, 276-285
- **Wu et al.** *Nucleic Acids Res.,* 1989, vol. 17, 3057-3063
- **Wu et al.** *Plant Mol. Biol.,* 1988, vol. 11, 641-649
- **An et al.** *Plant J.,* 1996, vol. 10 (1), 107-121
- **Zhang et al.** *Plant Science,* 1997, vol. 129, 81-89
- **Hajela et al.** *Plant Physiol.,* 1990, vol. 93, 1246-1252
- **Wlihelm et al.** *Plant Mol Biol.,* 1993, vol. 23, 1073-1077
- **Baker et al.** *Plant Mol Biol.,* 1994, vol. 24, 701-713
- **Kasuga et al.** *Nature Biotechnology,* 1998, vol. 18, 287-291
- **Barros et al.** *Plant Mol Biol,* 1992, vol. 19, 665-75
- **Marrs et al.** *Dev Genet.,* 1993, vol. 14, 27-41
- **Schoffl et al.** *Mol Gen Gent,* 1989, vol. 217, 246-53
- **Waters et al.** *J Experimental Botany,* 1996, vol. 47, 325-338
- **Ouellet et al.** *FEBS Lett.,* 1998, vol. 423, 324-328
- **Kirch et al.** *Plant Mol Biol,* 1997, vol. 33, 897-909
- **Dolferus et al.** *Plant Physiol,* 1994, vol. 105, 1075-87
- **Joshee et al.** *Plant Cell Physiol,* 1998, vol. 39, 64-72
- **Schneider et al.** *Plant Physiol,* 1997, vol. 113, 335-45
- **Chaudhary et al.** *Plant Mol Biol,* 1996, vol. 30, 1247-57
- **Raghothama et al.** *Plant Mol Biol,* 1993, vol. 23, 1117-28
- **Vilardell et al.** *Plant Mol Biol,* 1991, vol. 17, 985-93
- **Opperman et al.** *Science,* 1994, vol. 263, 221-23
- **Ward et al.** *Plant Cell,* 1991, vol. 3, 1085-1094
- **Lebel et al.** *Plant J,* 1998, vol. 16 (2), 223-33
- **Melchers et al.** *Plant J,* 1994, vol. 5 (4), 469-80
- **Lawton et al.** *Plant Mol Biol,* 1992, vol. 19 (5), 735-43
- **Barthels et al.** *The Plant Cell,* 1997, vol. 9, 2119-2134
- **Strittmatter et al.** *Mol. Plant-Microbe Interact,* 1996, vol. 9, 68-73
- **Manners et al.** *Plant Mol Biol,* 1998, vol. 38 (6), 1071-80
- **Vignutelli et al.** *Plant J,* 1998, vol. 14 (3), 285-95
- **Bird ; Wilson.** *Mol. Plant-Microbe Interact.,* 1994, vol. 7, 419-42
- **Niebel et al.** *Mol Plant Microbe Interact,* 1995, vol. 8, 371-8
- **Aristizabal et al.** *8th International Congress on Plant-Microbe Interaction,* 1996
- **Fenoll et al.** *Cellular and molecular aspects of plant-nematode interactions,* 1997
- **Fenoll et al.** Cellular and molecular aspects of plant-nematode interactions. Kluwer Academic, 1997
- **Molina ; Garcia-Olmedo.** *FEBS Lett,* 1993, vol. 316 (2), 119-22
- **Turner et al.** *Proc Natl Acad Sci U S A,* 1997, vol. 94 (8), 3866-71
- **Suzuki et al.** *Plant Mol Biol,* 1993, vol. 21, 109-119
- **Yamamoto et al.** *Plant Cell,* 1991, vol. 3, 371-382
- **Okada et al.** *Plant Physiol,* 2000, vol. 122, 1045-1056
- **Wanapu ; Shinmyo.** *Ann N Y Acad Sci,* 1996, vol. 782, 107-114

- **Okumura et al.** *Plant Mol Biol,* 1994, vol. 25, 705-719
- **Fowler et al.** *Plant Physiol,* 1999, vol. 121, 1081-1092
- **Olszewski.** *Proteins,* 1996, vol. 25, 286-299
- **Hoffman.** *Comput. Appl. Biosci.,* 1995, vol. 1, 675-679
- **Monge.** *J. Mol. Biol.,* 1995, vol. 247, 995-1012
- **Renouf.** *Adv. Exp. Med. Biol.,* 1995, vol. 376, 37-45
- **Fassina.** *Immunomethods,* 1994, vol. 5, 114-120
- **Berry.** *Biochem. Soc. Trans.,* 1994, vol. 22, 1033-1036
- **Wodak.** *Ann, N. Y. Acac. Sci.,* 1987, vol. 501, 1-13
- **Pabo.** *Biochemistry,* 1986, vol. 25, 5987-5991
- **Benkirane.** *J. Biol. Chem.,* 1996, vol. 271, 33218-33224
- **Banerjee.** *Biopolymers,* 1996, vol. 39, 769-777
- **Zhang.** *Biochem. Biophys. Res. Commun.,* 1996, vol. 224, 327-331
- **Ostresh.** *Methods in Enzymology,* 1996, vol. 267, 220-234
- **Dorner.** *Bioorg. Med. Chem.,* 1996, vol. 4, 709-715
- **Rose.** *Biochemistry,* 1996, vol. 35, 12933-12944
- **Ruterber.** *Bioorg. Med. Chem.,* 1996, vol. 4, 1545-1558
- **Alberts et al.** Molecular Biology of the Cell. 1994
- **Altschul et al.** *Nucleic Acids Res.,* 1997, vol. 25, 3389-3402
- **Morris et al.** *Biochem Biophys Res Comm,* 1999, vol. 255, 328-333
- **Houda-Herin et al.** *Plant J,* vol. 17, 615-626
- **Alberts, B. ; Bray, D. ; Lewis, J. ; Raff, M. ; Roberts, K. ; Watson, J. D.** Molecular Biology of the Cell. Garland Publishing Inc, 1994
- **Altschul, S.F. ; Madden, T.L. ; Schäffer, A.A. ; Zhang, J. ; Zhang, Z. ; Miller, W. ; Lipman, D.J.** Gapped BLAST and PSI-BLAST: a new generation of protein database search programs. *Nucl. Acids Res.,* 1997, vol. 25, 3389-3402
- **An, G. ; Watson, B. D. ; Stachel, S. ; Gordon, M. P. ; Nester, E. W.** New cloning vehicles for transformation of higher plants. *EMBO J.,* 1985, vol. 4, 277-284
- **Armstrong, C. L. ; Petersen, W. P. ; Buchholz, W. G. ; Bowen, B. A. ; Sulc, S. L.** Factors affecting PEG-mediated stable transformation of maize protoplasts. *Plant Cell Reports,* 1990, vol. 9, 335-339
- **Banerjee, A. ; Pramanik, A. ; Bhattacharjya, S. ; Balaram, P.** Omega amino acids in peptide design: incorporation into helices. *Biopolymers,* 1996, vol. 39, 769-777
- **Baron, M. H. ; Baltimore, D.** Antibodies against the chemically synthesized genome-linked protein of poliovirus react with native virus-specific proteins. *Cell,* 1982, vol. 28, 395-404
- **Bartel, P. L. ; Fields, S.** The Yeast Two-Hybrid System. Oxford University Press, 1997
- **Benkirane, N. ; Guichard, G. ; Briand, J. P. ; Muller, S.** Exploration of requirements for peptidomimetic immune recognition. Antigenic and immunogenic properties of reduced peptide bond pseudopeptide analogues of a histone hexapeptide. *J. Biol Chem.,* 1996, vol. 271, 33218-33224
- **Berry, A. ; Brenner, S. E.** A prototype computer system for de novo protein design. *Biochem.Soc. Trans.,* 1994, vol. 22, 1033-1036
- **Christou, P. ; McCabe, D. E. ; Swain, W. F.** Stable transformation of soybean callus by DNA-coated gold particles. *Plant Physiol.,* 1988, vol. 87, 671-674
- **Crossway, A. ; Oakes, J. V. ; Irvine, J. M. ; Ward, B. ; Knauf, V. C. ; Shewmaker, C. K.** Integration of foreign DNA following microinjection of tobacco mesophyll protoplasts. *Mol. Gen. Genet.,* 1986, vol. 202, 179-185
- **Dale, E. C. ; Ow, D. W.** Intra- and intermolecular site-specific recombination in plant cells mediated by bacteriophage P1 recombinase. *Gene,* 1990, vol. 91, 79-85
- **Dodds, J. H.** Plant genetic engineering. Cambridge University Press, 1985
- **Doerner, P. ; Jorgensen, J. E. ; You, R. ; Steppuhn, J. ; Lamb, C.** Control of root growth and development by cyclin expression. *Nature,* 1996, vol. 380, 520-523
- **Dorner, B. ; Husar, G. M. ; Ostresh, J. M. ; Houghten, R. A.** The synthesis of peptidomimetic combinatorial libraries through successive amide alkylations. *Bioorg.Med.Chem.,* 1996, vol. 4, 709-715
- **Ellis, J. G. ; Llewellyn, D. J. ; Dennis, E. S. ; Peacock, W. J.** Maize Adh-1 promoter sequences control anaerobic regulation: addition of upstream promoter elements from constitutive genes is necessary for expression in tobacco. *EMBO J.,* 1987, vol. 6, 11-16
- **Faiss, M. ; Zalubilová, J. ; Strnad, M. ; Schmülling, T.** Conditional transgenic expression of the ipt gene indicates a function for cytokinins in paracrine signaling in whole tobacco plants. *Plant J.,* 1997, vol. 12, 401-415
- **Fassina, G. ; Melli, M.** Identification of interactive sites of proteins and protein receptors by computer-assisted searches for complementary peptide sequences. *Immunomethods,* 1994, vol. 5, 114-120

- **Fedoroff, N. V. ; Smith, D. L.** A versatile system for detecting transposition in Arabidopsis. *Plant J.,* 1993, vol. 3, 273-289
- **Hanahan, D.** Studies on transformation of Escherichia coli with plasmids. *J.Mol.Biol,* 1983, vol. 166, 557-580
- **Hansen, G. ; Chilton, M. D.** ''Agrolistic'' transformation of plant cells: integration of T-strands generated in planta. *Proc.Natl.Acad.Sci.U.S.A,* 1996, vol. 93, 14978-14983
- **Hansen, G. ; Shillito, R. D. ; Chilton, M. D.** T-strand integration in maize protoplasts after codelivery of a T-DNA substrate and virulence genes. *Proc.Natl.Acad.Sci.U.S.A,* 1997, vol. 94, 11726-11730
- **Hanson, B. ; Engler, D. ; Moy, Y. ; Newman, B. ; Ralston, E. ; Gutterson, N.** A simple method to enrich an Agrobacterium-transformed population for plants containing only T-DNA sequences. *Plant J.,* 1999, vol. 19, 727-734
- **Harlow, E. ; Lane, D.** Antibodies: A Laboratory Manual. Cold Spring Harbor Laboratory Press, 1988
- **Herrera-Estrella, L. ; De Block, M. ; Messens, E. H. J. P. ; Van Montagu, M. ; Schell, J.** Chimeric genes as dominant selectable markers in plant cells. *EMBO J.,* 1983, vol. 2, 987-995
- **Hoffman, D. L. ; Laiter, S. ; Singh, R. K. ; Vaisman, I. I. ; Tropsha, A.** Rapid protein structure classification using one-dimensional structure profiles on the bioSCAN parallel computer. *Comput.Appl.Biosci.,* 1995, vol. 11, 675-679
- **Klee, H.J. ; Lanehon, M.B.** Plant Hormones:Physiology, Biochemisry and Molecular Biology. Kluwer, 1995, 340-353
- **Krens, F. A. ; Molendijk, L. ; Wullems, G. J. ; Schilperoort, R. A.** In vitro transformation of plant protoplasts with Ti-plasmid DNA. *Nature,* 1982, vol. 296, 72-74
- **Lerner, R. A.** Tapping the immunological repertoire to produce antibodies of predetermined specificity. *Nature,* 1982, vol. 299, 593-596
- **Lerner, R. A. ; Green, N. ; Alexander, H. ; Liu, F. T. ; Sutcliffe, J. G. ; Shinnick, T. M.** Chemically synthesized peptides predicted from the nucleotide sequence of the hepatitis B virus genome elicit antibodies reactive with the native envelope protein of Dane particles. *Proc.Natl.Acad.Sci.U.S.A,* 1981, vol. 78, 3403-3407
- **Liddle, J. E. ; Cryer, A.** A Practical Guide to Monoclonal Antibodies. Wiley, 1991
- **Loffler, J. ; Langui, D. ; Probst, A. ; Huber, G.** Accumulation of a 50 kDa N-terminal fragment of beta-APP695 in Alzheimer's disease hippocampus and neocortex. *Neurochem.Int.,* 1994, vol. 24, 281-288
- **Monge, A. ; Lathrop, E. J. ; Gunn, J. R. ; Shenkin, P. S. ; Friesner, R. A.** Computer modeling of protein folding: conformational and energetic analysis of reduced and detailed protein models. *J.Mol.Biol,* 1995, vol. 247, 995-1012
- **Morris, R.O. et al.** Isolation of a gene encoding a glycosylated cytokinin oxidase from maize. Bioechem. *Biophys. Res. Commun.,* 1999, vol. 255, 328-333
- **Motyka, V. ; Faiss, M. ; Strnad, M. ; Kaminek, M. ; Schmuelling, T.** Changes in cytokinin content and cytokinin oxidase activity in response to derepression of ipt gene transcription in transgenic tobacco calli and plants. *Plant Physiol.,* 1996, vol. 112, 1035-1043
- **Murakami, T. ; Simonds, W. F. ; Spiegel, A. M.** Site-specific antibodies directed against G protein beta and gamma subunits: effects on alpha and beta gamma subunit interaction. *Biochemistry,* 1992, vol. 31, 2905-2911
- **Olszewski, K. A. ; Kolinski, A. ; Skolnick, J.** Folding simulations and computer redesign of protein A three-helix bundle motifs. *Proteins,* 1996, vol. 25, 286-299
- **Osborne, B. I. ; Wirtz, U. ; Baker, B.** A system for insertional mutagenesis and chromosomal rearrangement using the Ds transposon and Cre-lox. *Plant J.,* 1995, vol. 7, 687-701
- **Ostresh, J. M. ; Blondelle, S. E. ; Dorner, B. ; Houghten, R. A.** Generation and use of nonsupport-bound peptide and peptidomimetic combinatorial libraries. *Methods Enzymol.,* 1996, vol. 267, 220-234
- **Pabo, C. O. ; Suchanek, E. G.** Computer-aided model-building strategies for protein design. *Biochemistry,* 1986, vol. 25, 5987-5991
- **Paszkowski, J. ; Shillito, R. D. ; Saul, M. ; Mandak, V. ; Hohn, T. H. B. P. I.** Direct gene transfer to plants. *EMBO J.,* 1984, vol. 3, 2717-2722
- **Peralta, E. G. ; Hellmiss, R. ; Ream, W.** Overdrive, a T-DNA transmission enhancer on the A. tumefaciens tumour-inducing plasmid. *EMBO J.,* 1986, vol. 5, 1137-1142
- **Quirino, B.F. ; Noh, Y.-S. ; Himelbau, E. ; Amasino, R.M.** Molecular aspects of leaf senescence. *Trends in Plant Science,* 2000, vol. 5, 278-282
- **Renouf, D. V. ; Hounsell, E. F.** Molecular modelling of glycoproteins by homology with non-glycosylated protein domains, computer simulated glycosylation and molecular dynamics. *Adv.Exp.Med.Biol,* 1995, vol. 376, 37-45
- **Rinaldi, A.C. ; Comandini, O.** Cytokinin oxidase strikes again. *Trends in Plant Sc.,* 1999, vol. 4, 300
- **Rose, R. B. ; Craik, C. S. ; Douglas, N. L. ; Stroud, R. M.** Three-dimensional structures of HIV-1 and SIV protease product complexes. *Biochemistry,* 1996, vol. 35, 12933-12944

- **Rutenber, E. E. ; McPhee, F. ; Kaplan, A. P. ; Gallion, S. L. ; Hogan, J. C., Jr. ; Craik, C. S. ; Stroud, R. M.** A new class of HIV-1 protease inhibitor: the crystallographic structure, inhibition and chemical synthesis of an aminimide peptide isostere. *Bioorg.Med.Chem.,* 1996, vol. 4, 1545-1558
- **Sambrook, J. ; Fritsch, E. F. ; Maniatis, T.** Molecular Cloning: A Laboratory Manual. Cold Spring Harbor Laboratory Press, 1989
- **Schlappi, M. ; Smith, D. ; Fedoroff, N.** TnpA trans-activates methylated maize Suppressor-mutator transposable elements in transgenic tobacco. *Genetics,* 1993, vol. 133, 1009-1021
- **Shioda, T. ; Andriole, S. ; Yahata, T. ; Isselbacher, K. J.** A green fluorescent protein-reporter mammalian two-hybrid system with extrachromosomal maintenance of a prey expression plasmid: Application to interaction screening. *Proc.Natl.Acad.Sci.U.S.A,* 2000, vol. 97, 5220-5224
- **Smulling, T. ; Rupp, H.M. ; Frank, M ; Schafer, S..** Advances in Regulation of Plant Growth and Development. Peres, Prague, 1999, 85-96
- **Tamura, R. N. ; Cooper, H. M. ; Collo, G. ; Quaranta, V.** Cell type-specific integrin variants with alternative alpha chain cytoplasmic domains. *Proc.Natl.Acad.Sci.U.S.A,* 1991, vol. 88, 10183-10187
- **Werner, T. ; Vadau Motyka ; Miroslav Strnad ; Thomas Schmülling.** Regulation of plant growth by cytokinin. *Proc. Nat. Acad. Sci.,* 2001, vol. 58 (18), 10487-10492
- **Van Haaren, M. J. ; Sedee, N. J. ; Schilperoort, R. A. ; Hooykaas, P. J.** Overdrive is a T-region transfer enhancer which stimulates T-strand production in Agrobacterium tumefaciens. *Nucleic Acids Res.,* 1987, vol. 15, 8983-8997
- **Van Sluys, M. A. ; Tempe, J. ; Fedoroff, N.** Studies on the introduction and mobility of the maize Activator element in Arabidopsis thaliana and Daucus carota. *EMBO J.,* 1987, vol. 6, 3881-3889
- **Wang, K. ; Genetello, C. ; Van Montagu, M. ; Zambryski, P. C.** Sequence context of the T-DNA border repeat element determines its relative activity during T-DNA transfer to plant cells. *Mol. Gen. Genet.,* 1987, vol. 210, 338-346
- **Woulfe, J. ; Lafortune, L. ; de Nadai, F. ; Kitabgi, P. ; Beaudet, A.** Post-translational processing of the neurotensin/neuromedin N precursor in the central nervous system of the rat-II. Immunohistochemical localization of maturation products. *Neuroscience,* 1994, vol. 60, 167-181
- **Zhang, Y. L. ; Dawe, A. L. ; Jiang, Y. ; Becker, J. M. ; Naider, F.** A superactive peptidomimetic analog of a farnesylated dodecapeptide yeast pheromone. *Biochem.Biophys.Res.Commun.,* 1996, vol. 224, 327-331

SEQUENCE LISTING

<110> SCHMULLING, Thomas WERNER, Tomas

<120> Method for modifying plant morphology, biochemistry and physiology comprising expression of plant cytokinin oxidase

<130> CROP-005-EP2-DIV1

<150> US 10/014,101

<151> 2001-10-12

<160> 36

<170> PatentIn version 3.3

<210> 1
<211> 2236
<212> DNA
<213> Arabidopsis thaliana

<400> 1

atgggattga cctcatcctt acggttccat agacaaaaca acaagacttt cctcggaatc 60
ttcatgatct tagttctaag ctgtatacca ggtagaacca atctttgttc caatcattct 120
gttagtaccc caaaagaatt accttcttca aatccttcag atattcgttc ctcattagtt 180
tcactagatt tggagggtta tataagcttc gacgatgtcc acaatgtggc caaggacttt 240
ggcaacagat accagttacc acctttggca attctacatc caaggtcagt ttttgatatt 300
tcatcgatga tgaagcatat agtacatctg ggctccacct caaatcttac agtagcagct 360
agaggccatg gtcactcgct tcaaggacaa gctctagctc atcaaggtgt tgtcatcaaa 420
atggagtcac ttcgaagtcc tgatatcagg atttataagg ggaagcaacc atatgttgat 480
gtctcaggtg gtgaaatatg gataaacatt ctacgcgaga ctctaaaata cggtctttca 540
ccaaagtcct ggacagacta ccttcatttg accgttggag gtacactatc taatgctgga 600
atcagcggtc aagcattcaa gcatggaccc caaatcaaca acgtctacca gctagagatt 660
gttacaggta tttcattcat gctttatctc tgcggtagtc tcaaaaaaat atgcacctgt 720
aaagaatatc catctcttca tgagcaaaaa cactgacgac tttaaataat ttttgactat 780
aaaacaagag tgcataggca caaatgtgaa atatgcaaca cacaattgta acttgcacca 840
agaaaaaagt tataaaaaca aacaactgat aagcaatata tttccaatat ttaatcaggg 900
aaaggagaag tcgtaacctg ttctgagaag cggaattctg aacttttctt cagtgttctt 960
ggcgggcttg gacagtttgg cataatcacc cgggcacgga tctctcttga accagcaccg 1020
catatggtaa agttctatct tgaacaaagt tcaaacaata tacgctatga ttctaagaac 1080
cactttcctg acacagtcaa ataactttta ataggttaaa tggatcaggg tactctactc 1140
tgacttttct gcattttcaa gggaccaaga atatctgatt tcgaaggaga aaacttttga 1200
ttacgttgaa ggatttgtga taatcaatag aacagacctt ctcaataatt ggcgatcgtc 1260
attcagtccc aacgattcca cacaggcaag cagattcaag tcagatggga aaactcttta 1320
ttgcctagaa gtggtcaaat atttcaaccc agaagaagct agctctatgg atcaggtaag 1380

atgtgaaagc aatatataac tagacttagt ttccacagag agctccaaat caaccgttgg 1440
ctactagcct actaacataa tgaatggttg ccgtgcagga aactggcaag ttactttcag 1500
agttaaatta tattccatcc actttgtttt catctgaagt gccatatatc gagtttctgg 1560
atcgcgtgca tatcgcagag agaaaactaa gagcaaaggg tttatgggag gttccacatc 1620
cctggctgaa tctcctgatt cctaagagca gcatatacca atttgctaca gaagttttca 1680
acaacattct cacaagcaac aacaacggtc ctatccttat ttatccagtc aatcaatcca 1740
agtaagtgag caaaatgcca aaagcaaatg cgtccagtga ttctgaaaca taaattacta 1800
accatatcca acattttgtg gtttcaggtg gaagaaacat acatctttga taactccaaa 1860
tgaagatata ttctatctcg tagcctttct cccctctgca gtgccaaatt cctcagggaa 1920
aaacgatcta gagtaccttt tgaaacaaaa ccaaagagtt atgaacttct gcgcagcagc 1980
aaacctcaac gtgaagcagt atttgcccca ttatgaaact caaaaagagt ggaaatcaca 2040
ctttggcaaa agatgggaaa catttgcaca gaggaaacaa gcctacgacc ctctagcgat 2100
tctagcacct ggccaaagaa tattccaaaa gacaacagga aaattatctc ccatccaact 2160
cgcaaagtca aaggcaacag gaagtcctca aaggtaccat tacgcatcaa tactgccgaa 2220
acctagaact gtataa 2236

<210> 2
<211> 575
<212> PRT
<213> Arabidopsis thaliana

<400> 2

Met Gly Leu Thr Ser Ser Leu Arg Phe His Arg Gln Asn Asn Lys Thr
1 5 10 15

Phe Leu Gly Ile Phe Met Ile Leu Val Leu Ser Cys Ile Pro Gly Arg
20 25 30

Thr Asn Leu Cys Ser Asn His Ser Val Ser Thr Pro Lys Glu Leu Pro
35 40 45

Ser Ser Asn Pro Ser Asp Ile Arg Ser Ser Leu Val Ser Leu Asp Leu
50 55 60

Glu Gly Tyr Ile Ser Phe Asp Asp Val His Asn Val Ala Lys Asp Phe
65 70 75 80

Gly Asn Arg Tyr Gln Leu Pro Pro Leu Ala Ile Leu His Pro Arg Ser
85 90 95

Val Phe Asp Ile Ser Ser Met Met Lys His Ile Val His Leu Gly Ser
100 105 110

Thr Ser Asn Leu Thr Val Ala Ala Arg Gly His Gly His Ser Leu Gln
115 120 125

Gly Gln Ala Leu Ala His Gln Gly Val Val Ile Lys Met Glu Ser Leu
    130                 135                 140

Arg Ser Pro Asp Ile Arg Ile Tyr Lys Gly Lys Gln Pro Tyr Val Asp
145                 150                 155                 160

Val Ser Gly Gly Glu Ile Trp Ile Asn Ile Leu Arg Glu Thr Leu Lys
                165                 170                 175

Tyr Gly Leu Ser Pro Lys Ser Trp Thr Asp Tyr Leu His Leu Thr Val
            180                 185                 190

Gly Gly Thr Leu Ser Asn Ala Gly Ile Ser Gly Gln Ala Phe Lys His
        195                 200                 205

Gly Pro Gln Ile Asn Asn Val Tyr Gln Leu Glu Ile Val Thr Gly Lys
    210                 215                 220

Gly Glu Val Val Thr Cys Ser Glu Lys Arg Asn Ser Glu Leu Phe Phe
225                 230                 235                 240

Ser Val Leu Gly Gly Leu Gly Gln Phe Gly Ile Ile Thr Arg Ala Arg
                245                 250                 255

Ile Ser Leu Glu Pro Ala Pro His Met Val Lys Trp Ile Arg Val Leu
            260                 265                 270

Tyr Ser Asp Phe Ser Ala Phe Ser Arg Asp Gln Glu Tyr Leu Ile Ser
        275                 280                 285

Lys Glu Lys Thr Phe Asp Tyr Val Glu Gly Phe Val Ile Ile Asn Arg
    290                 295                 300

Thr Asp Leu Leu Asn Asn Trp Arg Ser Ser Phe Ser Pro Asn Asp Ser
305                 310                 315                 320

Thr Gln Ala Ser Arg Phe Lys Ser Asp Gly Lys Thr Leu Tyr Cys Leu
                325                 330                 335

Glu Val Val Lys Tyr Phe Asn Pro Glu Glu Ala Ser Ser Met Asp Gln
            340                 345                 350

Glu Thr Gly Lys Leu Leu Ser Glu Leu Asn Tyr Ile Pro Ser Thr Leu
        355                 360                 365

Phe Ser Ser Glu Val Pro Tyr Ile Glu Phe Leu Asp Arg Val His Ile
    370                 375                 380

Ala Glu Arg Lys Leu Arg Ala Lys Gly Leu Trp Glu Val Pro His Pro
385                 390                 395                 400

Trp Leu Asn Leu Leu Ile Pro Lys Ser Ser Ile Tyr Gln Phe Ala Thr
405 410 415

Glu Val Phe Asn Asn Ile Leu Thr Ser Asn Asn Asn Gly Pro Ile Leu
420 425 430

Ile Tyr Pro Val Asn Gln Ser Lys Trp Lys Lys His Thr Ser Leu Ile
435 440 445

Thr Pro Asn Glu Asp Ile Phe Tyr Leu Val Ala Phe Leu Pro Ser Ala
450 455 460

Val Pro Asn Ser Ser Gly Lys Asn Asp Leu Glu Tyr Leu Leu Lys Gln
465 470 475 480

Asn Gln Arg Val Met Asn Phe Cys Ala Ala Ala Asn Leu Asn Val Lys
485 490 495

Gln Tyr Leu Pro His Tyr Glu Thr Gln Lys Glu Trp Lys Ser His Phe
500 505 510

Gly Lys Arg Trp Glu Thr Phe Ala Gln Arg Lys Gln Ala Tyr Asp Pro
515 520 525

Leu Ala Ile Leu Ala Pro Gly Gln Arg Ile Phe Gln Lys Thr Thr Gly
530 535 540

Lys Leu Ser Pro Ile Gln Leu Ala Lys Ser Lys Ala Thr Gly Ser Pro
545 550 555 560

Gln Arg Tyr His Tyr Ala Ser Ile Leu Pro Lys Pro Arg Thr Val
565 570 575

<210> 3
<211> 2991
<212> DNA
<213> Arabidopsis thaliana

<400> 3

```
atggctaatc ttcgtttaat gatcacttta atcacggttt taatgatcac caaatcatca     60
aacggtatta aaattgattt acctaaatcc cttaacctca ccctctctac cgatccttcc    120
atcatctccg cagcctctca tgacttcgga aacataacca ccgtgacccc cggcggcgta    180
atctgcccct cctccaccgc tgatatctct cgtctcctcc aatacgccgc aaacggaaaa    240
agtacattcc aagtagcggc tcgtggccaa ggccactcct taaacggcca agcctcggtc    300
tccggcggag taatcgtcaa catgacgtgt atcactgacg tggtggtttc aaaagacaag    360
aagtacgctg acgtggcggc cgggacgtta tgggtggatg tgcttaagaa gacggcggag    420
aaaggggtgt cgccggtttc ttggacggat tatttgcata taaccgtcgg aggaacgttg    480
tcgaatggtg gaattggtgg tcaagtgttt cgaaacggtc ctcttgttag taacgtcctt    540
gaattggacg ttattactgg tacgcatctt ctaaactttg atgtacatac aacaacaaaa    600
```

actgtttttg ttttatagta tttttcattt tttgtaccat aggttttatg ttttatagtt 660
gtgctaaact tcttgcacca cacgtaagtc ttcgaaacac aaaatgcgta acgcatctat 720
atgtttttg tacatattga atgttgttca tgagaaataa agtaattaca tatacacaca 780
tttattgtcg tacatatata aataattaaa gacaaatttt cacaattggt agcgtgttaa 840
tttgggattt ttgtaatgta catgcatgac gcatgcatat ggagcttttc ggttttctta 900
gatttgtgta gtatttcaaa tatatcattt attttctttc gaataaagag gtggtatatt 960
tttaaaatag caacatttca gaatttttct ttgaatttac actttttaaa ttgttattgt 1020
taatatggat tttgaataaa taatttcagg gaaaggtgaa atgttgacat gctcgcgaca 1080
gctaaaccca gaattgttct atggagtgtt aggaggtttg ggtcaatttg gaattataac 1140
gagagccaga attgttttgg accatgcacc taaacgggta cgtatcatca tattttacca 1200
tttgttttag tcagcattca tttttcatta gtaattccgt ttcaatttct aaattttttt 1260
agtcaataga aaatgattct tatgtcagag cttgattatt tagtgatttt tattgagata 1320
aaataaaata taacctaacg gaaataatta ttttactaat cggataatgt ctgattaaaa 1380
cattttatga tattacacta agagagttag agacgtatgg atcacaaaac atgaagcttt 1440
cttagatggt atcctaaaac taaagttagg tacaagtttg gaatttaggt caaatgctta 1500
agttgcatta atttgaacaa aatctatgca ttgaataaaa aaaagatatg gattatttta 1560
taaagtatag tccttgtaat cctaggactt gttgtctaat cttgtcttat gcgtgcaaat 1620
ctttttgatg tcaatatata atccttgttt attagagtca agctctttca ttagtcaact 1680
actcaaatat actccaaagt ttagaatata gtcttctgac taattagaat cttacaaccg 1740
ataaacgtta caatttggtt atcattttaa aaaacagatt tggtcataat atacgatgac 1800
gttctgtttt agtttcatct attcacaaat tttatataat tattttcaag aaaatattga 1860
aatactatac tgtaatatgg tttctttata tatgtgtgta taaattaaat gggattgttt 1920
tctctaaatg aaattgtgta ggccaaatgg tttcggatgc tctacagtga tttcacaact 1980
tttacaaagg accaagaacg tttgatatca atggcaaacg atattggagt cgactattta 2040
gaaggtcaaa tatttctatc aaacggtgtc gttgacacct ctttttttccc accttcagat 2100
caatctaaag tcgctgatct agtcaagcaa cacggtatca tctatgttct tgaagtagcc 2160
aagtattatg atgatcccaa tctccccatc atcagcaagg tactacacat ttacattttc 2220
atcatcgttt ttatcatacc ataagatatt taaatgattc atcattgcac cacattaaga 2280
tattcatcat catcatcgtt acattttttt ttgcatctta tgcttctcat aatctactat 2340
tgtgtaggtt attgacacat taacgaaaac attaagttac ttgcccgggt tcatatcaat 2400
gcacgacgtg gcctacttcg atttcttgaa ccgtgtacat gtcgaagaaa ataaactcag 2460
atctttggga ttatgggaac ttcctcatcc ttggcttaac ctctacgttc ctaaatctcg 2520
gattctcgat tttcataacg gtgttgtcaa agacattctt cttaagcaaa aatcagcttc 2580
gggactcgct cttctctatc caacaaaccg gaataagtac atacttctct tcattcatat 2640

```
ttatcttcaa gaaccaaagt aaataaattt ctatgaactg attatgctgt tattgttaga    2700
tgggacaatc gtatgtcggc gatgatacca gagatcgatg aagatgttat atatattatc    2760
ggactactac aatccgctac cccaaaggat cttccagaag tggagagcgt taacgagaag    2820
ataattaggt tttgcaagga ttcaggtatt aagattaagc aatatctaat gcattatact    2880
agtaaagaag attggattga gcattttgga tcaaaatggg atgatttttc gaagaggaaa    2940
gatctatttg atcccaagaa actgttatct ccagggcaag acatcttttg a             2991
```

<210> 4
<211> 501
<212> PRT
<213> Arabidopsis thaliana

<400> 4

Met Ala Asn Leu Arg Leu Met Ile Thr Leu Ile Thr Val Leu Met Ile
1               5                   10                  15

Thr Lys Ser Ser Asn Gly Ile Lys Ile Asp Leu Pro Lys Ser Leu Asn
            20                  25                  30

Leu Thr Leu Ser Thr Asp Pro Ser Ile Ile Ser Ala Ala Ser His Asp
        35                  40                  45

Phe Gly Asn Ile Thr Thr Val Thr Pro Gly Gly Val Ile Cys Pro Ser
    50                  55                  60

Ser Thr Ala Asp Ile Ser Arg Leu Leu Gln Tyr Ala Ala Asn Gly Lys
65                  70                  75                  80

Ser Thr Phe Gln Val Ala Ala Arg Gly Gln Gly His Ser Leu Asn Gly
                85                  90                  95

Gln Ala Ser Val Ser Gly Gly Val Ile Val Asn Met Thr Cys Ile Thr
            100                 105                 110

Asp Val Val Val Ser Lys Asp Lys Lys Tyr Ala Asp Val Ala Ala Gly
        115                 120                 125

Thr Leu Trp Val Asp Val Leu Lys Lys Thr Ala Glu Lys Gly Val Ser
    130                 135                 140

Pro Val Ser Trp Thr Asp Tyr Leu His Ile Thr Val Gly Gly Thr Leu
145                 150                 155                 160

Ser Asn Gly Gly Ile Gly Gly Gln Val Phe Arg Asn Gly Pro Leu Val
                165                 170                 175

Ser Asn Val Leu Glu Leu Asp Val Ile Thr Gly Lys Gly Glu Met Leu
            180                 185                 190

Thr Cys Ser Arg Gln Leu Asn Pro Glu Leu Phe Tyr Gly Val Leu Gly

195 200 205

Gly Leu Gly Gln Phe Gly Ile Ile Thr Arg Ala Arg Ile Val Leu Asp
210 215 220

His Ala Pro Lys Arg Ala Lys Trp Phe Arg Met Leu Tyr Ser Asp Phe
225 230 235 240

Thr Thr Phe Thr Lys Asp Gln Glu Arg Leu Ile Ser Met Ala Asn Asp
245 250 255

Ile Gly Val Asp Tyr Leu Glu Gly Gln Ile Phe Leu Ser Asn Gly Val
260 265 270

Val Asp Thr Ser Phe Phe Pro Pro Ser Asp Gln Ser Lys Val Ala Asp
275 280 285

Leu Val Lys Gln His Gly Ile Ile Tyr Val Leu Glu Val Ala Lys Tyr
290 295 300

Tyr Asp Asp Pro Asn Leu Pro Ile Ile Ser Lys Val Ile Asp Thr Leu
305 310 315 320

Thr Lys Thr Leu Ser Tyr Leu Pro Gly Phe Ile Ser Met His Asp Val
325 330 335

Ala Tyr Phe Asp Phe Leu Asn Arg Val His Val Glu Glu Asn Lys Leu
340 345 350

Arg Ser Leu Gly Leu Trp Glu Leu Pro His Pro Trp Leu Asn Leu Tyr
355 360 365

Val Pro Lys Ser Arg Ile Leu Asp Phe His Asn Gly Val Val Lys Asp
370 375 380

Ile Leu Leu Lys Gln Lys Ser Ala Ser Gly Leu Ala Leu Leu Tyr Pro
385 390 395 400

Thr Asn Arg Asn Lys Trp Asp Asn Arg Met Ser Ala Met Ile Pro Glu
405 410 415

Ile Asp Glu Asp Val Ile Tyr Ile Ile Gly Leu Leu Gln Ser Ala Thr
420 425 430

Pro Lys Asp Leu Pro Glu Val Glu Ser Val Asn Glu Lys Ile Ile Arg
435 440 445

Phe Cys Lys Asp Ser Gly Ile Lys Ile Lys Gln Tyr Leu Met His Tyr
450 455 460

Thr Ser Lys Glu Asp Trp Ile Glu His Phe Gly Ser Lys Trp Asp Asp
465 470 475 480

Phe Ser Lys Arg Lys Asp Leu Phe Asp Pro Lys Lys Leu Leu Ser Pro
485 490 495

Gly Gln Asp Ile Phe
500

<210> 5
<211> 3302
<212> DNA
<213> Arabidopsis thaliana

<400> 5

atggcgagtt ataatcttcg ttcacaagtt cgtcttatag caataacaat agtaatcatc 60
attactctct caactccgat cacaaccaac acatcaccac aaccatggaa tatcctttca 120
cacaacgaat tcgccggaaa actcacctcc tcctcctcct ccgtcgaatc agccgccaca 180
gatttcggcc acgtcaccaa aatcttccct tccgccgtct taatcccttc ctccgttgaa 240
gacatcacag atctcataaa actctctttt gactctcaac tgtcttttcc tttagccgct 300
cgtggtcacg gacacagcca ccgtggccaa gcctcggcta aagacggagt tgtggtcaac 360
atgcggtcca tggtaaaccg ggatcgaggt atcaaggtgt ctaggacctg tttatatgtt 420
gacgtggacg ctgcgtggct atggattgag gtgttgaata aaactttgga gttagggtta 480
acgccggttt cttggacgga ttatttgtat ttaacagtcg gtgggacgtt atcaaacggc 540
ggaattagtg gacaaacgtt tcggtacggt ccacagatca ctaatgttct agagatggat 600
gttattactg gtacgtacca cgatcttttt cacacagaga ttaaaaaaaa cagtaatagt 660
gattttaact tcgtacgttt ctgatagaca acaaagaact tcgtacgttt ttcgaagttt 720
tttcgtcttt ttcattttag atctgcgcgg ccatttttgg ttatgctatt gtttgtttgt 780
attgtttgtc tctgtttatt tatttctcga acttgttgat agcttttctt cttttcacac 840
atcaatctaa tcaccttttt tggtcttaag attagaaaga agatacggac taggtaaaaa 900
taggtggttg taaacgtaga cgcattaaaa aaatattggt tttttatttt tttgataagc 960
aaaattggtg gttggtctaa gattataaac ttgatattaa tgcaaaggtc gatctagcaa 1020
tagaagatta atcaatattc ttggtgtttt aacaacagat tatttcatca ttaaaatcgt 1080
gaaacaaaga aattttggta gtatacatta cgtgtagttt tgttagttta ttaaaaaaaa 1140
tagtatatag ttttgttaaa acgcgattta tttagtaaca cattagtata ttacacgttt 1200
aaccaactaa actttttttt ttgaataatt atgttctata tttcttactc aaattatgca 1260
aatttcgtgg attcgaagtc aaatttctgc gaaatttaca tggtcatata ttataaaact 1320
gttcatataa cccggtgaac aaacagacaa ttaagggttt gaatggttac ggcggttggg 1380
gcggacacaa ccgtcaatag atcagaccgt tttttattta ccattcatca attatattcc 1440
gcagtggttt ggggtaaaaa aaatagaaga aaaccgcagc ggaccaattc cataccgttt 1500
ttacatacaa ataaacatgg tgcgcaacgg tttattgtcc gcctcaaaaa tgaaatggac 1560
taaaccgcag ataaattaga ccgctttgtc cgctgcctcc attcatagac taaaaaaaaa 1620

```
caaccaaaaa aaaaatggtc ccacgcccat gattttacac gaggtttctt gtggcgtaag    1680
gacaaaactc aaaagttcat aacgtttggt cctaaccagg tgtaatggat taagtaacag    1740
tcaattttct tattatagct gtatccatta tgtccacata tgcatccata tacattacac    1800
tgttggtctc aagtgtagtt agattacgaa gactttcaag ttccattttt tggttaggag    1860
ataaacataa tttaatgata ccgactttag cactctaggc tcaaaacaag tacagaagag    1920
aatagtttta tttcaaactc gttgcattgt tgtatcaatt aattgtgtta gtctttgtat    1980
attcttacat aacggtccaa gtttgttgaa atagtttact tactaaactt ttcctaatgg    2040
ggtcaaattt tattttatag gaaaaggaga gattgcaact tgttccaagg acatgaactc    2100
ggatcttttc ttcgcggtgt taggaggttt gggtcaattc ggcattataa caagagccag    2160
aattaaactt gaagtagctc cgaaaagggt atgttaaatt tgtaaattat gcaactacag    2220
aaaattctat gaaatttatg aatgaacata tatgcatttt tggatttttg taggccaagt    2280
ggttaaggtt tctatacata gatttctccg aattcacaag agatcaagaa cgagtgatat    2340
cgaaaacgga cggtgtagat ttcttagaag gttccattat ggtggaccat ggcccaccgg    2400
ataactggag atccacgtat tatccaccgt ccgatcactt gaggatcgcc tcaatggtca    2460
aacgacatcg tgtcatctac tgccttgaag tcgtcaagta ttacgacgaa acttctcaat    2520
acacagtcaa cgaggtccgt acatacatac aatcataaat catacatgta taattgggag    2580
atctttatgc attattcaat tatattaatt tactttagtt atttaactta tgcaggaaat    2640
ggaggagtta agcgatagtt taaaccatgt aagagggttt atgtacgaga aagatgtgac    2700
gtatatggat ttcctaaacc gagttcgaac cggagagcta aacctgaaat ccaaaggcca    2760
atgggatgtt ccacatccat ggcttaatct cttcgtacca aaaactcaaa tctccaaatt    2820
tgatgatggt gtttttaagg gtattatcct aagaaataac atcactagcg gtcctgttct    2880
tgtttatcct atgaatcgca acaagtaagt ttaactcgat attgcaaaat ttactatcta    2940
cattttcgtt ttggaatccg aaatattctt acaagctaat tttatgcggc gtttttaggt    3000
ggaatgatcg gatgtctgcc gctatacccg aggaagatgt attttatgcg gtagggtttt    3060
taagatccgc gggttttgac aattgggagg cttttgatca agaaaacatg gaaatactga    3120
agttttgtga ggatgctaat atgggggtta tacaatatct tccttatcat tcatcacaag    3180
aaggatgggt tagacatttt ggtccgaggt ggaatatttt cgtagagaga aaatataaat    3240
atgatcccaa aatgatatta tcaccgggac aaaatatatt tcaaaaaata aactcgagtt    3300
ag                                                                   3302
```

<210> 6
<211> 523
<212> PRT
<213> Arabidopsis thaliana

<400> 6

```
Met Ala Ser Tyr Asn Leu Arg Ser Gln Val Arg Leu Ile Ala Ile Thr
1               5                   10                  15
```

Ile Val Ile Ile Ile Thr Leu Ser Thr Pro Ile Thr Thr Asn Thr Ser
20 25 30

Pro Gln Pro Trp Asn Ile Leu Ser His Asn Glu Phe Ala Gly Lys Leu
35 40 45

Thr Ser Ser Ser Ser Ser Val Glu Ser Ala Ala Thr Asp Phe Gly His
50 55 60

Val Thr Lys Ile Phe Pro Ser Ala Val Leu Ile Pro Ser Ser Val Glu
65 70 75 80

Asp Ile Thr Asp Leu Ile Lys Leu Ser Phe Asp Ser Gln Leu Ser Phe
85 90 95

Pro Leu Ala Ala Arg Gly His Gly His Ser His Arg Gly Gln Ala Ser
100 105 110

Ala Lys Asp Gly Val Val Val Asn Met Arg Ser Met Val Asn Arg Asp
115 120 125

Arg Gly Ile Lys Val Ser Arg Thr Cys Leu Tyr Val Asp Val Asp Ala
130 135 140

Ala Trp Leu Trp Ile Glu Val Leu Asn Lys Thr Leu Glu Leu Gly Leu
145 150 155 160

Thr Pro Val Ser Trp Thr Asp Tyr Leu Tyr Leu Thr Val Gly Gly Thr
165 170 175

Leu Ser Asn Gly Gly Ile Ser Gly Gln Thr Phe Arg Tyr Gly Pro Gln
180 185 190

Ile Thr Asn Val Leu Glu Met Asp Val Ile Thr Gly Lys Gly Glu Ile
195 200 205

Ala Thr Cys Ser Lys Asp Met Asn Ser Asp Leu Phe Phe Ala Val Leu
210 215 220

Gly Gly Leu Gly Gln Phe Gly Ile Ile Thr Arg Ala Arg Ile Lys Leu
225 230 235 240

Glu Val Ala Pro Lys Arg Ala Lys Trp Leu Arg Phe Leu Tyr Ile Asp
245 250 255

Phe Ser Glu Phe Thr Arg Asp Gln Glu Arg Val Ile Ser Lys Thr Asp
260 265 270

Gly Val Asp Phe Leu Glu Gly Ser Ile Met Val Asp His Gly Pro Pro
275 280 285

Asp Asn Trp Arg Ser Thr Tyr Tyr Pro Pro Ser Asp His Leu Arg Ile
290 295 300

Ala Ser Met Val Lys Arg His Arg Val Ile Tyr Cys Leu Glu Val Val
305 310 315 320

Lys Tyr Tyr Asp Glu Thr Ser Gln Tyr Thr Val Asn Glu Glu Met Glu
325 330 335

Glu Leu Ser Asp Ser Leu Asn His Val Arg Gly Phe Met Tyr Glu Lys
340 345 350

Asp Val Thr Tyr Met Asp Phe Leu Asn Arg Val Arg Thr Gly Glu Leu
355 360 365

Asn Leu Lys Ser Lys Gly Gln Trp Asp Val Pro His Pro Trp Leu Asn
370 375 380

Leu Phe Val Pro Lys Thr Gln Ile Ser Lys Phe Asp Asp Gly Val Phe
385 390 395 400

Lys Gly Ile Ile Leu Arg Asn Asn Ile Thr Ser Gly Pro Val Leu Val
405 410 415

Tyr Pro Met Asn Arg Asn Lys Trp Asn Asp Arg Met Ser Ala Ala Ile
420 425 430

Pro Glu Glu Asp Val Phe Tyr Ala Val Gly Phe Leu Arg Ser Ala Gly
435 440 445

Phe Asp Asn Trp Glu Ala Phe Asp Gln Glu Asn Met Glu Ile Leu Lys
450 455 460

Phe Cys Glu Asp Ala Asn Met Gly Val Ile Gln Tyr Leu Pro Tyr His
465 470 475 480

Ser Ser Gln Glu Gly Trp Val Arg His Phe Gly Pro Arg Trp Asn Ile
485 490 495

Phe Val Glu Arg Lys Tyr Lys Tyr Asp Pro Lys Met Ile Leu Ser Pro
500 505 510

Gly Gln Asn Ile Phe Gln Lys Ile Asn Ser Ser
515 520

<210> 7
<211> 2782
<212> DNA
<213> Arabidopsis thaliana

<400> 7

atgactaata ctctctgttt aagcctcatc accctaataa cgctttttat aagtttaacc 60

ccaaccttaa tcaaatcaga tgagggcatt gatgttttct tacccatatc actcaacctt 120
acggtcctaa ccgatccctt ctccatctct gccgcttctc acgacttcgg taacataacc 180
gacgaaaatc ccggcgccgt cctctgccct tcctccacca cggaggtggc tcgtctcctc 240
cgtttcgcta acggaggatt ctcttacaat aaaggctcaa ccagccccgc gtctactttc 300
aaaagtggctg ctcgaggcca aggccactcc ctccgtggcc aagcctctgc acccggaggt 360
gtcgtcgtga acatgacgtg tctcgccatg gcggctaaac cagcggcggt tgttatctcg 420
gcagacggga cttacgctga cgtggctgcc gggacgatgt gggtggatgt tctgaaggcg 480
gcggtggata gaggcgtctc gccggttaca tggacggatt atttgtatct cagcgtcggc 540
gggacgttgt cgaacgctgg aatcggtggt cagacgttta gacacggccc tcagattagt 600
aacgttcatg agcttgacgt tattaccggt acgtaaatac caaaacttca ctaatctcgt 660
tacaattttt taattttttg gtaatataaa ttttgtacgg ctcaactctt aattaagaat 720
gaaacagtat ctatgatctt ctagatgctc tttttttgtc tgcaagcttt aattgtagta 780
acatcagcga tatatatatc acatgcatgt gtattattga tgataatata taatgtttta 840
gttacaaatt tgattctcaa ggtaaaactc acacgccata accagtataa aactccaaaa 900
atcacgtttt ggtcagaaat acatatcctt cattaacagt agttatgcta taatttgtga 960
ttataaataa ctccggagtt tgttcacaat actaaatttc aggaaaaggt gaaatgatga 1020
cttgctctcc aaagttaaac cctgaattgt tctatggagt tttaggaggt ttgggtcaat 1080
tcggtattat aacgagggcc aggattgcgt tggatcatgc acccacaagg gtatgtatca 1140
tgcatctata gtgtaatcaa tttataattt taatgtagtg gtcctaaatc caaaatttga 1200
tttgatttgg ttggaacgta cgtatatata ataagtcaaa aggctgattt tgaagacgaa 1260
tttatatact tttgttgaat taaatctgat tttgcttacg ttttattaga ttctgcgtaa 1320
taaatcctag gacttgctcg agtgtaatct tgtcttatgc ttgcaaatct tgttgatgtc 1380
aatatctaat ctttttttatt atatttccct acgtaagttt tagatatagt tattttaaac 1440
tgctataaat tgtgtacgta tagactttag ataaaaagtt gtggtcgctt gcacctattt 1500
gtttatcgct atagtgattc aaaggtctat atatgattct tggtttttct ttttgaaaaa 1560
aatagaccat acaatccaag gaagatgatc ttaaatggac taatttatgg atataaattg 1620
atatacaaat ctgcaggtga aatggtctcg catactctac agtgacttct cggcttttaa 1680
aagagaccaa gagcgtttaa tatcaatgac caatgatctc ggagttgact ttttggaagg 1740
tcaacttatg atgtcaaatg gcttcgtaga cacctctttc ttcccactct ccgatcaaac 1800
aagagtcgca tctcttgtga atgaccaccg gatcatctat gttctcgaag tagccaagta 1860
ttatgacaga accacccttc ccattattga ccaggtacta aaatccatta ttcatgatga 1920
ttatcttcac acaatcagta tcatcaccaa ttaccatcat cacttgtcat atatgatcca 1980
aagtaaatat atcacatgat ataaataaat cgttcaaatc ttttttttta aagaataaaa 2040
gaatcatttt caagcattac tcatacacat ctacgaatca ccgtgaccat atataaccat 2100
acgcttatta aataatcatt tttgtttgta ggtgattgac acgttaagta gaactctagg 2160

tttcgctcca gggtttatgt tcgtacaaga tgttccgtat ttcgatttct tgaaccgtgt 2220
ccgaaacgaa gaagataaac tcagatcttt aggactatgg gaagttcctc atccatggct 2280
taacatcttt gtcccggggt ctcgaatcca agattttcat gatggtgtta ttaatggcct 2340
tcttctaaac caaacctcaa cttctggtgt tactctcttc tatcccacaa accgaaacaa 2400
gtaaatattt actttttgat tttgttttat ttgaaagtat atcccaataa tgtatgttaa 2460
attgttaaca agaatttatt ttattaatag atggaacaac cgcatgtcaa cgatgacacc 2520
ggacgaagat gtttttttatg tgatcggatt actgcaatca gctggtggat ctcaaaattg 2580
gcaagaactt gaaaatctca acgacaaggt tattcagttt tgtgaaaact cgggaattaa 2640
gattaaggaa tatttgatgc actatacaag aaaagaagat tgggttaaac attttggacc 2700
aaaatgggat gattttttaa gaaagaaaat tatgtttgat cccaaaagac tattgtctcc 2760
aggacaagac atatttaatt aa 2782

<210> 8
<211> 524
<212> PRT
<213> Arabidopsis thaliana

<400> 8

Met Thr Asn Thr Leu Cys Leu Ser Leu Ile Thr Leu Ile Thr Leu Phe
1 5 10 15

Ile Ser Leu Thr Pro Thr Leu Ile Lys Ser Asp Glu Gly Ile Asp Val
20 25 30

Phe Leu Pro Ile Ser Leu Asn Leu Thr Val Leu Thr Asp Pro Phe Ser
35 40 45

Ile Ser Ala Ala Ser His Asp Phe Gly Asn Ile Thr Asp Glu Asn Pro
50 55 60

Gly Ala Val Leu Cys Pro Ser Ser Thr Thr Glu Val Ala Arg Leu Leu
65 70 75 80

Arg Phe Ala Asn Gly Gly Phe Ser Tyr Asn Lys Gly Ser Thr Ser Pro
85 90 95

Ala Ser Thr Phe Lys Val Ala Ala Arg Gly Gln Gly His Ser Leu Arg
100 105 110

Gly Gln Ala Ser Ala Pro Gly Gly Val Val Val Asn Met Thr Cys Leu
115 120 125

Ala Met Ala Ala Lys Pro Ala Ala Val Val Ile Ser Ala Asp Gly Thr
130 135 140

Tyr Ala Asp Val Ala Ala Gly Thr Met Trp Val Asp Val Leu Lys Ala
145 150 155 160

Ala Val Asp Arg Gly Val Ser Pro Val Thr Trp Thr Asp Tyr Leu Tyr
165 170 175

Leu Ser Val Gly Gly Thr Leu Ser Asn Ala Gly Ile Gly Gly Gln Thr
180 185 190

Phe Arg His Gly Pro Gln Ile Ser Asn Val His Glu Leu Asp Val Ile
195 200 205

Thr Gly Lys Gly Glu Met Met Thr Cys Ser Pro Lys Leu Asn Pro Glu
210 215 220

Leu Phe Tyr Gly Val Leu Gly Gly Leu Gly Gln Phe Gly Ile Ile Thr
225 230 235 240

Arg Ala Arg Ile Ala Leu Asp His Ala Pro Thr Arg Val Lys Trp Ser
245 250 255

Arg Ile Leu Tyr Ser Asp Phe Ser Ala Phe Lys Arg Asp Gln Glu Arg
260 265 270

Leu Ile Ser Met Thr Asn Asp Leu Gly Val Asp Phe Leu Glu Gly Gln
275 280 285

Leu Met Met Ser Asn Gly Phe Val Asp Thr Ser Phe Phe Pro Leu Ser
290 295 300

Asp Gln Thr Arg Val Ala Ser Leu Val Asn Asp His Arg Ile Ile Tyr
305 310 315 320

Val Leu Glu Val Ala Lys Tyr Tyr Asp Arg Thr Thr Leu Pro Ile Ile
325 330 335

Asp Gln Val Ile Asp Thr Leu Ser Arg Thr Leu Gly Phe Ala Pro Gly
340 345 350

Phe Met Phe Val Gln Asp Val Pro Tyr Phe Asp Phe Leu Asn Arg Val
355 360 365

Arg Asn Glu Glu Asp Lys Leu Arg Ser Leu Gly Leu Trp Glu Val Pro
370 375 380

His Pro Trp Leu Asn Ile Phe Val Pro Gly Ser Arg Ile Gln Asp Phe
385 390 395 400

His Asp Gly Val Ile Asn Gly Leu Leu Leu Asn Gln Thr Ser Thr Ser
405 410 415

Gly Val Thr Leu Phe Tyr Pro Thr Asn Arg Asn Lys Trp Asn Asn Arg
420 425 430

Met Ser Thr Met Thr Pro Asp Glu Asp Val Phe Tyr Val Ile Gly Leu
435 440 445

Leu Gln Ser Ala Gly Gly Ser Gln Asn Trp Gln Glu Leu Glu Asn Leu
450 455 460

Asn Asp Lys Val Ile Gln Phe Cys Glu Asn Ser Gly Ile Lys Ile Lys
465 470 475 480

Glu Tyr Leu Met His Tyr Thr Arg Lys Glu Asp Trp Val Lys His Phe
485 490 495

Gly Pro Lys Trp Asp Asp Phe Leu Arg Lys Lys Ile Met Phe Asp Pro
500 505 510

Lys Arg Leu Leu Ser Pro Gly Gln Asp Ile Phe Asn
515 520

<210> 9
<211> 2805
<212> DNA
<213> Arabidopsis thaliana

<400> 9

atgacgtcaa gctttcttct cctgacgttc gccatatgta aactgatcat agccgtgggt 60
ctaaacgtgg gccccagtga gctcctccgc atcggagcca tagatgtcga cggccacttc 120
accgtccacc cttccgactt agcctccgtc tcctcagact tcggtatgct gaagtcacct 180
gaagagccat tggccgtgct tcatccatca tcggccgaag acgtggcacg actcgtcaga 240
acagcttacg gttcagccac ggcgtttccg gtctcagccc gaggccacgg ccattccata 300
aacggacaag ccgcggcggg gaggaacggt gtggtggttg aaatgaacca cggcgtaacc 360
gggacgccca agccactcgt ccgaccggat gaaatgtatg tggatgtatg gggtggagag 420
ttatgggtcg atgtgttgaa gaaaacgttg gagcatggct tagcaccaaa atcatggacg 480
gattacttgt atctaaccgt tggaggtaca ctctccaatg caggaatcag tggtcaagct 540
tttcaccatg gtcctcaaat tagtaacgtc cttgagctcg acgttgtaac tggttagtat 600
taaaacattc aagttcatat attttaaatg cttttgtctg aagttttact aataacaaga 660
aattgatacc aaaaagtagg gaaaggagag gtgatgagat gctcagaaga agagaacaca 720
aggctattcc atggagttct tggtggatta ggtcaatttg ggatcatcac tcgagcacga 780
atctctctcg aaccagctcc ccaaagggta atattttttt aatgactagc tatcaaaaat 840
ccctggcggg tccatacgtt gtaatctttt tagtttttac tgttgatggt atttttttata 900
tattttggat aataaaaccc taaaatggta tattgtgatg acaggtgaga tggatacggg 960
tattgtattc gagcttcaaa gtgtttacgg aggaccaaga gtacttaatc tcaatgcatg 1020
gtcaattaaa gtttgattac gtggaaggtt ttgtgattgt ggacgaagga ctcgtcaaca 1080
attggagatc ttctttcttc tctccacgta accccgtcaa gatctcctct gttagttcca 1140

```
acggctctgt tttgtattgc cttgagatca ccaagaacta ccacgactcc gactccgaaa   1200
tcgttgatca ggtcactttc attattcact tagaaaaaag cgatattttc atttttttata   1260
ttgatgaata tctggaagga tttaacgcta tgcgactatt gggaaatcat tatgaaaaaa   1320
tatttagttt atatgattga aagtggtctc catagtattt ttgttgtgtc gactttatta   1380
taacttaaat ttggaagagg acatgaagaa gaagccagag aggatctaca gagatctagc   1440
ttttccacct gaacttaata atgcacattt atataattat ttttcttctt ctaaagttta   1500
gtttatcact agcgaattaa tcatggttac taattaagta gtggacaggg tcatggacca   1560
ctcactcacc aaataatgat tcctctttac tcttaagttt aattttaata aaaccaactc   1620
tactggaatc ttaacttatc cttggttttg gtaggctttt atagcaacac ggttttttta   1680
attttcctat tccagatttt gtatattaaa tgtcgatttt ttttcttttt gtttcaggaa   1740
gttgagattc tgatgaagaa attgaatttc ataccgacat cggtctttac aacggattta   1800
caatatgtgg actttctcga ccgggtacac aaggccgaat tgaagctccg gtccaagaat   1860
ttatgggagg ttccacaccc atggctcaac ctcttcgtgc caaaatcaag aatctctgac   1920
ttcgataaag gcgttttcaa gggcattttg ggaaataaaa caagtggccc tattcttatc   1980
taccccatga acaaagacaa gtaagtcttg acattaccat tgattactac ttctaaattt   2040
cttctctaga aaaaagaata aaacgagttt tgcattgcat gcatgcaaag ttacacttgt   2100
ggggattaat tagtggtcca agaaaaaaag tttgtcaaaa ttgaaaaaaa ctagacacgt   2160
ggtacatggg attgtccgaa aaacgttgtc cacatgtgca tcgaaccagc taagattgac   2220
aacaacactt cgtcggctcg tatttctctt tttgttttgt gaccaaatcc gatggtccag   2280
attgggttta tttgttttta agttcctaga actcatggtg ggtgggtccc aatcagattc   2340
tcctagacca aaccgatctc aacgaaccct ccgcacatca ttgattatta cattaatata   2400
gatattgtcg ttgctgacgt gtcgtaattt gatgttattg tcagatggga cgagaggagc   2460
tcagccgtga cgccggatga ggaagttttc tatctggtgg ctctattgag atcagcttta   2520
acggacggtg aagagacaca gaagctagag tatctgaaag atcagaaccg tcggatcttg   2580
gagttctgtg aacaagccaa gatcaatgtg aagcagtatc ttcctcacca cgcaacacag   2640
gaagagtggg tggctcattt tggggacaag tgggatcggt tcagaagctt aaaggctgag   2700
tttgatccgc gacacatact cgctactggt cagagaatct ttcaaaaccc atctttgtct   2760
ttgtttcctc cgtcgtcgtc ttcttcgtca gcggcttcat ggtga                    2805
```

<210> 10
<211> 536
<212> PRT
<213> Arabidopsis thaliana

<400> 10

Met Thr Ser Ser Phe Leu Leu Leu Thr Phe Ala Ile Cys Lys Leu Ile
1               5                   10                  15

Ile Ala Val Gly Leu Asn Val Gly Pro Ser Glu Leu Leu Arg Ile Gly

20 25 30

Ala Ile Asp Val Asp Gly His Phe Thr Val His Pro Ser Asp Leu Ala
35 40 45

Ser Val Ser Ser Asp Phe Gly Met Leu Lys Ser Pro Glu Glu Pro Leu
50 55 60

Ala Val Leu His Pro Ser Ser Ala Glu Asp Val Ala Arg Leu Val Arg
65 70 75 80

Thr Ala Tyr Gly Ser Ala Thr Ala Phe Pro Val Ser Ala Arg Gly His
85 90 95

Gly His Ser Ile Asn Gly Gln Ala Ala Ala Gly Arg Asn Gly Val Val
100 105 110

Val Glu Met Asn His Gly Val Thr Gly Thr Pro Lys Pro Leu Val Arg
115 120 125

Pro Asp Glu Met Tyr Val Asp Val Trp Gly Gly Glu Leu Trp Val Asp
130 135 140

Val Leu Lys Lys Thr Leu Glu His Gly Leu Ala Pro Lys Ser Trp Thr
145 150 155 160

Asp Tyr Leu Tyr Leu Thr Val Gly Gly Thr Leu Ser Asn Ala Gly Ile
165 170 175

Ser Gly Gln Ala Phe His His Gly Pro Gln Ile Ser Asn Val Leu Glu
180 185 190

Leu Asp Val Val Thr Gly Lys Gly Glu Val Met Arg Cys Ser Glu Glu
195 200 205

Glu Asn Thr Arg Leu Phe His Gly Val Leu Gly Gly Leu Gly Gln Phe
210 215 220

Gly Ile Ile Thr Arg Ala Arg Ile Ser Leu Glu Pro Ala Pro Gln Arg
225 230 235 240

Val Arg Trp Ile Arg Val Leu Tyr Ser Ser Phe Lys Val Phe Thr Glu
245 250 255

Asp Gln Glu Tyr Leu Ile Ser Met His Gly Gln Leu Lys Phe Asp Tyr
260 265 270

Val Glu Gly Phe Val Ile Val Asp Glu Gly Leu Val Asn Asn Trp Arg
275 280 285

Ser Ser Phe Phe Ser Pro Arg Asn Pro Val Lys Ile Ser Ser Val Ser
290 295 300

```
Ser Asn Gly Ser Val Leu Tyr Cys Leu Glu Ile Thr Lys Asn Tyr His
305                 310                 315                 320

Asp Ser Asp Ser Glu Ile Val Asp Gln Glu Val Glu Ile Leu Met Lys
                325                 330                 335

Lys Leu Asn Phe Ile Pro Thr Ser Val Phe Thr Thr Asp Leu Gln Tyr
            340                 345                 350

Val Asp Phe Leu Asp Arg Val His Lys Ala Glu Leu Lys Leu Arg Ser
        355                 360                 365

Lys Asn Leu Trp Glu Val Pro His Pro Trp Leu Asn Leu Phe Val Pro
    370                 375                 380

Lys Ser Arg Ile Ser Asp Phe Asp Lys Gly Val Phe Lys Gly Ile Leu
385                 390                 395                 400

Gly Asn Lys Thr Ser Gly Pro Ile Leu Ile Tyr Pro Met Asn Lys Asp
                405                 410                 415

Lys Trp Asp Glu Arg Ser Ser Ala Val Thr Pro Asp Glu Glu Val Phe
            420                 425                 430

Tyr Leu Val Ala Leu Leu Arg Ser Ala Leu Thr Asp Gly Glu Glu Thr
        435                 440                 445

Gln Lys Leu Glu Tyr Leu Lys Asp Gln Asn Arg Arg Ile Leu Glu Phe
    450                 455                 460

Cys Glu Gln Ala Lys Ile Asn Val Lys Gln Tyr Leu Pro His His Ala
465                 470                 475                 480

Thr Gln Glu Glu Trp Val Ala His Phe Gly Asp Lys Trp Asp Arg Phe
                485                 490                 495

Arg Ser Leu Lys Ala Glu Phe Asp Pro Arg His Ile Leu Ala Thr Gly
            500                 505                 510

Gln Arg Ile Phe Gln Asn Pro Ser Leu Ser Leu Phe Pro Pro Ser Ser
        515                 520                 525

Ser Ser Ser Ser Ala Ala Ser Trp
    530                 535
```

<210> 11
<211> 1936
<212> DNA
<213> Arabidopsis thaliana

<400> 11

```
atgcttatag taagaagttt caccatcttg cttctcagct gcatagcctt taagttggct      60
```

```
tgctgcttct ctagcagcat ttcttctttg aaggcgcttc ccctagtagg ccatttggag     120
tttgaacatg tccatcacgc ctccaaagat tttggaaatc gataccagtt gatccctttg     180
gcggtcttac atcccaaatc ggtaagcgac atcgcctcaa cgatacgaca catctggatg     240
atgggcactc attcacagct tacagtggca gcgagaggtc gtggacattc actccaaggc     300
caagctcaaa caagacatgg aattgttata cacatggaat cactccatcc ccagaagctg     360
caggtctaca gtgtggattc ccctgctcca tatgttgatg tgtctggtgg tgagctgtgg     420
ataaacattt tgcatgagac cctcaagtac gggcttgcac caaaatcatg gacggattac     480
ctgcatttaa ctgtaggtgg tactctgtcc aatgctggaa taagcggcca ggcattccga     540
catggaccac agatcagcaa tgttcatcaa ctggagattg tcacaggtta gttcagagtt     600
gcagtattcg tgttttgaaa gcatagactc tatatggttg gtgactatta acaacatgaa     660
gagattcccg agaatagcta cccactaatg tcatgcctat ttattgactg caggaaaagg     720
cgagatccta aactgtacaa agaggcagaa cagcgactta tttaatggtg ttcttggtgg     780
tttaggtcag tttggcatca taacgcgggc aagaatagca ttggaaccag caccaaccat     840
ggtaaacaat aaataaataa aaaacttaaa aactgaacac gcgtgtgtcc tcctaactct     900
gtataatgga caggtaaaat ggataagagt gttatacctg gattttgcag cttttgccaa     960
ggaccaagag caactaatat ctgcccaggg ccacaaattc gattacatag aagggtttgt    1020
gataataaac aggacaggcc tcctgaacag ctggaggttg tctttcaccg cagaagagcc    1080
tttagaagca agccaattca agtttgatgg aaggactctg tattgtctgg agctagccaa    1140
gtatttgaag caagataaca aagacgtaat caaccaggtg agaaaacaga gtagaagcaa    1200
tcggtagaat cttctttggt agatgacatt cattggaact gaaaatatat atatatttgt    1260
ccaatccagg aagtgaaaga aacattatca gagctaagct acgtgacgtc gacactgttt    1320
acaacggagg tagcatatga agcattcttg gacagggtac atgtgtctga ggtaaaactc    1380
cgatcgaaag ggcagtggga ggtgccacat ccatggctga acctcctggt accaagaagc    1440
aaaatcaatg aatttgcaag aggtgtattt ggaaacatac taacggatac aagcaacggc    1500
ccagtcatcg tctacccagt gaacaaatca aagtaagaaa gaaagaaaga aagagctagt    1560
catgattttg tttcttttca cttgttgaca aaacaaaagc atgttggtga gcaggtggga    1620
caatcaaaca tcagcagtaa caccggagga agaggtattc tacctggtgg cgatcctaac    1680
atcggcatct ccagggtcgg caggaaagga tggagtagaa gagatcttga ggcggaacag    1740
aagaatactg gaattcagtg aagaagcagg gatagggttg aagcagtatc tgccacatta    1800
cacgacaaga gaagagtgga gatcccattt cggggacaag tggggagaat ttgtgaggag    1860
gaaatccaga tatgatccat tggcaattct tgcgcctggc caccgaattt ttcaaaaggc    1920
agtctcatac tcatga                                                    1936
```

<210> 12
<211> 504
<212> PRT
<213> Arabidopsis thaliana

<400> 12

```
Met Leu Ile Val Arg Ser Phe Thr Ile Leu Leu Leu Ser Cys Ile Ala
1               5                   10                  15

Phe Lys Leu Ala Cys Cys Phe Ser Ser Ser Ile Ser Ser Leu Lys Ala
            20                  25                  30

Leu Pro Leu Val Gly His Leu Glu Phe Glu His Val His His Ala Ser
        35                  40                  45

Lys Asp Phe Gly Asn Arg Tyr Gln Leu Ile Pro Leu Ala Val Leu His
    50                  55                  60

Pro Lys Ser Val Ser Asp Ile Ala Ser Thr Ile Arg His Ile Trp Met
65                  70                  75                  80

Met Gly Thr His Ser Gln Leu Thr Val Ala Ala Arg Gly Arg Gly His
                85                  90                  95

Ser Leu Gln Gly Gln Ala Gln Thr Arg His Gly Ile Val Ile His Met
            100                 105                 110

Glu Ser Leu His Pro Gln Lys Leu Gln Val Tyr Ser Val Asp Ser Pro
        115                 120                 125

Ala Pro Tyr Val Asp Val Ser Gly Gly Glu Leu Trp Ile Asn Ile Leu
    130                 135                 140

His Glu Thr Leu Lys Tyr Gly Leu Ala Pro Lys Ser Trp Thr Asp Tyr
145                 150                 155                 160

Leu His Leu Thr Val Gly Gly Thr Leu Ser Asn Ala Gly Ile Ser Gly
                165                 170                 175

Gln Ala Phe Arg His Gly Pro Gln Ile Ser Asn Val His Gln Leu Glu
            180                 185                 190

Ile Val Thr Gly Lys Gly Glu Ile Leu Asn Cys Thr Lys Arg Gln Asn
        195                 200                 205

Ser Asp Leu Phe Asn Gly Val Leu Gly Gly Leu Gly Gln Phe Gly Ile
    210                 215                 220

Ile Thr Arg Ala Arg Ile Ala Leu Glu Pro Ala Pro Thr Met Asp Gln
225                 230                 235                 240

Glu Gln Leu Ile Ser Ala Gln Gly His Lys Phe Asp Tyr Ile Glu Gly
                245                 250                 255

Phe Val Ile Ile Asn Arg Thr Gly Leu Leu Asn Ser Trp Arg Leu Ser
```

260 265 270

Phe Thr Ala Glu Glu Pro Leu Glu Ala Ser Gln Phe Lys Phe Asp Gly
275 280 285

Arg Thr Leu Tyr Cys Leu Glu Leu Ala Lys Tyr Leu Lys Gln Asp Asn
290 295 300

Lys Asp Val Ile Asn Gln Glu Val Lys Glu Thr Leu Ser Glu Leu Ser
305 310 315 320

Tyr Val Thr Ser Thr Leu Phe Thr Thr Glu Val Ala Tyr Glu Ala Phe
325 330 335

Leu Asp Arg Val His Val Ser Glu Val Lys Leu Arg Ser Lys Gly Gln
340 345 350

Trp Glu Val Pro His Pro Trp Leu Asn Leu Leu Val Pro Arg Ser Lys
355 360 365

Ile Asn Glu Phe Ala Arg Gly Val Phe Gly Asn Ile Leu Thr Asp Thr
370 375 380

Ser Asn Gly Pro Val Ile Val Tyr Pro Val Asn Lys Ser Lys Trp Asp
385 390 395 400

Asn Gln Thr Ser Ala Val Thr Pro Glu Glu Glu Val Phe Tyr Leu Val
405 410 415

Ala Ile Leu Thr Ser Ala Ser Pro Gly Ser Ala Gly Lys Asp Gly Val
420 425 430

Glu Glu Ile Leu Arg Arg Asn Arg Arg Ile Leu Glu Phe Ser Glu Glu
435 440 445

Ala Gly Ile Gly Leu Lys Gln Tyr Leu Pro His Tyr Thr Thr Arg Glu
450 455 460

Glu Trp Arg Ser His Phe Gly Asp Lys Trp Gly Glu Phe Val Arg Arg
465 470 475 480

Lys Ser Arg Tyr Asp Pro Leu Ala Ile Leu Ala Pro Gly His Arg Ile
485 490 495

Phe Gln Lys Ala Val Ser Tyr Ser
500

<210> 13
<211> 31
<212> DNA
<213> Artificial Sequence

<220>

<223> Description of Artificial Sequence:oligonucleotide : primer or probe

<400> 13
cggtcgacat gggattgacc tcatccttac g 31

<210> 14
<211> 35
<212> DNA
<213> Artificial Sequence

<220>
<223> Description of Artificial sequence:oligonucleotide : primer or probe

<400> 14
gcgtcgactt atacagttct aggtttcggc agtat 35

<210> 15
<211> 33
<212> DNA
<213> Artificial sequence

<220>
<223> Description of Artificial Sequence:oligonucleotide : primer or probe

<400> 15
gcggtaccag agagagaaac ataaacaaat ggc 33

<210> 16
<211> 31
<212> DNA
<213> Artificial Sequence

<220>
<223> Description of Artificial Sequence:oligonucleotide : primer or probe

<400> 16
gcggtaccca attttacttc caccaaaatg c 31

<210> 17
<211> 34
<212> DNA
<213> Artificial Sequence

<220>
<223> Description of Artificial sequence:oligonucleotide : primer or probe

<400> 17
gcggtacctt cattgataag aatcaagcta ttca 34

<210> 18
<211> 31
<212> DNA
<213> Artificial sequence

<220>
<223> Description of Artificial sequence:oligonucleotide : primer or probe

<400> 18

gcggtaccca aagtggtgag aacgactaac a 31

<210> 19
<211> 28
<212> DNA
<213> Artificial Sequence

<220>
<223> Description of Artificial Sequence:oligonucleotide : primer or probe

<400> 19
gcggtacccc cattaaccta cccgtttg 28

<210> 20
<211> 32
<212> DNA
<213> Artificial Sequence

<220>
<223> Description of Artificial Sequence:oligonucleotide : primer or probe

<400> 20
gcggtaccag acgatgaacg tacttgtctg ta 32

<210> 21
<211> 28
<212> DNA
<213> Artificial sequence

<220>
<223> Description of Artificial Sequence:oligonucleotide : primer or probe

<400> 21
ggggtacctt gatgaatcgt gaaatgac 28

<210> 22
<211> 31
<212> DNA
<213> Artificial Sequence

<220>
<223> Description of Artificial Sequence:oligonucleotide : primer or probe

<400> 22
ggggtaccct ttcctcttgg ttttgtcctg t 31

<210> 23
<211> 32
<212> DNA
<213> Artificial Sequence

<220>
<223> Description of Artificial Sequence:oligonucleotide : primer or probe

<400> 23
gctctagatc aggaaaagaa ccatgcttat ag 32

<210> 24

<211> 32
<212> DNA
<213> Artificial sequence

<220>
<223> Description of Artificial Sequence:oligonucleotide : primer or probe

<400> 24
gctctagatc atgagtatga gactgccttt tg 32

<210> 25
<211> 1728
<212> DNA
<213> Arabidopsis thaliana

<400> 25

```
atgggattga cctcatcctt acggttccat agacaaaaca acaagacttt cctcggaatc     60
ttcatgatct tagttctaag ctgtatacca ggtagaacca atctttgttc caatcattct    120
gttagtaccc caaaagaatt accttcttca aatccttcag atattcgttc ctcattagtt    180
tcactagatt tggagggtta tataagcttc gacgatgtcc acaatgtggc caaggacttt    240
ggcaacagat accagttacc acctttggca attctacatc caaggtcagt tttgatattt    300
tcatcgatga tgaagcatat agtacatctg ggctccacct caaatcttac agtagcagct    360
agaggccatg gtcactcgct tcaaggacaa gctctagctc atcaaggtgt tgtcatcaaa    420
atggagtcac ttcgaagtcc tgatatcagg atttataagg ggaagcaacc atatgttgat    480
gtctcaggtg gtgaaatatg gataaacatt ctacgcgaga ctctaaaata cggtctttca    540
ccaaagtcct ggacagacta ccttcatttg accgttggag gtacactatc taatgctgga    600
atcagcggtc aagcattcaa gcatggaccc caaatcaaca acgtctacca gctagagatt    660
gttacaggga aaggagaagt cgtaacctgt tctgagaagc ggaattctga acttttcttc    720
agtgttcttg gcgggcttgg acagtttggc ataatcaccc gggcacggat ctctcttgaa    780
ccagcaccgc atatggttaa atggatcagg gtactctact ctgacttttc tgcattttca    840
agggaccaag aatatctgat ttcgaaggag aaaacttttg attacgttga aggatttgtg    900
ataatcaata gaacagacct tctcaataat tggcgatcgt cattcagtcc caacgattcc    960
acacaggcaa gcagattcaa gtcagatggg aaaactcttt attgcctaga agtggtcaaa   1020
tatttcaacc cagaagaagc tagctctatg gatcaggaaa ctggcaagtt actttcagag   1080
ttaaattata ttccatccac tttgttttca tctgaagtgc catatatcga gtttctggat   1140
cgcgtgcata tcgcagagag aaaactaaga gcaaagggtt tatgggaggt tccacatccc   1200
tggctgaatc tcctgattcc taagagcagc atataccaat ttgctacaga agttttcaac   1260
aacattctca caagcaacaa caacggtcct atccttattt atccagtcaa tcaatccaag   1320
tggaagaaac atacatcttt gataactcca aatgaagata tattctatct cgtagccttt   1380
ctcccctctg cagtgccaaa ttcctcaggg aaaaacgatc tagagtacct tttgaaacaa   1440
aaccaaagag ttatgaactt ctgcgcagca gcaaacctca acgtgaagca gtatttgccc   1500
cattatgaaa ctcaaaaaga gtggaaatca cactttggca aaagatggga aacatttgca   1560
cagaggaaac aagcctacga ccctctagcg attctagcac ctggccaaag aatattccaa   1620
aagacaacag gaaaattatc tcccatccaa ctcgcaaagt caaaggcaac aggaagtcct   1680
caaaggtacc attacgcatc aatactgccg aaacctagaa ctgtataa                 1728
```

<210> 26
<211> 1506
<212> DNA
<213> Arabidopsis thaliana

<400> 26

atggctaatc ttcgtttaat gatcacttta atcacggttt taatgatcac caaatcatca 60
aacggtatta aaattgattt acctaaatcc cttaacctca ccctctctac cgatccttcc 120
atcatctccg cagcctctca tgacttcgga aacataacca ccgtgacccc cggcggcgta 180
atctgcccct cctccaccgc tgatatctct cgtctcctcc aatacgccgc aaacggaaaa 240
agtacattcc aagtagcggc tcgtggccaa ggccactcct taaacggcca agcctcggtc 300
tccggcggag taatcgtcaa catgacgtgt atcactgacg tggtggtttc aaaagacaag 360
aagtacgctg acgtggcggc cgggacgtta tgggtggatg tgcttaagaa gacggcggag 420
aaaggggtgt cgccggtttc ttggacggat tatttgcata taaccgtcgg aggaacgttg 480
tcgaatggtg gaattggtgg tcaagtgttt cgaaacggtc ctcttgttag taacgtcctt 540
gaattggacg ttattactgg gaaaggtgaa atgttgacat gctcgcgaca gctaaaccca 600
gaattgttct atggagtgtt aggaggtttg ggtcaatttg gaattataac gagagccaga 660
attgttttgg accatgcacc taaacgggcc aaatggtttc ggatgctcta cagtgatttc 720
acaactttta caaaggacca agaacgtttg atatcaatgg caaacgatat tggagtcgac 780
tatttagaag gtcaaatatt tctatcaaac ggtgtcgttg acacctcttt tttcccacct 840
tcagatcaat ctaaagtcgc tgatctagtc aagcaacacg gtatcatcta tgttcttgaa 900
gtagccaagt attatgatga tcccaatctc cccatcatca gcaaggttat tgacacatta 960
acgaaaacat taagttactt gcccgggttc atatcaatgc acgacgtggc ctacttcgat 1020
ttcttgaacc gtgtacatgt cgaagaaaat aaactcagat ctttgggatt atgggaactt 1080
cctcatcctt ggcttaacct ctacgttcct aaatctcgga ttctcgattt tcataacggt 1140
gttgtcaaag acattcttct taagcaaaaa tcagcttcgg gactcgctct tctctatcca 1200
acaaaccgga ataaatggga caatcgtatg tcggcgatga taccagagat cgatgaagat 1260
gttatatata ttatcggact actacaatcc gctaccccaa aggatcttcc agaagtggag 1320
agcgttaacg agaagataat taggttttgc aaggattcag gtattaagat taagcaatat 1380
ctaatgcatt atactagtaa agaagattgg attgagcatt ttggatcaaa atgggatgat 1440
ttttcgaaga ggaaagatct atttgatccc aagaaactgt tatctccagg gcaagacatc 1500
ttttga 1506

<210> 27
<211> 1572
<212> DNA
<213> Arabidopsis thaliana

<400> 27

```
atggcgagtt ataatcttcg ttcacaagtt cgtcttatag caataacaat agtaatcatc      60
attactctct caactccgat cacaaccaac acatcaccac aaccatggaa tatcctttca     120
cacaacgaat tcgccggaaa actcacctcc tcctcctcct ccgtcgaatc agccgccaca     180
gatttcggcc acgtcaccaa aatcttccct tccgccgtct taatcccttc ctccgttgaa     240
gacatcacag atctcataaa actctctttt gactctcaac tgtcttttcc tttagccgct     300
cgtggtcacg gacacagcca ccgtggccaa gcctcggcta aagacggagt tgtggtcaac     360
atgcggtcca tggtaaaccg ggatcgaggt atcaaggtgt ctaggacctg tttatatgtt     420
gacgtggacg ctgcgtggct atggattgag gtgttgaata aaactttgga gttagggtta     480
acgccggttt cttggacgga ttatttgtat ttaacagtcg gtgggacgtt atcaaacggc     540
ggaattagtg gacaaacgtt tcggtacggt ccacagatca ctaatgttct agagatggat     600
gttattactg gaaaaggaga gattgcaact tgttccaagg acatgaactc ggatcttttc     660
ttcgcggtgt taggaggttt gggtcaattc ggcattataa caagagccag aattaaactt     720
gaagtagctc cgaaaagggc caagtggtta aggtttctat acatagattt ctccgaattc     780
acaagagatc aagaacgagt gatatcgaaa acggacggtg tagatttctt agaaggttcc     840
attatggtgg accatggccc accggataac tggagatcca cgtattatcc accgtccgat     900
cacttgagga tcgcctcaat ggtcaaacga catcgtgtca tctactgcct tgaagtcgtc     960
aagtattacg acgaaacttc tcaatacaca gtcaacgagg aaatggagga gttaagcgat    1020
agtttaaacc atgtaagagg gtttatgtac gagaaagatg tgacgtatat ggatttccta    1080
aaccgagttc gaaccggaga gctaaacctg aaatccaaag gccaatggga tgttccacat    1140
ccatggctta atctcttcgt accaaaaact caaatctcca aatttgatga tggtgttttt    1200
aagggtatta tcctaagaaa taacatcact agcggtcctg ttcttgttta tcctatgaat    1260
cgcaacaagt ggaatgatcg gatgtctgcc gctatacccg aggaagatgt attttatgcg    1320
gtagggtttt taagatccgc gggttttgac aattgggagg cttttgatca agaaaacatg    1380
gaaatactga agttttgtga ggatgctaat atgggggtta tacaatatct tccttatcat    1440
tcatcacaag aaggatgggt tagacatttt ggtccgaggt ggaatatttt cgtagagaga    1500
aaatataaat atgatcccaa aatgatatta tcaccgggac aaaatatatt tcaaaaaata    1560
aactcgagtt ag                                                        1572
```

<210> 28
<211> 1575
<212> DNA
<213> Arabidopsis thaliana

<400> 28

```
atgactaata ctctctgttt aagcctcatc accctaataa cgctttttat aagtttaacc      60
ccaaccttaa tcaaatcaga tgagggcatt gatgttttct tacccatatc actcaacctt     120
```

acggtcctaa ccgatccctt ctccatctct gccgcttctc acgacttcgg taacataacc 180
gacgaaaatc ccggcgccgt cctctgccct tcctccacca cggaggtggc tcgtctcctc 240
cgtttcgcta acggaggatt ctcttacaat aaaggctcaa ccagccccgc gtctactttc 300
aaagtggctg ctcgaggcca aggccactcc ctccgtggcc aagcctctgc acccggaggt 360
gtcgtcgtga acatgacgtg tctcgccatg gcggctaaac cagcggcggt tgttatctcg 420
gcagacggga cttacgctga cgtggctgcc gggacgatgt gggtggatgt tctgaaggcg 480
gcggtggata gaggcgtctc gccggttaca tggacggatt atttgtatct cagcgtcggc 540
gggacgttgt cgaacgctgg aatcggtggt cagacgttta gacacggccc tcagattagt 600
aacgttcatg agcttgacgt tattaccgga aaaggtgaaa tgatgacttg ctctccaaag 660
ttaaaccctg aattgttcta tggagtttta ggaggtttgg gtcaattcgg tattataacg 720
agggccagga ttgcgttgga tcatgcaccc acaagggtga aatggtctcg catactctac 780
agtgacttct cggcttttaa aagagaccaa gagcgtttaa tatcaatgac caatgatctc 840
ggagttgact ttttggaagg tcaacttatg atgtcaaatg gcttcgtaga cacctctttc 900
ttcccactct ccgatcaaac aagagtcgca tctcttgtga atgaccaccg gatcatctat 960
gttctcgaag tagccaagta ttatgacaga accacccttc ccattattga ccaggtgatt 1020
gacacgttaa gtagaactct aggtttcgct ccagggttta tgttcgtaca agatgttccg 1080
tatttcgatt tcttgaaccg tgtccgaaac gaagaagata aactcagatc tttaggacta 1140
tgggaagttc ctcatccatg gcttaacatc tttgtcccgg ggtctcgaat ccaagatttt 1200
catgatggtg ttattaatgg ccttcttcta aaccaaacct caacttctgg tgttactctc 1260
ttctatccca caaaccgaaa caaatggaac aaccgcatgt caacgatgac accggacgaa 1320
gatgtttttt atgtgatcgg attactgcaa tcagctggtg gatctcaaaa ttggcaagaa 1380
cttgaaaatc tcaacgacaa ggttattcag ttttgtgaaa actcgggaat taagattaag 1440
gaatatttga tgcactatac aagaaaagaa gattgggtta aacattttgg accaaaatgg 1500
gatgattttt taagaaagaa aattatgttt gatcccaaaa gactattgtc tccaggacaa 1560
gacatattta attaa 1575

<210> 29
<211> 1611
<212> DNA
<213> Arabidopsis thaliana

<400> 29

atgacgtcaa gctttcttct cctgacgttc gccatatgta aactgatcat agccgtgggt 60
ctaaacgtgg gccccagtga gctcctccgc atcggagcca tagatgtcga cggccacttc 120
accgtccacc cttccgactt agcctccgtc tcctcagact tcggtatgct gaagtcacct 180
gaagagccat tggccgtgct tcatccatca tcggccgaag acgtggcacg actcgtcaga 240
acagcttacg gttcagccac ggcgtttccg gtctcagccc gaggccacgg ccattccata 300
aacggacaag ccgcggcggg gaggaacggt gtggtggttg aaatgaacca cggcgtaacc 360

gggacgccca agccactcgt ccgaccggat gaaatgtatg tggatgtatg gggtggagag 420
ttatgggtcg atgtgttgaa gaaaacgttg gagcatggct tagcaccaaa atcatggacg 480
gattacttgt atctaaccgt tggaggtaca ctctccaatg caggaatcag tggtcaagct 540
tttcaccatg gtcctcaaat tagtaacgtc cttgagctcg acgttgtaac tgggaaagga 600
gaggtgatga gatgctcaga agaagagaac acaaggctat tccatggagt tcttggtgga 660
ttaggtcaat ttgggatcat cactcgagca cgaatctctc tcgaaccagc tccccaaagg 720
gtgagatgga tacgggtatt gtattcgagc ttcaaagtgt ttacggagga ccaagagtac 780
ttaatctcaa tgcatggtca attaaagttt gattacgtgg aaggttttgt gattgtggac 840
gaaggactcg tcaacaattg gagatcttct ttcttctctc cacgtaaccc cgtcaagatc 900
tcctctgtta gttccaacgg ctctgttttg tattgccttg agatcaccaa gaactaccac 960
gactccgact ccgaaatcgt tgatcaggaa gttgagattc tgatgaagaa attgaatttc 1020
ataccgacat cggtctttac aacggattta caatatgtgg actttctcga ccgggtacac 1080
aaggccgaat tgaagctccg gtccaagaat ttatgggagg ttccacaccc atggctcaac 1140
ctcttcgtgc caaaatcaag aatctctgac ttcgataaag gcgttttcaa gggcattttg 1200
ggaaataaaa caagtggccc tattcttatc taccccatga acaaagacaa atgggacgag 1260
aggagctcag ccgtgacgcc ggatgaggaa gttttctatc tggtggctct attgagatca 1320
gctttaacgg acggtgaaga gacacagaag ctagagtatc tgaaagatca gaaccgtcgg 1380
atcttggagt tctgtgaaca agccaagatc aatgtgaagc agtatcttcc tcaccacgca 1440
acacaggaag agtgggtggc tcattttggg gacaagtggg atcggttcag aagcttaaag 1500
gctgagtttg atccgcgaca catactcgct actggtcaga gaatctttca aaacccatct 1560
ttgtctttgt ttcctccgtc gtcgtcttct tcgtcagcgg cttcatggtg a 1611

<210> 30
<211> 1515
<212> DNA
<213> Arabidopsis thaliana

<400> 30

atgcttatag taagaagttt caccatcttg cttctcagct gcatagcctt taagttggct 60
tgctgcttct ctagcagcat ttcttctttg aaggcgcttc ccctagtagg ccatttggag 120
tttgaacatg tccatcacgc ctccaaagat tttggaaatc gataccagtt gatccctttg 180
gcggtcttac atcccaaatc ggtaagcgac atcgcctcaa cgatacgaca catctggatg 240
atgggcactc attcacagct tacagtggca gcgagaggtc gtggacattc actccaaggc 300
caagctcaaa caagacatgg aattgttata cacatggaat cactccatcc ccagaagctg 360
caggtctaca gtgtggattc ccctgctcca tatgttgatg tgtctggtgg tgagctgtgg 420
ataaacattt tgcatgagac cctcaagtac gggcttgcac caaaatcatg gacggattac 480
ctgcatttaa ctgtaggtgg tactctgtcc aatgctggaa taagcggcca ggcattccga 540
catggaccac agatcagcaa tgttcatcaa ctggagattg tcacaggaaa aggcgagatc 600

ctaaactgta caaagaggca gaacagcgac ttatttaatg gtgttcttgg tggtttaggt 660
cagtttggca tcataacgcg ggcaagaata gcattggaac cagcaccaac catggaccaa 720
gagcaactaa tatctgccca gggccacaaa ttcgattaca tagaagggtt tgtgataata 780
aacaggacag gcctcctgaa cagctggagg ttgtctttca ccgcagaaga gcctttagaa 840
gcaagccaat tcaagtttga tggaaggact ctgtattgtc tggagctagc caagtatttg 900
aagcaagata acaaagacgt aatcaaccag gaagtgaaag aaacattatc agagctaagc 960
tacgtgacgt cgacactgtt tacaacggag gtagcatatg aagcattctt ggacagggta 1020
catgtgtctg aggtaaaact ccgatcgaaa gggcagtggg aggtgccaca tccatggctg 1080
aacctcctgg taccaagaag caaaatcaat gaatttgcaa gaggtgtatt tggaaacata 1140
ctaacggata caagcaacgg cccagtcatc gtctacccag tgaacaaatc aaagtgggac 1200
aatcaaacat cagcagtaac accggaggaa gaggtattct acctggtggc gatcctaaca 1260
tcggcatctc cagggtcggc aggaaaggat ggagtagaag agatcttgag gcggaacaga 1320
agaatactgg aattcagtga agaagcaggg atagggttga agcagtatct gccacattac 1380
acgacaagag aagagtggag atcccatttc ggggacaagt ggggagaatt tgtgaggagg 1440
aaatccagat atgatccatt ggcaattctt gcgcctggcc accgaatttt tcaaaaggca 1500
gtctcatact catga 1515

<210> 31
<211> 84
<212> DNA
<213> Arabidopsis thaliana

<400> 31

tcagcttcgg gactcgctct tctctatcca acaaaccgga ataaatggga caatcgtatg 60
tcggcgatga taccagagat cgat 84

<210> 32
<211> 28
<212> PRT

<213> Arabidopsis thaliana

<400> 32

Ser Ala Ser Gly Leu Ala Leu Leu Tyr Pro Thr Asn Arg Asn Lys Trp
1               5                   10                  15

Asp Asn Arg Met Ser Ala Met Ile Pro Glu Ile Asp
            20                  25

<210> 33
<211> 2814
<212> DNA
<213> Arabidopsis thaliana

<400> 33

atgaatcgta tgacgtcaag ctttcttctc ctgacgttcg ccatatgtaa actgatcata 60

gccgtgggtc taaacgtggg ccccagtgag ctcctccgca tcggagccat agatgtcgac 120

ggccacttca ccgtccaccc ttccgactta gcctccgtct cctcagactt cggtatgctg 180
aagtcacctg aagagccatt ggccgtgctt catccatcat cggccgaaga cgtggcacga 240
ctcgtcagaa cagcttacgg ttcagccacg gcgtttccgg tctcagcccg aggccacggc 300
cattccataa acggacaagc cgcggcgggg aggaacggtg tggtggttga aatgaaccac 360
ggcgtaaccg ggacgcccaa gccactcgtc cgaccggatg aaatgtatgt ggatgtatgg 420
ggtggagagt tatgggtcga tgtgttgaag aaaacgttgg agcatggctt agcaccaaaa 480
tcatggacgg attacttgta tctaaccgtt ggaggtacac tctccaatgc aggaatcagt 540
ggtcaagctt ttcaccatgg tcctcaaatt agtaacgtcc ttgagctcga cgttgtaact 600
ggttagtatt aaaacattca agttcatata ttttaaatgc ttttgtctga agttttacta 660
ataacaagaa attgatacca aaaagtaggg aaaggagagg tgatgagatg ctcagaagaa 720
gagaacacaa ggctattcca tggagttctt ggtggattag gtcaatttgg gatcatcact 780
cgagcacgaa tctctctcga accagctccc caaagggtaa tattttttta atgactagct 840
atcaaaaatc cctggcgggt ccatacgttg taatcttttt agtttttact gttgatggta 900
tttttatat attttggata ataaaaccct aaaatggtat attgtgatga caggtgagat 960
ggatacgggt attgtattcg agcttcaaag tgtttacgga ggaccaagag tacttaatct 1020
caatgcatgg tcaattaaag tttgattacg tggaaggttt tgtgattgtg gacgaaggac 1080
tcgtcaacaa ttggagatct tctttcttct ctccacgtaa ccccgtcaag atctcctctg 1140
ttagttccaa cggctctgtt ttgtattgcc ttgagatcac caagaactac cacgactccg 1200
actccgaaat cgttgatcag gtcactttca ttattcactt agaaaaaagc gatattttca 1260
ttttttatat tgatgaatat ctggaaggat ttaacgctat gcgactattg ggaaatcatt 1320
atgaaaaaat atttagttta tatgattgaa agtggtctcc atagtatttt tgttgtgtcg 1380
actttattat aacttaaatt tggaagagga catgaagaag aagccagaga ggatctacag 1440
agatctagct tttccacctg aacttaataa tgcacattta tataattatt tttcttcttc 1500
taaagtttag tttatcacta gcgaattaat catggttact aattaagtag tggacagggt 1560
catggaccac tcactcacca aataatgatt cctctttact cttaagttta attttaataa 1620
aaccaactct actggaatct taacttatcc ttggttttgg taggctttta tagcaacacg 1680
gtttttttaa ttttcctatt ccagattttg tatattaaat gtcgattttt tttctttttg 1740
tttcaggaag ttgagattct gatgaagaaa ttgaatttca taccgacatc ggtctttaca 1800
acggatttac aatatgtgga ctttctcgac cgggtacaca aggccgaatt gaagctccgg 1860
tccaagaatt tatgggaggt tccacaccca tggctcaacc tcttcgtgcc aaaatcaaga 1920
atctctgact tcgataaagg cgttttcaag ggcattttgg gaaataaaac aagtggccct 1980
attcttatct accccatgaa caaagacaag taagtcttga cattaccatt gattactact 2040
tctaaatttc ttctctagaa aaaagaataa aacgagtttt gcattgcatg catgcaaagt 2100
tacacttgtg gggattaatt agtggtccaa gaaaaaaagt ttgtcaaaat tgaaaaaaac 2160
tagacacgtg gtacatggga ttgtccgaaa aacgttgtcc acatgtgcat cgaaccagct 2220

```
aagattgaca acaacacttc gtcggctcgt atttctcttt ttgttttgtg accaaatccg   2280
atggtccaga ttgggtttat ttgtttttaa gttcctagaa ctcatggtgg gtgggtccca   2340
atcagattct cctagaccaa accgatctca acgaaccctc cgcacatcat tgattattac   2400
attaatatag atattgtcgt tgctgacgtg tcgtaatttg atgttattgt cagatgggac   2460
gagaggagct cagccgtgac gccggatgag gaagttttct atctggtggc tctattgaga   2520
tcagctttaa cggacggtga agagacacag aagctagagt atctgaaaga tcagaaccgt   2580
cggatcttgg agttctgtga acaagccaag atcaatgtga agcagtatct tcctcaccac   2640
gcaacacagg aagagtgggt ggctcatttt ggggacaagt gggatcggtt cagaagctta   2700
aaggctgagt ttgatccgcg acacatactc gctactggtc agagaatctt tcaaaaccca   2760
tctttgtctt tgtttcctcc gtcgtcgtct tcttcgtcag cggcttcatg gtga         2814
```

<210> 34
<211> 1620
<212> DNA
<213> Arabidopsis thaliana

<400> 34

```
atgaatcgta tgacgtcaag ctttcttctc ctgacgttcg ccatatgtaa actgatcata      60
gccgtgggtc taaacgtggg ccccagtgag ctcctccgca tcggagccat agatgtcgac     120
ggccacttca ccgtccaccc ttccgactta gcctccgtct cctcagactt cggtatgctg     180
aagtcacctg aagagccatt ggccgtgctt catccatcat cggccgaaga cgtggcacga     240
ctcgtcagaa cagcttacgg ttcagccacg gcgtttccgg tctcagcccg aggccacggc     300
cattccataa acggacaagc cgcggcgggg aggaacggtg tggtggttga aatgaaccac     360
ggcgtaaccg ggacgcccaa gccactcgtc cgaccggatg aaatgtatgt ggatgtatgg     420
ggtggagagt tatgggtcga tgtgttgaag aaaacgttgg agcatggctt agcaccaaaa     480
tcatggacgg attacttgta tctaaccgtt ggaggtacac tctccaatgc aggaatcagt     540
ggtcaagctt ttcaccatgg tcctcaaatt agtaacgtcc ttgagctcga cgttgtaact     600
gggaaaggag aggtgatgag atgctcagaa gaagagaaca caaggctatt ccatggagtt     660
cttggtggat taggtcaatt tgggatcatc actcgagcac gaatctctct cgaaccagct     720
ccccaaaggg tgagatggat acgggtattg tattcgagct tcaaagtgtt tacggaggac     780
caagagtact taatctcaat gcatggtcaa ttaaagtttg attacgtgga aggttttgtg     840
attgtggacg aaggactcgt caacaattgg agatcttctt tcttctctcc acgtaacccc     900
gtcaagatct cctctgttag ttccaacggc tctgttttgt attgccttga gatcaccaag     960
aactaccacg actccgactc cgaaatcgtt gatcaggaag ttgagattct gatgaagaaa    1020
ttgaatttca taccgacatc ggtctttaca acggatttac aatatgtgga ctttctcgac    1080
cgggtacaca aggccgaatt gaagctccgg tccaagaatt tatgggaggt tccacaccca    1140
tggctcaacc tcttcgtgcc aaaatcaaga atctctgact tcgataaagg cgttttcaag    1200
ggcattttgg gaaataaaac aagtggccct attcttatct accccatgaa caaagacaaa    1260

tgggacgaga ggagctcagc cgtgacgccg gatgaggaag ttttctatct ggtggctcta    1320
ttgagatcag ctttaacgga cggtgaagag acacagaagc tagagtatct gaaagatcag    1380
aaccgtcgga tcttggagtt ctgtgaacaa gccaagatca atgtgaagca gtatcttcct    1440
caccacgcaa cacaggaaga gtgggtggct cattttgggg acaagtggga tcggttcaga    1500
agcttaaagg ctgagtttga tccgcgacac atactcgcta ctggtcagag aatctttcaa    1560
aacccatctt tgtctttgtt tcctccgtcg tcgtcttctt cgtcagcggc ttcatggtga    1620
```

<210> 35
<211> 539
<212> PRT
<213> Arabidopsis thaliana

<400> 35

Met Asn Arg Met Thr Ser Ser Phe Leu Leu Leu Thr Phe Ala Ile Cys
1               5                   10                  15

Lys Leu Ile Ile Ala Val Gly Leu Asn Val Gly Pro Ser Glu Leu Leu
            20                  25                  30

Arg Ile Gly Ala Ile Asp Val Asp Gly His Phe Thr Val His Pro Ser
        35                  40                  45

Asp Leu Ala Ser Val Ser Ser Asp Phe Gly Met Leu Lys Ser Pro Glu
    50                  55                  60

Glu Pro Leu Ala Val Leu His Pro Ser Ser Ala Glu Asp Val Ala Arg
65                  70                  75                  80

Leu Val Arg Thr Ala Tyr Gly Ser Ala Thr Ala Phe Pro Val Ser Ala
                85                  90                  95

Arg Gly His Gly His Ser Ile Asn Gly Gln Ala Ala Ala Gly Arg Asn
            100                 105                 110

Gly Val Val Val Glu Met Asn His Gly Val Thr Gly Thr Pro Lys Pro
        115                 120                 125

Leu Val Arg Pro Asp Glu Met Tyr Val Asp Val Trp Gly Gly Glu Leu
    130                 135                 140

Trp Val Asp Val Leu Lys Lys Thr Leu Glu His Gly Leu Ala Pro Lys
145                 150                 155                 160

Ser Trp Thr Asp Tyr Leu Tyr Leu Thr Val Gly Gly Thr Leu Ser Asn
                165                 170                 175

Ala Gly Ile Ser Gly Gln Ala Phe His His Gly Pro Gln Ile Ser Asn
            180                 185                 190

Val Leu Glu Leu Asp Val Val Thr Gly Lys Gly Glu Val Met Arg Cys
195 200 205

Ser Glu Glu Glu Asn Thr Arg Leu Phe His Gly Val Leu Gly Gly Leu
210 215 220

Gly Gln Phe Gly Ile Ile Thr Arg Ala Arg Ile Ser Leu Glu Pro Ala
225 230 235 240

Pro Gln Arg Val Arg Trp Ile Arg Val Leu Tyr Ser Ser Phe Lys Val
245 250 255

Phe Thr Glu Asp Gln Glu Tyr Leu Ile Ser Met His Gly Gln Leu Lys
260 265 270

Phe Asp Tyr Val Glu Gly Phe Val Ile Val Asp Glu Gly Leu Val Asn
275 280 285

Asn Trp Arg Ser Ser Phe Phe Ser Pro Arg Asn Pro Val Lys Ile Ser
290 295 300

Ser Val Ser Ser Asn Gly Ser Val Leu Tyr Cys Leu Glu Ile Thr Lys
305 310 315 320

Asn Tyr His Asp Ser Asp Ser Glu Ile Val Asp Gln Glu Val Glu Ile
325 330 335

Leu Met Lys Lys Leu Asn Phe Ile Pro Thr Ser Val Phe Thr Thr Asp
340 345 350

Leu Gln Tyr Val Asp Phe Leu Asp Arg Val His Lys Ala Glu Leu Lys
355 360 365

Leu Arg Ser Lys Asn Leu Trp Glu Val Pro His Pro Trp Leu Asn Leu
370 375 380

Phe Val Pro Lys Ser Arg Ile Ser Asp Phe Asp Lys Gly Val Phe Lys
385 390 395 400

Gly Ile Leu Gly Asn Lys Thr Ser Gly Pro Ile Leu Ile Tyr Pro Met
405 410 415

Asn Lys Asp Lys Trp Asp Glu Arg Ser Ser Ala Val Thr Pro Asp Glu
420 425 430

Glu Val Phe Tyr Leu Val Ala Leu Leu Arg Ser Ala Leu Thr Asp Gly
435 440 445

Glu Glu Thr Gln Lys Leu Glu Tyr Leu Lys Asp Gln Asn Arg Arg Ile
450 455 460

Leu Glu Phe Cys Glu Gln Ala Lys Ile Asn Val Lys Gln Tyr Leu Pro

465 470 475 480

His His Ala Thr Gln Glu Glu Trp Val Ala His Phe Gly Asp Lys Trp
485 490 495

Asp Arg Phe Arg Ser Leu Lys Ala Glu Phe Asp Pro Arg His Ile Leu
500 505 510

Ala Thr Gly Gln Arg Ile Phe Gln Asn Pro Ser Leu Ser Leu Phe Pro
515 520 525

Pro Ser Ser Ser Ser Ser Ser Ala Ala Ser Trp
530 535

<210> 36
<211> 842
<212> DNA
<213> Arabidopsis thaliana

<400> 36

```
aagcttaaat gacaatttag taccttgggt tggtcatgat ttagagcgga acaaatatac      60
catacatcaa acgaggatat acagagaaaa ttcatggaag tatggaattt agaggacaat     120
ttctcttctg ggctacaacg gaccggccca ttcgctcatt tacccagagg tatcgagttt     180
gtggactttt gatgccgcta gagactattg gcatcggatt gaaaaaaatg tttacttcgt     240
tgttaacaat ttctgaatg caatattttc cttgtcatga atatttaaac ttgttattac      300
tttcttttag cttaggtgtg gacaattatg gagtttactt caaacgagga agaatcttaa     360
acgctcggtt caggtctcga aaacaaacca actcacaatc ctgacttaat tgaggaaaac     420
aatgcaaaac cacatgcatg cttccatatt tctatcataa tcttataaga aaaaacacta     480
ctaagtgaaa tgattctgta tatatataac caatgccttt tgttttgtga tattttatgt     540
atatataact attgactttt gtcatctatg gatagtgtct cgggctcttg gcaaacatat     600
ttcaaagaaa agttaatgac tgtaattaat taatctgaag ctagaaacag aaccccgagg     660
taaaagaaaa agacagagca catgaagttt agtactttta tatatttaat atatcattct     720
ttcttattgc ttatctctaa agcaaaaact tccctaaacc ctaagccaaa ggactcagat     780
cgatgcagaa ccaagaaggc ttgttttgga tttgagagcc aaatgcaaag aaaaaaactc     840
tt                                                                    842
```